# EUROPEAN PATENT APPLICATION

(11) **EP 4 501 357 A1**
(43) Date of publication of application: **05.02.2025**
(21) Application number: 23780834.0
(22) Date of filing: 30.03.2023
(51) Int. Cl.: A61K 45/00, A61K 31/551, A61P 1/00, A61P 1/06, A61P 1/08, A61P 1/12, A61P 7/10, A61P 9/00, A61P 9/02, A61P 9/06, A61P 11/00, A61P 11/04, A61P 11/16, A61P 17/00, A61P 17/04, A61P 25/00, A61P 25/04, A61P 37/00

(54) **PHARMACEUTICAL COMPOSITION FOR PREVENTING OR SUPPRESSING OR TREATING SYMPTOMS ACCOMPANYING PSEUDO-ALLERGIC REACTIONS**

(30) Priority: 31.03.2022 JP 2022060261
(71) Applicant: Takasaki University of Health and Welfare, Takasaki-shi, Gunma, 370-0033 (JP); Nippon Chemiphar Co., Ltd., Tokyo 101-0032 (JP)
(72) Inventor: MATSUOKA Isao, Takasaki City, Gunma 370-0033 (JP); ITO Masa-aki, Takasaki City, Gunma 370-0033 (JP); YOSHIDA Kazuki, Takasaki City, Gunma 370-0033 (JP); IMAI Toshiyasu, Misato City, Saitama 341-0005 (JP)
(74) Representative: V.O.
(86) International application number: PCT/JP2023/013084
(87) International publication number: WO 2023/190826

(57) **Abstract**

The present invention relates to a pharmaceutical composition for preventing, suppressing, or treating a symptom associated with a pseudo-allergic reaction, the pharmaceutical composition including a compound having a P2X4 receptor antagonizing action, or a pharmaceutically acceptable salt thereof, as an active ingredient.

## Description

### Technical Field

The present invention relates to a pharmaceutical composition for preventing, suppressing, or treating a symptom associated with a pseudo-allergic reaction, the pharmaceutical composition including a compound having a P2X4 receptor antagonizing action, or a pharmaceutically acceptable salt thereof, as an active ingredient.

### Background Art

Allergic diseases are a modern disease that continues to increase. Generally, in order to develop immediate allergy, an IgE antibody against an allergen such as pollen or food is produced, and is required to bind to an IgE receptor of mast cells. Next, when the allergen binds to the IgE antibody to stimulate the IgE receptor, the mast cells are activated to release (degranulate) intracellular granules such as histamine, and thus, allergic symptoms are caused. In this way, immediate allergy is induced through two steps.

On the other hand, certain cationic agents, such as antibiotics and anesthetics, bind directly to the receptors of mast cells and activate the mast cells, causing symptoms similar to those of the immediate allergy involving IgE. This phenomenon is called pseudo-allergy in which IgE is not involved and has become a major medical problem as a side effect of a drug (Non Patent Literature 1).

Pseudo-allergy is suggested to be mediated by Mas-related G protein coupled receptor (Mrgpr) X2 that recognizes a wide range of therapeutic agents having a cationic group and causes MC activation (Non Patent Literature 2). For example, for peptidic medicaments (Icatibant, Cetrorelixm, Leuprolide, Octreotide, Sermorelin, Mastoparan, Atracurium, Ciprofloxcin, and the like), and non-peptidic medicaments (Atracurium, Tubocurarine, Rocuronium, Succinylcholine, and Ciprofloxacin), MrgprX2 is demonstrated to be a receptor that mediates a pseudo-allergic reaction (Patent Literature 1, Non Patent Literature 3, and the like). The responsiveness of mast cells to signals via MrgprX2 may be upregulated under certain conditions, although a higher concentration of a stimulant is required compared to IgE antibody-dependent stimulation, but such a mechanism has not been elucidated.

Diazepinedione derivative compounds having a P2X4 receptor antagonizing action are disclosed (Patent Literature 2 and 3). Recently, aromatic sulfonamide derivative compounds that are P2X4 receptor antagonists have been reported (Patent Literature 4 and 5). A diazepinedione derivative compound having a P2X4 receptor antagonizing action inhibited degranulation of IgE antibody stimulation or prostaglandin E2 stimulation from mast cells and suppressed skin or systemic anaphylaxis (Patent Literature 6). However, no effect of any compound on pseudo-allergy to drugs was disclosed.

A pseudo-allergic reaction, which is one of side effects of drugs, not only interferes with various treatments, but also sometimes leads to life-threatening, such as anaphylactic shock. As a therapeutic agent for pseudo-allergy, MrgprB2 antagonists, lipid ceramides which are ligands of the immune receptor CD300f, and the like have attracted great attention, and there is a need to clarify the pathogenesis in order to develop a fundamental treatment.

### Citation List

### Patent Literature

Patent Literature 1: WO2016/019246A
Patent Literature 2: WO2010/093061A
Patent Literature 3: WO2013/105608A
Patent Literature 4: WO2016/198374A
Patent Literature 5: WO2017/191000A
Patent Literature 6: WO2022/030428A

### Non Patent Literature

Non Patent Literature 1: Turner PJ et al., J.Allergy Clin. Immunoly: In Practice. 2017, 5, 1169
Non Patent Literature 2: Bruhns P et al., J.Allergy Clin. Immunol. 2021,147, 1133
Non Patent Literature 3: McNeil BD et al., Nature 2015,519, 237

### Summary of Invention

### Technical Problem

An object of the present invention is to provide a pharmaceutical composition useful in preventing, suppressing, or treating a symptom associated with a pseudo-allergic reaction, and particularly, to provide a pharmaceutical composition useful in preventing, suppressing, or treating a symptom associated with a pseudo-allergic reaction to a drug.

### Solution to Problem

As a result of conducting intensive research in order to achieve the above object, the present inventors found that a P2X4 receptor antagonist, for example, compounds represented by general formulas (A) to (D,) or a pharmaceutically acceptable salt thereof, are useful in preventing, suppressing, or treating a symptom associated with a pseudo-allergic reaction, and particularly useful in preventing, suppressing, or treating a symptom associated with a pseudo-allergic reaction to a drug, thus completing the present invention.

That is, the present invention relates to the following:
[1] A pharmaceutical composition for preventing, suppressing, or treating a symptom associated with a pseudo-allergic reaction, the pharmaceutical composition including a compound having a P2X4 receptor antagonizing action, or a pharmaceutically acceptable salt thereof, as an active ingredient.
[2] The pharmaceutical composition according to [1], in which the symptom associated with the pseudo-allergic reaction is one or more selected from the group consisting of a skin and mucous membrane symptom (including wheals, erythema punctatum, urticaria, angioedema, eyelid edema, bulbar chemosis, and pruritus), a digestive symptom (including pharyngeal and laryngeal edema, vomiting, abdominal pain, and diarrhea), a respiratory symptom (including wheezing and dyspnea), a cardiovascular symptom (including arrhythmia, paleness, a drop in body temperature, and a drop in blood pressure), and a central nervous system symptom (including headache and loss of consciousness).
[3] The pharmaceutical composition according to [1] or [2], in which the pseudo-allergic reaction is caused by an allergen having a cationic group.
[4] The pharmaceutical composition according to [3], in which the allergen is amorolfine hydrochloride, atracurium, betadefensin, bleomycin, cetrorelix, C48/80 (monomer or cyclized monomer), ciprofloxacin, cisatracurium, codeine, colistimethate, complanadine A, cortistatin-14, degarelix, enfuvirtide, exenatide, fluoroquinolone, ganirelix, gentamicin sulfate, glatiramer acetate, glucagon, goserelin, histrelin, icatibant, ketoconazole, lanreotide, leuprolide, levofloxacin, liraglutide, LL-37, mafenide acetate, mastoparan, micronomicin sulfate, mivacurium, morphine, moxifloxacin, octreotide, ofloxacin, PAMP-12, PAMP-20, pasireotide, PMX-53, pramlintide, rocuronium, sermorelin, sisomicin sulfate, substance P, succinylcholine, sulfadoxine, sulfamethoxazole, terbinafine hydrochloride, teriparatide, tesamorelin, triptorelin, tubocurarine, or vancomycin.
[5] The pharmaceutical composition according to any one of [1] to [4], in which the compound is a compound represented by the following general formula (A): 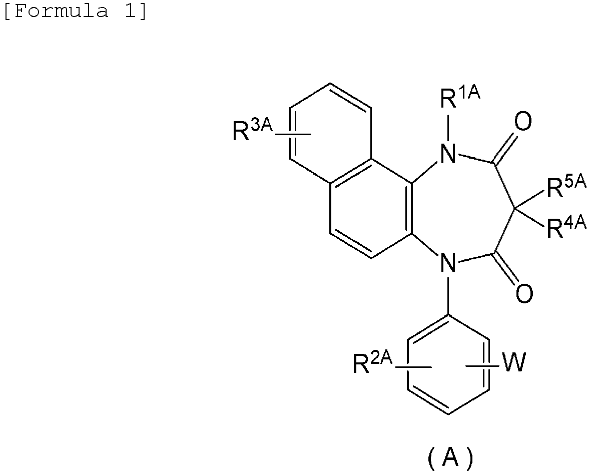 (In the formula R^{1A} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, or an alkyl group having 1 to 3 carbon atoms and substituted with a phenyl group,
   R^{2A} and R^{3A} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, an acylamino group having 2 to 8 carbon atoms and substituted with 1 to 3 halogen atoms, an alkylsulfonylamino group having 1 to 8 carbon atoms, a carboxyl group, an acyl group having 2 to 8 carbon atoms, an alkoxycarbonyl group (the alkoxy moiety has 1 to 8 carbon atoms), a carbamoyl group, an alkylthio group having 1 to 8 carbon atoms, an alkylsulfinyl group having 1 to 8 carbon atoms, an alkylsulfonyl group having 1 to 8 carbon atoms, or a sulfamoyl group,
   R^{4A} and R^{5A} may be the same or different, and represent a hydrogen atom, an alkyl group having 1 to 8 carbon atoms, an alkyl group having 1 to 8 carbon atoms and substituted with 1 to 3 halogen atoms, or an alkyl group having 1 to 3 carbon atoms substituted with a phenyl group, and
   W^{A} represents a five or six membered heterocyclic ring including 1 to 4 nitrogen atoms as the members of the ring, which may have a substituent).
[6] The pharmaceutical composition according to any one of [1] to [4], in which the compound is a compound represented by the following general formula (BI): 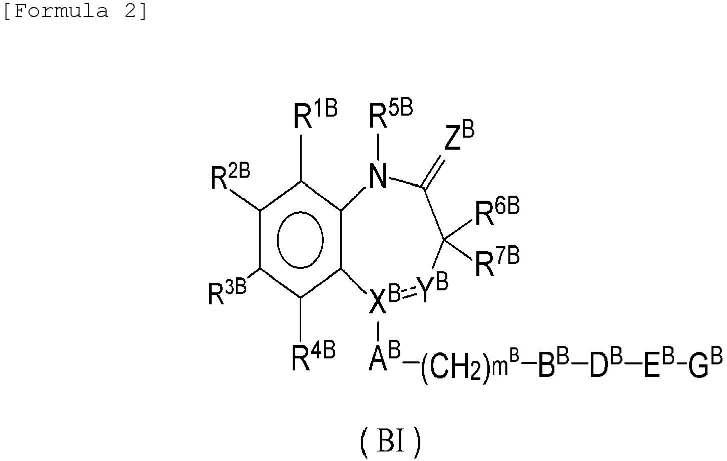 (In the formula R^{1B} and R^{2B} may be the same or different, and represent a hydrogen atom, an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), a phenyl group which may be substituted, a pyridyl group which may be substituted, or an aralkyl group (the aryl moiety has 6 to 10 carbon atoms, and the alkylene moiety has 1 to 8 carbon atoms), or
   R^{1B} and R^{2B} may bind together to form a condensed ring selected from a naphthalene ring, a quinoline ring, an isoquinoline ring, a tetrahydronaphthalene ring, an indane ring, a tetrahydroquinoline ring, and a tetrahydroisoquinoline ring together with the benzene ring to which they bind, and the ring constituted by R^{1B} and R^{2B} bound to each other, together with the carbon atoms to which R^{1B} and R^{2B} bind may be substituted with 1 to 4 of the same or different substituents selected from an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),
   R^{3B} and R^{4B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), or an aralkyl group (the aryl moiety has 6 to 10 carbon atoms, and the alkylene moiety has 1 to 8 carbon atoms),
   R^{5B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
   R^{6B} and R^{7B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, or an amino group,
   X^{B} represents C, CH, or N,
   Y^{B} represents N, NH, or C(=O),
   provided that when X^{B} is N, Y^{B} is not N or NH, and
   when X^{B} is C or CH, Y^{B} is not C(=O),
   the double line consisting of the solid line and the broken line represents a single bond or a double bond,
   Z^{B} represents an oxygen atom or a sulfur atom,
   A^{B} represents a benzene ring, a pyridine ring, a thiophene ring, a pyrimidine ring, a naphthalene ring, a quinoline ring, or an indole ring, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group, as a substituent, or represents an atomic bond,
   B^{B} represents N(R^{8B})C(=O), NHCONH, CON(R^{9B}), NHC (=S) NH, N (R^{10B}) SO₂, SO₂N(R^{11B}), or OSO₂, in the formula
   R^{BB}, R^{9B}, R^{10B}, and R^{11B} represent a hydrogen atom, an alkyl group having 1 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
   D^{B} represents an alkylene chain having 1 to 6 carbon atoms, which may have 1 to 4 of the same or different substituents selected from an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, and an aralkyl group (the aryl moiety has 6 to 10 carbon atoms, and the alkylene moiety has 1 to 8 carbon atoms), as a substituent, and may further have a double bond, or represents an atomic bond,
   E^{B} represents O, S, NR^{12B}, or an atomic bond, in the formula
   R^{12B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
   G^{B} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (the aryl moiety has 6 to 10 carbon atoms, and the alkylene moiety has 1 to 8 carbon atoms), a phenyl group which may be substituted, a pyridyl group which may be substituted, an imidazolyl group which may be substituted, an oxazolyl group which may be substituted, and a thiazolyl group which may be substituted, as a substituent, and
   m^{B} represents an integer of 0 to 5,
   provided that when R^{1B} and R^{2B} do not bind together to form a ring, those compounds are excluded in the formula, X^{B} is C, Y^{B} is N, the double line consisting of the solid line and the broken line is a double bond, Z^{B} is an oxygen atom, A^{B} is a benzene ring, m^{B} is 0, B^{B} is C(=O)NH, E^{B} is an atomic bond, and G^{B} is a phenyl group).
[7] The pharmaceutical composition according to any one of [1] to [4], in which the compound, or a pharmaceutically acceptable salt thereof, is a compound selected from the group consisting of the following (A1) to (A21) and (B1) to (B214) or a pharmaceutically acceptable salt thereof:
   (A1) 5-[3-(1H-tetrazol-5-yl)phenyl]-1H-naphtho[1,2-b][1,4]diazepine-2,4(3H,5H)-dione;
   (A2) 5-[3-(1H-tetrazol-5-yl)phenyl]-1H-naphtho[1,2-b][1,4]diazepine-2,4(3H,5H)-dione sodium salt;
   (A3) 5-[3-(1H-tetrazol-5-yl)phenyl]-1H-naphtho[1,2-b][1,4]diazepine-2,4(3H,5H)-dione potassium salt;
   (A4) 5-[4-(1H-tetrazol-5-yl)phenyl]-1H-naphtho[1,2-b][1,4]diazepine-2,4(3H,5H)-dione;
   (A5) 5-[4-(1H-tetrazol-5-yl)phenyl]-1H-naphtho[1,2-b][1,4]diazepine-2,4(3H,5H)-dione sodium salt;
   (A6) 1-methyl-5-[3-(1H-tetrazol-5-yl)phenyl]-1H-naphtho[1,2-b][1,4]diazepine-2,4(3H,5H)-dione;
   (A7) 1,3-dimethyl-5-[3-(1H-tetrazol-5-yl)phenyl]-1H-naphtho[1,2-b][1,4]diazepine-2,4(3H,5H)-dione;
   (A8) 5-[2-chloro-5-(1H-tetrazol-5-yl)phenyl]-1H-naphtho[1,2-b][1,4]diazepine-2,4(3H,5H)-dione;
   (A9) 5-[2-chloro-5-(1H-tetrazol-5-yl)phenyl]-1H-naphtho[1,2-b][1,4]diazepine-2,4(3H,5H)-dione sodium salt;
   (A10) 5-[2-methyl-5-(1H-tetrazol-5-yl)phenyl]-1H-naphtho[1,2-b][1,4]diazepine-2,4(3H,5H)-dione;
   (A11) 5-[2-methyl-5-(1H-tetrazol-5-yl)phenyl]-1H-naphtho[1,2-b][1,4]diazepine-2,4(3H,5H)-dione sodium salt;
   (A12) 5-[2-bromo-5-(1H-tetrazol-5-yl)phenyl]-1H-naphtho[1,2-b][1,4]diazepine-2,4(3H,5H)-dione;
   (A13) 5-[3-(2-methyl-2H-tetrazol-5-yl)phenyl]-1H-naphtho[1,2-b][1,4]diazepine-2,4(3H,5H)-dione;
   (A14) 5-[3-(1-methyl-1H-tetrazol-5-yl)phenyl]-1H-naphtho[1,2-b][1,4]diazepine-2,4(3H,5H)-dione;
   (A15) 5-[3-(5-oxo-4H-[1,2,4]oxadiazol-3-yl)phenyl]-1H-naphtho[1,2-b][1,4]diazepine-2,4(3H,5H)-dione;
   (A16) 5-[3-(5-thioxo-4H-[1,2,4]oxadiazol-3-yl)phenyl]-1H-naphtho[1,2-b][1,4]diazepine-2,4(3H,5H)-dione;
   (A17) 5-[3-(5-thioxo-4H-[1,2,4]oxadiazol-3-yl)phenyl]-1H-naphtho[1,2-b][1,4]diazepine-2,4(3H,5H)-dione sodium salt;
   (A18) 5-[3-(oxazol-2-yl)phenyl]-1H-naphtho[1,2-b][1,4]diazepine-2,4(3H,5H)-dione;
   (A19) 5-[3-(1H-pyrazol-4-yl)phenyl]-1H-naphtho[1,2-b][1,4]diazepine-2,4(3H,5H)-dione;
   (A20) 5-[4-fluoro-3-(1H-tetrazol-5-yl)phenyl]-1H-naphtho[1,2-b][1,4]diazepine-2,4(3H,5H)-dione; and
   (A21) 5-[4-fluoro-3-(1H-tetrazol-5-yl)phenyl]-1H-naphtho[1,2-b][1,4]diazepine-2,4(3H,5H)-dione sodium salt; and
   (B1) 5-(4-benzoylaminophenyl)-1H-naphtho[1,2-b][1,4]diazepine-2,4(3H,5H)-dione;
   (B2) 5-[4-[2-(trifluoromethyl)benzoyl]aminophenyl]-1H-naphtho[1,2-b][1,4]diazepine-2,4(3H,5H)-dione;
   (B3) 5-[4-(3-bromobenzoyl)aminophenyl]-1H-naphtho[1,2-b][1,4]diazepine-2,4(3H,5H)-dione;
   (B4) 5-[4-[4-(trifluoromethyl)benzoyl]aminophenyl]-1H-naphtho[1,2-b][1,4]diazepine-2,4(3H,5H)-dione;
   (B5) 5-[4-(2-methylbenzoyl)aminophenyl]-1H-naphtho[1,2-b][1,4]diazepine-2,4(3H,5H)-dione;
   (B6) 5-[4-(2,6-dimethylbenzoylamino)phenyl]-1H-naphtho[1,2-b][1,4]diazepine-2,4(3H,5H)-dione;
   (B7) 5-[4-(2,6-dichlorobenzoyl)aminophenyl]-1H-naphtho[1,2-b][1,4]diazepine-2,4(3H,5H)-dione;
   (B8) 5-[4-(3-chlorobenzoyl)aminophenyl]-1H-naphtho[1,2-b][1,4]diazepine-2,4(3H,5H)-dione;
   (B9) 5-[4-(2-phenylacetylamino)phenyl]-1H-naphtho[1,2-b][1,4]diazepine-2,4(3H,5H)-dione;
   (B10) 1-[4-(2,4-dioxo-1,2,3,4-tetrahydronaphtho[1,2-b][1,4]diazepine-5-yl)phenyl]-3-phenylthiourea;
   (B11) 5-[4-(2,3-dimethoxybenzoylamino)phenyl]-1H-naphtho[1,2-b][1,4]diazepine-2,4(3H,5H)-dione;
   (B12) 5-[4-(2-methoxybenzoylamino)phenyl]-1H-naphtho[1,2-b][1,4]diazepine-2,4(3H,5H)-dione;
   (B13) 5-[4-[(2-chlorophenylacetyl)amino]phenyl]-1H-naphtho[1,2-b][1,4]diazepine-2,4(3H,5H)-dione;
   (B14) 5-[4-(2,3-dimethylbenzoylamino)phenyl]-1H-naphtho[1,2-b][1,4]diazepine-2,4(3H,5H)-dione;
   (B15) 5-[4-(2,5-dimethylbenzoylamino)phenyl]-1H-naphtho[1,2-b][1,4]diazepine-2,4(3H,5H)-dione;
   (B16) 5-[4-(5-bromo-2-chlorobenzoylamino)phenyl]-1H-naphtho[1,2-b][1,4]diazepine-2,4(3H,5H)-dione;
   (B17) 5-[4-(2,4-dichlorobenzoylamino)phenyl]-1H-naphtho[1,2-b][1,4]diazepine-2,4(3H,5H)-dione;
   (B18) 5-[4-(2-hydroxybenzoylamino)phenyl]-1H-naphtho[1,2-b][1,4]diazepine-2,4(3H,5H)-dione;
   (B19) 5-[4-(2,3-dihydroxybenzoylamino)phenyl]-1H-naphtho[1,2-b][1,4]diazepine-2,4(3H,5H)-dione;
   (B20) 1-[4-(2,4-dioxo-1,2,3,4-tetrahydronaphtho[1,2-b][1,4]diazepine-5-yl)phenyl]-3-phenylurea;
   (B21) 5-[4-[(2,6-dichlorophenylacetyl)amino]phenyl]-1H-naphtho[1,2-b][1,4]diazepine-2,4(3H,5H)-dione;
   (B22) 5-[4-[(2-methoxyphenylacetyl)amino]phenyl]-1H-naphtho[1,2-b][1,4]diazepine-2,4(3H,5H)-dione;
   (B23) 5-[4-[(2-hydroxyphenylacetyl)amino]phenyl]-1H-naphtho[1,2-b][1,4]diazepine-2,4(3H,5H)-dione;
   (B24) 1-(2-chlorophenyl)-3-[4-(2,4-dioxo-1,2,3,4-tetrahydronaphtho[1,2-b][1,4]diazepine-5-yl)phenyl]thiourea;
   (B25) 5-[4-[3-(trifluoromethyl)benzoylamino]phenyl]-1H-naphtho[1,2-b][1,4]diazepine-2,4(3H,5H)-dione;
   (B26) 5-[4-[2-[2-(trifluoromethyl)phenyl]acetylamino]phenyl]-1H-naphtho[1,2-b][1,4]diazepine-2,4(3H,5H)-dione;
   (B27) 1-(2-chlorophenyl)-3-[4-(2,4-dioxo-1,2,3,4-tetrahydronaphtho[1,2-b][1,4]diazepine-5-yl)phenyl]urea;
   (B28) 5-[4-[(2-phenylpropionyl)amino]phenyl]-1H-naphtho[1,2-b][1,4]diazepine-2,4(3H,5H)-dione;
   (B29) 5-[4-(2-chloro-3-methoxybenzoylamino)phenyl]-1H-naphtho[1,2-b][1,4]diazepine-2,4(3H,5H)-dione;
   (B30) 5-[4-(3-phenylpropionylamino)phenyl]-1H-naphtho[1,2-b][1,4]diazepine-2,4(3H,5H)-dione;
   (B31) 5-[4-[(1H-indol-3-carbonyl)amino]phenyl]-1H-naphtho[1,2-b][1,4]diazepine-2,4(3H,5H)-dione;
   (B32) 5-[4-(2-chloro-3-hydroxybenzoylamino)phenyl]-1H-naphtho[1,2-b][1,4]diazepine-2,4(3H,5H)-dione;
   (B33) 5-[4-[(2-methyl-2-phenylpropionyl)amino]phenyl]-1H-naphtho[1,2-b][1,4]diazepine-2,4(3H,5H)-dione;
   (B34) 5-[4-(2-phenoxyacetylamino)phenyl]-1H-naphtho[1,2-b][1,4]diazepine-2,4(3H,5H)-dione;
   (B35) 5-[4-[2-(2-chloro-4-methoxyphenyl)acetylamino]phenyl]-1H-naphtho[1,2-b][1,4]diazepine-2,4(3H,5H)-dione;
   (B36) 5-[4-[(1-methyl-1H-imidazol-2-carbonyl)amino]phenyl]-1H-naphtho[1,2-b][1,4]diazepine-2,4(3H,5H)-dione;
   (B37) 5-[4-[2-(2,4-dichlorophenyl)acetylamino]phenyl]-1H-naphtho[1,2-b][1,4]diazepine-2,4(3H,5H)-dione;
   (B38) 5-[4-[2-(2-chloro-4-hydroxyphenyl)acetylamino]phenyl]-1H-naphtho[1,2-b][1,4]diazepine-2,4(3H,5H)-dione;
   (B39) 5-[4-(3-phenylpropenylamino)phenyl]-1H-naphtho[1,2-b][1,4]diazepine-2,4(3H,5H)-dione;
   (B40) 5-[4-[(3-pyridylacetyl)amino]phenyl]-1H-naphtho[1,2-b][1,4]diazepine-2,4(3H,5H)-dione hydrochloride;
   (B41) 5-[4-(1H-benzimidazol-2-carbonylamino)phenyl]-1H-naphtho[1,2-b][1,4]diazepine-2,4(3H,5H)-dione;
   (B42) 1-[4-(2,3-dimethylbenzoylamino)phenyl]-7-methoxy-1H-1,5-benzodiazepine-2,4(3H,5H)-dione;
   (B43) 5-[4-[(benzoylamino)methyl]phenyl]-1H-naphtho[1,2-b][1,4]diazepine-2,4(3H,5H)-dione;
   (B44) 5-[4-[(2-chlorobenzoylamino)methyl]phenyl]-1H-naphtho[1,2-b][1,4]diazepine-2,4(3H,5H)-dione;
   (B45) 1-[4-(2,3-dimethylbenzoylamino)phenyl]-7-hydroxy-1H-1,5-benzodiazepine-2,4(3H,5H)-dione;
   (B46) 5-[4-(2-chlorobenzoylamino)phenyl]-1H-naphtho[1,2-b][1,4]diazepine-2,4(3H,5H)-dione;
   (B47) 5-[4-(2-bromobenzoylamino)phenyl]-1H-naphtho[1,2-b][1,4]diazepine-2,4(3H,5H)-dione;
   (B48) 5-[4-(2-iodobenzoylamino)phenyl]-1H-naphtho[1,2-b][1,4]diazepine-2,4(3H,5H)-dione;
   (B49) 5-[4-(2,3-dimethylbenzoylamino)-3-fluorophenyl]-1H-naphtho[1,2-b][1,4]diazepine-2,4(3H,5H)-dione;
   (B50) 5-[4-[2-(2-methylphenyl)acetylamino]phenyl]-1H-naphtho[1,2-b][1,4]diazepine-2,4(3H,5H)-dione;
   (B51) 5-[4-[(quinoxalin-2-yl)carbonylamino]phenyl]-1H-naphtho[1,2-b][1,4]diazepine-2,4(3H,5H)-dione;
   (B52) 5-[4-[(5-methylthiophen-2-yl)carbonylamino]phenyl]-1H-naphtho[1,2-b][1,4]diazepine-2,4(3H,5H)-dione;
   (B53) 5-[3-[(2-chlorophenylacetyl)amino]phenyl]-1H-naphtho[1,2-b][1,4]diazepine-2,4(3H,5H)-dione;
   (B54) 5-[4-[(2,4,6-trimethylbenzoyl)amino]phenyl]-1H-naphtho[1,2-b][1,4]diazepine-2,4(3H,5H)-dione;
   (B55) 5-[4-(cyclohexylcarbonylamino)phenyl]-1,2,3,4,8,9,10,11-octahydronaphtho[1,2-b][1,4]diazepine-2,4(3H,5H)-dione;
   (B56) 1-[4-(2,3-dimethylbenzoyl)aminophenyl]-6-methyl-1H-1,5-benzodiazepine-2,4(3H,5H)-dione;
   (B57) 5-[4-[(2-ethylbenzoyl)amino]phenyl]-1H-naphtho[1,2-b][1,4]diazepine-2,4(3H,5H)-dione;
   (B58) 5-[4-[(6-methylpyridin-2-yl)carbonylamino]phenyl]-1,2,3,4,8,9,10,11-octahydronaphtho[1,2-b][1,4]diazepine-2,4(3H,5H)-dione;
   (B59) 5-[4-[(2-methylpyridin-3-yl)carbonylamino]phenyl]-1,2,3,4,8,9,10,11-octahydronaphtho[1,2-b][1,4]diazepine-2,4(3H,5H)-dione;
   (B60) 1-[4-(2,4-dioxo-1,2,3,4-tetrahydronaphtho[1,2-b][1,4]diazepine-5-yl)phenyl]-3-(2-methylphenyl)thiourea;
   (B61) 5-[4-(2-methoxy-3-methylbenzoyl)aminophenyl]-1,2,3,4,8,9,10,11-octahydronaphtho[1,2-b][1,4]diazepine-2,4(3H,5H)-dione;
   (B62) 5-[4-(2,3-dichlorobenzoyl)aminophenyl]-1,2,3,4,8,9,10,11-octahydronaphtho[1,2-b][1,4]diazepine-2,4(3H,5H)-dione;
   (B63) 5-[4-(2,3-dimethylbenzoylamino)-3-hydroxyphenyl]-1H-naphtho[1,2-b][1,4]diazepine-2,4(3H,5H)-dione;
   (B64) 5-[4-(2-chloro-3-methoxybenzoylamino)phenyl]-1,3-dihydronaphtho[1,2-e]-1,4-diazepine-2-one;
   (B65) 5-[4-[(4-dimethylaminobenzoyl)amino]phenyl]-1H-naphtho[1,2-b][1,4]diazepine-2,4(3H,5H)-dione;
   (B66) 5-[4-[2-(2,4-dichlorophenoxy)acetylamino]phenyl]-1H-naphtho[1,2-b][1,4]diazepine-2,4(3H,5H)-dione;
   (B67) 5-[4-[2-(2-methylphenoxy)acetylamino]phenyl]-1H-naphtho[1,2-b][1,4]diazepine-2,4(3H,5H)-dione;
   (B68) N-[4-(2,4-dioxo-1,2,3,4-tetrahydronaphtho[1,2-b][1,4]diazepin-5-yl)butyl]-2-chloro-3-methoxybenzamide;
   (B69) 5-[4-(2-chloro-3-hydroxybenzoylamino)phenyl]-1,3-dihydronaphtho[1,2-e]-1,4-diazepine-2-one;
   (B70) 5-[4-(2-acetylbenzoylamino)phenyl]-1H-naphtho[1,2-b][1,4]diazepine-2,4(3H,5H)-dione;
   (B71) 5-[4-(2-tert-butylbenzoylamino)phenyl]-1H-naphtho[1,2-b][1,4]diazepine-2,4(3H,5H)-dione;
   (B72) 5-[2-(2-iodobenzoyl)aminoethyl]-1H-naphtho[1,2-b][1,4]diazepine-2,4(3H,5H)-dione;
   (B73) 5-[3-[(2-iodobenzoyl)amino]phenyl]-1H-naphtho[1,2-b][1,4]diazepine-2,4(3H,5H)-dione;
   (B74) 6,7-dimethyl-1-[4-(2-iodobenzoyl)aminophenyl]-1H-1,5-benzodiazepine-2,4(3H,5H)-dione;
   (B75) 5-[4-[(1-methylpiperidin-4-yl)carbonylamino]phenyl]-1H-naphtho[1,2-b][1,4]diazepine-2,4(3H,5H)-dione hydrochloride;
   (B76) 5[4-[(benzofuran-2-yl)carbonylamino]phenyl]-1H-naphtho[1,2-b][1,4]diazepine-2,4(3H,5H)-dione;
   (B77) 5-[4-[(1-methyl-1H-indol-3-yl)carbonylamino]phenyl]-1H-naphtho[1,2-b][1,4]diazepine-2,4(3H,5H)-dione;
   (B78) 5-[4-(2-propenylbenzoylamino)phenyl]-1H-naphtho[1,2-b][1,4]diazepine-2,4(3H,5H)-dione;
   (B79) 5-[4-(2-propylbenzoylamino)phenyl]-1H-naphtho[1,2-b][1,4]diazepine-2,4(3H,5H)-dione;
   (B80) 5-[3-fluoro-4-(2-iodobenzoylamino)phenyl]-1H-naphtho[1,2-b][1,4]diazepine-2,4(3H,5H)-dione;
   (B81) 5-[4-(2-hydroxy-3-methylbenzoyl)aminophenyl]-1,2,3,4,8,9,10,11-octahydronaphtho[1,2-b][1,4]diazepine-2,4(3H,5H)-dione;
   (B82) 5-[4-[(2-isopropoxybenzoyl)amino]phenyl]-1H-naphtho[1,2-b][1,4]diazepine-2,4(3H,5H)-dione;
   (B83) 5-[4-[(3-methylthiophen-2-yl)carbonylamino]phenyl]-1H-naphtho[1,2-b][1,4]diazepine-2,4(3H,5H)-dione;
   (B84) 5-[4-(2-phenoxypropionylamino)phenyl]-1H-naphtho[1,2-b][1,4]diazepine-2,4(3H,5H)-dione;
   (B85) 5-[4-[2-(4-chloro-2-methylphenoxy)acetylamino]phenyl]-1H-naphtho[1,2-b][1,4]diazepine-2,4(3H,5H)-dione;
   (B86) 5-[4-[(4-fluoro-2-trifluoromethyl)benzoyl]aminophenyl]-1H-naphtho[1,2-b][1,4]diazepine-2,4(3H,5H)-dione;
   (B87) 5-[4-(4-fluoro-2-methoxybenzoyl)aminophenyl]-1H-naphtho[1,2-b][1,4]diazepine-2,4(3H,5H)-dione;
   (B88) 5-[4-(4-fluoro-2-hydroxybenzoyl)aminophenyl]-1H-naphtho[1,2-b][1,4]diazepine-2,4(3H,5H)-dione;
   (B89) 5-[3-[(2-iodophenylacetyl)amino]phenyl]-1H-naphtho[1,2-b][1,4]diazepine-2,4(3H,5H)-dione;
   (B90) 5-[4-(2-methyl-2-phenoxypropionylamino)phenyl]-1H-naphtho[1,2-b][1,4]diazepine-2,4(3H,5H)-dione;
   (B91) 5-[4-(2-tert-butylbenzoylamino)phenyl]-1,3-dihydronaphtho[1,2-e]-1,4-diazepine-2-one;
   (B92) 5-[4-[(3-dimethylaminobenzoyl)amino]phenyl]-1H-naphtho[1,2-b][1,4]diazepine-2,4(3H,5H)-dione;
   (B93) 5-[4-(4-iodo-2-methoxybenzoylamino)phenyl]-1H-naphtho[1,2-b][1,4]diazepine-2,4(3H,5H)-dione;
   (B94) 5-[4-(6-fluoro-2-methoxybenzoylamino)phenyl]-1H-naphtho[1,2-b][1,4]diazepine-2,4(3H,5H)-dione;
   (B95) 5-[4-(2-hydroxy-4-iodobenzoylamino)phenyl]-1H-naphtho[1,2-b][1,4]diazepine-2,4(3H,5H)-dione;
   (B96) 5-[4-(6-fluoro-2-hydroxybenzoylamino)phenyl]-1H-naphtho[1,2-b][1,4]diazepine-2,4(3H,5H)-dione;
   (B97) 5-[4-(2-fluorobenzoyl)aminophenyl]-1H-naphtho[1,2-b][1,4]diazepine-2,4(3H,5H)-dione;
   (B98) 5-[4-[(2-dimethylaminobenzoyl)amino]phenyl]-1H-naphtho[1,2-b][1,4]diazepine-2,4(3H,5H)-dione;
   (B99) 5-[4-(2-methoxy-6-methylbenzoylamino)phenyl]-1H-naphtho[1,2-b][1,4]diazepine-2,4(3H,5H)-dione;
   (B100) 5-[4-(2-hydroxy-6-methylbenzoylamino)phenyl]-1H-naphtho[1,2-b][1,4]diazepine-2,4(3H,5H)-dione;
   (B101) 5-[4-[3-(2-methylphenyl)propionylamino]phenyl]-1H-naphtho[1,2-b][1,4]diazepine-2,4(3H,5H)-dione;
   (B102) 5-(4-phenylcarbamoylphenyl)-1H-naphtho[1,2-b][1,4]diazepine-2,4(3H,5H)-dione;
   (B103) 5-(4-benzylcarbamoylphenyl)-1H-naphtho[1,2-b][1,4]diazepine-2,4(3H,5H)-dione;
   (B104) 5-[4-[3-(2-methylphenyl)propenoylamino]phenyl]-1H-naphtho[1,2-b][1,4]diazepine-2,4(3H,5H)-dione;
   (B105) 5-[4-[3-(2-chlorophenyl)propionylamino]phenyl]-1H-naphtho[1,2-b][1,4]diazepine-2,4(3H,5H)-dione;
   (B106) 5-[4-(2-iodobenzoyl)aminophenyl]-1,2,3,4,8,9,10,11-octahydronaphtho[1,2-b][1,4]diazepine-2,4(3H,5H)-dione;
   (B107) 5-[4-[(1-methyl-1H-pyrrol-2-ylacetyl)amino]phenyl]-1,2,3,4,8,9,10,11-octahydronaphtho[1,2-b][1,4]diazepine-2,4(3H,5H)-dione;
   (B108) 5-[4-(2-chlorobenzyl)carbamoylphenyl]-1H-naphtho[1,2-b][1,4]diazepine-2,4(3H,5H)-dione;
   (B109) 5-[4-[3-(2-chlorophenyl)propenoylamino]phenyl]-1H-naphtho[1,2-b][1,4]diazepine-2,4(3H,5H)-dione;
   (B110) 5-[4-(2-chlorophenyl)carbamoylphenyl]-1H-naphtho[1,2-b][1,4]diazepine-2,4(3H,5H)-dione;
   (Bill) 5-[4-(6-bromo-2,3-methylenedioxybenzoylamino)phenyl]-1H-naphtho[1,2-b][1,4]diazepine-2,4(3H,5H)-dione;
   (B112) 5-[4-(6-bromo-2-methoxybenzoylamino)phenyl]-1H-naphtho[1,2-b][1,4]diazepine-2,4(3H,5H)-dione;
   (B113) 5-[4-[(2-tert-butylbenzoyl)amino]phenyl]-1,2,3,4,8,9,10,11-octahydronaphtho[1,2-b][1,4]diazepine-2,4(3H,5H)-dione;
   (B114) 5-[2-(2-iodobenzoyl)aminopyridin-5-yl]-1H-naphtho[1,2-b][1,4]diazepine-2,4(3H,5H)-dione;
   (B115) 5-[4-(6-bromo-2-hydroxybenzoylamino)phenyl]-1H-naphtho[1,2-b][1,4]diazepine-2,4(3H,5H)-dione;
   (B116) 5-[4-(6-chloro-2-methoxybenzoylamino)phenyl]-1H-naphtho[1,2-b][1,4]diazepine-2,4(3H,5H)-dione;
   (B117) 5-[4-(2-iodobenzoylamino)phenyl]-1H-[1,4]diazepino[2,3-h]quinoline-2,4(3H,5H)-dione;
   (B118) 5-[4-(6-chloro-2-hydroxybenzoylamino)phenyl]-1H-naphtho[1,2-b][1,4]diazepine-2,4(3H,5H)-dione;
   (B119) 5-[4-(2-hydroxy-6-methoxybenzoylamino)phenyl]-1H-naphtho[1,2-b][1,4]diazepine-2,4(3H,5H)-dione;
   (B120) 5-[4-[2-methoxy-6-(trifluoromethyl)benzoylamino]phenyl]-1H-naphtho[1,2-b][1,4]diazepine-2,4(3H,5H)-dione;
   (B121) 5-[4-[2-hydroxy-6-(trifluoromethyl)benzoylamino]phenyl]-1H-naphtho[1,2-b][1,4]diazepine-2,4(3H,5H)-dione;
   (B122) 5-[4-[(2-isopropenylbenzoyl)amino]phenyl]-1,2,3,4,8,9,10,11-octahydronaphtho[1,2-b][1,4]diazepine-2,4(3H,5H)-dione;
   (B123) 5-[4-[(2-isopropylbenzoyl)amino]phenyl]-1,2,3,4,8,9,10,11-octahydronaphtho[1,2-b][1,4]diazepine-2,4(3H,5H)-dione;
   (B124) 5-[4-[2-chloro-5-(methylthio)benzoylamino]phenyl]-1H-naphtho[1,2-b][1,4]diazepine-2,4(3H,5H)-dione;
   (B125) 5-[4-[2-(methylthio)benzoylamino]phenyl]-1H-naphtho[1,2-b][1,4]diazepine-2,4(3H,5H)-dione;
   (B126) 5-[4-[3-(methylthio)benzoylamino]phenyl]-1H-naphtho[1,2-b][1,4]diazepine-2,4(3H,5H)-dione;
   (B127) 5-[4-[2-ethyl-6-methoxybenzoylamino]phenyl]-1H-naphtho[1,2-b][1,4]diazepine-2,4(3H,5H)-dione;
   (B128) 5-[4-(3-methanesulfonylbenzoylamino)phenyl]-1H-naphtho[1,2-b][1,4]diazepine-2,4(3H,5H)-dione;
   (B129) 6-ethyl-1-[4-(2-iodobenzoyl)aminophenyl]-1H-1,5-benzodiazepine-2,4(3H,5H)-dione;
   (B130) 5-[4-[2-ethyl-6-hydroxybenzoylamino]phenyl]-1H-naphtho[1,2-b][1,4]diazepine-2,4(3H,5H)-dione;
   (B131) 5-[4-(3-methanesulfinylbenzoylamino)phenyl]-1H-naphtho[1,2-b][1,4]diazepine-2,4(3H,5H)-dione;
   (B132) 5-[4-(2-chloro-5-methanesulfinylbenzoylamino)phenyl]-1H-naphtho[1,2-b][1,4]diazepine-2,4(3H,5H)-dione;
   (B133) 5-[4-(2-methanesulfinylbenzoylamino)phenyl]-1H-naphtho[1,2-b][1,4]diazepine-2,4(3H,5H)-dione;
   (B134) 5-[4-[[2-(4-morpholinyl)acetyl]amino]phenyl]-1,2,3,4,8,9,10,11-octahydronaphtho[1,2-b][1,4]diazepine-2,4(3H,5H)-dione hydrochloride;
   (B135) 5-[4-(2-chloro-6-methoxybenzoylamino)phenyl]-1,3-dihydronaphtho[1,2-e]-1,4-diazepine-2-one;
   (B136) 5-[4-[[(3-chloropyridin-2-yl)carbonyl]amino]phenyl]-1H-naphtho[1,2-b][1,4]diazepine-2,4(3H,5H)-dione;
   (B137) 5-[4-(2-chloro-6-hydroxybenzoylamino)phenyl]-1,3-dihydronaphtho[1,2-e]-1,4-diazepine-2-one;
   (B138) 5-[4-(3-chloro-2-methoxybenzoylamino)phenyl]-1H-naphtho[1,2-b][1,4]diazepine-2,4(3H,5H)-dione;
   (B139) 5-[4-[(3-methylpyridin-2-yl)carbonylamino]phenyl]-1H-naphtho[1,2-b][1,4]diazepine-2,4(3H,5H)-dione;
   (B140) 5-[4-[[(3-chloropyridin-2-yl)carbonyl]amino]phenyl]-1,2,3,4,8,9,10,11-octahydronaphtho[1,2-b][1,4]diazepine-2,4(3H,5H)-dione;
   (B141) 5-[4-(3-chloro-2-hydroxybenzoylamino)phenyl]-1H-naphtho[1,2-b][1,4]diazepine-2,4(3H,5H)-dione;
   (B142) 5-[4-[[(3-hydroxypyridin-2-yl)carbonyl]amino]phenyl]-1H-naphtho[1,2-b][1,4]diazepine-2,4(3H,5H)-dione;
   (B143) 5-[4-[(3-vinylpyridin-2-yl)carbonylamino]phenyl]-1H-naphtho[1,2-b][1,4]diazepine-2,4(3H,5H)-dione;
   (B144) 5-[4-[(3-ethylpyridin-2-yl)carbonylamino]phenyl]-1H-naphtho[1,2-b][1,4]diazepine-2,4(3H,5H)-dione;
   (B145) N-[4-(2,4-dioxo-1,2,3,4-tetrahydronaphtho-[1,2-b][1,4]-diazepin-5-yl)phenyl]-2-nitrobenzenesulfonamide;
   (B146) N-[4-(2,4-dioxo-1,2,3,4-tetrahydronaphtho[1,2-b][1,4]diazepin-5-yl)phenyl]benzenesulfonamide;
   (B147) 3-bromo-N-[4-(2,4-dioxo-1,2,3,4-tetrahydronaphtho[1,2-b][1,4]diazepin-5-yl)phenyl]benzenesulfonamide;
   (B148) N-[4-(2,4-dioxo-1,2,3,4-tetrahydronaphtho[1,2-b][1,4]diazepin-5-yl)phenyl]-3-methoxybenzenesulfonamide;
   (B149) N-[3-(2-oxo-2,3-dihydro-1H-naphtho[1,2-e][1,4]diazepin-5-yl)phenyl]benzenesulfonamide;
   (B150) N-[3-(2,4-dioxo-1,2,3,4-tetrahydronaphtho-[1,2-b][1,4]-diazepin-5-yl)phenyl]-2-nitrobenzenesulfonamide;
   (B151) N-[4-(2,4-dioxo-1,2,3,4,8,9,10,11-octahydro-naphtho[1,2-b][1,4]-diazepin-5-yl)phenyl]-2-nitrobenzenesulfonamide;
   (B152) N-[4-(2,4-dioxo-1,2,3,4,8,9,10,11-octahydro-naphtho[1,2-b][1,4]-diazepin-5-yl)phenyl]-N-methyl-2-nitrobenzenesulfonamide;
   (B153) N-[3-(2,4-dioxo-1,2,3,4-tetrahydronaphtho-[1,2-b][1,4]-diazepin-5-yl)phenyl]-N-methyl-2-nitrobenzenesulfonamide;
   (B154) 4-(2,4-dioxo-1,2,3,4,8,9,10,11-octahydronaphtho[1,2-b][1,4]diazepin-5-yl)-N-phenylbenzenesulfonamide;
   (B155) N-[3-(2,4-dioxo-1,2,3,4-tetrahydronaphtho[1,2-b][1,4]diazepin-5-yl)phenyl]-2-naphthalenesulfonamide;
   (B156) N-[3-(2,4-dioxo-1,2,3,4-tetrahydronaphtho[1,2-b][1,4]diazepin-5-yl)phenyl]-1-naphthalenesulfonamide;
   (B157) N-[4-(2,4-dioxo-1,2,3,4-tetrahydronaphtho[1,2-b][1,4]diazepin-5-yl)phenyl]cyclohexanesulfonamide;
   (B158) N-[4-(2,4-dioxo-1,2,3,4-tetrahydronaphtho[1,2-b][1,4]diazepin-5-yl)phenyl]-3-pyridinesulfonamide hydrochloride;
   (B159) N-[4-(2,4-dioxo-1,2,3,4-tetrahydronaphtho[1,2-b][1,4]diazepin-5-yl)phenyl]-4-isopropylbenzenesulfonamide;
   (B160) N-[4-(2,4-dioxo-1,2,3,4-tetrahydronaphtho[1,2-b][1,4]diazepin-5-yl)phenyl]phenylmethanesulfonamide;
   (B161) N-[4-(2,4-dioxo-1,2,3,4-tetrahydronaphtho[1,2-b][1,4]diazepin-5-yl)phenyl]-2-thiophenesulfonamide;
   (B162) N-[4-(2,4-dioxo-1,2,3,4-tetrahydronaphtho[1,2-b][1,4]diazepin-5-yl)phenyl]-2-naphthalenesulfonamide;
   (B163) 4-(2,4-dioxo-1,2,3,4,8,9,10,11-octahydronaphtho[1,2-b][1,4]diazepin-5-yl)phenyl 3-bromobenzenesulfonate;
   (B164) N-benzyl-N-[4-(1-benzyl-2,4-dioxo-1,2,3,4-tetrahydronaphtho[1,2-b][1,4]diazepin-5-yl)phenyl]-2-nitrobenzenesulfonamide;
   (B165) N-benzyl-N-[4-(2,4-dioxo-1,2,3,4-tetrahydronaphtho[1,2-b][1,4]diazepin-5-yl)phenyl]-2-nitrobenzenesulfonamide;
   (B166) 3-bromo-N-[4-(2,4-dioxo-1,2,3,4-tetrahydronaphtho[1,2-b][1,4]diazepin-5-yl)phenyl]-N-methylbenzenesulfonamide;
   (B167) N-[4-(2,4-dioxo-1,2,3,4-tetrahydronaphtho-[1,2-b][1,4]-diazepin-5-yl)phenyl]-N-methyl-2-nitrobenzenesulfonamide;
   (B168) N-[4-(2,4-dioxo-1,2,3,4-tetrahydronaphtho-[1,2-b][1,4]-diazepin-5-yl)phenyl]-N-(2-hydroxyethyl)-2-nitrobenzenesulfonamide;
   (B169) N-[4-(7-chloro-2,4-dioxo-2,3,4,5-tetrahydro-1H-benzo[b][1,4]diazepin-1-yl)phenyl]benzenesulfonamide;
   (B170) N-[4-(7-bromo-2,4-dioxo-2,3,4,5-tetrahydro-1H-benzo[b][1,4]diazepin-1-yl)phenyl]benzenesulfonamide;
   (B171) N-[4-[(2,4-dioxo-7-(trifluoromethyl)-2,3,4,5-tetrahydro-1H-benzo[b][1,4]diazepin-1-yl)]phenyl]benzenesulfonamide;
   (B172) N-[4-(2,4-dioxo-2,3,4,5-tetrahydro-1H-benzo[b][1,4]diazepin-1-yl)phenyl]benzenesulfonamide;
   (B173) 1-(2-chlorophenyl)-N-[4-(2,4-dioxo-1,2,3,4-tetrahydronaphtho[1,2-b][1,4]diazepin-5-yl)phenyl]methanesulfonamide;
   (B174) 1-(3-bromophenyl)-N-[4-(2,4-dioxo-1,2,3,4-tetrahydronaphtho[1,2-b][1,4]diazepin-5-yl)phenyl]methanesulfonamide;
   (B175) N-[4-(2,4-dioxo-1,2,3,4-tetrahydronaphtho-[1,2-b][1,4]-diazepin-5-yl)phenyl]-2-trifluoromethylbenzenesulfonamide;
   (B176) N-[4-(7-bromo-6-methyl-2,4-dioxo-2,3,4,5-tetrahydro-1H-benzo[b][1,4]diazepin-1-yl)phenyl]benzenesulfonamide;
   (B177) 1-(2-chlorophenyl)-N-[4-(2,4-dioxo-1,2,3,4,8,9,10,11-octahydronaphtho[1,2-b][1,4]diazepin-5-yl)phenyl]methanesulfonamide;
   (B178) 3-bromo-N-[4-(2,4-dioxo-1,2,3,4,8,9,10,11-octahydronaphtho[1,2-b][1,4]diazepin-5-yl)phenyl]benzenesulfonamide;
   (B179) N-[4-(2,4-dioxo-1,2,3,4,8,9,10,11-octahydronaphtho[1,2-b][1,4]diazepin-5-yl)phenyl]-3-methoxybenzenesulfonamide;
   (B180) 1-(2-bromophenyl)-N-[4-(2,4-dioxo-1,2,3,4-tetrahydronaphtho[1,2-b][1,4]diazepin-5-yl)phenyl]methanesulfonamide;
   (B181) N-[4-(2,4-dioxo-1,2,3,4-tetrahydronaphtho[1,2-b][1,4]diazepin-5-yl)phenyl]-1-(2-methylphenyl)methanesulfonamide;
   (B182) N-[4-(2,4-dioxo-1,2,3,4-tetrahydronaphtho[1,2-b][1,4]diazepin-5-yl)phenyl]-1-(2-nitrophenyl)methanesulfonamide;
   (B183) N-[4-(2,4-dioxo-1,2,3,4-tetrahydronaphtho[1,2-b][1,4]diazepin-5-yl)phenyl]-2-phenylethanesulfonamide;
   (B184) 1-(2,3-dichlorophenyl)-N-[4-(2,4-dioxo-1,2,3,4,8,9,10,11-octahydronaphtho[1,2-b][1,4]diazepin-5-yl)phenyl]methanesulfonamide;
   (B185) 1-(2-chlorophenyl)-N-[4-(2,4-dioxo-7-methoxy-1H-benzo[1,2-b][1,4]diazepin-1-yl)phenyl]methanesulfonamide;
   (B186) 1-(2-chlorophenyl)-N-[4-(2,4-dioxo-7-hydroxy-1H-benzo[1,2-b][1,4]diazepin-1-yl)phenyl]methanesulfonamide;
   (B187) 1-(4-chlorophenyl)-N-[4-(2,4-dioxo-1,2,3,4-tetrahydronaphtho[1,2-b][1,4]diazepin-5-yl)phenyl]methanesulfonamide;
   (B188) 1-(2-chlorophenyl)-N-[4-(2,4-dioxo-1,2,3,4-tetrahydronaphtho[1,2-b][1,4]diazepin-5-yl)benzyl]methanesulfonamide;
   (B189) 1-(2-chlorophenyl)-N-[4-(2,4-dioxo-1,2,3,4-tetrahydronaphtho[1,2-b][1,4]diazepin-5-yl)-2-methoxyphenyl]methanesulfonamide;
   (B190) 1-(2-chlorophenyl)-N-[4-(2,4-dioxo-1,2,3,4-tetrahydronaphtho[1,2-b][1,4]diazepin-5-yl)-2-hydroxyphenyl]methanesulfonamide;
   (B191) 1-(2,6-dichlorophenyl)-N-[4-(2,4-dioxo-1,2,3,4,8,9,10,11-octahydronaphtho[1,2-b][1,4]diazepin-5-yl)phenyl]methanesulfonamide;
   (B192) 1-(2-chlorophenyl)-N-[4-(2,4-dioxo-6-methyl-1H-benzo[1,2-b][1,4]diazepin-1-yl)phenyl]methanesulfonamide;
   (B193) 1-(2-chlorophenyl)-N-[3-(2,4-dioxy-1,2,3,4-tetrahydronaphtho[1,2-b][1,4]diazepin-5-yl)propyl]methanesulfonamide;
   (B194) 1-(2-chlorophenyl)-N-[2-(2,4-dioxo-1,2,3,4-tetrahydronaphtho[1,2-b][1,4]diazepin-5-yl)ethyl]methanesulfonamide;
   (B195) N-[4-(2,4-dioxo-1,2,3,4,8,9,10,11-octahydronaphtho[1,2-b][1,4]diazepin-5-yl)phenyl]-1-(2-iodophenyl)methanesulfonamide;
   (B196) 1-(2-chlorophenyl)-N-[4-(2,4-dioxo-1,2,3,4,8,9,10,11-octahydronaphtho[1,2-b][1,4]diazepin-5-yl)phenyl]-N-methylmethanesulfonamide;
   (B197) 1-(2-chlorophenyl)-N-[4-(2-oxo-2,3-dihydro-1H-naphtho[1,2-e][1,4]diazepin-5-yl)phenyl]methanesulfonamide;
   (B198) 1-[2-(trifluoromethyl)phenyl]-N-[4-(2,4-dioxo-1,2,3,4-tetrahydronaphtho[1,2-b][1,4]diazepin-5-yl)phenyl]phenyl-N-methylmethanesulfonamide;
   (B199) 1-(2-ethylphenyl)-N-[4-(2,4-dioxo-1,2,3,4-tetrahydronaphtho[1,2-b][1,4]diazepin-5-yl)phenyl]phenyl-N-methylmethanesulfonamide;
   (B200) 1-(2,3-dimethylphenyl)-N-[4-(2,4-dioxo-1,2,3,4-tetrahydronaphtho[1,2-b][1,4]diazepin-5-yl)phenyl]phenyl-N-methylmethanesulfonamide;
   (B201) 2-(2-chlorophenyl)-N-[4-(2,4-dioxo-1,2,3,4-tetrahydronaphtho[1,2-b][1,4]diazepin-5-yl)phenyl]phenyl-N-methylethanesulfonamide;
   (B202) 1-(2-nitrophenyl)-N-[4-(2,4-dioxo-1,2,3,4-tetrahydronaphtho[1,2-b][1,4]diazepin-5-yl)phenyl]phenyl-N-methylmethanesulfonamide;
   (B203) 1-(2-aminophenyl)-N-[4-(2,4-dioxo-1,2,3,4-tetrahydronaphtho[1,2-b][1,4]diazepin-5-yl)phenyl]phenyl-N-methylmethanesulfonamide;
   (B204) 1-(2-dimethylaminophenyl)-N-[4-(2,4-dioxo-1,2,3,4-tetrahydronaphtho[1,2-b][1,4]diazepin-5-yl)phenyl]phenyl-N-methylmethanesulfonamide;
   (B205) 5-[4-[(pyridin-4-yl)carbonylamino]phenyl]-1H-naphtho[1,2-b][1,4]diazepine-2,4(3H,5H)-dione hydrochloride;
   (B206) 5-[4-[2-[(pyridin-3-yl)oxy]acetylamino]phenyl]-1H-naphtho[1,2-b][1,4]diazepine-2,4(3H,5H)-dione hydrochloride;
   (B207) 5-[4-[(pyridin-3-yl)carbonylamino]phenyl]-1H-naphtho[1,2-b][1,4]diazepine-2,4(3H,5H)-dione hydrochloride;
   (B208) 5-[4-[(2-methylpyridin-3-yl)carbonylamino]phenyl]-1H-naphtho[1,2-b][1,4]diazepine-2,4(3H,5H)-dione hydrochloride;
   (B209) 5-[4-[(2-chloropyridin-3-yl)carbonylamino]phenyl]-1H-naphtho[1,2-b][1,4]diazepine-2,4(3H,5H)-dione;
   (B210) 5-[4-[2-[(pyridin-2-yl)oxy]acetylamino]phenyl]-1H-naphtho[1,2-b][1,4]diazepine-2,4(3H,5H)-dione;
   (B211) 5-[4-[[4-(trifluoromethyl)pyridin-3-yl]carbonylamino]phenyl]-1H-naphtho[1,2-b][1,4]diazepine-2,4(3H,5H)-dione;
   (B212) 5-[4-[(2-chloropyridin-3-yl)carbonylamino]phenyl]-1H-[1,4]diazepino[2,3-f]isoquinoline-2,4(3H,5H)-dione;
   (B213) 5-[4-[(2-chloropyridin-3-yl)carbonylamino]phenyl]-8 , 9, 10, 11-tetrahydro-1H-[1,4]diazepino[2,3-f]isoquinoline-2,4(3H,5H)-dione; and
   (B214) 5-[4-[(2-isopropylbenzoyl)amino]phenyl]-1H-naphtho[1,2-b][1,4]diazepine-2,4(3H,5H)-dione.
[8] The pharmaceutical composition according to any one of [1] to [4], in which the compound is a compound selected from the group consisting of the following (C1) to (C297):
   (C1) N-[4-(3-chloro-5-cyanophenoxy)-3-sulfamoylphenyl]-2-(2-chlorophenyl)acetamide;
   (C2) 2-(2-chlorophenyl)-N-4-[3-(dimethylamino)phenoxy]-3-sulfamoylphenylacetamide;
   (C3) 2-(2-chlorophenyl)-N-4-[(2-chloropyridin-4-yl)oxy]-3-sulfamoylphenylacetamide;
   (C4) 2-(2-chlorophenyl)-N-[4-(3-isopropylphenoxy)-3-sulfamoylphenyl]acetamide;
   (C5) 2-(2-chlorophenyl)-N-3-sulfamoyl-4-[3-(trifluoromethyl)phenoxy]phenylacetamide;
   (C6) 2-(2-chlorophenyl)-N-3-sulfamoyl-4-[3-(trifluoromethoxy)phenoxy]phenylacetamide;
   (C7) N-[4-(3-acetylphenoxy)-3-sulfamoylphenyl]-2-(2-chlorophenyl) acetamide;
   (C8) N-[4-(1,3-benzodioxol-5-yloxy)-3-sulfamoylphenyl]-2-(2-chlorophenyl)acetamide;
   (C9) N-[4-(3-acetamidophenoxy)-3-sulfamoylphenyl]-2-(2-chlorophenyl) acetamide;
   (C10) 2-(2-chlorophenyl)-N-[4-(2-fluorophenoxy)-3-sulfamoylphenyl]acetamide;
   (C11) 2-(2-chlorophenyl)-N-[4-(3-fluorophenoxy)-3-sulfamoylphenyl]acetamide;
   (C12) 2-(2-chlorophenyl)-N-[4-(4-fluorophenoxy)-3-sulfamoylphenyl]acetamide;
   (C13) 2-(2-chlorophenyl)-N-[4-(pyridin-2-yloxy)-3-sulfamoylphenyl]acetamide;
   (C14) 2-(2-chlorophenyl)-N-(4-phenoxy-3-sulfamoylphenyl)acetamide;
   (C15) 2-(2-chlorophenyl)-N-[4-(3-cyanophenoxy)-3-sulfamoylphenyl]acetamide;
   (C16) 2-(2-chlorophenyl)-N-4-[3-(methylsulfonyl)phenoxy]-3-sulfamoylphenylacetamide;
   (C17) 3-(4-[(2-chlorophenyl)acetyl]amino-2-sulfamoylphenoxy)benzamide;
   (C18) 2-(2-chlorophenyl)-N-[4-(3-methylphenoxy)-3-sulfamoylphenyl]acetamide;
   (C19) 2-(2-chlorophenyl)-N-[4-(pyrimidin-5-yloxy)-3-sulfamoylphenyl]acetamide;
   (C20) 2-(2-chlorophenyl)-N-3-sulfamoyl-4-[3-(4H-1,2,4-triazol-4-yl)phenoxy]phenylacetamide;
   (C21) 2-(2-chlorophenyl)-N-3-sulfamoyl-4-[3-(1H-tetrazol-5-yl)phenoxy]phenylacetamide;
   (C22) 2-(2-chlorophenyl)-N-[4-(3-methoxyphenoxy)-3-sulfamoylphenyl]acetamide;
   (C23) 2-(2-chlorophenyl)-N-[4-(4-methoxyphenoxy)-3-sulfamoylphenyl]acetamide;
   (C24) 2-(2-chlorophenyl)-N-4-[3-(difluoromethoxy)phenoxy]-3-sulfamoylphenylacetamide;
   (C25) 2-(2-chlorophenyl)-N-[4-(3,4-dicyanophenoxy)-3-sulfamoylphenyl]acetamide;
   (C26) 2-(2-chlorophenyl)-N-4-[3-(morpholin-4-yl)phenoxy]-3-sulfamoylphenylacetamide;
   (C27) 2-(2-chlorophenyl)-N-[4-(3-4-[(2-chlorophenyl)acetyl]piperazin-1-ylphenoxy)-3-sulfamoylphenyl]acetamide;
   (C28) 2-(2-chlorophenyl)-N-[4-(pyridin-3-yloxy)-3-sulfamoylphenyl]acetamide;
   (C29) 2-(2-chlorophenyl)-N-4-[(5-chloropyridin-3-yl)oxy]-3-sulfamoylphenylacetamide;
   (C30) 2-(2-chlorophenyl)-N-[4-(4-cyanophenoxy)-3-sulfamoylphenyl]acetamide;
   (C31) 2-(2-chlorophenyl)-N-4-[3-(difluoromethyl)phenoxy]-3-sulfamoylphenylacetamide;
   (C32) 2-(2-chlorophenyl)-N-[4-(2-methoxyphenoxy)-3-sulfamoylphenyl]acetamide;
   (C33) 2-(2-chlorophenyl)-N-[4-(3,5-dicyanophenoxy)-3-sulfamoylphenyl]acetamide;
   (C34) 2-(2-chlorophenyl)-N-[4-(5-cyano-2-methoxyphenoxy)-3-sulfamoylphenyl]acetamide;
   (C35) 2-(2-chlorophenyl)-N-4-[(2,5-dichloropyridin-3-yl)oxy]-3-sulfamoylphenylacetamide;
   (C36) 2-(2-chlorophenyl)-N-4-[(5,6-dichloropyridin-3-yl)oxy]-3-sulfamoylphenylacetamide;
   (C37) 3-(4-[(2-chlorophenyl)acetyl]amino-2-sulfamoylphenoxy)-N-cyclopropylbenzamide;
   (C38) 2-(2-chlorophenyl)-N-4-[(3-chloropyridin-2-yl)oxy]-3-sulfamoylphenylacetamide;
   (C39) 2-(2-chlorophenyl)-N-4-[(4-chloropyridin-2-yl)oxy]-3-sulfamoylphenylacetamide;
   (C40) 2-(2-chlorophenyl)-N-4-[(6-chloropyridin-2-yl)oxy]-3-sulfamoylphenylacetamide;
   (C41) 2-(2-chlorophenyl)-N-4-[3-(1-methyl-4,5-dihydro-1H-imidazol-2-yl)phenoxy]-3-sulfamoylphenylacetamide;
   (C42) 2-(2-chlorophenyl)-N-4-[4-(1H-imidazol-1-yl)phenoxy]-3-sulfamoylphenylacetamide;
   (C43) 2-(2-chlorophenyl)-N-4-[4-(2-oxopyrrolidin-1-yl)phenoxy]-3-sulfamoylphenylacetamide;
   (C44) 2-(2-chlorophenyl)-N-4-[4-(morpholin-4-yl)phenoxy]-3-sulfamoylphenylacetamide;
   (C45) 2-(2-chlorophenyl)-N-[4-(5-cyano-2-methylphenoxy)-3-sulfamoylphenyl]acetamide;
   (C46) 2-(2-chlorophenyl)-N-[4-(3-cyano-2-methylphenoxy)-3-sulfamoylphenyl]acetamide;
   (C47) 2-(2-chlorophenyl)-N-[4-(3-cyano-4-fluorophenoxy)-3-sulfamoylphenyl]acetamide;
   (C48) N-4-[(5-chloro-2-cyanopyridin-3-yl)oxy]-3-sulfamoylphenyl-2-(2-chlorophenyl)acetamide;
   (C49) 2-(2-chlorophenyl)-N-4-[3-(piperidin-1-yl)phenoxy]-3-sulfamoylphenylacetamide;
   (C50) 2-(2-chlorophenyl)-N-4-[3-(2-oxopyrrolidin-1-yl)phenoxy]-3-sulfamoylphenylacetamide;
   (C51) 2-(2-chlorophenyl)-N-4-[3-(2-oxo-1,3-oxazolidin-3-yl)phenoxy]-3-sulfamoylphenylacetamide;
   (C52) 2-(2-chlorophenyl)-N-4-[3-(morpholin-4-ylcarbonyl)phenoxy]-3-sulfamoylphenylacetamide;
   (C53) 2-(2-chlorophenyl)-N-4-[(4-methyltetrahydro-2H-pyran-4-yl)methoxy]-3-sulfamoylphenylacetamide;
   (C54) 2-(2-chlorophenyl)-N-4-[(4-fluorotetrahydro-2H-pyran-4-yl)methoxy]-3-sulfamoylphenylacetamide;
   (C55) 2-(2-chlorophenyl)-N-4-[(4-cyanotetrahydro-2H-pyran-4-yl)methoxy]-3-sulfamoylphenylacetamide;
   (C56) 2-(2-chlorophenyl)-N-(3-sulfamoyl-4-[2-(trifluoromethyl)pyrimidin-5-yl]oxyphenyl)acetamide;
   (C57) 2-(2-chlorophenyl)-N-4-[(2-isopropylpyrimidin-5-yl)oxy]-3-sulfamoylphenylacetamide;
   (C58) 2-(2-chlorophenyl)-N-4-[(2-cyclopropyl-4-methylpyrimidin-5-yl)oxy]-3-sulfamoylphenylacetamide;
   (C59) N-[4-(3-bromophenoxy)-3-sulfamoylphenyl]-2-(2-chlorophenyl) acetamide;
   (C60) N-[4-(4-bromophenoxy)-3-sulfamoylphenyl]-2-(2-chlorophenyl) acetamide;
   (C61) 2-(2-chlorophenyl)-N-4-[3-(2-methyl-1,3-thiazol-4-yl)phenoxy]-3-sulfamoylphenylacetamide;
   (C62) 2-(2-chlorophenyl)-N-4-[4-(5-oxopyrrolidin-2-yl)phenoxy]-3-sulfamoylphenylacetamide;
   (C63) 2-(2-chlorophenyl)-N-4-[4-(2-oxo-1,3-oxazolidin-3-yl)phenoxy]-3-sulfamoylphenylacetamide;
   (C64) 2-(2-chlorophenyl)-N-3-sulfamoyl-4-[4-(1,3-thiazol-2-yl)phenoxy]phenylacetamide;
   (C65) N-[4-(2-chlorophenoxy)-3-sulfamoylphenyl]-2-(2-chlorophenyl) acetamide;
   (C66) N-[4-(4-chlorophenoxy)-3-sulfamoylphenyl]-2-(2-chlorophenyl) acetamide;
   (C67) 2-(2-chlorophenyl)-N-4-[3-(piperidin-1-ylcarbonyl)phenoxy]-3-sulfamoylphenylacetamide;
   (C68) 2-(2-chlorophenyl)-N-3-sulfamoyl-4-[4-(tetrahydrofuran-3-yl)phenoxy]phenylacetamide;
   (C69) 2-(2-chlorophenyl)-N-[4-(3-cyano-5-fluorophenoxy)-3-sulfamoylphenyl]acetamide;
   (C70) N-[4-(2-methoxyphenoxy)-3-sulfamoylphenyl]-2-phenylacetamide;
   (C71) N-[4-(2-methoxyphenoxy)-3-sulfamoylphenyl]-2-[4-(trifluoromethyl)phenyl]acetamide;
   (C72) N-3-sulfamoyl-4-[2-(trifluoromethoxy)phenoxy]phenyl-2-[4-(trifluoromethyl)phenyl]acetamide;
   (C73) N-[4-(2-chlorophenoxy)-3-sulfamoylphenyl]-2-[4-(trifluoromethyl)phenyl]acetamide;
   (C74) 2-phenyl-N-3-sulfamoyl-4-[2-(trifluoromethoxy)phenoxy]phenylacetamide;
   (C75) 2-(2-chlorophenyl)-N-4-[(2-oxo-1,2-dihydropyridin-3-yl)oxy]-3-sulfamoylphenylacetamide;
   (C76) N-[4-(2-chlorophenoxy)-3-sulfamoylphenyl]-2-phenylacetamide;
   (C77) N-[4-(4-chlorophenoxy)-3-sulfamoylphenyl]-2-phenylacetamide;
   (C78) N-4-[(5-chloropyridin-3-yl)oxy]-3-sulfamoylphenyl-2-phenylacetamide;
   (C79) 2-(2-chlorophenyl)-N-4-[(2-chloropyrimidin-5-yl)oxy]-3-sulfamoylphenylacetamide;
   (C80) 2-(2-chlorophenyl)-N-4-[(5-fluoropyridin-3-yl)oxy]-3-sulfamoylphenylacetamide;
   (C81) 2-(2-chlorophenyl)-N-4-[(6-chloropyridin-3-yl)oxy]-3-sulfamoylphenylacetamide;
   (C82) N-[2-chloro-4-(3-chlorophenoxy)-5-sulfamoylphenyl]-2-(2-chlorophenyl)acetamide;
   (C83) N-[2-chloro-4-(3-chlorophenoxy)-5-sulfamoylphenyl]-2-(2-chloro-3-fluorophenyl)acetamide;
   (C84) N-[4-(3-chlorophenoxy)-3-sulfamoylphenyl]-2-(3-fluorophenyl) acetamide;
   (C85) N-[4-(3-chlorophenoxy)-3-sulfamoylphenyl]-2-(4-fluorophenyl) acetamide;
   (C86) N-[4-(3-chlorophenoxy)-3-sulfamoylphenyl]-2-[2-(trifluoromethyl)phenyl]acetamide;
   (C87) N-[4-(3-chlorophenoxy)-3-sulfamoylphenyl]-2-(2-isopropylphenyl) acetamide;
   (C88) N-[4-(3-chlorophenoxy)-3-sulfamoylphenyl]-2-(2-ethoxyphenyl) acetamide;
   (C89) N-[4-(3-chlorophenoxy)-3-sulfamoylphenyl]-2-[2-(difluoromethyl)phenyl]acetamide;
   (C90) N-[4-(3-chlorophenoxy)-3-sulfamoylphenyl]-2-2-[(trifluoromethyl)sulfanyl]phenylacetamide;
   (C91) 2-(2-Bromophenyl)-N-[4-(3-chlorophenoxy)-3-sulfamoylphenyl]acetamide;
   (C92) N-[4-(3-chlorophenoxy)-3-sulfamoylphenyl]-2-(4-methylpyridin-3-yl)acetamide;
   (C93) N-[4-(3-chlorophenoxy)-3-sulfamoylphenyl]-2-(2-chloropyridin-3-yl)acetamide;
   (C94) N-[4-(3-chlorophenoxy)-3-sulfamoylphenyl]-2-(2-chlorophenyl)-2,2-difluoroacetamide;
   (C95) 2-(2-chloro-4-methylphenyl)-N-[4-(3-chlorophenoxy)-3-sulfamoylphenyl]acetamide;
   (C96) 2-(2-chloro-6-methylphenyl)-N-[4-(3-chlorophenoxy)-3-sulfamoylphenyl]acetamide;
   (C97) 2-(2-chloro-5-methylphenyl)-N-[4-(3-chlorophenoxy)-3-sulfamoylphenyl]acetamide;
   (C98) 2-(2-chloro-3-fluorophenyl)-N-[4-(3-chlorophenoxy)-3-sulfamoylphenyl]acetamide;
   (C99) 2-(2-chloro-5-fluorophenyl)-N-[4-(3-chlorophenoxy)-3-sulfamoylphenyl]acetamide;
   (C100) 2-(2-chloro-6-fluorophenyl)-N-[4-(3-chlorophenoxy)-3-sulfamoylphenyl]acetamide;
   (C101) 2-(2-chloro-6-methoxyphenyl)-N-[4-(3-chlorophenoxy)-3-sulfamoylphenyl]acetamide;
   (C102) 2-(2-chloro-5-methoxyphenyl)-N-[4-(3-chlorophenoxy)-3-sulfamoylphenyl]acetamide;
   (C103) N-[4-(3-chlorophenoxy)-3-sulfamoylphenyl]-2-(2,3-dichlorophenyl) acetamide;
   (C104) N-[4-(3-chlorophenoxy)-3-sulfamoylphenyl]-2-(2,6-dichlorophenyl) acetamide;
   (C105) N-[4-(3-chlorophenoxy)-3-sulfamoylphenyl]-2-[2-(trifluoromethoxy)phenyl]acetamide;
   (C106) N-[4-(3-chlorophenoxy)-3-sulfamoylphenyl]-2,2-difluoro-2-phenylacetamide;
   (C107) N-[4-(3-chlorophenoxy)-3-sulfamoylphenyl]-2-[2-chloro-3-(trifluoromethyl)phenyl]acetamide;
   (C108) N-[4-(3-chlorophenoxy)-3-sulfamoylphenyl]-2-[2-chloro-6-(trifluoromethyl)phenyl]acetamide;
   (C109) N-[4-(3-chlorophenoxy)-3-sulfamoylphenyl]-2-[2-chloro-5-(trifluoromethyl)phenyl]acetamide;
   (C110) N-[4-(3-chlorophenoxy)-3-sulfamoylphenyl]-2-(2,4-dichlorophenyl) acetamide;
   (C111) N-[4-(3-chlorophenoxy)-3-sulfamoylphenyl]-2-(4,6-dichloropyridin-3-yl)acetamide;
   (C112) N-[4-(3-chlorophenoxy)-3-sulfamoylphenyl]-2-(3-chloropyridin-2-yl)acetamide;
   (C113) N-[4-(3-chlorophenoxy)-3-sulfamoylphenyl]-2-[2-(difluoromethoxy)phenyl]acetamide;
   (C114) N-[4-(3-chlorophenoxy)-3-sulfamoylphenyl]-2-(2,5-dichlorophenyl)acetamide;
   (C115) 2-[6-chloro-2,3-difluoro-4-(trifluoromethyl)phenyl]-N-[4-(3-chlorophenoxy)-3-sulfamoylphenyl]acetamide;
   (C116) N-[4-(3-chlorophenoxy)-3-sulfamoylphenyl]-2-[4-(trifluoromethyl)phenyl]acetamide;
   (C117) 2-(5-bromo-2-chlorophenyl)-N-[4-(3-chlorophenoxy)-3-sulfamoylphenyl]acetamide;
   (C118) 2-(4-bromo-2-chloro-5-methylphenyl)-N-[4-(3-chlorophenoxy)-3-sulfamoylphenyl]acetamide;
   (C119) N-[4-(3-chlorophenoxy)-3-sulfamoylphenyl]-2-(3-chloropyridin-4-yl)acetamide;
   (C120) 2-(2-chloro-6-fluoro-3-methylphenyl)-N-[4-(3-chlorophenoxy)-3-sulfamoylphenyl]acetamide;
   (C121) 2-(6-chloro-2-fluoro-3-methylphenyl)-N-[4-(3-chlorophenoxy)-3-sulfamoylphenyl]acetamide;
   (C122) 2-(2-chloro-3,6-difluorophenyl)-N-[4-(3-chlorophenoxy)-3-sulfamoylphenyl]acetamide;
   (C123) 2-(2-chloro-4,5-difluorophenyl)-N-[4-(3-chlorophenoxy)-3-sulfamoylphenyl]acetamide;
   (C124) N-[4-(3-chlorophenoxy)-3-sulfamoylphenyl]-2-(2,3-dichloro-6-fluorophenyl)acetamide;
   (C125) N-[4-(3-chlorophenoxy)-3-sulfamoylphenyl]-2-(2,3,6-trichlorophenyl)acetamide;
   (C126) N-[4-(3-chlorophenoxy)-3-sulfamoylphenyl]-2-(2,6-dichloro-4-methylphenyl)acetamide;
   (C127) N-[4-(3-chlorophenoxy)-3-sulfamoylphenyl]-2-[2,3-dichloro-6-(trifluoromethyl)phenyl]acetamide;
   (C128) N-[4-(3-chlorophenoxy)-3-sulfamoylphenyl]-2-(2,6-dichloro-3-methylphenyl)acetamide;
   (C129) N-[4-(3-chlorophenoxy)-3-sulfamoylphenyl]-2-(2,6-dichloro-3-cyclopropylphenyl)acetamide;
   (C130) N-[4-(3-chlorophenoxy)-3-sulfamoylphenyl]-2-[2,6-dichloro-3-(trifluoromethyl)phenyl]acetamide;
   (C131) 2-(3-bromo-2,6-dichlorophenyl)-N-[4-(3-chlorophenoxy)-3-sulfamoylphenyl]acetamide;
   (C132) 2-(3-bromo-2-chloro-6-methylphenyl)-N-[4-(3-chlorophenoxy)-3-sulfamoylphenyl]acetamide;
   (C133) 2-(3-bromo-6-chloro-2-methylphenyl)-N-[4-(3-chlorophenoxy)-3-sulfamoylphenyl]acetamide;
   (C134) N-[4-(3-chlorophenoxy)-3-sulfamoylphenyl]-2-[2-chloro-5-(1,1,2,2-tetrafluoroethoxy)phenyl]acetamide;
   (C135) N-[4-(3-chlorophenoxy)-3-sulfamoylphenyl]-2-[2-chloro-4-(trifluoromethyl)phenyl]acetamide;
   (C136) 2-(2-chlorophenyl)-N-(4-[3-(methylsulfonyl)benzyl]oxy-3-sulfamoylphenyl)acetamide;
   (C137) 2-(2-chlorophenyl)-N-4-[(2-fluorobenzyl)oxy]-3-sulfamoylphenylacetamide;
   (C138) 2-(2-chlorophenyl)-N-4-[(4-cyanobenzyl)oxy]-3-sulfamoylphenylacetamide;
   (C139) N-4-[(3-chlorobenzyl)oxy]-3-sulfamoylphenyl-2-(2-chlorophenyl) acetamide;
   (C140) 2-(2-chlorophenyl)-N-4-[(3-methoxybenzyl)oxy]-3-sulfamoylphenylacetamide;
   (C141) N-[4-(benzyloxy)-3-sulfamoylphenyl]-2-(2-chlorophenyl) acetamide;
   (C142) 2-(2-chlorophenyl)-N-4-[(3-cyanobenzyl)oxy]-3-sulfamoylphenylacetamide;
   (C143) 2-(2-chlorophenyl)-N-4-[(4-fluorobenzyl)oxy]-3-sulfamoylphenylacetamide;
   (C144) N-4-[(2-chlorobenzyl)oxy]-3-sulfamoylphenyl-2-(2-chlorophenyl) acetamide;
   (C145) 2-(2-chlorophenyl)-N-4-[(2-cyanobenzyl)oxy]-3-sulfamoylphenylacetamide;
   (C146) N-[4-(benzyloxy)-3-sulfamoylphenyl]-2-phenylacetamide;
   (C147) 2-(2-chlorophenyl)-N-(4-[4-(methylsulfonyl)benzyl]oxy-3-sulfamoylphenyl)acetamide;
   (C148) 2-(2-chlorophenyl)-N-[4-(1-phenylethoxy)-3-sulfamoylphenyl]acetamide;
   (C149) 2-(2-chlorophenyl)-N-[4-(1-phenylethoxy)-3-sulfamoylphenyl]acetamide;
   (C150) 2-(2-chlorophenyl)-N-[4-(pyridin-3-ylmethoxy)-3-sulfamoylphenyl]acetamide;
   (C151) 2-(2-chlorophenyl)-N-[4-(pyridin-2-ylmethoxy)-3-sulfamoylphenyl]acetamide;
   (C152) 2-(2-chlorophenyl)-N-[4-(pyridin-4-ylmethoxy)-3-sulfamoylphenyl]acetamide;
   (C153) N-[4-(pyridin-2-ylmethoxy)-3-sulfamoylphenyl]-2-[4-(trifluoromethyl)phenyl]acetamide;
   (C154) 2-(2-chlorophenyl)-N-[4-(pyrimidin-4-ylmethoxy)-3-sulfamoylphenyl]acetamide;
   (C155) 2-(2-chlorophenyl)-N-[4-(pyrimidin-2-ylmethoxy)-3-sulfamoylphenyl]acetamide;
   (C156) 2-(2-chlorophenyl)-N-[4-(2-phenylethoxy)-3-sulfamoylphenyl]acetamide;
   (C157) 2-(2-chlorophenyl)-N-4-[2-(3-chlorophenyl)ethoxy]-3-sulfamoylphenylacetamide;
   (C158) 2-(2-chlorophenyl)-N-[4-(cyclobutylmethoxy)-3-sulfamoylphenyl]acetamide;
   (C159) 2-(2-chlorophenyl)-N-[4-(oxetan-2-ylmethoxy)-3-sulfamoylphenyl]acetamide;
   (C160) 2-(2-chlorophenyl)-N-[4-(oxetan-3-ylmethoxy)-3-sulfamoylphenyl]acetamide;
   (C161) 2-(2-chlorophenyl)-N-[4-(cyclopentylmethoxy)-3-sulfamoylphenyl]acetamide;
   (C162) 2-(2-chlorophenyl)-N-[3-sulfamoyl-4-(tetrahydrofuran-2-ylmethoxy)phenyl]acetamide;
   (C163) 2-(2-chlorophenyl)-N-[3-sulfamoyl-4-(tetrahydrofuran-3-ylmethoxy)phenyl]acetamide;
   (C164) 2-(2-chloro-5-fluorophenyl)-N-[3-sulfamoyl-4-(tetrahydro-2H-pyran-4-ylmethoxy)phenyl]acetamide;
   (C165) 2-(2-chlorophenyl)-N-[3-sulfamoyl-4-(tetrahydro-2H-pyran-4-ylmethoxy)phenyl]acetamide;
   (C166) 2-(2-chlorophenyl)-N-[3-sulfamoyl-4-(tetrahydro-2H-pyran-3-ylmethoxy)phenyl]acetamide;
   (C167) 2-(2-chloro-6-fluorophenyl)-N-[3-sulfamoyl-4-(tetrahydro-2H-pyran-4-ylmethoxy)phenyl]acetamide;
   (C168) 2-(2-chloro-3-fluorophenyl)-N-[3-sulfamoyl-4-(tetrahydro-2H-pyran-4-ylmethoxy)phenyl]acetamide;
   (C169) 2-(2-Chlorophenyl)-N-5-sulfamoyl-6-[3-(trifluoromethyl)phenoxy]pyridin-3-ylacetamide;
   (C170) 2-phenyl-N-5-sulfamoyl-6-[3-(trifluoromethyl)phenoxy]pyridin-3-ylacetamide;
   (C171) N-[4-(3-chlorophenoxy)-3-fluoro-5-sulfamoylphenyl]-2-phenylacetamide;
   (C172) N-[4-(3-chlorophenoxy)-3-fluoro-5-sulfamoylphenyl]-2-(2-methylphenyl)acetamide;
   (C173) N-[4-(3-chlorophenoxy)-3-fluoro-5-sulfamoylphenyl]-2-(3-methylphenyl)acetamide;
   (C174) 2-(2-chlorophenyl)-N-4-[3-(3-oxomorpholin-4-yl)phenoxy]-3-sulfamoylphenylacetamide;
   (C175) 2-(2-chlorophenyl)-N-4-[4-(3-oxomorpholin-4-yl)phenoxy]-3-sulfamoylphenylacetamide;
   (C176) 2-(2-chlorophenyl)-N-4-[4-(2-oxopiperidin-1-yl)phenoxy]-3-sulfamoylphenylacetamide;
   (C177) 2-(2-chlorophenyl)-N-4-[3-(2-oxopiperidin-1-yl)phenoxy]-3-sulfamoylphenylacetamide;
   (C178) 2-(2-chlorophenyl)-N-4-[3-(prop-1-en-2-yl)phenoxy]-3-sulfamoylphenylacetamide;
   (C179) 2-(2-chlorophenyl)-N-4-[2-(prop-1-en-2-yl)phenoxy]-3-sulfamoylphenylacetamide;
   (C180) N-[4-(3-chlorophenoxy)-3-sulfamoylphenyl]-2-(4-methylphenyl) acetamide;
   (C181) N-[4-(3-chlorophenoxy)-3-sulfamoylphenyl]-2-(4-chlorophenyl) acetamide;
   (C182) N-[4-(3-chlorophenoxy)-3-sulfamoylphenyl]-2-(pyridin-3-yl)acetamide;
   (C183) N-[4-(3-chlorophenoxy)-3-sulfamoylphenyl]-2-(2-methylphenyl) acetamide;
   (C184) N-[4-(3-chlorophenoxy)-3-sulfamoylphenyl]-2-(3-methylphenyl) acetamide;
   (C185) N-[4-(3-chlorophenoxy)-3-sulfamoylphenyl]-2-phenylpropanamide;
   (C186) N-[4-(3-chlorophenoxy)-3-sulfamoylphenyl]-2-(pyridin-2-yl)acetamide;
   (C187) N-[4-(3-chlorophenoxy)-3-sulfamoylphenyl]-2-(3-chlorophenyl) acetamide;
   (C188) N-[4-(3-chlorophenoxy)-3-sulfamoylphenyl]-2-(2-chlorophenyl) acetamide;
   (C189) N-[4-(3-chlorophenoxy)-3-sulfamoylphenyl]-2-(pyridin-4-yl)acetamide;
   (C190) N-[4-(3-chlorophenoxy)-3-sulfamoylphenyl]-2-(6-methylpyridin-2-yl)acetamide;
   (C191) N-[4-(3-chlorophenoxy)-3-sulfamoylphenyl]-2-(4-methoxyphenyl) acetamide;
   (C192) N-[4-(3-chlorophenoxy)-3-sulfamoylphenyl]-2-(3-methoxyphenyl) acetamide;
   (C193) N-[4-(3-chlorophenoxy)-3-sulfamoylphenyl]-2-(2-methoxyphenyl) acetamide;
   (C194) N-[4-(3-chlorophenoxy)-3-sulfamoylphenyl]-2-(5-methylpyridin-2-yl)acetamide;
   (C195) (2S)-N-[4-(3-chlorophenoxy)-3-sulfamoylphenyl]-2-phenylpropanamide;
   (C196) (2R)-N-[4-(3-chlorophenoxy)-3-sulfamoylphenyl]-2-phenylpropanamide;
   (C197) N-[4-(3-chlorophenoxy)-3-sulfamoylphenyl]-2-(2-chlorophenyl)propanamide;
   (C198) 2-(2-[4-(3-chlorophenoxy)-3-sulfamoylphenyl]amino-2-oxoethyl)-N-(2-methoxyethyl)-N-methylbenzamide;
   (C199) 2-(2-[4-(3-chlorophenoxy)-3-sulfamoylphenyl]amino-2-oxoethyl)-N,N-dimethylbenzamide;
   (C200) N-[4-(cyclohexyloxy)-3-sulfamoylphenyl]-2-phenylacetamide;
   (C201) 2-(2-chlorophenyl)-N-[4-(cyclohexyloxy)-3-sulfamoylphenyl]acetamide;
   (C202) 3-(2-[4-(3-chlorophenoxy)-3-sulfamoylphenyl]amino-2-oxoethyl)-N-(2-methoxyethyl)benzamide;
   (C203) 3-(2-[4-(3-chlorophenoxy)-3-sulfamoylphenyl]amino-2-oxoethyl)-N,N-dimethylbenzamide;
   (C204) 3-(2-[4-(3-chlorophenoxy)-3-sulfamoylphenyl]amino-2-oxoethyl)-N-methylbenzamide;
   (C205) N-[4-(cyclobutyloxy)-3-sulfamoylphenyl]-2-phenylacetamide;
   (C206) 2-(2-chlorophenyl)-N-[4-(cyclobutyloxy)-3-sulfamoylphenyl]acetamide;
   (C207) 2-phenyl-N-[3-sulfamoyl-4-(tetrahydro-2H-pyran-4-yloxy)phenyl]acetamide;
   (C208) 2-(2-chlorophenyl)-N-[3-sulfamoyl-4-(tetrahydro-2H-pyran-4-yloxy)phenyl]acetamide;
   (C209) 3-(2-[4-(3-chlorophenoxy)-3-sulfamoylphenyl]amino-2-oxoethyl)-N-(2-methoxyethyl)-N-methylbenzamide;
   (C210) N-[4-(3-chlorophenoxy)-3-sulfamoylphenyl]-2-(5-chloropyridin-2-yl)acetamide;
   (C211) N-[4-(3-chlorophenoxy)-3-sulfamoylphenyl]-2-[3-(2-methoxyethoxy)phenyl]acetamide;
   (C212) N-[4-(3-chlorophenoxy)-3-sulfamoylphenyl]-2-[2-(2-methoxyethoxy)phenyl]acetamide;
   (C213) N-[4-(3-chlorophenoxy)-3-sulfamoylphenyl]-2-[3-(2-hydroxyethoxy)phenyl]acetamide;
   (C214) N-[4-(3-chlorophenoxy)-3-sulfamoylphenyl]-2-[2-(2-hydroxyethoxy)phenyl]acetamide;
   (C215) N-[4-(3-chlorophenoxy)-3-sulfamoylphenyl]-2-(2-fluorophenyl) acetamide;
   (C216) N-[4-(oxetan-3-yloxy)-3-sulfamoylphenyl]-2-phenylacetamide;
   (C217) 2-(2-chlorophenyl)-N-[4-(oxetan-3-yloxy)-3-sulfamoylphenyl]acetamide;
   (C218) N-[4-(cyclopentyloxy)-3-sulfamoylphenyl]-2-phenylacetamide;
   (C219) 2-(2-chlorophenyl)-N-[4-(cyclopentyloxy)-3-sulfamoylphenyl]acetamide;
   (C220) N-4-[(1-methylpiperidin-3-yl)oxy]-3-sulfamoylphenyl-2-phenylacetamide;
   (C221) 2-(2-chlorophenyl)-N-4-[(1-methylpiperidin-3-yl)oxy]-3-sulfamoylphenylacetamide;
   (C222) N-4-[(1-methylpyrrolidin-3-yl)oxy]-3-sulfamoylphenyl-2-phenylacetamide;
   (C223) 2-(2-chlorophenyl)-N-4-[(1-methylpyrrolidin-3-yl)oxy]-3-sulfamoylphenylacetamide;
   (C224) N-[4-(4-chlorophenoxy)-3-sulfamoylphenyl]-2-(4-fluorophenyl) acetamide;
   (C225) N-[4-(3-chlorophenoxy)-3-sulfamoylphenyl]-2-(4-cyanophenyl)acetamide;
   (C226) N-[4-(3-chlorophenoxy)-3-sulfamoylphenyl]-2-(2-cyanophenyl)acetamide;
   (C227) N-[4-(3-chlorophenoxy)-3-sulfamoylphenyl]-2-(3-cyanophenyl)acetamide;
   (C228) N-[4-(4-chlorophenoxy)-3-sulfamoylphenyl]-2-[4-(trifluoromethyl)phenyl]acetamide;
   (C229) N-[4-(4-chlorophenoxy)-3-sulfamoylphenyl]-2-(4-chlorophenyl) acetamide;
   (C230) N-[4-(4-chlorophenoxy)-3-sulfamoylphenyl]-2-(4-methoxyphenyl) acetamide;
   (C231) N-[4-(4-chlorophenoxy)-3-sulfamoylphenyl]-2-(2-fluorophenyl) acetamide;
   (C232) 2-(2-chloro-4-fluorophenyl)-N-[4-(4-chlorophenoxy)-3-sulfamoylphenyl]acetamide;
   (C233) 2-(2-chlorophenyl)-N-4-[(1,1-dioxidetetrahydrothiophen-3-yl)oxy]-3-sulfamoylphenylacetamide;
   (C234) 2-(2-chlorophenyl)-N-4-[(1-methyl-1H-pyrazol-4-yl)oxy]-3-sulfamoylphenylacetamide;
   (C235) N-[4-(4-chlorophenoxy)-3-sulfamoylphenyl]-2-[4-(difluoromethyl)phenyl]acetamide;
   (C236) 2-(2-chloro-4-methoxyphenyl)-N-[4-(4-chlorophenoxy)-3-sulfamoylphenyl]acetamide;
   (C237) 2-(2-chlorophenyl)-N-4-[(1-methyl-1H-pyrazol-3-yl)oxy]-3-sulfamoylphenylacetamide;
   (C238) 2-(2-chlorophenyl)-N-4-[(1-methyl-1H-pyrazol-5-yl)oxy]-3-sulfamoylphenylacetamide;
   (C239) 2-(2-chlorophenyl)-N-4-[(1-methylpiperidin-4-yl)oxy]-3-sulfamoylphenylacetamide;
   (C240) 2-(2-chlorophenyl)-N-(4-[5-methyl-2-(pyridin-3-yl)-1,3-thiazol-4-yl]oxy-3-sulfamoylphenyl)acetamide;
   (C241) N-[4-(3-chlorophenoxy)-2-methyl-5-sulfamoylphenyl]-2-(2-chlorophenyl)acetamide;
   (C242) 2-(2-Chlorophenyl)-N-{4-[(1-oxidetetrahydrothiophen-3-yl)oxy]-3-sulfamoylphenyl}acetamide;
   (C243) N-[4-(4-chlorophenoxy)-3-sulfamoylphenyl]-2-[2,6-dichloro-4-(trifluoromethyl)phenyl]acetamide;
   (C244) N-[4-(4-chlorophenoxy)-3-sulfamoylphenyl]-2-(2,5-dichloro-4-cyanophenyl)acetamide;
   (C245) N-[4-(3-chlorophenoxy)-3-sulfamoylphenyl]-2-phenylacetamide;
   (C246) N-[4-(cyclopropylmethoxy)-3-sulfamoylphenyl]-2-phenylacetamide;
   (C247) N-[4-(3,5-dimethylphenoxy)-3-sulfamoylphenyl]-2-phenylacetamide;
   (C248) N-[4-(2,4-difluorophenoxy)-3-sulfamoylphenyl]-2-phenylacetamide;
   (C249) N-[4-(4-fluorophenoxy)-3-sulfamoylphenyl]-2-phenylacetamide;
   (C250) N-[4-(3-fluorophenoxy)-3-sulfamoylphenyl]-2-phenylacetamide;
   (C251) N-[4-(3-methoxyphenoxy)-3-sulfamoylphenyl]-2-phenylacetamide;
   (C252) N-[4-(2-fluoro-5-methylphenoxy)-3-sulfamoylphenyl]-2-phenylacetamide;
   (C253) 2-phenyl-N-3-sulfamoyl-4-[4-(trifluoromethoxy)phenoxy]phenylacetamide;
   (C254) 2-phenyl-N-3-sulfamoyl-4-[3-(trifluoromethyl)phenoxy]phenylacetamide;
   (C255) N-[4-(3,5-dimethoxyphenoxy)-3-sulfamoylphenyl]-2-phenylacetamide;
   (C256) N-[4-(3-cyanophenoxy)-3-sulfamoylphenyl]-2-phenylacetamide;
   (C257) N-[4-(3-chlorophenoxy)-3-sulfamoylphenyl]-2-(4-hydroxyphenyl) acetamide;
   (C258) 2-(2-chloro-6-methoxy-4-methylphenyl)-N-[4-(3-chlorophenoxy)-3-sulfamoylphenyl]acetamide;
   (C259) 2-(2-chloro-6-fluorophenyl)-N-[4-(3-chlorophenoxy)-3-sulfamoylphenyl]propanamide;
   (C260) 2-(2-chloro-4,6-difluorophenyl)-N-[4-(3-chlorophenoxy)-3-sulfamoylphenyl]acetamide;
   (C261) N-[4-(3-chlorophenoxy)-3-sulfamoylphenyl]-2-(2,6-dichlorophenyl)propanamide;
   (C262) 2-(2-chlorophenyl)-N-4-[(2H5)phenyloxy]-3-sulfamoylphenylacetamide;
   (C263) 2-(2-chlorophenyl)-N-(4-{[4-chloro(2H4)phenyl]oxy}-3-sulfamoylphenyl)acetamide;
   (C264) 2-(2-chlorophenyl)-N-(4-{[2-chloro(2H4)phenyl]oxy}-3-sulfamoylphenyl)acetamide;
   (C265) 2-(2-chlorophenyl)-N-4-[4-(2-hydroxypropan-2-yl)phenoxy]-3-sulfamoylphenylacetamide;
   (C266) 2-(2-chlorophenyl)-N-4-[(2,2-dimethyltetrahydro-2H-pyran-4-yl)methoxy]-3-sulfamoylphenylacetamide;
   (C267) 2-(2-chlorophenyl)-N-{4-[(1R,5S,6r)-3-oxabicyclo[3.1.0]hex-6-ylmethoxy]-3-sulfamoylphenyl}acetamide;
   (C268) 2-(2-chlorophenyl)-N-4-[(4-chlorotetrahydro-2H-pyran-4-yl)methoxy]-3-sulfamoylphenylacetamide;
   (C269) 2-(2-chlorophenyl)-N-[4-(1,4-dioxane-2-ylmethoxy)-3-sulfamoylphenyl]acetamide;
   (C270) 2-(2-chlorophenyl)-N-3-sulfamoyl-4-[(2,2,6,6-tetramethyltetrahydro-2H-pyran-4-yl)oxy]phenylacetamide;
   (C271) N-[4-(3-chlorophenoxy)-3-methyl-5-sulfamoylphenyl]-2-(2-chlorophenyl)acetamide;
   (C272) N-[4-(3-chlorophenoxy)-3-methyl-5-sulfamoylphenyl]-2-phenylacetamide;
   (C273) methyl 2-(4-[(2-chlorophenyl)acetyl]amino-2-sulfamoylphenoxy)benzoate;
   (C274) methyl 4-(4-[(2-chlorophenyl)acetyl]amino-2-sulfamoylphenoxy)benzoate;
   (C275) 2-(2-chlorophenyl)-N-4-[3-(2-hydroxypropan-2-yl)phenoxy]-3-sulfamoylphenylacetamide;
   (C276) 2-(2-chlorophenyl)-N-4-[2-(2-hydroxypropan-2-yl)phenoxy]-3-sulfamoylphenylacetamide;
   (C277) N-[4-(4-chlorophenoxy)-3-sulfamoylphenyl]-2-(2,3-dihydro-1,4-benzodioxin-6-yl)acetamide;
   (C278) 2-(7-chloro-2,3-dihydro-1,4-benzodioxin-6-yl)-N-[4-(3-chlorophenoxy)-3-sulfamoylphenyl]acetamide;
   (C279) 2-(5-chloro-2,3-dihydro-1-benzofuran-4-yl)-N-[4-(3-chlorophenoxy)-3-sulfamoylphenyl]acetamide;
   (C280) 2-(2-fluorophenyl)-N-[3-sulfamoyl-4-(tetrahydro-2H-pyran-4-ylmethoxy)phenyl]acetamide;
   (C281) N-[3-sulfamoyl-4-(tetrahydro-2H-pyran-4-ylmethoxy)phenyl]-2-[2-(trifluoromethyl)phenyl]acetamide;
   (C282) 2-[2-(difluoromethyl)phenyl]-N-[3-sulfamoyl-4-(tetrahydro-2H-pyran-4-ylmethoxy)phenyl]acetamide;
   (C283) 2-(2-chloro-4-fluorophenyl)-N-[3-sulfamoyl-4-(tetrahydro-2H-pyran-4-ylmethoxy)phenyl]acetamide;
   (C284) 2-(2-chlorophenyl)-N-(3-sulfamoyl-4-{[6-(trifluoromethyl)pyridin-3-yl]oxy}phenyl)acetamide;
   (C285) 2-(2-chlorophenyl)-N-(4-{[5-chloro-4-(trifluoromethyl)pyridin-2-yl]oxy}-3-sulfamoyl-phenyl)acetamide;
   (C286) N-[4-(3-chlorophenoxy)-3-sulfamoylphenyl]-2-phenyl(2H2)acetamide;
   (C287) N-{4-[(6-chloro-5-fluoropyridin-3-yl)oxy]-3-sulfamoylphenyl}-2-(2-chlorophenyl)acetamide;
   (C288) 2-(2-chlorophenyl)-N-{4-[(4,4-difluoro-1-hydroxycyclohexyl)methoxy]-3-sulfamoylphenyl}acetamide;
   (C289) 2-(2-chlorophenyl)-N-{4-[(1-hydroxycyclohexyl)methoxy]-3-sulfamoylphenyl}acetamide;
   (C290) N-[4-(3-chlorophenoxy)-3-fluoro-5-sulfamoylphenyl]-2-(2-fluorophenyl)acetamide;
   (C291) N-[4-(3-chlorophenoxy)-3-fluoro-5-sulfamoylphenyl]-2-[2-(difluoromethyl)phenyl]acetamide;
   (C292) N-[4-(3-chlorophenoxy)-3-fluoro-5-sulfamoylphenyl]-2-(2-chlorophenyl)acetamide;
   (C293) 2-(2-chloro-5-fluorophenyl)-N-[4-(3-chlorophenoxy)-3-fluoro-5-sulfamoylphenyl]acetamide;
   (C294) N-[6-(3-chlorophenoxy)-5-sulfamoylpyridin-3-yl]-2-(2-fluorophenyl)acetamide;
   (C295) N-[6-(3-chlorophenoxy)-5-sulfamoylpyridin-3-yl]-2-[2-(trifluoromethyl)phenyl]acetamide;
   (C296) N-[6-(3-chlorophenoxy)-5-sulfamoylpyridin-3-yl]-2-[2-(difluoromethyl)phenyl]acetamide; and
   (C297) 2-(2-chloro-5-fluorophenyl)-N-[6-(3-chlorophenoxy)-5-sulfamoylpyridin-3-yl]acetamide.
[9] The pharmaceutical composition according to any one of [1] to [4], in which the compound is a compound selected from the group consisting of the following (D1) to (D558):
   (D1) 2-(2-chlorophenyl)-N-[4-(2-oxopyridin-1(2H)-yl)-3-sulfamoylphenyl]acetamide;
   (D2) N-[4-(4-chloro-2-oxopyridin-1(2H)-yl)-3-sulfamoylphenyl]-2-(2-chlorophenyl)acetamide;
   (D3) 2-(2-chlorophenyl)-N-[4-(3,5-dichloro-2-oxopyridin-1(2H)-yl)-3-sulfamoylphenyl]acetamide;
   (D4) N-[4-(3-chloro-2-oxopyridin-1(2H)-yl)-3-sulfamoylphenyl]-2-(2-chlorophenyl)acetamide;
   (D5) 2-(2-chlorophenyl)-N-[4-(3-methyl-1H-1,2,4-triazol-1-yl)-3-sulfamoylphenyl]acetamide;
   (D6) 2-(2-chlorophenyl)-N-[4-(5-methyl-1H-1,2,4-triazol-1-yl)-3-sulfamoylphenyl]acetamide;
   (D7) 2-(2-chlorophenyl)-N-{3-sulfamoyl-4-[3-(trifluoromethyl)-1H-1,2,4-triazol-1-yl]phenyl}acetamide;
   (D8) 2-(2-chlorophenyl)-N-{4-[5-methyl-3-(trifluoromethyl)-1H-1,2,4-triazol-1-yl]-3-sulfamoylphenyl}acetamide;
   (D9) 2-(2-chlorophenyl)-N-[4-(1H-imidazol-1-yl)-3-sulfamoylphenyl]acetamide;
   (D10) 2-(2-chlorophenyl)-N-{3-sulfamoyl-4-[4-(trifluoromethyl)-1H-imidazol-1-yl]phenyl}acetamide;
   (D11) 2-(2-chlorophenyl)-N-{3-sulfamoyl-4-[3-(trifluoromethyl)-1H-pyrazol-1-yl]phenyl}acetamide;
   (D12) 2-(2-chlorophenyl)-N-{4-[3-(difluoromethyl)-1H-pyrazol-1-yl]-3-sulfamoylphenyl}acetamide;
   (D13) 2-(2-chlorophenyl)-N-{4-[5-cyclopropyl-3-(difluoromethyl)-1H-pyrazol-1-yl]-3-sulfamoylphenyl}acetamide;
   (D14) 2-(2-chlorophenyl)-N-{4-[4-methyl-3-(trifluoromethyl)-1H-pyrazol-1-yl]-3-sulfamoylphenyl}acetamide;
   (D15) 2-(2-chlorophenyl)-N-{4-[5-methyl-3-(trifluoromethyl)-1H-pyrazol-1-yl]-3-sulfamoylphenyl}acetamide;
   (D16) 2-(2-chlorophenyl)-N-[3-sulfamoyl-4-(1H-1,2,4-triazol-1-yl)phenyl]acetamide;
   (D17) 2-(2-chlorophenyl)-N-{4-[3-(difluoromethyl)-1H-1,2,4-triazol-1-yl]-3-sulfamoylphenyl}acetamide;
   (D18) N-{4-[3-(difluoromethyl)-1H-1,2,4-triazol-1-yl]-3-sulfamoylphenyl}-2-(2-fluorophenyl)acetamide;
   (D19) 2-(2-chlorophenyl)-N-{3-sulfamoyl-4-[4-(trifluoromethyl)-1H-pyrazol-1-yl]phenyl}acetamide;
   (D20) 2-(2-fluorophenyl)-N-{3-sulfamoyl-4-[4-(trifluoromethyl)-1H-pyrazol-1-yl]phenyl}acetamide;
   (D21) 2-[2-(difluoromethyl)phenyl]-N-{3-sulfamoyl-4-[4-(trifluoromethyl)-1H-pyrazol-1-yl]phenyl}acetamide;
   (D22) N-{3-sulfamoyl-4-[4-(trifluoromethyl)-1H-pyrazol-1-yl]phenyl}-2-[2-(trifluoromethyl)phenyl]acetamide;
   (D23) N-[4-(3-tert-butyl-1H-1,2,4-triazol-1-yl)-3-sulfamoylphenyl]-2-(2-chlorophenyl)acetamide;
   (D24) 2-(2-chlorophenyl)-N-[4-(3-chloro-1H-1,2,4-triazol-1-yl)-3-sulfamoylphenyl]acetamide;
   (D25) 2-(2-chlorophenyl)-N-[4-(4-chloro-1H-pyrazol-1-yl)-3-sulfamoylphenyl]acetamide;
   (D26) 2-(2-chlorophenyl)-N-[4-(4-fluoro-1H-pyrazol-1-yl)-3-sulfamoylphenyl]acetamide;
   (D27) 2-(2-chlorophenyl)-N-[4-(4-isopropoxy-1H-pyrazol-1-yl)-3-sulfamoylphenyl]acetamide;
   (D28) N-[4-(4-bromo-1H-pyrazol-1-yl)-3-sulfamoylphenyl]-2-(2-chlorophenyl)acetamide;
   (D29) 2-(2-chlorophenyl)-N-[4-(3-isobutyl-1H-1,2,4-triazol-1-yl)-3-sulfamoylphenyl]acetamide;
   (D30) 2-(2-chlorophenyl)-N-{4-[4-(methylsulfanyl)-1H-pyrazol-1-yl]-3-sulfamoylphenyl}acetamide;
   (D31) 2-(2-chlorophenyl)-N-[4-(4-methoxy-1H-pyrazol-1-yl)-3-sulfamoylphenyl]acetamide;
   (D32) N-[4-(1H-benzoimidazol-1-yl)-3-sulfamoylphenyl]-2-(2-chlorophenyl)acetamide;
   (D33) N-[4-(4-chloro-1H-imidazol-1-yl)-3-sulfamoylphenyl]-2-(2-chlorophenyl)acetamide;
   (D34) 2-(2-chlorophenyl)-N-{4-[3-(dimethylamino)-1H-1,2,4-triazol-1-yl]-3-sulfamoylphenyl}acetamide;
   (D35) 2-(2-chlorophenyl)-N-[4-(3-ethyl-1H-1,2,4-triazol-1-yl)-3-sulfamoylphenyl]acetamide;
   (D36) (2-chlorophenyl)-N-[4-(3-cyclopropyl-1H-1,2,4-triazol-1-yl)-3-sulfamoylphenyl]acetamide;
   (D37) 2-(2-chlorophenyl)-N-[4-(5-cyclopropyl-1H-1,2,4-triazol-1-yl)-3-sulfamoylphenyl]acetamide;
   (D38) 2-(2-chlorophenyl)-N-{4-[3-(methoxymethyl)-1H-1,2,4-triazol-1-yl]-3-sulfamoylphenyl}acetamide;
   (D39) 2-(2-chlorophenyl)-N-[4-(4-cyano-1H-pyrazol-1-yl)-3-sulfamoylphenyl]acetamide;
   (D40) N-[4-(4-tert-butyl-1H-imidazol-1-yl)-3-sulfamoylphenyl]-2-(2-chlorophenyl)acetamide;
   (D41) 2-(2-chlorophenyl)-N-[4-(3-cyano-1H-1,2,4-triazol-1-yl)-3-sulfamoylphenyl]acetamide;
   (D42) N-[4-(4-bromo-1H-imidazol-1-yl)-3-sulfamoylphenyl]-2-(2-chlorophenyl)acetamide;
   (D43) 2-(2-chlorophenyl)-N-[4-(3H-imidazo[4,5-b]pyridin-3-yl)-3-sulfamoylphenyl]acetamide;
   (D44) 2-(2-chlorophenyl)-N-[4-(1H-imidazo[4,5-b]pyridin-1-yl)-3-sulfamoylphenyl]acetamide;
   (D45) 2-(2-chlorophenyl)-N-[4-(1H-imidazo[4,5-c]pyridin-1-yl)-3-sulfamoylphenyl]acetamide;
   (D46) 2-(2-chlorophenyl)-N-[4-(3H-imidazo[4,5-c]pyridin-3-yl)-3-sulfamoylphenyl]acetamide;
   (D47) 2-(2-chlorophenyl)-N-[4-(2,4-dimethyl-1H-imidazol-1-yl)-3-sulfamoylphenyl]acetamide;
   (D48) 2-(2-fluorophenyl)-N-[4-(4-fluoro-1H-pyrazol-1-yl)-3-sulfamoylphenyl]acetamide;
   (D49) N-[4-(4-chloro-1H-pyrazol-1-yl)-3-sulfamoylphenyl]-2-[2-(difluoromethyl)phenyl]acetamide;
   (D50) N-[4-(4-chloro-1H-pyrazol-1-yl)-3-sulfamoylphenyl]-2-(2-fluorophenyl)acetamide;
   (D51) 2-[2-(difluoromethyl)phenyl]-N-[4-(4-fluoro-1H-pyrazol-1-yl)-3-sulfamoylphenyl]acetamide;
   (D52) N-[4-(4-cyano-1H-pyrazol-1-yl)-3-sulfamoylphenyl]-2-(2-fluorophenyl)acetamide;
   (D53) 2-(2-methoxyphenyl)-N-{3-sulfamoyl-4-[3-(trifluoromethyl)-1H-1,2,4-triazol-1-yl]phenyl}acetamide;
   (D54) 2-(2-fluorophenyl)-N-{3-sulfamoyl-4-[3-(trifluoromethyl)-1H-1,2,4-triazol-1-yl]phenyl}acetamide;
   (D55) 2-(3-fluorophenyl)-N-{3-sulfamoyl-4-[3-(trifluoromethyl)-1H-1,2,4-triazol-1-yl]phenyl}acetamide;
   (D56) 2-(2-chloro-4-fluorophenyl)-N-{3-sulfamoyl-4-[3-(trifluoromethyl)-1H-1,2,4-triazol-1-yl]phenyl}acetamide;
   (D57) 2-[2-(difluoromethoxy)phenyl]-N-{3-sulfamoyl-4-[3-(trifluoromethyl)-1H-1,2,4-triazol-1-yl]phenyl}acetamide;
   (D58) 2-(2-chloro-5-fluorophenyl)-N-{3-sulfamoyl-4-[3-(trifluoromethyl)-1H-1,2,4-triazol-1-yl]phenyl}acetamide;
   (D59) 2-(3-chloropyridin-4-yl)-N-{3-sulfamoyl-4-[3-(trifluoromethyl)-1H-1,2,4-triazol-1-yl]phenyl}acetamide;
   (D60) 2-[4-(difluoromethyl)phenyl]-N-{3-sulfamoyl-4-[3-(trifluoromethyl)-1H-1,2,4-triazol-1-yl]phenyl}acetamide;
   (D61) 2-(2-chlorophenyl)-2,2-difluoro-N-{3-sulfamoyl-4-[3-(trifluoromethyl)-1H-1,2,4-triazol-1-yl]phenyl}acetamide;
   (D62) N-[4-(4-bromo-1H-pyrazol-1-yl)-3-sulfamoylphenyl]-2-(2-chlorophenyl)-2,2-difluoroacetamide;
   (D63) N-[4-(4-bromo-1H-pyrazol-1-yl)-3-sulfamoylphenyl]-2-(2-fluorophenyl)acetamide;
   (D64) N-[4-(4-bromo-1H-pyrazol-1-yl)-3-sulfamoylphenyl]-2-[2-(trifluoromethyl)phenyl]acetamide;
   (D65) N-[4-(4-bromo-1H-pyrazol-1-yl)-3-sulfamoylphenyl]-2-(3-fluorophenyl)acetamide;
   (D66) N-[4-(4-bromo-1H-pyrazol-1-yl)-3-sulfamoylphenyl]-2-(2-methylphenyl)acetamide;
   (D67) N-[4-(4-bromo-1H-pyrazol-1-yl)-3-sulfamoylphenyl]-2-(2-chloropyridin-3-yl)acetamide;
   (D68) N-[4-(4-bromo-1H-pyrazol-1-yl)-3-sulfamoylphenyl]-2-(2-chloro-4-fluorophenyl)acetamide;
   (D69) N-[4-(4-bromo-1H-pyrazol-1-yl)-3-sulfamoylphenyl]-2-[2-(difluoromethoxy)phenyl]acetamide;
   (D70) N-[4-(4-bromo-1H-pyrazol-1-yl)-3-sulfamoylphenyl]-2-[2-(trifluoromethoxy)phenyl]acetamide;
   (D71) N-[4-(4-bromo-1H-pyrazol-1-yl)-3-sulfamoylphenyl]-2-(2-chloro-4,5-difluorophenyl)acetamide;
   (D72) N-[4-(4-bromo-1H-pyrazol-1-yl)-3-sulfamoylphenyl]-2-(3-chloropyridin-4-yl)acetamide;
   (D73) N-[4-(4-bromo-1H-pyrazol-1-yl)-3-sulfamoylphenyl]-2-[4-(difluoromethyl)phenyl]acetamide;
   (D74) 2-(2-bromophenyl)-N-[4-(4-chloro-1H-pyrazol-1-yl)-3-sulfamoylphenyl]acetamide;
   (D75) N-[4-(4-chloro-1H-pyrazol-1-yl)-3-sulfamoylphenyl]-2-(2-methoxyphenyl)acetamide;
   (D76) 2-(4-chlorophenyl)-N-[4-(4-chloro-1H-pyrazol-1-yl)-3-sulfamoylphenyl]acetamide;
   (D77) 2-(2-chloro-6-fluorophenyl)-N-[4-(4-chloro-1H-pyrazol-1-yl)-3-sulfamoylphenyl]acetamide;
   (D78) N-[4-(4-chloro-1H-pyrazol-1-yl)-3-sulfamoylphenyl]-2-(2-nitrophenyl)acetamide;
   (D79) N-[4-(4-chloro-1H-pyrazol-1-yl)-3-sulfamoylphenyl]-2-(2,4-dichlorophenyl)acetamide;
   (D80) N-[4-(4-chloro-1H-pyrazol-1-yl)-3-sulfamoylphenyl]-2-(2,6-dichlorophenyl)acetamide;
   (D81) N-[4-(4-chloro-1H-pyrazol-1-yl)-3-sulfamoylphenyl]-2-(3,4-difluorophenyl)acetamide;
   (D82) 2-(3-chlorophenyl)-N-[4-(4-chloro-1H-pyrazol-1-yl)-3-sulfamoylphenyl]acetamide;
   (D83) N-[4-(4-chloro-1H-pyrazol-1-yl)-3-sulfamoylphenyl]-2-(3,5-difluorophenyl)acetamide;
   (D84) N-[4-(4-chloro-1H-pyrazol-1-yl)-3-sulfamoylphenyl]-2-(4-fluorophenyl)acetamide;
   (D85) N-[4-(4-chloro-1H-pyrazol-1-yl)-3-sulfamoylphenyl]-2-(2-hydroxyphenyl)acetamide;
   (D86) N-[4-(4-chloro-1H-pyrazol-1-yl)-3-sulfamoylphenyl]-2-(4-hydroxyphenyl)acetamide;
   (D87) N-[4-(4-chloro-1H-pyrazol-1-yl)-3-sulfamoylphenyl]-2-(2,3-difluorophenyl)acetamide;
   (D88) 2-(2-chloro-4-fluorophenyl)-N-[4-(4-chloro-1H-pyrazol-1-yl)-3-sulfamoylphenyl]acetamide;
   (D89) N-[4-(4-chloro-1H-pyrazol-1-yl)-3-sulfamoylphenyl]-2-(2-chloropyridin-3-yl)acetamide;
   (D90) N-[4-(4-chloro-1H-pyrazol-1-yl)-3-sulfamoylphenyl]-2-[2-chloro-3-(trifluoromethyl)phenyl]acetamide;
   (D91) N-[4-(4-chloro-1H-pyrazol-1-yl)-3-sulfamoylphenyl]-2-[2-(difluoromethoxy)phenyl]acetamide;
   (D92) N-[4-(4-chloro-1H-pyrazol-1-yl)-3-sulfamoylphenyl]-2-[2-(trifluoromethoxy)phenyl]acetamide;
   (D93) 2-(2-chloro-4,5-difluorophenyl)-N-[4-(4-chloro-1H-pyrazol-1-yl)-3-sulfamoylphenyl]acetamide;
   (D94) 2-(2-chloro-4-methoxyphenyl)-N-[4-(4-chloro-1H-pyrazol-1-yl)-3-sulfamoylphenyl]acetamide;
   (D95) 2-(2-chloro-6-fluoro-3-methylphenyl)-N-[4-(4-chloro-1H-pyrazol-1-yl)-3-sulfamoylphenyl]acetamide;
   (D96) 2-(2-chloro-3,6-difluorophenyl)-N-[4-(4-chloro-1H-pyrazol-1-yl)-3-sulfamoylphenyl]acetamide;
   (D97) 2-(2-chloro-5-methylphenyl)-N-[4-(4-chloro-1H-pyrazol-1-yl)-3-sulfamoylphenyl]acetamide;
   (D98) 2-(2-chloro-5-fluorophenyl)-N-[4-(4-chloro-1H-pyrazol-1-yl)-3-sulfamoylphenyl]acetamide;
   (D99) N-[4-(4-chloro-1H-pyrazol-1-yl)-3-sulfamoylphenyl]-2-(2,5-dichlorophenyl)acetamide;
   (D100) N-[4-(4-chloro-1H-pyrazol-1-yl)-3-sulfamoylphenyl]-2-(2-isopropylphenyl)acetamide;
   (D101) 2-(2-chloro-5-methoxyphenyl)-N-[4-(4-chloro-1H-pyrazol-1-yl)-3-sulfamoylphenyl]acetamide;
   (D102) 2-(2-chloro-4,6-difluorophenyl)-N-[4-(4-chloro-1H-pyrazol-1-yl)-3-sulfamoylphenyl]acetamide;
   (D103) N-[4-(4-chloro-1H-pyrazol-1-yl)-3-sulfamoylphenyl]-2-[2-chloro-6-(trifluoromethyl)phenyl]acetamide;
   (D104) 2-(5-bromo-4-fluoro-2-methylphenyl)-N-[4-(4-chloro-1H-pyrazol-1-yl)-3-sulfamoylphenyl]acetamide;
   (D105) 2-(2-chloro-6-methoxyphenyl)-N-[4-(4-chloro-1H-pyrazol-1-yl)-3-sulfamoylphenyl]acetamide;
   (D106) N-[4-(4-chloro-1H-pyrazol-1-yl)-3-sulfamoylphenyl]-2-(2,6-difluorophenyl)propanamide;
   (D107) N-[4-(4-chloro-1H-pyrazol-1-yl)-3-sulfamoylphenyl]-2-[4-(difluoromethyl)phenyl]acetamide;
   (D108) 2-(4-chlorophenyl)-N-{3-sulfamoyl-4-[4-(trifluoromethyl)-1H-pyrazol-1-yl]phenyl}acetamide;
   (D109) 2-(2-chlorophenyl)-N-[4-(4-chloro-1H-pyrazol-1-yl)-3-sulfamoylphenyl]-2,2-difluoroacetamide;
   (D110) 2-(2-nitrophenyl)-N-{3-sulfamoyl-4-[4-(trifluoromethyl)-1H-pyrazol-1-yl]phenyl}acetamide;
   (Dill) 2-(2,4-dichlorophenyl)-N-{3-sulfamoyl-4-[4-(trifluoromethyl)-1H-pyrazol-1-yl]phenyl}acetamide;
   (D112) 2-(2-methoxyphenyl)-N-{3-sulfamoyl-4-[4-(trifluoromethyl)-1H-pyrazol-1-yl]phenyl}acetamide;
   (D113) 2-(2-chloro-6-fluorophenyl)-N-{3-sulfamoyl-4-[4-(trifluoromethyl)-1H-pyrazol-1-yl]phenyl}acetamide;
   (D114) 2-(3-fluorophenyl)-N-{3-sulfamoyl-4-[4-(trifluoromethyl)-1H-pyrazol-1-yl]phenyl}acetamide;
   (D115) 2-(3,5-difluorophenyl)-N-{3-sulfamoyl-4-[4-(trifluoromethyl)-1H-pyrazol-1-yl]phenyl}acetamide;
   (D116) 2-(2,6-dichlorophenyl)-N-{3-sulfamoyl-4-[4-(trifluoromethyl)-1H-pyrazol-1-yl]phenyl}acetamide;
   (D117) 2-(2-bromophenyl)-N-{3-sulfamoyl-4-[4-(trifluoromethyl)-1H-pyrazol-1-yl]phenyl}acetamide;
   (D118) 2-(3,4-difluorophenyl)-N-{3-sulfamoyl-4-[4-(trifluoromethyl)-1H-pyrazol-1-yl]phenyl}acetamide;
   (D119) 2-(4-fluorophenyl)-N-{3-sulfamoyl-4-[4-(trifluoromethyl)-1H-pyrazol-1-yl]phenyl}acetamide;
   (D120) 2-(3-chlorophenyl)-N-{3-sulfamoyl-4-[4-(trifluoromethyl)-1H-pyrazol-1-yl]phenyl}acetamide;
   (D121) 2-(4-methoxyphenyl)-N-{3-sulfamoyl-4-[4-(trifluoromethyl)-1H-pyrazol-1-yl]phenyl}acetamide;
   (D122) 2-(2,3-difluorophenyl)-N-{3-sulfamoyl-4-[4-(trifluoromethyl)-1H-pyrazol-1-yl]phenyl}acetamide;
   (D123) 2-(2-methylphenyl)-N-{3-sulfamoyl-4-[4-(trifluoromethyl)-1H-pyrazol-1-yl]phenyl}acetamide;
   (D124) 2-(3-methylphenyl)-N-{3-sulfamoyl-4-[4-(trifluoromethyl)-1H-pyrazol-1-yl]phenyl}acetamide;
   (D125) 2-[2-(difluoromethoxy)phenyl]-N-{3-sulfamoyl-4-[4-(trifluoromethyl)-1H-pyrazol-1-yl]phenyl}acetamide;
   (D126) 2-(2-chloropyridin-3-yl)-N-{3-sulfamoyl-4-[4-(trifluoromethyl)-1H-pyrazol-1-yl]phenyl}acetamide;
   (D127) 2-(2-chloro-4-fluorophenyl)-N-{3-sulfamoyl-4-[4-(trifluoromethyl)-1H-pyrazol-1-yl]phenyl}acetamide;
   (D128) 2-(2-chloro-5-methylphenyl)-N-{3-sulfamoyl-4-[4-(trifluoromethyl)-1H-pyrazol-1-yl]phenyl}acetamide;
   (D129) N-{3-sulfamoyl-4-[4-(trifluoromethyl)-1H-pyrazol-1-yl]phenyl}-2-[2-(trifluoromethoxy)phenyl]acetamide;
   (D130) 2-(2-chloro-4,5-difluorophenyl)-N-{3-sulfamoyl-4-[4-(trifluoromethyl)-1H-pyrazol-1-yl]phenyl}acetamide;
   (D131) 2-[2-chloro-3-(trifluoromethyl)phenyl]-N-{3-sulfamoyl-4-[4-(trifluoromethyl)-1H-pyrazol-1-yl]phenyl}acetamide;
   (D132) 2-(2-chloro-6-fluoro-3-methylphenyl)-N-{3-sulfamoyl-4-[4-(trifluoromethyl)-1H-pyrazol-1-yl]phenyl}acetamide;
   (D133) 2-(2-chloro-3,6-difluorophenyl)-N-{3-sulfamoyl-4-[4-(trifluoromethyl)-1H-pyrazol-1-yl]phenyl}acetamide;
   (D134) 2-[2-chloro-6-(trifluoromethyl)phenyl]-N-{3-sulfamoyl-4-[4-(trifluoromethyl)-1H-pyrazol-1-yl]phenyl}acetamide;
   (D135) 2-(2-chloro-4-methoxyphenyl)-N-{3-sulfamoyl-4-[4-(trifluoromethyl)-1H-pyrazol-1-yl]phenyl}acetamide;
   (D136) 2-(2,5-dichlorophenyl)-N-{3-sulfamoyl-4-[4-(trifluoromethyl)-1H-pyrazol-1-yl]phenyl}acetamide;
   (D137) 2-(2-isopropylphenyl)-N-{3-sulfamoyl-4-[4-(trifluoromethyl)-1H-pyrazol-1-yl]phenyl}acetamide;
   (D138) 2-(2-chloro-5-methoxyphenyl)-N-{3-sulfamoyl-4-[4-(trifluoromethyl)-1H-pyrazol-1-yl]phenyl}acetamide;
   (D139) 2-(2-chloro-5-fluorophenyl)-N-{3-sulfamoyl-4-[4-(trifluoromethyl)-1H-pyrazol-1-yl]phenyl}acetamide;
   (D140) N-[4-(4-fluoro-1H-pyrazol-1-yl)-3-sulfamoylphenyl]-2-[2-(trifluoromethyl)phenyl]acetamide;
   (D141) 2-(5-bromo-4-fluoro-2-methylphenyl)-N-{3-sulfamoyl-4-[4-(trifluoromethyl)-1H-pyrazol-1-yl]phenyl}acetamide;
   (D142) 2-(2-chloro-6-methoxyphenyl)-N-{3-sulfamoyl-4-[4-(trifluoromethyl)-1H-pyrazol-1-yl]phenyl}acetamide;
   (D143) 2-(2-chloro-4,6-difluorophenyl)-N-{3-sulfamoyl-4-[4-(trifluoromethyl)-1H-pyrazol-1-yl]phenyl}acetamide;
   (D144) 2-(3-fluorophenyl)-N-[4-(4-fluoro-1H-pyrazol-1-yl)-3-sulfamoylphenyl]acetamide;
   (D145) 2-(3,4-difluorophenyl)-N-[4-(4-fluoro-1H-pyrazol-1-yl)-3-sulfamoylphenyl]acetamide;
   (D146) 2-(3,5-difluorophenyl)-N-[4-(4-fluoro-1H-pyrazol-1-yl)-3-sulfamoylphenyl]acetamide;
   (D147) N-[4-(4-fluoro-1H-pyrazol-1-yl)-3-sulfamoylphenyl]-2-(2-hydroxyphenyl)acetamide;
   (D148) 2-(3-chlorophenyl)-N-[4-(4-fluoro-1H-pyrazol-1-yl)-3-sulfamoylphenyl]acetamide;
   (D149) 2-(4-fluorophenyl)-N-[4-(4-fluoro-1H-pyrazol-1-yl)-3-sulfamoylphenyl]acetamide;
   (D150) N-[4-(4-fluoro-1H-pyrazol-1-yl)-3-sulfamoylphenyl]-2-(4-hydroxyphenyl)acetamide;
   (D151) N-[4-(4-fluoro-1H-pyrazol-1-yl)-3-sulfamoylphenyl]-2-(3-methylphenyl)acetamide;
   (D152) N-[4-(4-fluoro-1H-pyrazol-1-yl)-3-sulfamoylphenyl]-2-(4-methylphenyl)acetamide;
   (D153) N-[4-(4-fluoro-1H-pyrazol-1-yl)-3-sulfamoylphenyl]-2-(4-methoxyphenyl)acetamide;
   (D154) N-[4-(4-fluoro-1H-pyrazol-1-yl)-3-sulfamoylphenyl]-2-(2-methylphenyl)acetamide;
   (D155) 2-(2,3-difluorophenyl)-N-[4-(4-fluoro-1H-pyrazol-1-yl)-3-sulfamoylphenyl]acetamide;
   (D156) 2-(2-ethoxyphenyl)-N-[4-(4-fluoro-1H-pyrazol-1-yl)-3-sulfamoylphenyl]acetamide;
   (D157) 2-[2-(difluoromethoxy)phenyl]-N-[4-(4-fluoro-1H-pyrazol-1-yl)-3-sulfamoylphenyl]acetamide;
   (D158) 2-(2-chloropyridin-3-yl)-N-[4-(4-fluoro-1H-pyrazol-1-yl)-3-sulfamoylphenyl]acetamide;
   (D159) 2-(2-chloro-6-fluoro-3-methylphenyl)-N-[4-(4-fluoro-1H-pyrazol-1-yl)-3-sulfamoylphenyl]acetamide;
   (D160) N-[4-(4-fluoro-1H-pyrazol-1-yl)-3-sulfamoylphenyl]-2-[2-(trifluoromethoxy)phenyl]acetamide;
   (D161) 2-(2-chloro-4,5-difluorophenyl)-N-[4-(4-fluoro-1H-pyrazol-1-yl)-3-sulfamoylphenyl]acetamide;
   (D162) 2-[2-chloro-3-(trifluoromethyl)phenyl]-N-[4-(4-fluoro-1H-pyrazol-1-yl)-3-sulfamoylphenyl]acetamide;
   (D163) 2-(2,5-dichlorophenyl)-N-[4-(4-fluoro-1H-pyrazol-1-yl)-3-sulfamoylphenyl]acetamide;
   (D164) 2-(2-chloro-3,6-difluorophenyl)-N-[4-(4-fluoro-1H-pyrazol-1-yl)-3-sulfamoylphenyl]acetamide;
   (D165) 2-(2-chloro-5-methylphenyl)-N-[4-(4-fluoro-1H-pyrazol-1-yl)-3-sulfamoylphenyl]acetamide;
   (D166) 2-(5-bromo-4-fluoro-2-methylphenyl)-N-[4-(4-fluoro-1H-pyrazol-1-yl)-3-sulfamoylphenyl]acetamide;
   (D167) N-[4-(4-fluoro-1H-pyrazol-1-yl)-3-sulfamoylphenyl]-2-(2-isopropylphenyl)acetamide;
   (D168) 2-(2-chloro-5-fluorophenyl)-N-[4-(4-fluoro-1H-pyrazol-1-yl)-3-sulfamoylphenyl]acetamide;
   (D169) 2-[2-chloro-6-(trifluoromethyl)phenyl]-N-[4-(4-fluoro-1H-pyrazol-1-yl)-3-sulfamoylphenyl]acetamide;
   (D170) N-[4-(4-chloro-1H-pyrazol-1-yl)-3-sulfamoylphenyl]-2-(4-methoxyphenyl)acetamide;
   (D171) 2-(2,6-difluorophenyl)-N-[4-(4-fluoro-1H-pyrazol-1-yl)-3-sulfamoylphenyl]propanamide;
   (D172) N-[4-(4-chloro-1H-pyrazol-1-yl)-3-sulfamoylphenyl]-2-(4-methylphenyl)acetamide;
   (D173) N-[4-(4-chloro-1H-pyrazol-1-yl)-3-sulfamoylphenyl]-2-(2-methylphenyl)acetamide;
   (D174) N-[4-(4-chloro-1H-pyrazol-1-yl)-3-sulfamoylphenyl]-2-(3-methylphenyl)acetamide;
   (D175) N-[4-(4-chloro-1H-pyrazol-1-yl)-3-sulfamoylphenyl]-2-(2-ethoxyphenyl)acetamide;
   (D176) 2-(2-ethoxyphenyl)-N-{3-sulfamoyl-4-[4-(trifluoromethyl)-1H-pyrazol-1-yl]phenyl}acetamide;
   (D177) 2-(2-bromophenyl)-N-[4-(4-fluoro-1H-pyrazol-1-yl)-3-sulfamoylphenyl]acetamide;
   (D178) 2-(2,4-dichlorophenyl)-N-[4-(4-fluoro-1H-pyrazol-1-yl)-3-sulfamoylphenyl]acetamide;
   (D179) 2-(2,6-dichlorophenyl)-N-[4-(4-fluoro-1H-pyrazol-1-yl)-3-sulfamoylphenyl]acetamide;
   (D180) N-[4-(4-cyano-1H-pyrazol-1-yl)-3-sulfamoylphenyl]-2-(2-nitrophenyl)acetamide;
   (D181) 2-(4-chlorophenyl)-N-[4-(4-cyano-1H-pyrazol-1-yl)-3-sulfamoylphenyl]acetamide;
   (D182) N-[4-(4-cyano-1H-pyrazol-1-yl)-3-sulfamoylphenyl]-2-(2-methoxyphenyl)acetamide;
   (D183) 2-(2-chloro-6-fluorophenyl)-N-[4-(4-cyano-1H-pyrazol-1-yl)-3-sulfamoylphenyl]acetamide;
   (D184) N-[4-(4-cyano-1H-pyrazol-1-yl)-3-sulfamoylphenyl]-2-[2-(trifluoromethyl)phenyl]acetamide;
   (D185) N-[4-(4-cyano-1H-pyrazol-1-yl)-3-sulfamoylphenyl]-2-(3-fluorophenyl)acetamide;
   (D186) N-[4-(4-cyano-1H-pyrazol-1-yl)-3-sulfamoylphenyl]-2-(2,4-dichlorophenyl)acetamide;
   (D187) N-[4-(4-cyano-1H-pyrazol-1-yl)-3-sulfamoylphenyl]-2-(2,6-dichlorophenyl)acetamide;
   (D188) 2-(2-bromophenyl)-N-[4-(4-cyano-1H-pyrazol-1-yl)-3-sulfamoylphenyl]acetamide;
   (D189) N-[4-(4-cyano-1H-pyrazol-1-yl)-3-sulfamoylphenyl]-2-(3,4-difluorophenyl)acetamide;
   (D190) N-[4-(4-cyano-1H-pyrazol-1-yl)-3-sulfamoylphenyl]-2-(3,5-difluorophenyl)acetamide;
   (D191) 2-(3-chlorophenyl)-N-[4-(4-cyano-1H-pyrazol-1-yl)-3-sulfamoylphenyl]acetamide;
   (D192) N-[4-(4-cyano-1H-pyrazol-1-yl)-3-sulfamoylphenyl]-2-(4-fluorophenyl)acetamide;
   (D193) N-[4-(4-cyano-1H-pyrazol-1-yl)-3-sulfamoylphenyl]-2-(2-hydroxyphenyl)acetamide;
   (D194) N-[4-(4-cyano-1H-pyrazol-1-yl)-3-sulfamoylphenyl]-2-(4-hydroxyphenyl)acetamide;
   (D195) N-[4-(4-cyano-1H-pyrazol-1-yl)-3-sulfamoylphenyl]-2-(4-methylphenyl)acetamide;
   (D196) N-[4-(4-cyano-1H-pyrazol-1-yl)-3-sulfamoylphenyl]-2-(4-methoxyphenyl)acetamide;
   (D197) N-[4-(4-cyano-1H-pyrazol-1-yl)-3-sulfamoylphenyl]-2-(2-methylphenyl)acetamide;
   (D198) N-[4-(4-cyano-1H-pyrazol-1-yl)-3-sulfamoylphenyl]-2-(3-methylphenyl)acetamide;
   (D199) N-[4-(4-cyano-1H-pyrazol-1-yl)-3-sulfamoylphenyl]-2-(2-ethoxyphenyl)acetamide;
   (D200) N-[4-(4-cyano-1H-pyrazol-1-yl)-3-sulfamoylphenyl]-2-(2,3-difluorophenyl)acetamide;
   (D201) 2-(2-chloropyridin-3-yl)-N-[4-(4-cyano-1H-pyrazol-1-yl)-3-sulfamoylphenyl]acetamide;
   (D202) 2-(2-chloro-4-fluorophenyl)-N-[4-(4-cyano-1H-pyrazol-1-yl)-3-sulfamoylphenyl]acetamide;
   (D203) N-[4-(4-cyano-1H-pyrazol-1-yl)-3-sulfamoylphenyl]-2-[2-(difluoromethoxy)phenyl]acetamide;
   (D204) N-[4-(4-cyano-1H-pyrazol-1-yl)-3-sulfamoylphenyl]-2-[2-(trifluoromethoxy)phenyl]acetamide;
   (D205) 2-(2-chloro-4,5-difluorophenyl)-N-[4-(4-cyano-1H-pyrazol-1-yl)-3-sulfamoylphenyl]acetamide;
   (D206) 2-[2-chloro-3-(trifluoromethyl)phenyl]-N-[4-(4-cyano-1H-pyrazol-1-yl)-3-sulfamoylphenyl]acetamide;
   (D207) 2-(2-chloro-6-fluoro-3-methylphenyl)-N-[4-(4-cyano-1H-pyrazol-1-yl)-3-sulfamoylphenyl]acetamide;
   (D208) 2-(2-chloro-3,6-difluorophenyl)-N-[4-(4-cyano-1H-pyrazol-1-yl)-3-sulfamoylphenyl]acetamide;
   (D209) 2-(2-chloro-5-methylphenyl)-N-[4-(4-cyano-1H-pyrazol-1-yl)-3-sulfamoylphenyl]acetamide;
   (D210) 2-(2-chloro-4-methoxyphenyl)-N-[4-(4-cyano-1H-pyrazol-1-yl)-3-sulfamoylphenyl]acetamide;
   (D211) N-[4-(4-cyano-1H-pyrazol-1-yl)-3-sulfamoylphenyl]-2-(2,5-dichlorophenyl)acetamide;
   (D212) 2-(5-chloro-2-methoxyphenyl)-N-[4-(4-cyano-1H-pyrazol-1-yl)-3-sulfamoylphenyl]acetamide;
   (D213) N-[4-(4-cyano-1H-pyrazol-1-yl)-3-sulfamoylphenyl]-2-[2-(propan-2-yl)phenyl]acetamide;
   (D214) 2-(2-chloro-5-fluorophenyl)-N-[4-(4-cyano-1H-pyrazol-1-yl)-3-sulfamoylphenyl]acetamide;
   (D215) 2-[2-chloro-6-(trifluoromethyl)phenyl]-N-[4-(4-cyano-1H-pyrazol-1-yl)-3-sulfamoylphenyl]acetamide;
   (D216) 2-(2-chloro-6-methoxyphenyl)-N-[4-(4-cyano-1H-pyrazol-1-yl)-3-sulfamoylphenyl]acetamide;
   (D217) 2-(2-chloro-4,6-difluorophenyl)-N-[4-(4-cyano-1H-pyrazol-1-yl)-3-sulfamoylphenyl]acetamide;
   (D218) 2-(3-chloropyridin-4-yl)-N-[4-(4-cyano-1H-pyrazol-1-yl)-3-sulfamoylphenyl]acetamide;
   (D219) N-[4-(4-cyano-1H-pyrazol-1-yl)-3-sulfamoylphenyl]-2-[4-(difluoromethyl)phenyl]acetamide;
   (D220) 2-(2-chlorophenyl)-N-[4-(4-cyano-1H-pyrazol-1-yl)-3-sulfamoylphenyl]-2,2-difluoroacetamide;
   (D221) 2-(2-fluorophenyl)-N-{3-sulfamoyl-4-[5-(trifluoromethyl)pyridin-3-yl]phenyl}acetamide;
   (D222) N-{3-sulfamoyl-4-[5-(trifluoromethyl)pyridin-3-yl]phenyl}-2-[2-(trifluoromethoxy)phenyl]acetamide;
   (D223) N-{4-[1-(difluoromethyl)-1H-pyrazol-4-yl]-3-sulfamoylphenyl}-2-(2-methylphenyl)acetamide;
   (D224) N-{4-[1-(difluoromethyl)-1H-pyrazol-4-yl]-3-sulfamoylphenyl}-2-[2-(trifluoromethyl)phenyl]acetamide;
   (D225) 2-[2-(difluoromethyl)phenyl]-N-{4-[1-(difluoromethyl)-1H-pyrazol-4-yl]-3-sulfamoylphenyl}acetamide;
   (D226) N-{4-[1-(difluoromethyl)-1H-pyrazol-4-yl]-3-sulfamoylphenyl}-2-(2-methoxyphenyl)acetamide;
   (D227) N-{4-[1-(difluoromethyl)-1H-pyrazol-4-yl]-3-sulfamoylphenyl}-2-(4-fluorophenyl)acetamide;
   (D228) 2-(2-chloro-5-fluorophenyl)-N-{4-[1-(difluoromethyl)-1H-pyrazol-4-yl]-3-sulfamoylphenyl}acetamide;
   (D229) 2-(2,3-dichlorophenyl)-N-{4-[1-(difluoromethyl)-1H-pyrazol-4-yl]-3-sulfamoylphenyl}acetamide;
   (D230) 2-(3-chloropyridin-4-yl)-N-{4-[1-(difluoromethyl)-1H-pyrazol-4-yl]-3-sulfamoylphenyl}acetamide;
   (D231) 2-(2-chlorophenyl)-N-[4-(3-cyclobutyl-1H-1,2,4-triazol-1-yl)-3-sulfamoylphenyl]acetamide;
   (D232) N-[4-(4-acetyl-1H-pyrazol-1-yl)-3-sulfamoylphenyl]-2-(2-chlorophenyl)acetamide;
   (D233) 2-(2-chlorophenyl)-N-[4-(3-isopropyl-1H-1,2,4-triazol-1-yl)-3-sulfamoylphenyl]acetamide;
   (D234) N-[4-(4-chloro-1H-pyrazol-1-yl)-3-sulfamoylphenyl]-2-[2-(trifluoromethyl)phenyl]acetamide;
   (D235) ethyl 1-(4-{[(2-chlorophenyl)acetyl]amino}-2-sulfamoylphenyl)-1H-pyrazole-4-carboxylate;
   (D236) ethyl 1-(4-{[(2-fluorophenyl)acetyl]amino}-2-sulfamoylphenyl)-1H-pyrazole-4-carboxylate;
   (D237) 2-(2-fluorophenyl)-N-{4-[4-(2-hydroxypropan-2-yl)-1H-pyrazol-1-yl]-3-sulfamoylphenyl}acetamide;
   (D238) 2-(2-chlorophenyl)-N-{4-[4-(2-hydroxypropan-2-yl)-1H-pyrazol-1-yl]-3-sulfamoylphenyl}acetamide;
   (D239) 2-(2-chloropyridin-3-yl)-N-{3-sulfamoyl-4-[3-(trifluoromethyl)-1H-1,2,4-triazol-1-yl]phenyl}acetamide;
   (D240) 2-(2-chlorophenyl)-N-methyl-N-{3-sulfamoyl-4-[4-(trifluoromethyl)-1H-pyrazol-1-yl]phenyl}acetamide;
   (D241) 2-(2-chlorophenyl)-N-{3-sulfamoyl-4-[4-(trifluoromethyl)-2H-1,2,3-triazol-2-yl]phenyl}acetamide;
   (D242) 2-(2-chlorophenyl)-N-{3-sulfamoyl-4-[4-(trifluoromethyl)-1H-1,2,3-triazol-1-yl]phenyl}acetamide;
   (D243) 2-(2-chloro-5-cyanophenyl)-N-{3-sulfamoyl-4-[4-(trifluoromethyl)-1H-pyrazol-1-yl]phenyl}acetamide;
   (D244) 2-(2-chlorophenyl)-N-{3-cyano-5-sulfamoyl-4-[4-(trifluoromethyl)-1H-pyrazol-1-yl]phenyl}acetamide;
   (D245) 2-(5-bromo-2-chlorophenyl)-N-{3-sulfamoyl-4-[4-(trifluoromethyl)-1H-pyrazol-1-yl]phenyl}acetamide;
   (D246) N-[4-(3-chloro-1H-1,2,4-triazol-1-yl)-3-sulfamoylphenyl]-2-(2-fluorophenyl)acetamide;
   (D247) 2-(2-chlorophenyl)-N-[3-cyano-4-(4-fluoro-1H-pyrazol-1-yl)-5-sulfamoylphenyl]acetamide;
   (D248) 2-(2-chlorophenyl)-N-[4-(4-methyl-1H-pyrazol-1-yl)-3-sulfamoylphenyl]acetamide;
   (D249) 2-(2-chlorophenyl)-N-[4-(3-methoxy-1H-1,2,4-triazol-1-yl)-3-sulfamoylphenyl]acetamide;
   (D250) 2-(2-chlorophenyl)-N-[4-(4-cyclopropyl-1H-imidazol-1-yl)-3-sulfamoylphenyl]acetamide;
   (D251) 2-(2-chlorophenyl)-N-[4-(4-methyl-1H-imidazol-1-yl)-3-sulfamoylphenyl]acetamide;
   (D252) 2-(2-chlorophenyl)-N-[4-(3-cyclopropyl-1H-pyrazol-1-yl)-3-sulfamoylphenyl]acetamide;
   (D253) 2-(2-chlorophenyl)-N-[4-(2H-pyrazolo[3,4-b]pyridin-2-yl)-3-sulfamoylphenyl]acetamide;
   (D254) 2-(2-chlorophenyl)-N-[4-(2H-pyrazolo[3,4-c]pyridin-2-yl)-3-sulfamoylphenyl]acetamide;
   (D255) 2-(2-chlorophenyl)-N-[4-(2H-pyrazolo[4,3-b]pyridin-2-yl)-3-sulfamoylphenyl]acetamide;
   (D256) 2-(2-chlorophenyl)-N-{4-[4-(2-methoxyethyl)-1H-pyrazol-1-yl]-3-sulfamoylphenyl}acetamide;
   (D257) 2-(2-chlorophenyl)-N-[4-(3-fluoro-1H-pyrazol-1-yl)-3-sulfamoylphenyl]acetamide;
   (D258) 2-(2-chlorophenyl)-N-(4-{4-[(2,2-difluoroethyl)amino]-1H-pyrazol-1-yl}-3-sulfamoylphenyl) acetamide;
   (D259) 2-(2-chlorophenyl)-N-{4-[4-(2,2-difluoroethyl)-1H-pyrazol-1-yl]-3-sulfamoylphenyl}acetamide;
   (D260) 2-(2-chlorophenyl)-N-[4-(4-{[(2,2-difluoroethyl)amino]methyl}-1H-pyrazol-1-yl)-3-sulfamoylphenyl]acetamide;
   (D261) 2-(2-chlorophenyl)-N-{3-sulfamoyl-4-[5-(trifluoromethyl)-1,2,4-oxadiazol-3-yl]phenyl}acetamide;
   (D262) 2-(2-fluorophenyl)-N-[4-(4-methyl-1H-pyrazol-1-yl)-3-sulfamoylphenyl]acetamide;
   (D263) N-[4-(4-cyclopropyl-1H-imidazol-1-yl)-3-sulfamoylphenyl]-2-(2-fluorophenyl)acetamide;
   (D264) N-[4-(3-cyclopropyl-1H-pyrazol-1-yl)-3-sulfamoylphenyl]-2-(2-fluorophenyl)acetamide;
   (D265) 2-(2-fluorophenyl)-N-{4-[4-(2-methoxyethyl)-1H-pyrazol-1-yl]-3-sulfamoylphenyl}acetamide;
   (D266) 2-(2-chlorophenyl)-N-(2-sulfamoylbiphenyl-4-yl)acetamide;
   (D267) 2-(2-chlorophenyl)-N-{4-[1-(cyclopropylmethyl)-1H-pyrazol-4-yl]-3-sulfamoylphenyl}acetamide;
   (D268) 2-(2-chlorophenyl)-N-{4-[1-(2-methoxyethyl)-1H-pyrazol-4-yl]-3-sulfamoylphenyl}acetamide;
   (D269) 2-(2-chlorophenyl)-N-{4-[1-(piperidin-4-yl)-1H-pyrazol-4-yl]-3-sulfamoylphenyl}acetamide;
   (D270) 2-(2-fluorophenyl)-N-{4-[1-(piperidin-4-yl)-1H-pyrazol-4-yl]-3-sulfamoylphenyl}acetamide;
   (D271) N-{4-[1-(azetidin-3-yl)-1H-pyrazol-4-yl]-3-sulfamoylphenyl}-2-(2-chlorophenyl)acetamide;
   (D272) 2-(3-chlorophenyl)-N-(2-sulfamoylbiphenyl-4-yl)acetamide;
   (D273) 2-(2-chlorophenyl)-N-[4-(3-methyl-1H-pyrazol-1-yl)-3-sulfamoylphenyl]acetamide;
   (D274) 2-(2-chlorophenyl)-N-[4-(6-chloropyridin-3-yl)-3-sulfamoylphenyl] acetamide;
   (D275) 2-(2-chlorophenyl)-N-[4-(3,5-dimethyl-1,2-oxazol-4-yl)-3-sulfamoylphenyl]acetamide;
   (D276) 2-(2-chlorophenyl)-N-(4-{1-[(3-methyloxetan-3-yl)methyl]-1H-pyrazol-4-yl}-3-sulfamoylphenyl)acetamide;
   (D277) 2-(2-chloro-4-fluorophenyl)-N-{4-[1-(2-methoxyethyl)-1H-pyrazol-4-yl]-3-sulfamoylphenyl}acetamide;
   (D278) 2-(2-chloro-4-fluorophenyl)-N-[4-(1-cyclopropyl-1H-pyrazol-4-yl)-3-sulfamoylphenyl]acetamide;
   (D279) 2-(2-chlorophenyl)-N-(3-sulfamoyl-4-{1-[2-(trifluoromethoxy)ethyl]-1H-pyrazol-4-yl}phenyl)acetamide;
   (D280) 2-(2-chlorophenyl)-N-[4-(1-cyclobutyl-1H-pyrazol-4-yl)-3-sulfamoylphenyl]acetamide;
   (D281) 2-(2-chlorophenyl)-N-(4-{1-[2-(pyrrolidin-1-yl)ethyl]-1H-pyrazol-4-yl}-3-sulfamoylphenyl)acetamide;
   (D282) 2-(2-chlorophenyl)-N-[4-(5-cyanopyridin-3-yl)-3-sulfamoylphenyl]acetamide;
   (D283) 2-(2-chlorophenyl)-N-(4-{1-[oxetan-2-ylmethyl]-1H-pyrazol-4-yl}-3-sulfamoylphenyl) acetamide;
   (D284) 2-(2-chlorophenyl)-N-{3-sulfamoyl-4-[2-(2,2,2-trifluoroethoxy)pyrimidin-5-yl]phenyl}acetamide;
   (D285) 2-(2-chlorophenyl)-N-[4-(2-methoxypyrimidin-5-yl)-3-sulfamoylphenyl]acetamide;
   (D286) 2-(2-chlorophenyl)-N-(4-{1-[2-(propan-2-yloxy)ethyl]-1H-pyrazol-4-yl}-3-sulfamoylphenyl)acetamide;
   (D287) 2-(2-chlorophenyl)-N-{4-[1-(3-hydroxy-3-methylbutyl)-1H-pyrazol-4-yl]-3-sulfamoylphenyl}acetamide;
   (D288) 2-(2-chlorophenyl)-N-{4-[5-(2-hydroxypropan-2-yl)-1-methyl-1H-pyrazol-3-yl]-3-sulfamoylphenyl}acetamide;
   (D289) 2-(2-chlorophenyl)-N-{4-[5-(difluoromethoxy)pyridin-3-yl]-3-sulfamoylphenyl}acetamide;
   (D290) N-[4-(2-chloro-5-methoxypyridin-3-yl)-3-sulfamoylphenyl]-2-(2-chlorophenyl)acetamide;
   (D291) 2-(2-chlorophenyl)-N-{4-[1-(2-hydroxyethyl)-1H-pyrazol-4-yl]-3-sulfamoylphenyl}acetamide;
   (D292) N-[4-(5-tert-butyl-1H-pyrazol-3-yl)-3-sulfamoylphenyl]-2-(2-chlorophenyl)acetamide;
   (D293) N-[4-(1-benzyl-1H-pyrazol-4-yl)-3-sulfamoylphenyl]-2-(2-chlorophenyl)acetamide;
   (D294) 2-(2-chlorophenyl)-N-[4-(6-methylpyridazin-4-yl)-3-sulfamoylphenyl] acetamide;
   (D295) 2-(2-chlorophenyl)-N-{3-sulfamoyl-4-[6-(trifluoromethyl)pyridin-2-yl]phenyl}acetamide;
   (D296) N-{4-[1-(difluoromethyl)-1H-pyrazol-4-yl]-3-sulfamoylphenyl}-2-(2-fluorophenyl)acetamide;
   (D297) 2-(2-chlorophenyl)-N-{6-[1-(difluoromethyl)-1H-pyrazol-4-yl]-5-sulfamoylpyridin-3-yl}acetamide;
   (D298) N-[4-(6-chloro-5-methylpyridin-3-yl)-3-sulfamoylphenyl]-2-(2-chlorophenyl)acetamide;
   (D299) 2-(2-chlorophenyl)-N-{4-[2-(cyclopropylamino)pyrimidin-5-yl]-3-sulfamoylphenyl}acetamide;
   (D300) 2-(2-chlorophenyl)-N-{3-sulfamoyl-4-[1-(tetrahydrofuran-3-yl)-1H-pyrazol-4-yl]phenyl}acetamide;
   (D301) 2-(2-chlorophenyl)-N-{4-[2-(methylamino)pyrimidin-5-yl]-3-sulfamoylphenyl}acetamide;
   (D302) 2-(2-chlorophenyl)-N-[6-(1-methyl-1H-pyrazol-4-yl)-5-sulfamoylpyridin-3-yl]acetamide;
   (D303) 2-(2-chlorophenyl)-N-{4-[1-(2,2-difluoroethyl)-1H-pyrazol-4-yl]-3-sulfamoylphenyl}acetamide;
   (D304) N-{3-sulfamoyl-4-[5-(trifluoromethyl)pyridin-3-yl]phenyl}-2-[2-(trifluoromethyl)phenyl]acetamide;
   (D305) 2-(2-chlorophenyl)-N-[3-sulfamoyl-5'-(trifluoromethyl)-2,3'-bipyridin-5-yl]acetamide;
   (D306) 2-(2-chlorophenyl)-N-{3-sulfamoyl-4-[2-(trifluoromethyl)pyrimidin-5-yl]phenyl}acetamide;
   (D307) 2-(2-chlorophenyl)-N-{3-sulfamoyl-4-[5-(trifluoromethoxy)pyridin-3-yl]phenyl}acetamide;
   (D308) 2-(2-chlorophenyl)-N-[4-(2-cyclopropylpyrimidin-5-yl)-3-sulfamoylphenyl]acetamide;
   (D309) 2-(2-chlorophenyl)-N-[4-(2-ethoxypyrimidin-5-yl)-3-sulfamoylphenyl]acetamide;
   (D310) 2-(2-chlorophenyl)-N-{4-[2-(propan-2-ylamino)pyrimidin-5-yl]-3-sulfamoylphenyl}acetamide;
   (D311) 2-(2-chlorophenyl)-N-{4-[2-(propan-2-yloxy)pyrimidin-5-yl]-3-sulfamoylphenyl}acetamide;
   (D312) 2-(2-chlorophenyl)-N-{4-[2-(ethylamino)pyrimidin-5-yl]-3-sulfamoylphenyl}acetamide;
   (D313) 2-(2-chlorophenyl)-N-[4-(2-methylpyrimidin-5-yl)-3-sulfamoylphenyl]acetamide;
   (D314) 2-(2-chlorophenyl)-N-{4-[2-(propylamino)pyrimidin-5-yl]-3-sulfamoylphenyl}acetamide;
   (D315) 2-(2-chlorophenyl)-N-(3-sulfamoyl-4-{2-[(2,2,2-trifluoroethyl)amino]pyrimidin-5-yl}phenyl)acetamide;
   (D316) 2-(2-chlorophenyl)-N-{4-[2-(cyclobutyloxy)pyrimidin-5-yl]-3-sulfamoylphenyl}acetamide;
   (D317) N-[4-(2-chloro-4-methylpyrimidin-5-yl)-3-sulfamoylphenyl]-2-(2-chlorophenyl)acetamide;
   (D318) 2-(2-Chlorophenyl)-N-{3-sulfamoyl-4-[4-(trifluoromethyl)pyridin-2-yl]phenyl}acetamide;
   (D319) 2-(2-chlorophenyl)-N-[4-(5-chloropyridin-2-yl)-3-sulfamoylphenyl]acetamide;
   (D320) 2-(2-chlorophenyl)-N-[4-(1,2-dimethyl-1H-imidazol-4-yl)-3-sulfamoylphenyl]acetamide;
   (D321) N-{6-[1-(difluoromethyl)-1H-pyrazol-4-yl]-5-sulfamoylpyridin-3-yl}-2-(2-fluorophenyl)acetamide;
   (D322) 2-(2-chlorophenyl)-N-{4-[5-(pyrrolidin-1-yl)pyridin-3-yl]-3-sulfamoylphenyl}acetamide;
   (D323) 2-(2-chlorophenyl)-N-[4-(1-cyclopropyl-1H-pyrazol-4-yl)-3-sulfamoylphenyl]-2-hydroxyethanamide;
   (D324) 2-(2-chlorophenyl)-N-{4-[1-(difluoromethyl)-1H-pyrazol-4-yl]-3-sulfamoylphenyl}-2-hydroxyethanamide;
   (D325) 2-(2-chlorophenyl)-N-[4-(5-chloropyridin-3-yl)-3-sulfamoylphenyl]acetamide;
   (D326) 2-(2-chlorophenyl)-N-{4-[1-(difluoromethyl)-1H-pyrazol-4-yl]-3-sulfamoylphenyl}acetamide;
   (D327) 2-(2-chlorophenyl)-N-[4-(5-fluoropyridin-3-yl)-3-sulfamoylphenyl]acetamide;
   (D328) 2-(2-chlorophenyl)-N-{4-[1-(2-methylpropyl)-1H-pyrazol-4-yl]-3-sulfamoylphenyl}acetamide;
   (D329) 2-(2-chlorophenyl)-N-[4-(1-cyclopentyl-1H-pyrazol-4-yl)-3-sulfamoylphenyl]acetamide;
   (D330) 2-(2-chlorophenyl)-N-[2'-fluoro-3'-(propan-2-yloxy)-2-sulfamoylbiphenyl-4-yl]acetamide;
   (D331) 2-(2-chlorophenyl)-N-{3-sulfamoyl-4-[5-(trifluoromethyl)pyridin-3-yl]phenyl}acetamide;
   (D332) N-[4-(6-chloro-5-methoxypyridin-3-yl)-3-sulfamoylphenyl]-2-(2-chlorophenyl)acetamide;
   (D333) 2-(2-chloro-6-fluorophenyl)-N-[4-(1-cyclopropyl-1H-pyrazol-4-yl)-3-sulfamoylphenyl]acetamide;
   (D334) 2-(2-chloro-3-fluorophenyl)-N-[4-(1-methyl-1H-pyrazol-4-yl)-3-sulfamoylphenyl]acetamide;
   (D335) N-[4-(1-tert-butyl-1H-pyrazol-4-yl)-3-sulfamoylphenyl]-2-(2-chlorophenyl)acetamide;
   (D336) 2-(2-chlorophenyl)-N-{4-[2-(propan-2-yloxy)pyridin-3-yl]-3-sulfamoylphenyl}acetamide;
   (D337) 2-(2-chloro-3-fluorophenyl)-N-[4-(1-cyclopropyl-1H-pyrazol-4-yl)-3-sulfamoylphenyl]acetamide;
   (D338) 2-(2-fluorophenyl)-N-[4-(pyridin-3-yl)-3-sulfamoylphenyl]acetamide;
   (D339) 2-(2-chloro-6-fluorophenyl)-N-[4-(1-methyl-1H-pyrazol-4-yl)-3-sulfamoylphenyl]acetamide;
   (D340) 2-(2-chlorophenyl)-N-[3'-fluoro-5'-(2-hydroxypropan-2-yl)-2-sulfamoylbiphenyl-4-yl]acetamide;
   (D341) 2-(2-chlorophenyl)-N-[4-(5-methoxypyridin-3-yl)-3-sulfamoylphenyl]acetamide;
   (D342) 2-(2-chlorophenyl)-N-[3'-(2-hydroxypropan-2-yl)-2-sulfamoylbiphenyl-4-yl]acetamide;
   (D343) 2-(2-fluorophenyl)-N-{4-[1-(propan-2-yl)-1H-pyrazol-4-yl]-3-sulfamoylphenyl}acetamide;
   (D344) N-[4-(1-cyclopropyl-1H-pyrazol-4-yl)-3-sulfamoylphenyl]-2-(2-fluorophenyl)acetamide;
   (D345) 2-(2-chlorophenyl)-N-{3-sulfamoyl-4-[1-(tetrahydro-2H-pyran-2-yl)-1H-pyrazol-4-yl]phenyl}acetamide;
   (D346) N-[4-(1-tert-butyl-1H-pyrazol-4-yl)-3-sulfamoylphenyl]-2-(2-fluorophenyl)acetamide;
   (D347) N-[3'-fluoro-5'-(2-hydroxypropan-2-yl)-2-sulfamoylbiphenyl-4-yl]-2-(2-fluorophenyl)acetamide;
   (D348) N-[4-(1-cyclopentyl-1H-pyrazol-4-yl)-3-sulfamoylphenyl]-2-(2-fluorophenyl)acetamide;
   (D349) 2-(2-chlorophenyl)-N-[4-(1-cyclopropyl-1H-pyrazol-4-yl)-3-sulfamoylphenyl]acetamide;
   (D350) 2-(2-chloro-6-fluorophenyl)-N-{4-[1-(2-methoxyethyl)-1H-pyrazol-4-yl]-3-sulfamoylphenyl}acetamide;
   (D351) 2-(2-chlorophenyl)-N-[4-(1,3-dimethyl-1H-pyrazol-5-yl)-3-sulfamoylphenyl]acetamide;
   (D352) 2-(2-chlorophenyl)-N-(4'-chloro-2-sulfamoylbiphenyl-4-yl)acetamide;
   (D353) 2-(2-chlorophenyl)-N-{4-[1-methyl-3-(trifluoromethyl)-1H-pyrazol-5-yl]-3-sulfamoylphenyl}acetamide;
   (D354) 2-(2-chlorophenyl)-N-[4-(pyridin-3-yl)-3-sulfamoylphenyl]acetamide;
   (D355) 2-(2-chlorophenyl)-N-{4-[1-methyl-3-(trifluoromethyl)-1H-pyrazol-4-yl]-3-sulfamoylphenyl}acetamide;
   (D356) 2-(2-chlorophenyl)-N-(3'-chloro-2-sulfamoylbiphenyl-4-yl)acetamide;
   (D357) 2-(2-chlorophenyl)-N-(4-{1-[(2,2-dichlorocyclopropyl)methyl]-1H-pyrazol-4-yl}-3-sulfamoylphenyl) acetamide;
   (D358) 2-(2-chlorophenyl)-N-[4-(1,3-dimethyl-1H-pyrazol-4-yl)-3-sulfamoylphenyl]acetamide;
   (D359) 2-(2-chlorophenyl)-N-{4-[1-(propan-2-yl)-1H-pyrazol-4-yl]-3-sulfamoylphenyl}acetamide;
   (D360) 2-(2-chlorophenyl)-N-[4-(1-methyl-1H-pyrazol-4-yl)-3-sulfamoylphenyl]acetamide;
   (D361) 2-(2-chlorophenyl)-N-{4-[1-(2-hydroxy-3,3-dimethylbutyl)-1H-pyrazol-4-yl]-3-sulfamoylphenyl}acetamide;
   (D362) 2-(2-fluorophenyl)-N-[4-(5-fluoropyridin-3-yl)-3-sulfamoylphenyl]acetamide;
   (D363) 2-(2-chlorophenyl)-N-[4-(5-methyl-1-phenyl-1H-pyrazol-4-yl)-3-sulfamoylphenyl]acetamide;
   (D364) 4'-{[(2-chlorophenyl)acetyl]amino}-N-[2-(dimethylamino)ethyl]-2'-sulfamoylbiphenyl-3-carboxamide;
   (D365) 2-(2-chlorophenyl)-N-[4-(pyrazolo[1,5-a]pyrimidin-3-yl)-3-sulfamoylphenyl]acetamide;
   (D366) 2-(2-chlorophenyl)-N-{4-[5-(pyrrolidin-1-ylcarbonyl)pyridin-3-yl]-3-sulfamoylphenyl}acetamide;
   (D367) 2-(2-chlorophenyl)-N-[4-(1H-pyrazol-4-yl)-3-sulfamoylphenyl]acetamide;
   (D368) 2-(2-fluorophenyl)-N-{4-[1-methyl-3-(trifluoromethyl)-1H-pyrazol-5-yl]-3-sulfamoylphenyl}acetamide;
   (D369) 2-(2-fluorophenyl)-N-{4-[1-(2-methoxyethyl)-1H-pyrazol-4-yl]-3-sulfamoylphenyl}acetamide;
   (D370) 2-(2-chlorophenyl)-N-{4-[1-(2-hydroxy-2-methylpropyl)-1H-pyrazol-4-yl]-3-sulfamoylphenyl}acetamide;
   (D371) 2-(5-chloro-2-fluorophenyl)-N-[4-(1-cyclopropyl-1H-pyrazol-4-yl)-3-sulfamoylphenyl]acetamide;
   (D372) 2-(2-chlorophenyl)-N-[4-(6-methylpyridin-3-yl)-3-sulfamoylphenyl]acetamide;
   (D373) 2-(2-chlorophenyl)-N-{4-[1-(oxetan-3-yl)-1H-pyrazol-4-yl]-3-sulfamoylphenyl}acetamide;
   (D374) 2-(2-fluorophenyl)-N-[4-(1-methyl-1H-pyrazol-4-yl)-3-sulfamoylphenyl]acetamide;
   (D375) N-[4-(1,3-dimethyl-1H-pyrazol-4-yl)-3-sulfamoylphenyl]-2-(2-fluorophenyl)acetamide;
   (D376) N-[4-(5-cyclopropyl-1,3,4-oxadiazol-2-yl)-3-sulfamoylphenyl]-2-(2-fluorophenyl)acetamide;
   (D377) 2-(2-chlorophenyl)-N-[4-(5-cyclopropyl-1,3,4-oxadiazol-2-yl)-3-sulfamoylphenyl]acetamide;
   (D378) N-{4-[4-(difluoromethyl)-1H-pyrazol-1-yl]-3-sulfamoylphenyl}-2-(2-fluorophenyl)acetamide;
   (D379) 2-(2-chlorophenyl)-N-{4-[4-(difluoromethyl)-1H-pyrazol-1-yl]-3-sulfamoylphenyl}acetamide;
   (D380) 2-(2-chlorophenyl)-N-{5-sulfamoyl-6-[4-(trifluoromethyl)-1H-pyrazol-1-yl]pyridin-3-yl}acetamide;
   (D381) 2-(2-fluorophenyl)-N-{5-sulfamoyl-6-[4-(trifluoromethyl)-1H-pyrazol-1-yl]pyridin-3-yl}acetamide;
   (D382) N-[6-(4-cyano-1H-pyrazol-1-yl)-5-sulfamoylpyridin-3-yl]-2-(2-fluorophenyl)acetamide;
   (D383) 2-(2-chlorophenyl)-N-[6-(4-cyano-1H-pyrazol-1-yl)-5-sulfamoylpyridin-3-yl]acetamide;
   (D384) 2-(2-fluorophenyl)-N-[6-(4-fluoro-1H-pyrazol-1-yl)-5-sulfamoylpyridin-3-yl]acetamide;
   (D385) 2-(2-chlorophenyl)-N-[6-(4-fluoro-1H-pyrazol-1-yl)-5-sulfamoylpyridin-3-yl]acetamide;
   (D386) N-[6-(4-bromo-1H-pyrazol-1-yl)-5-sulfamoylpyridin-3-yl]-2-(2-fluorophenyl)acetamide;
   (D387) 2-(2-chlorophenyl)-N-[6-(4-chloro-1H-pyrazol-1-yl)-5-sulfamoylpyridin-3-yl]acetamide;
   (D388) N-[6-(4-chloro-1H-pyrazol-1-yl)-5-sulfamoylpyridin-3-yl]-2-(2-fluorophenyl)acetamide;
   (D389) N-[6-(4-bromo-1H-pyrazol-1-yl)-5-sulfamoylpyridin-3-yl]-2-(2-chlorophenyl)acetamide;
   (D390) 2-(2-fluorophenyl)-N-{3-sulfamoyl-4-[5-(trifluoromethyl)-1,3,4-oxadiazol-2-yl]phenyl}acetamide;
   (D391) 2-(2-chlorophenyl)-N-{3-sulfamoyl-4-[5-(trifluoromethyl)-1,3,4-oxadiazol-2-yl]phenyl}acetamide;
   (D392) 2-(2-chlorophenyl)-N-{4-[3-(pyridin-3-yl)-1H-1,2,4-triazol-1-yl]-3-sulfamoylphenyl}acetamide;
   (D393) 2-(2-fluorophenyl)-N-[4-(1H-pyrazol-1-yl)-3-sulfamoylphenyl]acetamide;
   (D394) N-[4-(4-tert-butyl-1H-pyrazol-1-yl)-3-sulfamoylphenyl]-2-(2-chlorophenyl)acetamide;
   (D395) 2-(2-chlorophenyl)-N-[4-(1H-indazol-1-yl)-3-sulfamoylphenyl]acetamide;
   (D396) 2-(2-chlorophenyl)-N-[4-(2H-indazol-2-yl)-3-sulfamoylphenyl]acetamide;
   (D397) 2-(2-fluorophenyl)-N-[4-(2H-indazol-2-yl)-3-sulfamoylphenyl]acetamide;
   (D398) 2-(2-fluorophenyl)-N-[4-(1H-indazol-1-yl)-3-sulfamoylphenyl]acetamide;
   (D399) 1-(4-{[(2-chlorophenyl)acetyl]amino}-2-sulfamoylphenyl)-N,N-dimethyl-1H-pyrazol-4-carboxamide;
   (D400) N-[1-(4-{[(2-chlorophenyl)acetyl]amino}-2-sulfamoylphenyl)-1H-pyrazol-4-yl]cyclopropanecarboxamide;
   (D401) 2-(2-chlorophenyl)-N-{4-[4-(pyrazin-2-yl)-1H-pyrazol-1-yl]-3-sulfamoylphenyl}acetamide;
   (D402) 2-(2-chlorophenyl)-N-{4-[4-(pyridin-2-yl)-1H-pyrazol-1-yl]-3-sulfamoylphenyl}acetamide;
   (D403) 2-(2-chlorophenyl)-N-{4-[4-(pyridin-3-yl)-1H-pyrazol-1-yl]-3-sulfamoylphenyl}acetamide;
   (D404) 2-(2-chlorophenyl)-N-{4-[4-(pyrimidin-4-yl)-1H-pyrazol-1-yl]-3-sulfamoylphenyl}acetamide;
   (D405) 2-(2-fluorophenyl)-N-{4-[4-(pyrimidin-4-yl)-1H-pyrazol-1-yl]-3-sulfamoylphenyl}acetamide;
   (D406) 2-(4-methoxyphenyl)-N-{3-sulfamoyl-4-[4-(trifluoromethyl)-2H-1,2,3-triazol-2-yl]phenyl}acetamide;
   (D407) 2-(4-methoxyphenyl)-N-{3-sulfamoyl-4-[4-(trifluoromethyl)-1H-1,2,3-triazol-1-yl]phenyl}acetamide;
   (D408) 2-(4-methylphenyl)-N-{3-sulfamoyl-4-[4-(trifluoromethyl)-2H-1,2,3-triazol-(D)2-yl]phenyl}acetamide;
   (D409) 2-(4-methylphenyl)-N-{3-sulfamoyl-4-[4-(trifluoromethyl)-1H-1,2,3-triazol-1-yl]phenyl}acetamide;
   (D410) 2-[4-(difluoromethyl)phenyl]-N-{3-sulfamoyl-4-[4-(trifluoromethyl)-2H-1,2,3-triazol-2-yl]phenyl}acetamide;
   (D411) 2-[4-(difluoromethyl)phenyl]-N-{3-sulfamoyl-4-[4-(trifluoromethyl)-1H-1,2,3-triazol-1-yl]phenyl}acetamide;
   (D412) 2-(2-fluorophenyl)-N-{3-sulfamoyl-4-[4-(trifluoromethyl)-2H-1,2,3-triazol-2-yl]phenyl}acetamide;
   (D413) 2-(2-fluorophenyl)-N-{3-sulfamoyl-4-[4-(trifluoromethyl)-1H-1,2,3-triazol-1-yl]phenyl}acetamide;
   (D414) 2-[2-chloro-4-(trifluoromethyl)phenyl]-N-{3-sulfamoyl-4-[4-(trifluoromethyl)-2H-1,2,3-triazol-2-yl]phenyl}acetamide;
   (D415) 2-[2-chloro-4-(trifluoromethyl)phenyl]-N-{3-sulfamoyl-4-[4-(trifluoromethyl)-1H-1,2,3-triazol-1-yl]phenyl}acetamide;
   (D416) 2-(2,4-dichlorophenyl)-N-{3-sulfamoyl-4-[4-(trifluoromethyl)-2H-1,2,3-triazol-2-yl]phenyl}acetamide;
   (D417) 2-(2,4-dichlorophenyl)-N-{3-sulfamoyl-4-[4-(trifluoromethyl)-1H-1,2,3-triazol-1-yl]phenyl}acetamide;
   (D418) 2-(2-chlorophenyl)-N-[3-sulfamoyl-4-(1,3-thiazol-5-yl)phenyl]acetamide;
   (D419) 2-(2-chlorophenyl)-N-[3-sulfamoyl-4-(1,2-thiazol-3-yl)phenyl]acetamide;
   (D420) 2-(2-chlorophenyl)-N-[4-(2-methyl-1,3-thiazol-4-yl)-3-sulfamoylphenyl]acetamide;
   (D421) 2-(2-chlorophenyl)-N-[4-(2-methoxy-1,3-thiazol-4-yl)-3-sulfamoylphenyl]acetamide;
   (D422) 2-(2-chlorophenyl)-N-[4-(2-methoxy-1,3-thiazol-5-yl)-3-sulfamoylphenyl]acetamide;
   (D423) 2-(2-chlorophenyl)-N-[4-(2-methyl-1,3-thiazol-5-yl)-3-sulfamoylphenyl]acetamide;
   (D424) 2-(2-chlorophenyl)-N-[4-(3-methyl-1,2-thiazol-5-yl)-3-sulfamoylphenyl]acetamide;
   (D425) 2-(2-chlorophenyl)-N-[4-(4-methyl-1,3-thiazol-2-yl)-3-sulfamoylphenyl]acetamide;
   (D426) 2-(2-chlorophenyl)-N-[3-sulfamoyl-4-(1,2-thiazol-4-yl)phenyl]acetamide;
   (D427) 2-(2-chlorophenyl)-N-[3-sulfamoyl-4-(1,3-thiazol-2-yl)phenyl]acetamide;
   (D428) 2-(2-chlorophenyl)-N-[4-(4-cyano-1,3-thiazol-2-yl)-3-sulfamoylphenyl]acetamide;
   (D429) 2-(2-chlorophenyl)-N-{4-[2-(difluoromethyl)-1,3-thiazol-5-yl]-3-sulfamoylphenyl}acetamide;
   (D430) 2-(2-chlorophenyl)-N-[4-(2-cyclopropyl-1,3-thiazol-5-yl)-3-sulfamoylphenyl]acetamide;
   (D431) 2-(2-chlorophenyl)-N-[4-(2-cyclopropyl-1,3-thiazol-4-yl)-3-sulfamoylphenyl]acetamide;
   (D432) 2-(2-chlorophenyl)-N-[4-(4-methyl-1,3-oxazol-2-yl)-3-sulfamoylphenyl]acetamide;
   (D433) 2-(2-chlorophenyl)-N-{3-sulfamoyl-4-[2-(trifluoromethyl)-1,3-thiazol-4-yl]phenyl}acetamide;
   (D434) 2-(2-chlorophenyl)-N-{3-sulfamoyl-4-[4-(trifluoromethyl)-1,3-thiazol-2-yl]phenyl}acetamide;
   (D435) 2-(2-chlorophenyl)-N-{4-[2-(2-hydroxypropan-2-yl)-1,3-thiazol-5-yl]-3-sulfamoylphenyl}acetamide;
   (D436) 2-(2-chlorophenyl)-N-{4-[2-(2-hydroxypropan-2-yl)-1,3-thiazol-4-yl]-3-sulfamoylphenyl}acetamide;
   (D437) 2-(2-chlorophenyl)-N-[3-sulfamoyl-4-(1,3-thiazol-4-yl)phenyl]acetamide;
   (D438) 2-(2-chlorophenyl)-N-[4-(5-cyclopropyl-1,2-oxazol-3-yl)-3-sulfamoylphenyl]acetamide;
   (D439) 2-(2-chlorophenyl)-N-[4-(2-cyclopropyl-1,3-oxazol-5-yl)-3-sulfamoylphenyl]acetamide;
   (D440) 2-(2-chlorophenyl)-N-(4-{4-[(3,3-difluoroazetidin-1-yl)carbonyl]-1H-pyrazol-1-yl}-3-sulfamoylphenyl)acetamide;
   (D441) N-{4-[4-(azetidin-1-ylcarbonyl)-1H-pyrazol-1-yl]-3-sulfamoylphenyl}-2-(2-chlorophenyl)acetamide;
   (D442) 2-(2-chlorophenyl)-N-{4-[4-(pyrrolidin-1-ylcarbonyl)-1H-pyrazol-1-yl]-3-sulfamoylphenyl}acetamide;
   (D443) 2-(2-chlorophenyl)-N-(4-{4-[(3,3-difluoropyrrolidin-1-yl)carbonyl]-1H-pyrazol-1-yl}-3-sulfamoylphenyl) acetamide;
   (D444) 2-(4-chlorophenyl)-N-{3-sulfamoyl-4-[5-(trifluoromethyl)pyridin-3-yl]phenyl}acetamide;
   (D445) 2-(2-chloro-6-fluorophenyl)-N-{3-sulfamoyl-4-[5-(trifluoromethyl)pyridin-3-yl]phenyl}acetamide;
   (D446) N-{3-sulfamoyl-4-[5-(trifluoromethyl)pyridin-3-yl]phenyl}-2-[4-(trifluoromethyl)phenyl]acetamide;
   (D447) 2-(3-fluorophenyl)-N-{3-sulfamoyl-4-[5-(trifluoromethyl)pyridin-3-yl]phenyl}acetamide;
   (D448) 2-(2,4-dichlorophenyl)-N-{3-sulfamoyl-4-[5-(trifluoromethyl)pyridin-3-yl]phenyl}acetamide;
   (D449) 2-(2-bromophenyl)-N-{3-sulfamoyl-4-[5-(trifluoromethyl)pyridin-3-yl]phenyl}acetamide;
   (D450) 2-(2,4-difluorophenyl)-N-{3-sulfamoyl-4-[5-(trifluoromethyl)pyridin-3-yl]phenyl}acetamide;
   (D451) 2-(3,4-difluorophenyl)-N-{3-sulfamoyl-4-[5-(trifluoromethyl)pyridin-3-yl]phenyl}acetamide;
   (D452) 2-(3,5-difluorophenyl)-N-{3-sulfamoyl-4-[5-(trifluoromethyl)pyridin-3-yl]phenyl}acetamide;
   (D453) 2-(3-chlorophenyl)-N-{3-sulfamoyl-4-[5-(trifluoromethyl)pyridin-3-yl]phenyl}acetamide;
   (D454) 2-(4-methylphenyl)-N-{3-sulfamoyl-4-[5-(trifluoromethyl)pyridin-3-yl]phenyl}acetamide;
   (D455) 2-(4-methoxyphenyl)-N-{3-sulfamoyl-4-[5-(trifluoromethyl)pyridin-3-yl]phenyl}acetamide;
   (D456) 2-(2-fluoro-4-methylphenyl)-N-{3-sulfamoyl-4-[5-(trifluoromethyl)pyridin-3-yl]phenyl}acetamide;
   (D457) 2-(2-fluoro-4-methoxyphenyl)-N-{3-sulfamoyl-4-[5-(trifluoromethyl)pyridin-3-yl]phenyl}acetamide;
   (D458) 2-[4-(difluoromethyl)phenyl]-N-{3-sulfamoyl-4-[5-(trifluoromethyl)pyridin-3-yl]phenyl}acetamide;
   (D459) 2-(4-chlorophenyl)-N-[4-(6-chloropyridin-3-yl)-3-sulfamoylphenyl]acetamide;
   (D460) N-[4-(6-chloropyridin-3-yl)-3-sulfamoylphenyl]-2-(2-fluorophenyl)acetamide;
   (D461) 2-(2-chloro-6-fluorophenyl)-N-[4-(6-chloropyridin-3-yl)-3-sulfamoylphenyl]acetamide;
   (D462) N-[4-(6-chloropyridin-3-yl)-3-sulfamoylphenyl]-2-[4-(trifluoromethyl)phenyl]acetamide;
   (D463) N-[4-(6-chloropyridin-3-yl)-3-sulfamoylphenyl]-2-[2-(trifluoromethyl)phenyl]acetamide;
   (D464) N-[4-(6-chloropyridin-3-yl)-3-sulfamoylphenyl]-2-(3-fluorophenyl)acetamide;
   (D465) N-[4-(6-chloropyridin-3-yl)-3-sulfamoylphenyl]-2-(2,4-dichlorophenyl)acetamide;
   (D466) N-[4-(6-chloropyridin-3-yl)-3-sulfamoylphenyl]-2-(2,4-difluorophenyl)acetamide;
   (D467) 2-(2-bromophenyl)-N-[4-(6-chloropyridin-3-yl)-3-sulfamoylphenyl]acetamide;
   (D468) N-[4-(6-chloropyridin-3-yl)-3-sulfamoylphenyl]-2-(3,4-difluorophenyl)acetamide;
   (D469) N-[4-(6-chloropyridin-3-yl)-3-sulfamoylphenyl]-2-(4-methylphenyl)acetamide;
   (D470) N-[4-(6-chloropyridin-3-yl)-3-sulfamoylphenyl]-2-(4-methoxyphenyl)acetamide;
   (D471) N-[4-(6-chloropyridin-3-yl)-3-sulfamoylphenyl]-2-(2-fluoro-4-methylphenyl)acetamide;
   (D472) N-[4-(6-chloropyridin-3-yl)-3-sulfamoylphenyl]-2-(2-fluoro-4-methoxyphenyl)acetamide;
   (D473) N-[4-(6-chloropyridin-3-yl)-3-sulfamoylphenyl]-2-[4-(difluoromethyl)phenyl]acetamide;
   (D474) N-[4-(6-chloropyridin-3-yl)-3-sulfamoylphenyl]-2-[2-chloro-4-(trifluoromethyl)phenyl]acetamide;
   (D475) 2-(2,4-dichlorophenyl)-N-[4-(5-fluoropyridin-3-yl)-3-sulfamoylphenyl]acetamide;
   (D476) 2-(2-fluoro-4-methylphenyl)-N-[4-(5-fluoropyridin-3-yl)-3-sulfamoylphenyl]acetamide;
   (D477) 2-[4-(difluoromethyl)phenyl]-N-[4-(5-fluoropyridin-3-yl)-3-sulfamoylphenyl]acetamide;
   (D478) 2-(2-chlorophenyl)-N-[4-(4-cyclopropyl-1H-pyrazol-1-yl)-3-sulfamoylphenyl]acetamide;
   (D479) N-[4-(4,6-difluoro-2H-benzotriazol-2-yl)-3-sulfamoylphenyl]-2-(2-fluorophenyl)acetamide;
   (D480) N-[4-(4,6-difluoro-1H-benzotriazol-1-yl)-3-sulfamoylphenyl]-2-(2-fluorophenyl)acetamide;
   (D481) 2-(2-chlorophenyl)-N-[4-(4,6-difluoro-1H-benzotriazol-1-yl)-3-sulfamoylphenyl]acetamide;
   (D482) 2-(2-chlorophenyl)-N-[4-(4,6-difluoro-2H-benzotriazol-2-yl)-3-sulfamoylphenyl]acetamide;
   (D483) 2-(2-chlorophenyl)-N-{3-sulfamoyl-4-[2-(trifluoromethyl)-1,3-thiazol-5-yl]phenyl}acetamide;
   (D484) 2-(2-chlorophenyl)-N-[4-(4-cyano-1H-pyrazol-1-yl)-5-sulfamoyl-2-(trifluoromethyl)phenyl]acetamide;
   (D485) 2-[2-chloro-5-(trifluoromethyl)phenyl]-N-[4-(4-cyano-1H-pyrazol-1-yl)-3-sulfamoylphenyl]acetamide;
   (D486) 2-[2-chloro-5-(trifluoromethyl)phenyl]-N-[4-(4-cyano-1H-pyrazol-1-yl)-5-sulfamoyl-2-(trifluoromethyl)phenyl]acetamide;
   (D487) 2-(2-chlorophenyl)-N-[4-(4-cyano-1H-pyrazol-1-yl)-3-sulfamoyl-2-(trifluoromethyl)phenyl]acetamide;
   (D488) 2-[2-chloro-3-(trifluoromethyl)phenyl]-N-[4-(4-cyano-1H-pyrazol-1-yl)-5-sulfamoyl-2-(trifluoromethyl)phenyl]acetamide;
   (D489) 2-(2-chlorophenyl)-N-{4-[1-(difluoromethyl)-1H-pyrazol-4-yl]-3-sulfamoyl-2-(trifluoromethyl)phenyl}acetamide;
   (D490) 2-[2-chloro-3-(trifluoromethyl)phenyl]-N-{4-[1-(difluoromethyl)-1H-pyrazol-4-yl]-3-sulfamoylphenyl}acetamide;
   (D491) 2-(2-chlorophenyl)-N-{4-[1-(difluoromethyl)-1H-pyrazol-4-yl]-5-sulfamoyl-2-(trifluoromethyl)phenyl}acetamide;
   (D492) 2-(2-chlorophenyl)-N-{4-[1-(difluoromethyl)-5-(trifluoromethyl)-1H-pyrazol-4-yl]-3-sulfamoylphenyl}acetamide;
   (D493) 2-(2-chlorophenyl)-N-{4-[1-(difluoromethyl)-1H-pyrazol-4-yl]-2-fluoro-3-sulfamoylphenyl}acetamide;
   (D494) 2-(2-chlorophenyl)-N-{4-[1-(difluoromethyl)-1H-pyrazol-4-yl]-2-fluoro-5-sulfamoylphenyl}acetamide;
   (D495) 2-(2-chlorophenyl)-N-{4-[2-(dimethylamino)-1,3-thiazol-4-yl]-3-sulfamoylphenyl}acetamide;
   (D496) 2-(2-chlorophenyl)-N-[3-sulfamoyl-4-(1,2-thiazol-5-yl)phenyl]acetamide;
   (D497) 1-(4-{[(2-chlorophenyl)acetyl]amino}-2-sulfamoylphenyl)-N-cyclopropyl-N-methyl-1H-pyrazol-4-carboxamide;
   (D498) 2-(2-chlorophenyl)-2-hydroxy-N-{3-sulfamoyl-4-[4-(trifluoromethyl)-1H-pyrazol-1-yl]phenyl}ethanamide;
   (D499) 2-(2-chlorophenyl)-N-{3-chloro-5-sulfamoyl-4-[4-(trifluoromethyl)-1H-pyrazol-1-yl]phenyl}acetamide;
   (D500) 2-(2-chlorophenyl)-N-[4-(4-cyano-3-hydroxy-1H-pyrazol-1-yl)-3-sulfamoylphenyl]acetamide;
   (D501) 2-(2-chlorophenyl)-N-[4-(1H-pyrazolo[4,3-c]pyridin-1-yl)-3-sulfamoylphenyl]acetamide;
   (D502) 2-(2-chlorophenyl)-N-[4-(4,5-dimethyl-1,3-thiazol-2-yl)-3-sulfamoylphenyl]acetamide;
   (D503) 2-(2-chlorophenyl)-N-[4-(2,4-dimethyl-1,3-thiazol-5-yl)-3-sulfamoylphenyl]acetamide;
   (D504) 2-(2-chlorophenyl)-N-[4-(4-methyl-1,3-thiazol-5-yl)-3-sulfamoylphenyl]acetamide;
   (D505) N-{4-(4-amino-1H-pyrazol-1-yl)-3-[(2,4-dimethoxybenzyl)sulfamoyl]phenyl}-2-(2-chlorophenyl)acetamide;
   (D506) N-[1-(4-{[(2-chlorophenyl)acetyl]amino}-2-sulfamoylphenyl)-1H-pyrazol-4-yl]-2,2-difluoroacetamide;
   (D507) N-[1-(4-{[(2-chlorophenyl)acetyl]amino}-2-sulfamoylphenyl)-1H-pyrazol-4-yl]-3,3,3-trifluoropropanamide;
   (D508) N-[1-(4-{[(2-chlorophenyl)acetyl]amino}-2-sulfamoylphenyl)-1H-pyrazol-4-yl]-3,3,3-trifluoro-2-methylpropanamide (racemate);
   (D509) N-[1-(4-{[(2-chlorophenyl)acetyl]amino}-2-sulfamoylphenyl)-1H-pyrazol-4-yl]-3,3,3-trifluoro-2-methylpropanamide (enantiomer A);
   (D510) N-[1-(4-{[(2-chlorophenyl)acetyl]amino}-2-sulfamoylphenyl)-1H-pyrazol-4-yl]-3,3,3-trifluoro-2-methylpropanamide (enantiomer B);
   (D511) 2-(2-chlorophenyl)-N-(4-{4-[(cis)-2,5-dimethylpyrrolidin-1-yl]-1H-pyrazol-1-yl}-3-sulfamoylphenyl) acetamide;
   (D512) 2-(2-chlorophenyl)-N-(4-{4-[(trans)-2,5-dimethylpyrrolidin-1-yl]-1H-pyrazol-1-yl}-3-sulfamoylphenyl)acetamide (enantiomer A);
   (D513) 2-(2-chlorophenyl)-N-(4-{4-[(trans)-2,5-dimethylpyrrolidin-1-yl]-1H-pyrazol-1-yl}-3-sulfamoylphenyl)acetamide (enantiomer B);
   (D514) N-(4-{4-[(2,2-difluoroethyl)amino]-1H-pyrazol-1-yl}-3-sulfamoylphenyl)-2-(2-fluorophenyl)acetamide;
   (D515) 2-(2-chlorophenyl)-N-(3-sulfamoyl-4-{4-[(2,2,2-trifluoroethyl)amino]-1H-pyrazol-1-yl}phenyl)acetamide;
   (D516) 2-(2-chlorophenyl)-N-[4-(4-isopropyl-1H-pyrazol-1-yl)-3-sulfamoylphenyl]acetamide;
   (D517) 2-(2-fluorophenyl)-N-[4-(4-isopropyl-1H-pyrazol-1-yl)-3-sulfamoylphenyl]acetamide;
   (D518) 2-(2-chlorophenyl)-N-{3-sulfamoyl-4-[4-(2,2,2-trifluoroethyl)-1H-pyrazol-1-yl]phenyl}acetamide;
   (D519) 2-(2-fluorophenyl)-N-{3-sulfamoyl-4-[4-(2,2,2-trifluoroethyl)-1H-pyrazol-1-yl]phenyl}acetamide;
   (D520) 2-(2-chlorophenyl)-N-[4-(5-cyclopropyl-1,2,4-oxadiazol-3-yl)-3-sulfamoylphenyl]acetamide;
   (D521) 2-(2-chlorophenyl)-N-{3-sulfamoyl-4-[3-(trifluoromethyl)-1,2,4-oxadiazol-5-yl]phenyl}acetamide;
   (D522) 2-(2-chlorophenyl)-N-{4-[5-(difluoromethyl)-1,3,4-oxadiazol-2-yl]-3-sulfamoylphenyl}acetamide;
   (D523) N-{4-[5-(difluoromethyl)-1,3,4-oxadiazol-2-yl]-3-sulfamoylphenyl}-2-(2-fluorophenyl)acetamide;
   (D524) 2-(2-chlorophenyl)-N-[4-(5-methyl-1,3,4-oxadiazol-2-yl)-3-sulfamoylphenyl]acetamide;
   (D525) 2-(2-fluorophenyl)-N-[4-(5-methyl-1,3,4-oxadiazol-2-yl)-3-sulfamoylphenyl]acetamide;
   (D526) N-[4-(5-methyl-1,3,4-oxadiazol-2-yl)-3-sulfamoylphenyl]-2-(4-methylphenyl)acetamide;
   (D527) 2-(2-chlorophenyl)-N-[4-(1H-pyrrol-3-yl)-3-sulfamoylphenyl]acetamide;
   (D528) 2-(2-chlorophenyl)-N-{4-[5-(difluoroacetyl)-1H-pyrrol-3-yl]-3-sulfamoylphenyl}acetamide;
   (D529) 2-(2-chlorophenyl)-N-[4-(1-methyl-1H-pyrrol-3-yl)-3-sulfamoylphenyl]acetamide;
   (D530) 2-(2-chlorophenyl)-N-[4-(5-cyano-1-methyl-1H-pyrrol-2-yl)-3-sulfamoylphenyl]acetamide;
   (D531) 2-(2-chlorophenyl)-N-{3-sulfamoyl-2-(trifluoromethyl)-4-[4-(trifluoromethyl)-1H-pyrazol-1-yl]phenyl}acetamide;
   (D532) 2-(2-chlorophenyl)-N-{5-sulfamoyl-2-(trifluoromethyl)-4-[4-(trifluoromethyl)-1H-pyrazol-1-yl]phenyl}acetamide;
   (D533) 2-[2-chloro-3-(trifluoromethyl)phenyl]-N-{5-sulfamoyl-2-(trifluoromethyl)-4-[4-(trifluoromethyl)-1H-pyrazol-1-yl]phenyl}acetamide;
   (D534) 2-[2-chloro-5-(trifluoromethyl)phenyl]-N-{5-sulfamoyl-2-(trifluoromethyl)-4-[4-(trifluoromethyl)-1H-pyrazol-1-yl]phenyl}acetamide;
   (D535) 2-[2-chloro-6-(trifluoromethyl)phenyl]-N-[4-(4-fluoro-1H-pyrazol-1-yl)-5-sulfamoyl-2-(trifluoromethyl)phenyl]acetamide;
   (D536) 2-[2-chloro-3-(trifluoromethyl)phenyl]-N-[4-(4-fluoro-1H-pyrazol-1-yl)-5-sulfamoyl-2-(trifluoromethyl)phenyl]acetamide;
   (D537) 2-[2-chloro-5-(trifluoromethyl)phenyl]-N-[4-(4-fluoro-1H-pyrazol-1-yl)-5-sulfamoyl-2-(trifluoromethyl)phenyl]acetamide;
   (D538) 2-[2-chloro-4-(trifluoromethyl)phenyl]-N-[4-(4-fluoro-1H-pyrazol-1-yl)-5-sulfamoyl-2-(trifluoromethyl)phenyl]acetamide;
   (D539) N-[4-(3-tert-butyl-4-cyano-1H-pyrazol-1-yl)-3-sulfamoylphenyl]-2-(2-chlorophenyl)acetamide;
   (D540) N-[4-(3-bromo-4-cyano-1H-pyrazol-1-yl)-3-sulfamoylphenyl]-2-(2-chlorophenyl)acetamide;
   (D541) 2-(2-chlorophenyl)-N-{4-[4-chloro-3-(trifluoromethyl)-1H-pyrazol-1-yl]-3-sulfamoylphenyl}acetamide;
   (D542) 2-(2-chlorophenyl)-N-[4-(3,4-dimethyl-1H-pyrazol-1-yl)-3-sulfamoylphenyl]acetamide;
   (D543) N-[4-(4-chloro-3,5-dimethyl-1H-pyrazol-1-yl)-3-sulfamoylphenyl]-2-(2-chlorophenyl)acetamide;
   (D544) 2-(2-chlorophenyl)-N-[4-(1H-pyrazol-1-yl)-3-sulfamoylphenyl]acetamide;
   (D545) 2-(2-chlorophenyl)-N-[4-(3-cyano-5-methyl-1H-pyrazol-1-yl)-3-sulfamoylphenyl]acetamide;
   (D546) 2-(2-chlorophenyl)-N-[4-(3-hydroxy-5-methyl-1H-pyrazol-1-yl)-3-sulfamoylphenyl]acetamide;
   (D547) 2-(2-chlorophenyl)-N-[4-(4-cyano-5-methyl-1H-pyrazol-1-yl)-3-sulfamoylphenyl]acetamide;
   (D548) 2-(2-chlorophenyl)-N-[4-(4-cyano-3-methyl-1H-pyrazol-1-yl)-3-sulfamoylphenyl]acetamide;
   (D549) 2-(2-chlorophenyl)-N-{4-[4-(morpholin-4-yl)-1,3-thiazol-2-yl]-3-sulfamoylphenyl}acetamide;
   (D550) 2-(2-chlorophenyl)-N-{4-[5-(morpholin-4-yl)-1,3-thiazol-2-yl]-3-sulfamoylphenyl}acetamide;
   (D551) 2-(2-chlorophenyl)-N-[4-(5-methyl-1,3-thiazol-2-yl)-3-sulfamoylphenyl]acetamide;
   (D552) 2-(2-chlorophenyl)-N-[4-(pyridin-4-yl)-3-sulfamoylphenyl]acetamide;
   (D553) 1-(4-{[(2-chlorophenyl)acetyl]amino}-2-sulfamoylphenyl)-1H-pyrazol-4-carboxamide;
   (D554) 2-(2-chloro-3-hydroxyphenyl)-N-[4-(4-cyano-1H-pyrazol-1-yl)-3-sulfamoylphenyl]acetamide;
   (D555) 2-(2-chloro-4-hydroxyphenyl)-N-[4-(4-cyano-1H-pyrazol-1-yl)-3-sulfamoylphenyl]acetamide;
   (D556) 2-(2-chloro-5-hydroxyphenyl)-N-[4-(4-cyano-1H-pyrazol-1-yl)-3-sulfamoylphenyl]acetamide;
   (D557) 2-(2-chloro-6-hydroxyphenyl)-N-[4-(4-cyano-1H-pyrazol-1-yl)-3-sulfamoylphenyl]acetamide; and
   (D558) 2-(2-chlorophenyl)-N-[4-(4-cyano-1H-pyrazol-1-yl)-3-sulfamoylphenyl]-2-hydroxyacetamide.

### Advantageous Effects of Invention

The pharmaceutical composition of the present invention is useful as a pharmaceutical composition useful in preventing, suppressing, or treating a symptom associated with a pseudo-allergic reaction. In particular, the pharmaceutical composition of the present invention is useful as a pharmaceutical composition useful in preventing, suppressing, or treating a symptom associated with a pseudo-allergic reaction to a drug, the drug is not particularly limited as long as it is a compound that stimulates MrgprX2, and high efficacy is expected regardless of the degree of symptom associated with a pseudo-allergic reaction.

### Brief Description of Drawings

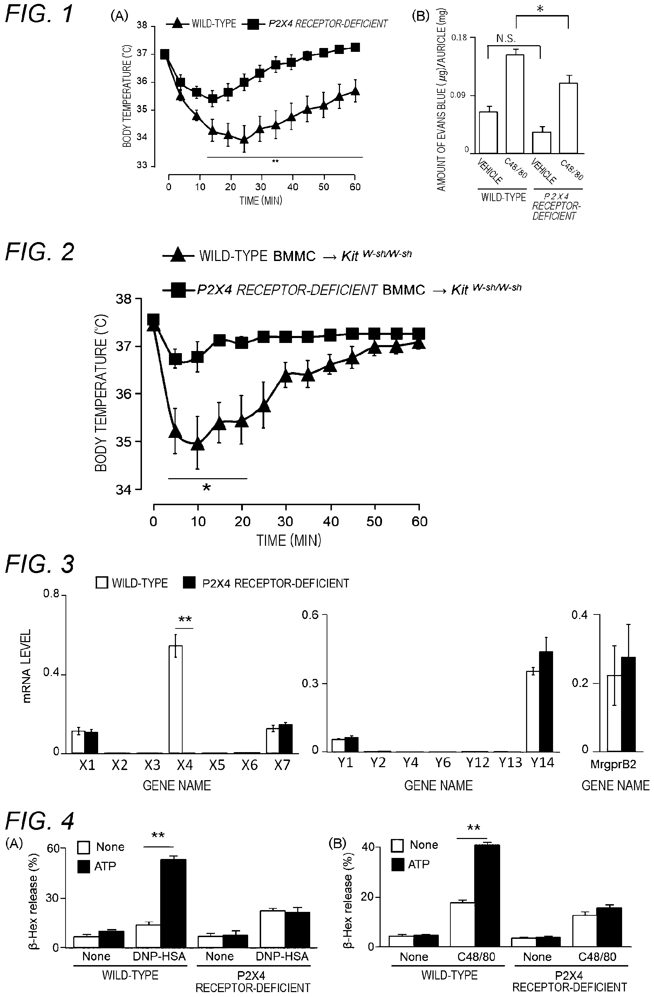

Fig. 1 shows a reduction of pseudo-allergy caused by P2X4 receptor deficiency.
   The reactions of (A) passive systemic anaphylaxis and (B) passive cutaneous anaphylaxis caused by administration of C48/80 were reduced by the deficiency of the P2X4 receptor gene. The data were mean ± SEM, and Student's t-test was performed (*: p < 0.05, **: p < 0.01, N.S.: not significant difference).
Fig. 2 shows involvement of mast cells in pseudo-allergy.
   Mast cells were reconstituted by transplanting BMMCs derived from wild-type mice or P2X4 receptor-deficient mice into mast cell-deficient mice (KitW-sh/W-sh). In mice reconstituted with mast cells from P2X4 receptor-deficient mice, pseudo-allergy caused by C48/80 was reduced (mean ± SEM). The data were mean ± SEM, and Student's t-test was performed (*: p < 0.05).
Fig. 3 shows gene expression of PMCs.
   Expression of a P2 receptor family (left: P2Xn receptor, center: P2Yn receptor) having a purine nucleotide derived from wild-type mice or P2X4 receptor-deficient mice as an endogenous ligand, and MrgprB2 gene was analyzed by a quantitative PCR method. The data were mean ± SEM, and Student's t-test was performed (**: p < 0.01).
Fig. 4 shows promotion of degranulation of PMCs by ATP.
   ATP promoted (A) IgE receptor stimulation, and (B) degranulation of PMCs by MrgprB2 stimulation with C48/80. The data were mean ± SEM, and Student's t-test was performed (**: p < 0.01). The promotion of degranulation by ATP was not observed in PMCs derived from P2X4 receptor-deficient mice.
Fig. 5 shows degranulation of PMCs by various MrgprB2 stimulators.
   ATP promoted degranulation of PMCs by (A) Substance P (Sub P) and PAMP-12 (PAMP), and (B) Vancomycin. The data were mean ± SEM, and Student's t-test was performed (**: p < 0.01). The promotion of degranulation by ATP was not observed in PMCs derived from P2X4 receptor-deficient mice.
Fig. 6 shows dose-dependency of allergen in degranulation of PMCs.
   Degranulation when PMCs derived from wild-type mice were stimulated with (A) DNP-HSA and (B) C48/80 at various concentrations in the presence and absence of ATP was measured. Note that, in (A), PMCs sensitized with IgE (SPE-7) in advance were used. The data were mean ± SEM, and Student's t-test was performed (*: p < 0.05, **: p < 0.01). ATP enhanced the sensitivity of the allergen of IgE receptor stimulation, whereas ATP enhanced the maximum response of MrgprB2 stimulation.

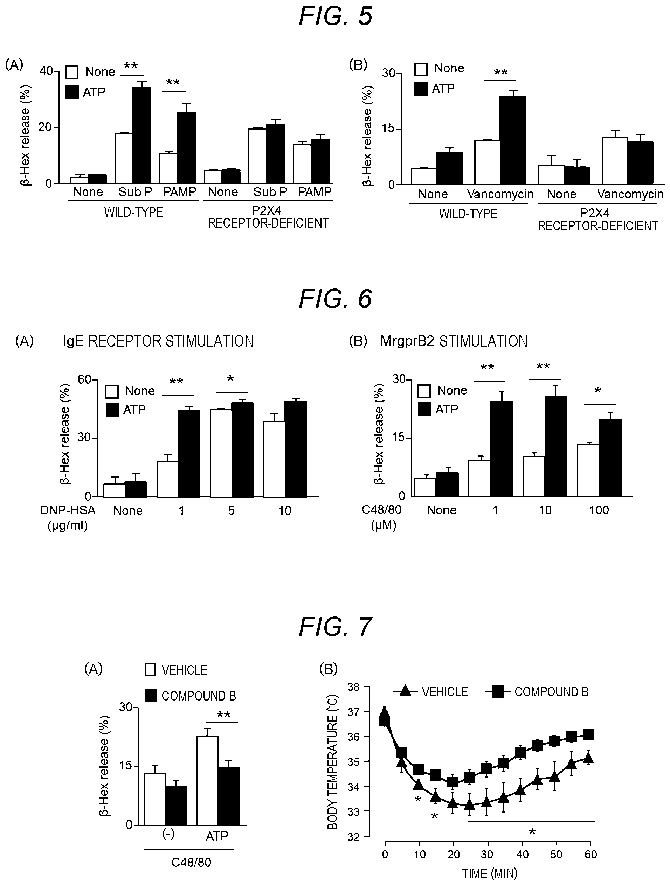

Fig. 7 shows treatments of pseudo-allergy with P2X4 receptor antagonists.
   Compound B, which inhibits a P2X4 receptor, suppressed (A) ATP-dependent degranulation of PMCs and (B) passive systemic anaphylaxis caused by C48/80. The data were mean ± SEM, and Student's t-test was performed (*: p < 0.05, **: p < 0.01).

### Description of Embodiments

The pharmaceutical composition of the present invention can be used as a pharmaceutical composition useful in preventing, suppressing, or treating a symptom associated with a pseudo-allergic reaction. In the present specification, the term "pseudo-allergic reaction" means an allergic reaction which is a biological reaction similar to a true allergy but is different from a true allergy and does not necessarily involve an IgE antibody. In other words, pseudo-allergy does not require IgE to induce an allergic reaction. Thus, pseudo-allergy may also be caused by a single exposure of an allergy-causing substance (allergen).

The symptom associated with the pseudo-allergic reaction is similar to that seen in the true allergy because it is caused by degranulation of mast cells and the like, and is a skin and mucous membrane symptom such as wheals, erythema punctatum, urticaria, angioedema, eyelid edema, bulbar chemosis, or pruritus, a digestive symptom such as pharyngeal and laryngeal edema, vomiting, abdominal pain, or diarrhea, a respiratory symptom such as wheezing or dyspnea, a cardiovascular symptom such as arrhythmia, paleness, a drop in body temperature, or a drop in blood pressure, or a central nervous system symptom such as headache or loss of consciousness. For example, flushing of the face, neck, or entire body observed when vancomycin is administered is called redman syndrome. The symptoms associated with the pseudo-allergic reaction include anaphylaxis in which these symptoms can occur suddenly across multiple organs and lead to life-threatening.

As another aspect of the present invention, the pharmaceutical composition of the present invention can be used for suppressing release of secretory granules within mast cells to the outside of the cells in a symptom associated with a pseudo-allergic reaction. The pharmaceutical composition of the present invention can be used in preventing, suppressing, or treating the symptom associated with a pseudo-allergic reaction which is accompanied by release of secretory granules within mast cells to the outside of the cells. Any of the symptoms associated with a pseudo-allergic reaction is a target for the application of the pharmaceutical composition of the present invention. The target for the application of the pharmaceutical composition of the present invention is not limited thereto.

Mast cells (also known as mastocytes (labrocytes)) are derived from bone marrow stem cells. Mast cells are part of the immune system and contain many granules rich in histamine and heparin. Although its role in allergy and anaphylaxis is most known, mast cells also play an important protective role and are closely involved in wound healing, including angiogenesis, and in protection against pathogens.

Mast cells are characteristically present in most tissues around blood vessels and nerves, and are particularly prominent at the boundary between the external and internal environments, such as in the skin, lung mucosa, and digestive tract, as well as near the mouth, conjunctiva, and nose. Mast cells in a living body may be classified into two types based on stainability and stimulation responsiveness. In rodents, mast cells are classified into connective tissue-type mast cells (CTMCs), which are distributed in the skin and abdominal cavity, and mucosal mast cells (MMCs), which are induced in the digestive tract during parasitic infection. CTMCs have heparinrich granules that stain positively for safranin and have a high histamine content, and MMCs have a low level of sulfation of proteoglycans and a low histamine content in granules.

Mast cells play a central role in the inflammatory process. Upon activation, mast cells rapidly release their characteristic granules and various hormone mediators into the interstitium. Mast cells can be stimulated to degranulate by direct damage (for example, physical or chemical (certain antibiotics such as opioids, alcohols, and polymyxins), crosslinking of immunoglobulin E (IgE) receptors, MrgprX2 agonists, or complement proteins.

In an allergic reaction or a pseudo-allergic reaction, mast cells remain inactive until the allergen binds to IgE or MrgprX2 that already coats the cells. Molecules released into the extracellular environment by allergen stimulation include pre-formed mediators (from granules) (for example, serine proteases such as tryptase, histamine (2 to 5 pg/cell), serotonin, proteoglycan, and heparin (active as an anticoagulant)), newly formed lipid mediators (eicosanoids) (that is, thromboxane, prostaglandin D2, leukotriene C4, and a platelet activating factor), and cytokines (for example, an eosinophil chemotactic factor).

Histamine dilates postcapillary venules, activates the endothelium, and increases vascular permeability. This leads to local edema (swelling), warmness, redness, and attraction of other inflammatory cells to the site of release. Histamine also depolarizes nerve endings (leading to pruritus or pain). Cutaneous manifestations of histamine release include "redness and wheal" reactions. Ridges and redness immediately after being bitten by a mosquito are preferred examples of these reactions, which occur a few seconds after loading of mast cells by the allergen.

In the present specification, the term "prevention" is a concept including preventing onset of a "diseased" or "abnormal" symptom, state, or disease before an outbreak thereof and an action or a method therefor.

In the present specification, the term "treatment" is a concept including eliminating, completely curing, healing, or remitting a "diseased" or "abnormal" symptom, state, or disease and an action or a method therefor, suppressing exacerbation of a "diseased" or "abnormal" symptom, state, or disease and an action or a method therefor, and improvement. Here, the term "improvement" is a concept including approach of a "diseased" or "abnormal" symptom, state, or disease to a "healthy" or "normal" state or an action or a method therefor and causing a "diseased" or "abnormal" symptom, state, or disease to be in a "healthy" or "normal" state or an action or a method therefor. Therefore, the term "improvement" in one embodiment includes a concept in which a numerical value which serves as an index of a "diseased" or "abnormal" symptom or state becomes small or large so as to approach a normal value or be the normal value in accordance with the "improvement". Furthermore, the term "suppression" is a concept including stopping or slowing down exacerbation or progression of a symptom, state, or disease and an action or a method therefor, and improving the symptom, state, or disease or an action or a method therefor. Here, the term "improvement" has the meaning described above. The expression "exacerbation or progression of symptom, state, or disease" includes exacerbation or progression of a "diseased" or "abnormal" symptom, state, or disease and exacerbation or progression from a "healthy" or "normal" state to a "diseased" or "abnormal" symptom, state, or disease. The term "suppression" in one embodiment is stopping or slowing down exacerbation or progression of a symptom, state, or disease or an action or a method therefor. The term "suppression" in another embodiment means stopping or slowing down exacerbation or progression of a symptom, state, or disease.

The term "treatment" in one embodiment means eliminating, completely curing, healing, or remitting a "diseased" or "abnormal" symptom, state, or disease and an action or a method therefor. The term "treatment" in another embodiment is eliminating, completely curing, healing, or remitting a "diseased" or "abnormal" symptom, state, or disease.

In the present specification, the symptom, state, or disease, or a numerical value, a state, or a function serving as indices of these can be evaluated by comparison of the indices before and after administration of the medicament provided by the present invention or by comparison of the indices between a group to which a placebo or a control that does not contain the medicament provided by the present invention is administered and a group to which the medicament provided by the present invention is administered.

The pseudo-allergy of the present invention is caused by substances having a cationic group, for example a cationic amphiphilic drug, and an endogenous or exogenous peptide consisting of a cationic head group and a hydrophobic core. Examples of the cationic group include various cyclic or chain functional groups having an amino group, an imino group, other primary to tertiary amine structures, and the like. The substance having the cationic group, which is an allergen of pseudo-allergy, may be induced as an endogenous substance (for example, substance P or PAMP-12), may be introduced into a living body as an exogenous substance (for example, mastoparan of wasp venom), or may be administered as a drug. Examples of the administration method of the drug include systemic administration and local administration, and include oral administration, sublingual administration, intravenous administration, intramuscular administration, subcutaneous administration, transdermal administration, intradermal administration, nasal administration, pulmonary administration, intra-airway administration, intraperitoneal administration, intrathecal administration, eye drops, and ear drops.

The pseudo-allergy of the present invention is caused by activation of MrgprX2. The mas-related G protein coupled receptor X2 (MrgprX2) is a mast cell specific receptor for substances having a cationic group, for example, a cationic amphiphilic drug and an endogenous or exogenous peptide consisting of a basic head group and a hydrophobic core. See McNeil B.D., 2015 Nature, 519:237-241, incorporated herein by reference. MrgprX2 recognizes and binds a small molecule containing a cyclized tetrahydroisoquinoline (THIQ), such as a non-steroidal neuromuscular blocker (NMBD), which includes tubocurarine and atracurium. In response to these compounds and the like, MrgprX2 mediates a pseudo-allergic reaction characterized by histamine release, inflammation, and airway constriction. As described in the present specification, MrgprX2 also acts as a receptor for peptides and alkaloids, such as cortistatin-14, pro-adrenomedulin N-terminal peptide (PAMP-12), and a few other ligands, including, to a lesser extent, PAMP-20, antimicrobial protein LL-37, PMX-53 peptide, beta-defensin, and complanadine A.

An exemplary human MrgprX2 amino acid sequence is known (NP_001290544.1 (GI:746816153), incorporated herein by reference (SEQ ID NO: 1)). An exemplary human MrgprX2 nucleic acid sequence is known (NM_001303615.1 (GI:746816152), incorporated herein by reference (SEQ ID NO:2)).

A mouse ortholog of human MrgprX2 is MrgprB2 (amino acid sequence: for example, NP_780740.2 (GI:229094244); nucleic acid sequence: for example, NM_175531.4 (GI:229094243)).

The allergy-causing substance (allergen) of the pseudo-allergy of the present invention is an MrgprX2 agonist. Whether an allergen of pseudo-allergy is an MrgprX2 agonist can be determined by a known method using, for example, recombinant cells expressing MrgprX2 (Patent Literature 1). When evaluation is performed using genetically modified HEK293 cells functionally expressing MrgprX2 (MrgprX2-HEK), EC50 as an MrgprX2 agonist of the allergen of the present invention is 50 uM or less, 10 uM or less, 5 uM or less, 1 uM or less, or 0.5 uM or less as a peptide, and 500 ug/mL or less, 100 ug/mL or less, 50 ug/mL or less, 10 ug/mL or less, 5 ug/mL or less, or 1 ug/mL or less as a compound.

The allergen of the pseudo-allergy of the present invention includes, but is not limited to, amorolfine hydrochloride, atracurium, beta-defensin, bleomycin, cetrorelix, C48/80 (monomer or cyclized monomer), ciprofloxacin, cisatracurium, codeine, colistimethate, complanadine A, cortistatin-14, degarelix, enfuvirtide, exenatide, fluoroquinolone, ganirelix, gentamicin sulfate, glatiramer acetate, glucagon, goserelin, histrelin, icatibant, ketoconazole, lanreotide, leuprolide, levofloxacin, liraglutide, LL-37, mafenide acetate, mastoparan, micronomicin sulfate, mivacurium, morphine, moxifloxacin, octreotide, ofloxacin, PAMP-12, PAMP-20, pasireotide, PMX-53, pramlintide, rocuronium, sermorelin, sisomicin sulfate, substance P, succinylcholine, sulfadoxine, sulfamethoxazole, terbinafine hydrochloride, teriparatide, tesamorelin, triptorelin, tubocurarine, and vancomycin.

A pharmaceutical composition for pseudo-allergy of the present invention includes a compound having a P2X4 receptor antagonizing action, or a pharmaceutically acceptable salt thereof, as an active ingredient.

### [Compound Having P2X4 Receptor Antagonizing Action or Pharmaceutically Acceptable Salt Thereof]

A compound capable of antagonizing and inhibiting the action of ATP on a subtype P2X4 receptor (Genebank No. X87763, incorporated herein by reference (SEQ ID NO: 3)) of a P2X family which is an ATP receptor, or a pharmaceutically acceptable salt thereof is meant. Whether or not there is an antagonizing action can be examined by a general method, for example, the method shown in Examples of the present application, and the compound having a P2X4 receptor antagonizing action of the present invention or the pharmaceutically acceptable salt thereof (in the present specification, also referred to as a "P2X4 receptor antagonist") has IC50 of 10 µM or less, 5 µM or less, or 1 µM or less. Examples of the pharmaceutically acceptable salt include an amine salt, an alkali metal salt (lithium, potassium, sodium, or the like), an alkaline earth metal salt (barium, calcium, magnesium, or the like), a transition metal salt (zinc or the like), a salt of a mineral acid (hydrochloride, sulfate, or the like), and a salt of an organic acid (acetate, lactate, malate, tartrate, citrate, ascorbate, succinate, butyrate, valerate, fumarate, organic sulfonate, or the like).

As an active ingredient of the pharmaceutical composition of the present invention, compounds represented by the following general formulas (A) to (D), or a pharmaceutically acceptable salt thereof, can be used.

Symbols used in the tables below and the like are as follows. Me: methyl group, Et: ethyl group, Pr: n-propyl group, iPr: isopropyl group, tBu: tert-butyl group, Ac: acetyl group, Ph: phenyl group

In the tables below and the like, a substituent may be marked together with a position number indicating a substitution position of the substituent. In addition, in order to distinguish position numbers of positions that appear to be identical to each other in a chemical formula, one position number may be indicated with a prime symbol "'" for convenience, however, as long as a definitive structure for a compound name can be specified, position numbers may be indicated without using the prime symbol.

(A-1) A compound represented by the following general formula (A) or a pharmaceutically acceptable salt thereof: 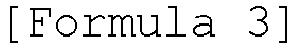 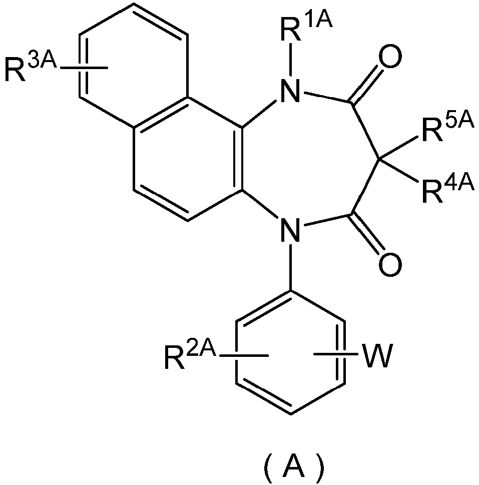 (In the formula R^{1A} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, or an alkyl group having 1 to 3 carbon atoms and substituted with a phenyl group;
R^{2A} and R^{3A} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, an acylamino group having 2 to 8 carbon atoms and substituted with 1 to 3 halogen atoms, an alkylsulfonylamino group having 1 to 8 carbon atoms, a carboxyl group, an acyl group having 2 to 8 carbon atoms, an alkoxycarbonyl group (the alkoxy moiety has 1 to 8 carbon atoms), a carbamoyl group, an alkylthio group having 1 to 8 carbon atoms, an alkylsulfinyl group having 1 to 8 carbon atoms, an alkylsulfonyl group having 1 to 8 carbon atoms, or a sulfamoyl group;
R^{4A} and R^{5A} may be the same or different, and represent a hydrogen atom, an alkyl group having 1 to 8 carbon atoms, an alkyl group having 1 to 8 carbon atoms and substituted with 1 to 3 halogen atoms, or an alkyl group having 1 to 3 carbon atoms and substituted with a phenyl group; and
W^{A} represents a five or six membered heterocyclic ring comprising 1 to 4 nitrogen atoms as the members of the ring, which may have a substituent)

In the general formula (A), examples of the alkyl group having 1 to 8 carbon atoms represented by R^{1A}, R^{2A}, R^{3A}, R^{4A}, and R^{5A} can comprise a methyl group, an ethyl group, a propyl group, an isopropyl group, a butyl group, an i-butyl group, a t-butyl group, a pentyl group, a hexyl group, and the like.

Examples of the alkenyl group having 2 to 8 carbon atoms represented by R^{1A} can comprise an allyl group and the like.

Examples of the alkyl group having 1 to 8 carbon atoms and substituted with 1 to 3 halogen atoms represented by R^{1A}, R^{2A}, R^{3A}, R^{4A}, and R^{5A} or R^{11A}, R^{12A}, R^{13A}, R^{14A}, and R^{15A} can comprise a methyl group, an ethyl group, a propyl group, an isopropyl group, a butyl group, a t-butyl group, and the like substituted with 1 to 3 halogen atoms such as a fluorine atom, a chlorine atom, and a bromine atom, and preferable examples thereof can comprise a trifluoromethyl group, a chloromethyl group, a 2-chloroethyl group, a 2-bromoethyl group, a 2-fluoroethyl group, and the like.

Examples of the alkyl group having 1 to 3 carbon atoms and substituted with a phenyl group represented by R^{1A}, R^{4A}, and R^{5A} can comprise a benzyl group and the like.

Examples of the alkoxy group having 1 to 8 carbon atoms represented by R^{2A} and R^{3A} can comprise a methoxy group, an ethoxy group, a propoxy group, an isopropoxy group, a butoxy group, an i-butoxy group, a t-butoxy group, a pentyloxy group, a hexyloxy group, and the like.

Examples of the alkoxy group having 1 to 8 carbon atoms and substituted with 1 to 3 halogen atoms represented by R^{2A} and R^{3A} can comprise a methyl group, an ethyl group, a propyl group, an isopropyl group, a butyl group, a t-butyl group, and the like substituted with 1 to 3 halogen atoms such as a fluorine atom, a chlorine atom, and a bromine atom, and preferable examples thereof can comprise a trifluoromethoxy group, a 2-chloroethoxy group, a 2-bromoethoxy group, a 2-fluoroethoxy group, and the like.

Examples of the halogen atom represented by R^{2A} and R^{3A} can comprise a fluorine atom, a chlorine atom, a bromine atom, and the like.

Examples of the alkylamino group having 1 to 8 carbon atoms represented by R^{2A} and R^{3A} can comprise a methylamino group, an ethylamino group, and the like.

Examples of the dialkylamino group having 1 to 8 carbon atoms represented by R^{2A} and R^{3A} can comprise a dimethylamino group, a diethylamino group, and the like.

Examples of the acylamino group having 2 to 8 carbon atoms represented by R^{2A} and R^{3A} can comprise an acetylamino group.

Examples of the acylamino group having 2 to 8 carbon atoms and substituted with 1 to 3 halogen atoms represented by R^{2A} and R^{3A} can comprise a trifluoromethylcarbonylamino group.

Examples of the alkylsulfonylamino group having 1 to 8 carbon atoms represented by R^{2A} and R^{3A} can comprise a methylsulfonylamino group.

Examples of the acyl group having 2 to 8 carbon atoms represented by R^{2A} and R^{3A} can comprise an acetyl group.

Examples of the alkoxycarbonyl group (the alkoxy moiety has 1 to 8 carbon atoms) represented by R^{2A} and R^{3A} can comprise a methoxycarbonyl group, an ethoxycarbonyl group, and the like.

Examples of the alkylthio group having 1 to 8 carbon atoms represented by R^{2A} and R^{3A} can comprise a methylthio group.

Examples of the alkylsulfinyl group having 1 to 8 carbon atoms represented by R^{2A} and R^{3A} can comprise a methylsulfinyl group.

Examples of the alkylsulfonyl group having 1 to 8 carbon atoms represented by R^{2A} and R^{3A} can comprise a methylsulfonyl group.

Examples of the five or six membered heterocyclic ring comprising 1 to 4 nitrogen atoms as the members of the ring, which may have a substituent, represented by W^{A} can comprise tetrazole, 1,2,4-triazole, 1,2,3-triazole, 1,2,4-oxadiazole, pyrazole, imidazole, oxazole, isoxazole, pyrrole, thiazole, pyridine, and pyrrolidine.

Examples of the substituent that may be comprised in the five or six membered heterocyclic ring comprising 1 to 4 nitrogen atoms as the members of the ring, which may have a substituent, represented by W^{A} can comprise an alkyl group having 1 to 8 carbon atoms such as a methyl group and an ethyl group, an alkyl group having 1 to 8 carbon atoms and substituted with 1 to 3 halogen atoms such as a trifluoromethyl group, a halogen atom such as a fluorine atom, a cyano group, an oxo group, a thioxo group, and the like.

R^{2A} and R^{3A} in the general formula (A) may have 1 to 3 same or different ones in the benzene ring substituted by R^{2A} and R3A .

As the compound represented by the general formula (A), the following compounds are preferable.
(A-2) The compound according to (A-1), in which W^{A} represents tetrazole, 1,2,4-triazole, 1,2,3-triazole, 1,2,4-oxadiazole, pyrazole, or imidazole that may have a substituent selected from an alkyl group having 1 to 8 carbon atoms, an alkyl group having 1 to 8 carbon atoms and substituted with 1 to 3 halogen atoms, a halogen atom, a cyano group, an oxo group, and a thioxo group.
(A-3) The compound according to (A-1) or (A-2), in which W^{A} represents tetrazole, 1,2,4-triazole, or 1,2,3-triazole that may have a substituent selected from an alkyl group having 1 to 8 carbon atoms, an alkyl group having 1 to 8 carbon atoms and substituted with 1 to 3 halogen atoms, a halogen atom, and a cyano group.
(A-4) The compound according to any one of (A-1) to (A-3), in which W^{A} represents 5-oxo-1,2,4-oxadiazole or 5-thioxo-1,2,4-oxadiazole.
(A-5) The compound according to any one of (A-1) to (A-4), in which W^{A} represents tetrazole.
(A-6) The compound according to any one of (A-1) to (A-5), in which R^{1A} represents a hydrogen atom or an alkyl group having 1 to 8 carbon atoms.
(A-7) The compound according to any one of (A-1) to (A-6), in which R^{1A} represents a hydrogen atom.
(A-8) The compound according to any one of (A-1) to (A-7), in which R^{4A} represents a hydrogen atom, and R^{5A} represents a hydrogen atom or an alkyl group having 1 to 8 carbon atoms.
(A-9) The compound according to any one of (A-1) to (A-8), in which R^{4A} and R^{5A} both represent hydrogen atoms.
(A-10) The compound according to any one of (A-1) to (A-9), in which R^{2A} represents a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, a carboxyl group, an acyl group having 2 to 8 carbon atoms, or an alkoxycarbonyl group (the alkoxy moiety has 1 to 8 carbon atoms).
(A-11) The compound according to any one of (A-1) to (A-10), in which R^{2A} represents a hydrogen atom.
(A-12) The compound according to any one of (A-1)(A-11), in which R^{3A} represents a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, a carboxyl group, an acyl group having 2 to 8 carbon atoms, or an alkoxycarbonyl group (the alkoxy moiety has 1 to 8 carbon atoms).
(A-13) The compound according to any one of (A-1) to (A-12), in which R^{3A} represents a hydrogen atom.

Examples of the pharmaceutically acceptable salt of the compound represented by the general formula (A) can comprise hydrochloride and the like, in a case where R^{2A} and R^{3A} in the general formula (A) represent amino groups or the like. In addition, in a case where R^{2A} and R^{3A} in the general formula (A) represent carboxyl groups, examples of the pharmaceutically acceptable salt of the compound represented by the general formula (A) can comprise a salt of an alkali metal such as sodium, potassium, and lithium.

Typical compounds comprised in the compounds represented by the general formula (A) are as follows.

### <Typical Compound A-100>

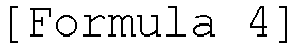

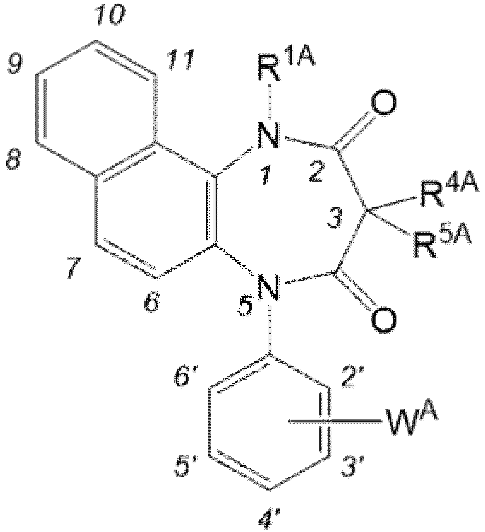

(In the formula R^{1A}, R^{4A}, R^{5A}, and W^{A} in the formula and a substitution position of W^{A} are indicated in Tables 1 to 3)

In Tables 1 to 3, the substitution position of W^{A} indicates a substitution position on a benzene ring. That is, positions 2, 3, and 4 in the tables correspond to positions 2', 3', and 4' in a formula of Typical Compound A-100, respectively.

**[Table 1]**

| R¹ | Position of W | W | R⁴/R⁵ |
|---|---|---|---|
| H | 2- | 1H-tetrazol-5-yl | H/H |
| H | 3- | 1H-tetrazol-5-yl | H/H |
| H | 3- | (1-methyl-1H-tetrazol)-5-yl | H/H |
| H | 4- | 1H-tetrazol-5-yl | H/H |
| CH₃ | 3- | 1H-tetrazol-5-yl | H/H |
| CH₃ | 3- | 1H-tetrazol-5-yl | CH₃/H |
| benzyl | 3- | 1H-tetrazol-5-yl | H/H |
| H | 3- | 1H-tetrazol-1-yl | H/H |
| H | 3- | 1H-tetrazol-1-yl | CH₃/CH₃ |
| H | 3- | (1,2,3-triazol)-5-yl | H/H |
| H | 3- | (1,2,4-triazol)-3-yl | H/H |
| H | 4- | (1,2,4-triazol)-3-yl | H/H |

**[Table 2]**

| R¹ | Position of W | W | R⁴/R⁵ |
|---|---|---|---|
| H | 2- | (1,2,4-triazol)-1-yl | H/H |
| H | 3- | (1,2,4-triazol)-1-yl | H/H |
| H | 3- | [5-(trifluoromethyl)-1,2,4-triazol]-3-yl | H/H |
| H | 3- | [5-(trifluoromethyl)-1,2,4-triazol]-3-yl | ethyl/H |
| H | 3- | [5-fluoro-1,2,3-triazol]-4-yl | H/H |
| H | 3- | [5-fluoro-1,2,3-triazol]-4-yl | CH₃/CH₃ |
| H | 3- | [5-cyano-1,2,3-triazol]-4-yl | H/H |
| H | 4- | 1H-imidazol-1-yl | H/H |
| H | 4- | 1H-imidazol-1-yl | Pr/H |
| H | 3- | 1H-imidazol-2-yl | H/H |
| H | 3- | 1H-imidazol-4-yl | H/H |
| H | 3- | imidazolin-2-yl | H/H |

**[Table 3]**

| R¹ | Position of W | W | R⁴/R⁵ |
|---|---|---|---|
| H | 2- | pyrazol-3-yl | H/H |
| H | 3- | pyrazol-4-yl | H/H |
| H | 3- | pyrazol-5-yl | CH₃/H |
| H | 3- | (1,2,4-oxadiazol)-3-yl | H/H |
| H | 3- | (1,3,4-oxadiazol)-2-yl | H/H |
| H | 3- | (5-oxo-1,2,4-oxadiazol)-3-yl | H/H |
| H | 3- | pyrrol-1-yl | H/H |
| H | 4- | pyrrolidin-2-yl | H/H |
| CH₃ | 4- | pyrrolidin-2-yl | CH₃/H |
| H | 4- | (1,3-oxazol)-5-yl | H/H |
| H | 3- | (1,3-oxazol)-5-yl | H/H |
| H | 2- | (1,3-thiazol)-5-yl | H/H |

### <Typical Compound A-200>

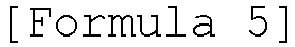

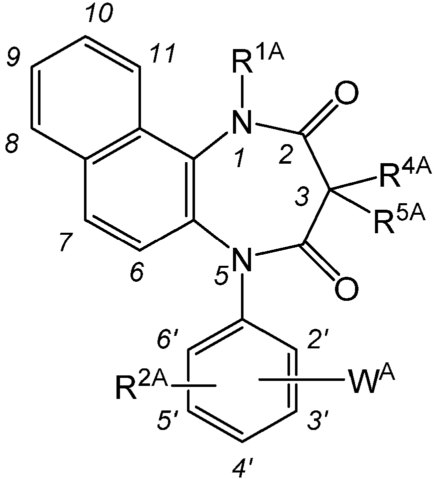

(In the formula R^{1A}, R^{2A}, R^{4A}, R^{5A}, and W^{A} in the formula and a substitution position of W^{A} are indicated in Tables 4 and 5)

In Tables 4 and 5, the substitution position of W^{A} indicates a substitution position on a benzene ring. That is, positions 2, 3, and 4 in the tables correspond to positions 2', 3', and 4' in a formula of Typical Compound A-200, respectively.

**[Table 4]**

| R¹ | R² | Position of W | W | R⁴/R⁵ |
|---|---|---|---|---|
| H | 4-OH | 3- | 1H-tetrazol-5-yl | H/H |
| H | 4-OCH₃ | 3- | 1H-tetrazol-5-yl | H/H |
| CH₃ | 2-Cl | 3- | 1H-tetrazol-5-yl | H/H |
| H | 2,6-Cl | 3- | 1H-tetrazol-5-yl | H/H |
| H | 4-F | 3- | 1H-tetrazol-5-yl | H/H |
| H | 4-Br | 3- | 1H-tetrazol-5-yl | ethyl/H |
| H | 3-OCH₃ | 4- | (1-methyl-1H-tetrazol)-5-yl | H/H |
| H | 4-CH₃ | 3- | 1H-tetrazol-5-yl | H/H |

**[Table 5]**

| R¹ | R² | Position of W | W | R⁴/R⁵ |
|---|---|---|---|---|
| H | 4-Cl | 3- | (1,2,3-triazol)-5-yl | CH₃/H |
| H | 4-CF₃ | 3- | (1,2,3-triazol)-5-yl | H/H |
| H | 3-SCH₃ | 4- | (1,2,4-triazol)-1-yl | H/H |
| H | 3-SO₂CH₃ | 4- | 1H-imidazol-1-yl | H/H |
| H | 3-NHSO₂CH₃ | 4- | 1H-imidazol-1-yl | H/H |
| H | 4-OCH₃ | 3- | 1H-imidazol-4-yl | H/H |
| H | 4-F | 2- | pyrazol-3-yl | H/H |

### <Typical Compound A-300>

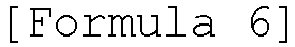

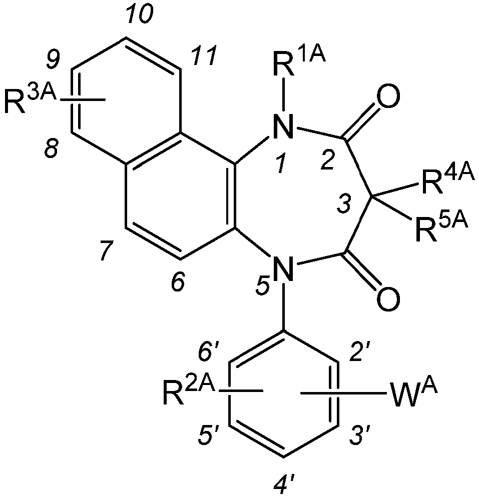

(In the formula R^{1A}, R^{2A}, R^{3A}, R^{4A}, R^{5A} and W^{A} in the formula and a substitution position of W^{A} are indicated in Tables 6 and 7)

In Tables 6 and 7, the substitution position of W^{A} indicates a substitution position on a benzene ring. That is, positions 3 and 4 in the tables correspond to positions 3' and 4' in a formula of Typical Compound A-300, respectively.

**[Table 6]**

| R¹ | R² | Position of W | W | R³ | R⁴/R⁵ |
|---|---|---|---|---|---|
| H | H | 3- | 1H-tetrazol-5-yl | 9-Br | H/H |
| H | 4-OCH₃ | 3- | 1H-tetrazol-5-yl | 9-Cl | H/H |
| H | 4-OH | 3- | 1H-tetrazol-5-yl | 10-OCH₃ | H/H |
| H | 2-Cl | 3- | 1H-tetrazol-5-yl | 9-Br | H/H |
| H | 2,6-Cl | 3- | 1H-tetrazol-5-yl | 9-CH₃ | H/H |
| H | H | 3- | 1H-tetrazol-5-yl | 10-Cl | CH₃/H |
| H | 3-OCH₃ | 4- | (1-methyl-1H-tetrazol)-5-yl | 9-CF₃ | H/H |

**[Table 7]**

| R¹ | R² | Position of W | W | R³ | R⁴/R⁵ |
|---|---|---|---|---|---|
| H | 4-CH₃ | 3- | 1H-tetrazol-1-yl | 9-CN | Pr/H |
| CH₃ | H | 3- | (1,2,3-triazol)-5-yl | 9-OH | H/H |
| ethyl | H | 3- | (1,2,3-triazol)-5-yl | 10-F | H/H |
| H | 3-Br | 4- | (1,2,4-triazol)-1-yl | 9-SCH₃ | H/H |
| allyl | H | 4- | 1H-imidazol-1-yl | 8-OCH₃ | H/H |
| H | H | 3- | 1H-imidazol-1-yl | 10-OCH₃ | CH₃/CH₃ |

(B-1) A compound represented by the following general formula (BI) or a pharmaceutically acceptable salt thereof: 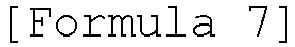 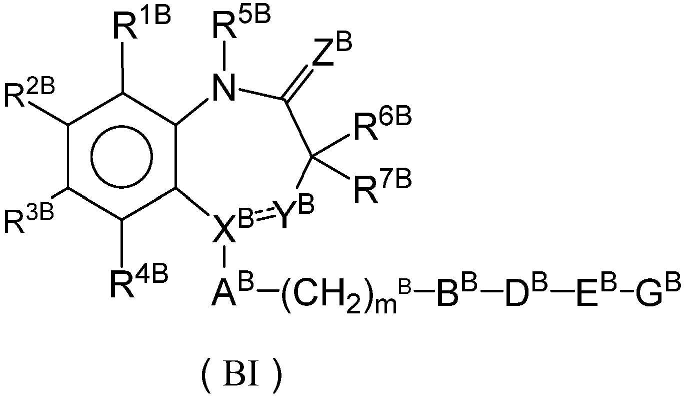 (In the formula R^{1B} and R^{2B} may be the same or different, and represent a hydrogen atom, an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), a phenyl group which may be substituted, a pyridyl group which may be substituted, or an aralkyl group (the aryl moiety has 6 to 10 carbon atoms, and the alkylene moiety has 1 to 8 carbon atoms), or
R^{1B} and R^{2B} may bind together to form a condensed ring selected from a naphthalene ring, a quinoline ring, an isoquinoline ring, a tetrahydronaphthalene ring, an indane ring, a tetrahydroquinoline ring, and a tetrahydroisoquinoline ring together with the benzene ring to which they bind, and the ring constituted by R^{1B} and R^{2B} bound to each other, together with the carbon atoms to which R^{1B} and R^{2B} bind may be substituted with 1 to 4 of the same or different substituents selected from an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),
R^{3B} and R^{4B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), or an aralkyl group (the aryl moiety has 6 to 10 carbon atoms, and the alkylene moiety has 1 to 8 carbon atoms),
R^{5B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
R^{6B} and R^{7B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, or an amino group,
X^{B} represents C, CH, or N,
Y^{B} represents N, NH, or C(=O),
provided that when X^{B} is N, Y^{B} is not N or NH, and
when X^{B} is C or CH, Y^{B} is not C(=O),
the double line consisting of the solid line and the broken line represents a single bond or a double bond,
Z^{B} represents an oxygen atom or a sulfur atom,
A^{B} represents a benzene ring, a pyridine ring, a thiophene ring, a pyrimidine ring, a naphthalene ring, a quinoline ring, or an indole ring, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group, as a substituent, or represents an atomic bond,
B^{B} represents N(R^{8B})C(=O), NHCONH, CON(R^{9B}), NHC (=S) NH, N(R^{10B})SO₂, SO₂N(R^{11B}), or OSO₂, in the formula
R^{8B}, R^{9B}, R^{10B}, and R^{11B} represent a hydrogen atom, an alkyl group having 1 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
D^{B} represents an alkylene chain having 1 to 6 carbon atoms, which may have 1 to 4 of the same or different substituents selected from an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, and an aralkyl group (the aryl moiety has 6 to 10 carbon atoms, and the alkylene moiety has 1 to 8 carbon atoms), as a substituent, and may further have a double bond, or represents an atomic bond,
E^{B} represents O, S, NR^{12B}, or an atomic bond, in the formula
R^{12B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
G^{B} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (the aryl moiety has 6 to 10 carbon atoms, and the alkylene moiety has 1 to 8 carbon atoms), a phenyl group which may be substituted, a pyridyl group which may be substituted, an imidazolyl group which may be substituted, an oxazolyl group which may be substituted, and a thiazolyl group which may be substituted, as a substituent, and
m^{B} represents an integer of 0 to 5,
provided that when R^{1B} and R^{2B} do not bind together to form a ring, those compounds are excluded in the formula, X^{B} is C, Y^{B} is N, the double line consisting of the solid line and the broken line is a double bond, Z^{B} is an oxygen atom, A^{B} is a benzene ring, m^{B} is 0, B^{B} is C(=O)NH, E^{B} is an atomic bond, and G^{B} is a phenyl group).

(B-2) A compound represented by the following general formula (BII) or a pharmaceutically acceptable salt thereof: 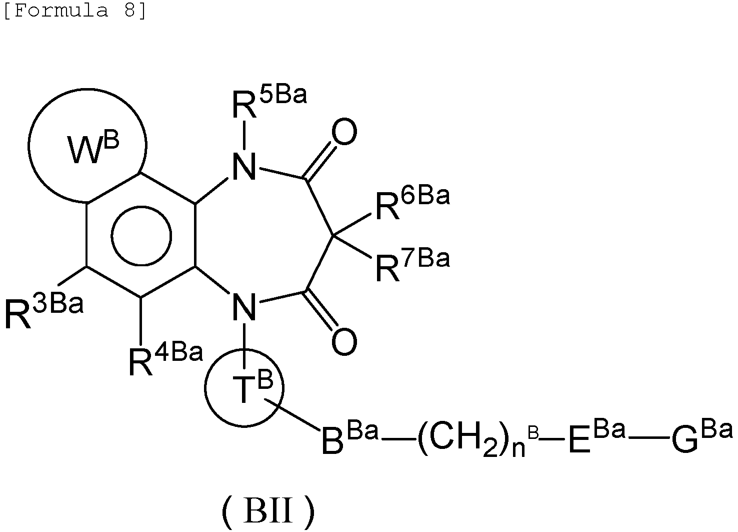 (In the formula 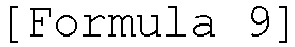 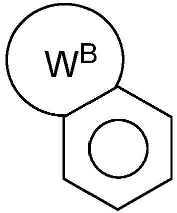
represents a naphthalene ring, a quinoline ring, an isoquinoline ring, a tetrahydronaphthalene ring, an indane ring, a tetrahydroquinoline ring, or a tetrahydroisoquinoline ring, and
these rings may be substituted with 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),
R^{3Ba} and R^{4Ba} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), or an aralkyl group (the aryl moiety has 6 to 10 carbon atoms, and the alkylene moiety has 1 to 8 carbon atoms),
R^{5Ba} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
R^{6Ba} and R^{7Ba} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, or an amino group, 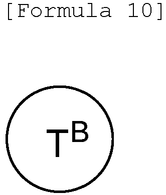
represents a benzene ring, a pyridine ring, a thiophene ring, a pyrimidine ring, a naphthalene ring, a quinoline ring, or an indole ring, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group, as a substituent,
B^{Ba} represents N(R^{8Ba})C(=O), NHCONH, CON(R^{9Ba}), NHC(=S)NH, N(R^{10Ba})SO₂, SO₂N(R^{11Ba}), or OSO₂, in the formula
R^{8Ba}, R^{9Ba}, R^{10Ba}, and R^{11Ba} represent a hydrogen atom, an alkyl group having 1 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
E^{Ba} represents O, S, NR^{12Ba}, or an atomic bond, in the formula
R^{12Ba} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
G^{Ba} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (the aryl moiety has 6 to 10 carbon atoms, and the alkylene moiety has 1 to 8 carbon atoms), a phenyl group which may be substituted, a pyridyl group which may be substituted, an imidazolyl group which may be substituted, an oxazolyl group which may be substituted, and a thiazolyl group which may be substituted, as a substituent, and
n^{B} represents an integer of 0 to 5).

Next, the substituents in the general formulas (BI) and (BII) of the present specification is described.

Examples of the alkyl group having 1 to 8 carbon atoms can comprise a methyl group, an ethyl group, a propyl group, an isopropyl group, a butyl group, an i-butyl group, a t-butyl group, a pentyl group, a hexyl group, and the like.

Examples of the cycloalkyl group having carbon atoms 3 to 8 can comprise a cyclopropyl group, a cyclohexyl group, and the like.

Examples of the alkenyl group having 2 to 8 carbon atoms can comprise an allyl group, and the like.

Examples of the alkoxy group having 1 to 8 carbon atoms can comprise a methoxy group, an ethoxy group, a propoxy group, an isopropoxy group, a butoxy group, an i-butoxy group, a t-butoxy group, a pentyloxy group, a hexyloxy group, and the like.

Examples of the alkyl group having 1 to 8 carbon atoms and substituted with 1 to 3 halogen atoms can comprise a methyl group, an ethyl group, a propyl group, an isopropyl group, a butyl group, a t-butyl group, and the like substituted with 1 to 3 halogen atoms such as a fluorine atom, a chlorine atom, and a bromine atom, and preferable examples thereof can comprise a trifluoromethyl group, a chloromethyl group, a 2-chloroethyl group, a 2-bromoethyl group, a 2-fluoroethyl group, and the like.

Examples of the alkoxy group having 1 to 8 carbon atoms substituted with 1 to 3 halogen atoms can comprise a methoxy group, an ethoxy group, a propoxy group, an isopropoxy group, a butoxy group, a t-butoxy group, and the like substituted with 1 to 3 halogen atoms such as a fluorine atom, a chlorine atom, and a bromine atom, and preferable examples thereof can comprise a trifluoromethoxy group, a chloromethoxy group, a 2-chloroethoxy group, a 2-bromoethoxy group, a 2-fluoroethoxy group, and the like.

Examples of the halogen atom can comprise a fluorine atom, a chlorine atom, a bromine atom, and the like.

Examples of the alkylamino group having 1 to 8 carbon atoms can comprise a methylamino group, an ethylamino group, and the like.

Examples of the dialkylamino group having 2 to 8 carbon atoms can comprise a dimethylamino group, a diethylamino group, and the like.

Examples of the acylamino group having 2 to 8 carbon atoms can comprise an acetylamino group and the like.

Examples of the acyl group having 2 to 8 carbon atoms can comprise an acetyl group and the like.

Examples of the alkoxycarbonyl group (the alkoxy moiety has 1 to 8 carbon atoms) can comprise a methoxycarbonyl group and the like.

Examples of the aralkyl group (the aryl moiety has 6 to 10 carbon atoms, and the alkylene moiety has 1 to 8 carbon atoms) can comprise a benzyl group and the like.

Examples of the alkyl group having 1 to 8 carbon atoms and substituted with a hydroxyl group can comprise a 2-hydroxyethyl group and the like.

Examples of the alkylsulfinyl group having 1 to 6 carbon atoms can comprise a methanesulfinyl group and the like.

Examples of the alkylthio group having 1 to 6 carbon atoms can comprise a methylthio group and the like.

Examples of the alkylsulfonyl group having 1 to 6 carbon atoms can comprise a methanesulfonyl group and the like.

Examples of the substituent that may be comprised in the phenyl group which may be substituted, the pyridyl group which may be substituted, the imidazolyl group which may be substituted, the oxazolyl group which may be substituted, and the thiazolyl group which may be substituted can comprise a halogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, and the like.

As the compound represented by the general formula (BI), the following compounds are preferable.

### (B-1-1)

The compound according to (B-1), in which R^{1B} and R^{2B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, a phenyl group which may be substituted, a pyridyl group which may be substituted, or an aralkyl group (the aryl moiety has 6 to 10 carbon atoms, and the alkylene moiety has 1 to 8 carbon atoms).

### (B-1-2)

The compound according to (B-1) or (B-1-1), in which R^{1B} and R^{2B} bind together to form a naphthalene ring or a tetrahydronaphthalene ring together with the benzene ring to which they bind, and the benzene ring or the cyclohexene ring constituted by R^{1B} and R^{2B}, bound to each other, together with the carbon atoms to which R^{1B} and R^{2B} bind may be substituted with 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),

### (B-1-3)

The compound according to (B-1) or (B-1-1), in which R^{1B} and R^{2B} bind together to form a naphthalene ring together with the benzene ring to which they bind, and the benzene ring constituted by R^{1B} and R^{2B}, bound to each other, together with the carbon atoms to which R^{1B} and R^{2B} bind may be substituted with 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, and an amino group.

### (B-1-4)

The compound according to (B-1) or (B-1-1), in which R^{1B} and R^{2B} bind together to form a naphthalene ring or a tetrahydronaphthalene ring together with the benzene ring to which they bind.

### (B-1-5)

The compound according to any one of (B-1) and (B-1-1) to (B-1-4), in which R^{3B} and R^{4B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, or an aralkyl group (the aryl moiety has 6 to 10 carbon atoms, and the alkylene moiety has 1 to 8 carbon atoms).

### (B-1-6)

The compound according to any one of (B-1) and (B-1-1) to (B-1-5), in which R^{5B} represents a hydrogen atom, an alkyl group having 1 to 8 carbon atoms, or an aralkyl group (the aryl moiety has 6 to 10 carbon atoms, and the alkylene moiety has 1 to 8 carbon atoms).

### (B-1-7)

The compound according to any one of (B-1) and (B-1-1) to (B-1-6), in which R^{5B} represents a hydrogen atom.

### (B-1-8)

The compound according to any one of (B-1) and (B-1-1) to (B-1-7), in which R^{6B} and R^{7B} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, or an alkoxy group having 1 to 8 carbon atoms and substituted with 1 to 3 halogen atoms, or a pharmaceutically acceptable salt thereof.

### (B-1-9)

The compound according to any one of (B-1) and (B-1-1) to (B-1-8), in which R^{6B} and R^{7B} both represent hydrogen atoms.

### (B-1-10)

The compound according to any one of (B-1) and (B-1-1) to (B-1-9), in which R^{3B}, R^{4B}, R^{5B}, R^{6B}, and R^{7B} are hydrogen atoms.

### (B-1-11)

The compound according to any one of (B-1) and (B-1-1) to (B-1-10), in which X^{B} represents N, Y^{B} represents C(=O), and the double line consisting of the solid line and the broken line represents a single bond.

### (B-1-12)

The compound according to any one of (B-1) and (B-1-1) to (B-1-11), in which X^{B} represents C, Y^{B} represents N, and the double line consisting of the solid line and the broken line represents a double bond.

### (B-1-13)

The compound according to any one of (B-1) and (B-1-1) to (B-1-12), in which Z^{B} represents an oxygen atom.

### (B-1-14)

The compound according to any one of (B-1) and (B-1-1) to (B-1-13), in which A^{B} represents a phenyl group or a pyridyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group as a substituent.

### (B-1-15)

The compound according to any one of (B-1) and (B-1-1) to (B-1-14), in which A^{B} represents a phenyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, and an amino group as a substituent.

### (B-1-16)

The compound according to any one of (B-1) and (B-1-1) to (B-1-15), in which A^{B} represents a phenyl group or a pyridyl group.

### (B-1-17)

The compound according to any one of (B-1) and (B-1-1) to (B-1-16), in which A^{B} represents an atomic bond.

### (B-1-18)

The compound according to any one of (B-1) and (B-1-1) to (B-1-17), in which B^{B} represents NHC(=O), NHCONH, CONH, NHC (=S) NH, NHSO₂, SO₂NH, or OSO₂.

### (B-1-19)

The compound according to any one of (B-1) and (B-1-1) to (B-1-18), in which B^{B} represents NHC(=O), NHCONH, or NHSO₂.

### (B-1-20)

The compound according to any one of (B-1) and (B-1-1) to (B-1-19), in which B^{B} represents NHC(=O) or NHSO₂.

### (B-1-21)

The compound according to any one of (B-1) and (B-1-1) to (B-1-20), in which B^{B} represents NHC(=O).

### (B-1-22)

The compound according to any one of (B-1) and (B-1-1) to (B-1-21), in which D^{B} represents an alkylene chain having 1 to 6 carbon atoms that may have 1 to 4 of the same or different substituents selected from an alkyl group having 1 to 8 carbon atoms and an alkyl group having 1 to 8 carbon atoms and substituted with 1 to 3 halogen atoms as a substituent, and may further have a double bond.

### (B-1-23)

The compound according to any one of (B-1) and (B-1-1) to (B-1-22), in which D^{B} represents an atomic bond.

### (B-1-24)

The compound according to any one of (B-1) and (B-1-1) to (B-1-23), in which D^{B} has 1 to 4 of the same or different substituents selected from an alkyl group having 1 to 8 carbon atoms and an alkenyl group having 2 to 8 carbon atoms as a substituent.

### (B-1-25)

The compound according to any one of (B-1) and (B-1-1) to (B-1-24), in which D^{B} has 1 to 4 of the same or different substituents selected from an alkyl group having 1 to 3 carbon atoms and an alkenyl group having 2 or 3 carbon atoms as a substituent.

### (B-1-26)

The compound according to any one of (B-1) and (B-1-1) to (B-1-25), in which E^{B} represents an atomic bond.

### (B-1-27)

The compound according to any one of (B-1) and (B-1-1) to (B-1-26), in which G^{B} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-1-28)

The compound according to any one of (B-1) and (B-1-1) to (B-1-27), in which G^{B} represents benzene that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-1-29)

The compound according to any one of (B-1) and (B-1-1) to (B-1-28), in which G^{B} represents benzene or pyridine that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, a halogen atom, a hydroxyl group, a nitro group, an amino group, a dialkylamino group having 2 to 8 carbon atoms, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-1-30)

The compound according to any one of (B-1) and (B-1-1) to (B-1-29), in which G^{B} represents benzene that may have 1 to 4 of the same or different substituents selected from an alkyl group having 1 to 8 carbon atoms and substituted with 1 to 3 halogen atoms, a halogen atom, and a hydroxyl group as a substituent.

### (B-1-31)

The compound according to any one of (B-1) and (B-1-1) to (B-1-30), in which m^{B} represents 0.

### (B-1-32)

The compound according to any one of (B-1) and (B-1-1) to (B-1-31), in which A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene that may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

### (B-1-33)

The compound according to any one of (B-1) and (B-1-1) to (B-1-32), in which A^{B} represents a benzene ring, m^{B} represents 0, B^{B} represents NHC(=O), D^{B} represents an atomic bond, E^{B} represents an atomic bond, and G^{B} represents benzene that may have 1 to 4 of the same or different substituents selected from an alkyl group having 1 to 8 carbon atoms and substituted with 1 to 3 halogen atoms, a halogen atom, and a hydroxyl group as a substituent.

### (B-1-34)

The compound according to any one of (B-1) and (B-1-1) to (B-1-33), in which R^{1B} and R^{2B} bind together to form a naphthalene ring together with the benzene ring to which they bind, R^{3B}, R^{4B}, R^{5B}, R^{6B}, and R^{7B} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene that may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

### (B-1-35)

The compound according to any one of (B-1) and (B-1-1) to (B-1-34), in which, in the general formula (BI), R^{1B} and R^{2B} bind together to form a naphthalene ring together with the benzene ring to which they bind, R^{3B}, R^{4B}, R^{5B}, R^{6B}, and R^{7B} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O), D^{B} represents an atomic bond, E^{B} represents an atomic bond, and G^{B} represents benzene that may have 1 to 4 of the same or different substituents selected from an alkyl group having 1 to 8 carbon atoms and substituted with 1 to 3 halogen atoms, a halogen atom, and a hydroxyl group as a substituent.

As the compound represented the general formula (BII), the following compounds are preferable.

### (B-2-1)

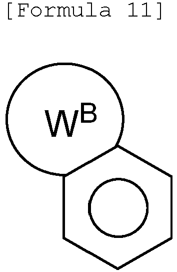

The compound according to (B-2) in which the above moiety represents a naphthalene ring or a tetrahydronaphthalene ring that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms) as a substituent.

### (B-2-2)

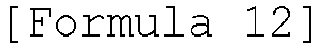

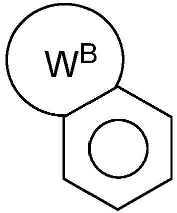

The compound according to (B-2) or (B-2-1) in which the above moiety represents a naphthalene ring that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, and an amino group as a substituent.

### (B-2-3)

The compound according to any one of (B-2), (B-2-1), and (B-2-2) in which R^{3Ba} and R^{4Ba} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, or an aralkyl group (the aryl moiety has 6 to 10 carbon atoms, and the alkylene moiety has 1 to 8 carbon atoms).

### (B-2-4)

The compound according to any one of (B-2) and (B-2-1) to (B-2-3), in which R^{5Ba} represents a hydrogen atom, an alkyl group having 1 to 8 carbon atoms, or an aralkyl group (the aryl moiety has 6 to 10 carbon atoms, and the alkylene moiety has 1 to 8 carbon atoms).

### (B-2-5)

The compound according to any one of (B-2) and (B-2-1) to (B-2-4), in which R^{5Ba} represents a hydrogen atom.

### (B-2-6)

The compound according to any one of (B-2) and (B-2-1) to (B-2-5), in which R^{6Ba} and R^{7Ba} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, or an alkoxy group having 1 to 8 carbon atoms and substituted with 1 to 3 halogen atoms.

### (B-2-7)

The compound according to any one of (B-2) and (B-2-1) to (B-2-6), in which R^{6Ba} and R^{7Ba} both represent hydrogen atoms.

### (B-2-8)

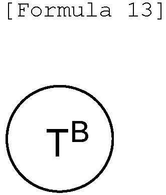

The compound according to any one of (B-2) and (B-2-1) to (B-2-7), in which the above moiety represents a phenyl group or a pyridyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group as a substituent.

### (B-2-9)

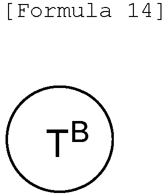

The compound according to any one of (B-2) and (B-2-1) to (B-2-8), in which the above moiety represents a phenyl group that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, and an amino group as a substituent.

### (B-2-10)

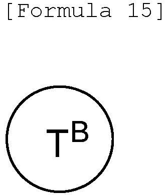

The compound according to any one of (B-2) and (B-2-1) to (B-2-9), in which the above moiety represents an atomic bond.

### (B-2-11)

The compound according to any one of (B-2) and (B-2-1) to (B-2-10), in which B^{Ba} represents NHC(=O), NHCONH, CONH, NHC (=S) NH, NHSO₂, SO₂NH, or OSO₂.

### (B-2-12)

The compound according to any one of (B-2) and (B-2-1) to (B-2-11), in which B^{Ba} represents NHC(=O), NHCONH, or NHSO₂.

### (B-2-13)

The compound according to any one of (B-2) and (B-2-1) to (B-2-12), in which E^{Ba} represents an atomic bond.

### (B-2-14)

The compound according to any one of (B-2) and (B-2-1) to (B-2-13), in which G^{Ba} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-2-15)

The compound according to any one of (B-2) and (B-2-1) to (B-2-14), in which G^{Ba} represents benzene that may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, and an alkylsulfonyl group having 1 to 6 carbon atoms as a substituent.

### (B-2-16)

The compound according to any one of (B-2) and (B-2-1) to (B-2-15), in which n^{B} represents 0.

In the general formula (BI), it is preferable that R^{B1} and R^{B2} bind together to form a condensed ring selected from a naphthalene ring or a tetrahydronaphthalene ring together with the benzene ring to which they bind, and it is particularly preferable that R^{B1} and R^{B2} bind together to form a naphthalene ring together with the benzene ring to which they bind.

In the general formula (BI), it is preferable that R^{B3}, R^{B4}, R^{B5}, R^{B6}, and R^{B7} represent hydrogen atoms.

In the general formula (BI), it is preferable that X^{B} represents N, Y^{B} represents C(=O), and the double line consisting of the solid line and the broken line represents a single bond.

In the general formula (BI), it is preferable that Z^{B} represents an oxygen atom.

In the general formula (BI), it is preferable that A^{B} represents a benzene ring or a pyridine ring, and it is particularly preferable that A^{B} represents a benzene ring.

In the general formula (BI), it is preferable that m^{B} represents 0 to 4, and it is particularly preferable that m^{B} represents 0.

In the general formula (BI), it is preferable that B^{B} represents N(R^{8B})C(=O) or N(R^{10B})SO₂, and in this case, it is more preferable that R^{8B} and R^{10B} represent hydrogen atoms. Furthermore, in the general formula (BI), it is particularly preferable that B^{B} represents NHC(=O).

In the general formula (BI), it is preferable that D^{B} represents an alkylene chain having 1 to 6 carbon atoms which has 1 to 4 of the same or different substituents selected from an alkyl group having 1 to 8 carbon atoms and an alkenyl group having 2 to 8 carbon atoms as a substituent, or represents an atomic bond, it is more preferable that D^{B} represents an alkylene chain having 1 to 8 carbon atoms which has 1 to 4 of the same or different substituents selected from an alkyl group having 1 to 3 carbon atoms and an alkenyl group having 2 or 3 carbon atoms as a substituent, or represents an atomic bond, and it is particularly preferable that D^{B} represents an atomic bond.

In the general formula (BI), it is preferable that E^{B} represents O or an atomic bond, and it is particularly preferable that E^{B} represents an atomic bond.

In the general formula (BI), it is preferable that G^{B} represents benzene or pyridine which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, a halogen atom, a hydroxyl group, a nitro group, an amino group, a dialkylamino group having 2 to 8 carbon atoms, a carboxyl group, an alkylsulfinyl group having 1 to 6 carbon atoms, an alkylthio group having 1 to 6 carbon atoms, and an alkylsulfonyl group having 1 to 6 carbon atoms as a substituent, and it is particularly preferable that G^{B} represents benzene which may have 1 to 4 of the same or different substituents selected from an alkyl group having 1 to 8 carbon atoms and substituted with 1 to 3 halogen atoms, a halogen atom, and a hydroxyl group as a substituent.

In the general formula (BI), it is particularly preferable that A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene which may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

In the general formula (BI), it is particularly preferable that A^{B} represents a benzene ring, m^{B} represents 0, B^{B} represents NHC(=O), D^{B} represents an atomic bond, E^{B} represents an atomic bond, and G^{B} represents benzene which may have 1 to 4 of the same or different substituents selected from an alkyl group having 1 to 8 carbon atoms and substituted with 1 to 3 halogen atoms, a halogen atom, and a hydroxyl group as a substituent.

In the general formula (BI), it is more preferable that R^{B1} and R^{B2} bind together to form a naphthalene ring together with the benzene ring to which they bind, R^{B3}, R^{B4}, R^{B5}, R^{B6}, and R^{B7} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O) or NHSO₂, D^{B} represents an alkyl group having 1 to 3 carbon atoms or an atomic bond, E^{B} represents an atomic bond, and G^{B} represents benzene which may have 1 to 4 of the same or different substituents selected from an alkyl group having 1 to 8 carbon atoms, an alkyl group having 1 to 8 carbon atoms and substituted with 1 to 3 halogen atoms, a halogen atom, and a hydroxyl group as a substituent.

In the general formula (BI), it is particularly preferable that R^{B1} and R^{B2} bind together to form a naphthalene ring together with the benzene ring to which they bind, R^{B3}, R^{B4}, R^{B5}, R^{B6}, and R^{B7} represent hydrogen atoms, X^{B} represents N, Y^{B} represents C(=O), the double line consisting of the solid line and the broken line represents a single bond, Z^{B} represents an oxygen atom, A^{B} represents a benzene ring, m^{B} represents 0, B^{B} represents NHC(=O), D^{B} represents an atomic bond, E^{B} represents an atomic bond, and G^{B} represents benzene which may have 1 to 4 of the same or different substituents selected from an alkyl group having 1 to 8 carbon atoms and substituted with 1 to 3 halogen atoms, a halogen atom, and a hydroxyl group as a substituent.

Typical compounds comprised in the compounds represented by the general formulas (BI) and/or (BII) are as follows.

### <Typical Compound Example B-100>

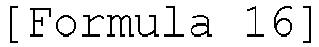

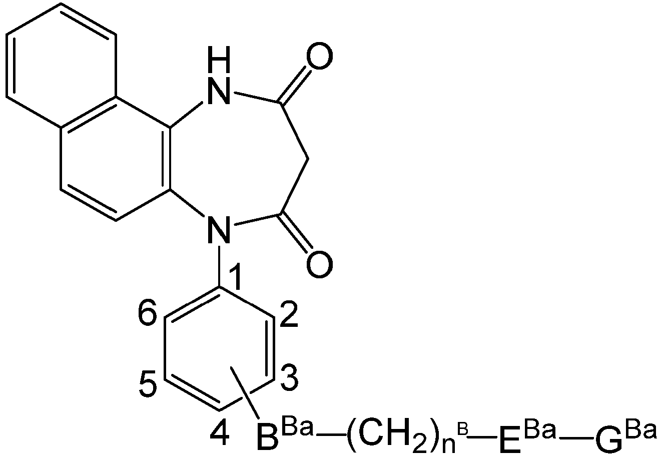

(In the formula B^{Ba} (substitution position), n^{B}, E^{Ba}, and G^{Ba} in the formula are indicated in Tables 8 to 17)

**[Table 8]**

| B^{Ba} (substitution position) | n^{B} | E^{Ba} | G^{Ba} |
|---|---|---|---|
| NHCO(4) | 0 | Atomic bond | Phenyl |
| NHCO(4) | 0 | Atomic bond | (2-CF₃)Phenyl |
| NHCO(4) | 0 | Atomic bond | (3-Br)Phenyl |
| NHCO(4) | 0 | Atomic bond | (4-CF₃)Phenyl |
| NHCO (4) | 0 | Atomic bond | (2-Me)Phenyl |
| NHCO(4) | 0 | Atomic bond | (2,6-Me)Phenyl |
| NHCO(4) | 0 | Atomic bond | (2,6-Cl)Phenyl |
| NHCO(4) | 0 | Atomic bond | (3-Cl)Phenyl |
| NHCO(4) | 1 | Atomic bond | Phenyl |
| NHC(=S)NH(4) | 0 | Atomic bond | Phenyl |
| NHCO(4) | 0 | Atomic bond | (2,3-OMe)Phenyl |
| NHCO(4) | 0 | Atomic bond | (2-OMe)Phenyl |
| NHCO(4) | 1 | Atomic bond | (2-Cl)Phenyl |
| NHCO(4) | 0 | Atomic bond | (2,3-Me)Phenyl |
| NHCO(4) | 0 | Atomic bond | (2,5-Me)Phenyl |
| NHCO(4) | 0 | Atomic bond | (2-C1,5-Br)Phenyl |

**[Table 9]**

| B^{Ba} (substitution position) | n^{B} | E^{Ba} | G^{Ba} |
|---|---|---|---|
| NHCO(4) | 0 | Atomic bond | (2,4-Cl)Phenyl |
| NHCO(4) | 0 | Atomic bond | (2-OH)Phenyl |
| NHCO(4) | 0 | Atomic bond | (2,3-OH)Phenyl |
| NHC(=O)NH(4) | 0 | Atomic bond | Phenyl |
| NHCO(4) | 1 | Atomic bond | (2,6-Cl)Phenyl |
| NHCO(4) | 1 | Atomic bond | (2-OMe)Phenyl |
| NHCO(4) | 1 | Atomic bond | (2-OH)Phenyl |
| NHC(=S)NH(4) | 0 | Atomic bond | (2-Cl)Phenyl |
| NHCO(4) | 0 | Atomic bond | (3-CF₃)Phenyl |
| NHCO(4) | 1 | Atomic bond | (2-CF₃) Phenyl |
| NHC(=O)NH(4) | 0 | Atomic bond | (2-Cl)Phenyl |
| NHCO(4) | 0 | Atomic bond | (2-Cl,3-OMe)Phenyl |
| NHCO(4) | 2 | Atomic bond | Phenyl |
| NHCO(4) | 0 | Atomic bond | 3-indolyl |
| NHCO(4) | 0 | Atomic bond | (2-Cl,3-OH)Phenyl |
| NHCO(4) | 1 | ○ | Phenyl |

**[Table 10]**

| B^{Ba} (substitution position) | n^{B} | E^{Ba} | G^{Ba} |
|---|---|---|---|
| NHCO(4) | 1 | Atomic bond | (2-Cl,4-OMe)Phenyl |
| NHCO (4) | 0 | Atomic bond | (1-Me)imidazol 2-yl |
| NHCO(4) | 1 | Atomic bond | (2,4-Cl)Phenyl |
| NHCO(4) | 1 | Atomic bond | (2-Cl,4-OH)Phenyl |
| NHCO(4) | 1 | Atomic bond | pyridin 3-yl |
| NHCO(4) | 0 | Atomic bond | Benzimidazol 2-yl |
| NHCO(4) | 0 | Atomic bond | (2-Cl)Phenyl |
| NHCO(4) | 0 | Atomic bond | (2-Br)Phenyl |
| NHCO(4) | 0 | Atomic bond | (2-I)Phenyl |
| NHCO(4) | 1 | Atomic bond | (2-Me) Phenyl |
| NHCO(4) | 0 | Atomic bond | quinoxalin 2-yl |
| NHCO(4) | 0 | Atomic bond | (5-Me)thiophen 2-yl |
| NHCO(3) | 1 | Atomic bond | (2-Cl)Phenyl |
| NHCO(4) | 0 | Atomic bond | (2,4,6-Me)Phenyl |
| NHCO(4) | 0 | Atomic bond | (2-Et)Phenyl |
| NHC(=S)NH(4) | 0 | Atomic bond | (2-Me) Phenyl |

**[Table 11]**

| B^{Ba} (substitution position) | n^{B} | E^{Ba} | G^{Ba} |
|---|---|---|---|
| NHCO(4) | 0 | Atomic bond | (4-NMe₂)Phenyl |
| NHCO(4) | 1 | O | (2,4-Cl)Phenyl |
| NHCO(4) | 1 | O | (2-Me) Phenyl |
| NHCO(4) | 0 | Atomic bond | (2-Ac)Phenyl |
| NHCO(4) | 0 | Atomic bond | (2-tBu)Phenyl |
| NHCO(3) | 0 | Atomic bond | (2-I)Phenyl |
| NHCO(4) | 0 | Atomic bond | (1-Me)piperidin 4-yl |
| NHCO(4) | 0 | Atomic bond | benzofuran 2-yl |
| NHCO(4) | 0 | Atomic bond | (1-Me)indol 3-yl |
| NHCO(4) | 0 | Atomic bond | (2-allyl)Phenyl |
| NHCO(4) | 0 | Atomic bond | (2-nPr)Phenyl |
| NHCO(4) | 0 | Atomic bond | (2-iPrO)Phenyl |
| NHCO(4) | 0 | Atomic bond | 3-Me thiophen 2-yl |
| NHCO(4) | 1 | O | (2-Me,3-Cl) Phenyl |
| NHCO(4) | 0 | Atomic bond | (2-CF₃,4-F)Phenyl |
| NHCO(4) | 0 | Atomic bond | (2-OMe,4-F)Phenyl |

**[Table 12]**

| B^{Ba}(substitution position) | n^{B} | E^{Ba} | G^{Ba} |
|---|---|---|---|
| NHCO(4) | 0 | Atomic bond | (2-OH,4-F)Phenyl |
| NHCO(3) | 1 | Atomic bond | (2-I)Phenyl |
| NHCO(4) | 0 | Atomic bond | (3-NMe₂)Phenyl |
| NHCO(4) | 0 | Atomic bond | (2-OMe,4-I)Phenyl |
| NHCO(4) | 0 | Atomic bond | (2-OMe,6-F)Phenyl |
| NHCO(4) | 0 | Atomic bond | (2-OH,4-I)Phenyl |
| NHCO(4) | 0 | Atomic bond | (2-OH,6-F)Phenyl |
| NHCO(4) | 0 | Atomic bond | (2-F)Phenyl |
| NHCO(4) | 0 | Atomic bond | (2-NMe₂)Phenyl |
| NHCO(4) | 0 | Atomic bond | (2-OMe,6-Me)Phenyl |
| NHCO(4) | 0 | Atomic bond | (2-OH,6-Me)Phenyl |
| NHCO(4) | 2 | Atomic bond | (2-Me) Phenyl |
| CONH(4) | 0 | Atomic bond | Phenyl |
| CONH(4) | 1 | Atomic bond | Phenyl |
| NHCO(4) | 2 | Atomic bond | (2-Cl)Phenyl |
| CONH(4) | 1 | Atomic bond | (2-Cl)Phenyl |

**[Table 13]**

| B^{Ba}(substitution position) | n^{B} | E^{Ba} | G^{Ba} |
|---|---|---|---|
| CONH(4) | 0 | Atomic bond | (2-Cl)Phenyl |
| NHCO(4) | 0 | Atomic bond | (5-Br,2,3-methylenedioxy)Phenyl |
| NHCO(4) | 0 | Atomic bond | (2-OMe,6-Br)Phenyl |
| NHCO(4) | 0 | Atomic bond | (2-OH,6-Br)Phenyl |
| NHCO(4) | 0 | Atomic bond | (2-OMe,6-Cl)Phenyl |
| NHCO(4) | 0 | Atomic bond | (2-OH, 6-Cl) Phenyl |
| NHCO(4) | 0 | Atomic bond | (2-OH,6-OMe)Phenyl |
| NHCO(4) | 0 | Atomic bond | (2-OMe,6-CF₃)Phenyl |
| NHCO(4) | 0 | Atomic bond | (2-OH,6-CF₃)Phenyl |
| NHCO(4) | 0 | Atomic bond | (2-Cl,5-SMe) Phenyl |
| NHCO(4) | 0 | Atomic bond | (2-SMe)Phenyl |
| NHCO(4) | 0 | Atomic bond | (3-SMe)Phenyl |
| NHCO(4) | 0 | Atomic bond | (2-OMe,6-Et)Phenyl |
| NHCO(4) | 0 | Atomic bond | (3-SO₂Me)Phenyl |
| NHCO(4) | 0 | Atomic bond | (2-OH,6-Et)Phenyl |
| NHCO(4) | 0 | Atomic bond | (3-S(=O)Me)Phenyl |

**[Table 14]**

| B^{Ba}(substitution position) | n^{B} | E^{Ba} | G^{Ba} |
|---|---|---|---|
| NHCO(4) | 0 | Atomic bond | (2-Cl,5-S(=O)Me)Phenyl |
| NHCO(4) | 0 | Atomic bond | (2-S(=O)Me)Phenyl |
| NHCO(4) | 0 | Atomic bond | (3-Cl) pyridin 2-yl |
| NHCO(4) | 0 | Atomic bond | (2-OMe,3-Cl)Phenyl |
| NHCO(4) | 0 | Atomic bond | (3-Me) pyridin 2-yl |
| NHCO(4) | 0 | Atomic bond | (2-OH,3-Cl)Phenyl |
| NHCO(4) | 0 | Atomic bond | (3-OH) pyridin 2-yl |
| NHCO(4) | 0 | Atomic bond | (3-Vinyl) pyridin 2-yl |
| NHCO(4) | 0 | Atomic bond | (2-Et)pyridin 2-yl |
| NHSO₂(4) | 0 | Atomic bond | (2-NO₂)Phenyl |
| NHSO₂(4) | 0 | Atomic bond | Phenyl |
| NHSO₂(4) | 0 | Atomic bond | (3-Br) Phenyl |
| NHSO₂(4) | 0 | Atomic bond | (3-OMe)Phenyl |
| NHSO₂(3) | 0 | Atomic bond | (2-NO₂)Phenyl |
| NMeSO₂(3) | 0 | Atomic bond | (2-NO₂)Phenyl |
| NHSO₂(3) | 0 | Atomic bond | naphthalen 2-yl |

**[Table 15]**

| B^{Ba}(substitution position) | n^{B} | E^{Ba} | G^{Ba} |
|---|---|---|---|
| NHSO₂(3) | 0 | Atomic bond | naphthalen 1-yl |
| NHSO₂(4) | 0 | Atomic bond | Cyclohexyl |
| NHSO₂(4) | 0 | Atomic bond | pyridin 3-yl |
| NHSO₂(4) | 0 | Atomic bond | (4-iPr)Phenyl |
| NHSO₂(4) | 1 | Atomic bond | Phenyl |
| NHSO₂(4) | 0 | Atomic bond | thiophen 2-yl |
| NHSO₂(4) | 0 | Atomic bond | naphthalen 2-yl |
| NBnSO₂(4) | 0 | Atomic bond | (2-NO₂)Phenyl |
| NMeSO₂(4) | 0 | Atomic bond | (3-Br) Phenyl |
| NMeSO₂(4) | 0 | Atomic bond | (2-NO₂)Phenyl |
| N(CH₂CH₂OH)SO₂(4) | 0 | Atomic bond | (2-NO₂)Phenyl |
| NHSO₂(4) | 1 | Atomic bond | (2-Cl)Phenyl |
| NHSO₂(4) | 1 | Atomic bond | (3-Br) Phenyl |
| NHSO₂(4) | 0 | Atomic bond | (2-CF₃) Phenyl |
| NHSO₂(4) | 1 | Atomic bond | (2-Br)Phenyl |
| NHSO₂(4) | 1 | Atomic bond | (2-Me)Phenyl |

**[Table 16]**

| B^{Ba}(substitution position) | n^{B} | E^{Ba} | G^{Ba} |
|---|---|---|---|
| NHSO₂(4) | 1 | Atomic bond | (2-NO₂)Phenyl |
| NHSO₂(4) | 2 | Atomic bond | Phenyl |
| NHSO₂(4) | 1 | Atomic bond | (4-Cl)Phenyl |
| NMeSO₂(4) | 1 | Atomic bond | (2-CF₃) Phenyl |
| NMeSO₂(4) | 1 | Atomic bond | (2-Et)Phenyl |
| NMeSO₂(4) | 1 | Atomic bond | (2,3-Me)Phenyl |
| NMeSO₂(4) | 2 | Atomic bond | (2-Cl)Phenyl |
| NMeSO₂(4) | 1 | Atomic bond | (2-NO₂)Phenyl |
| NMeSO₂(4) | 1 | Atomic bond | (2-NH₂)Phenyl |
| NMeSO₂(4) | 1 | Atomic bond | (2-NMe₂)Phenyl |

**[Table 17]**

| B^{Ba}(substitution position) | n^{B} | E^{Ba} | G^{Ba} |
|---|---|---|---|
| NHCO(4) | 0 | Atomic bond | Pyridin 4-yl |
| NHCO(4) | 1 | O | Pyridin 3-yl |
| NHCO(4) | 0 | Atomic bond | Pyridin 3-yl |
| NHCO(4) | 0 | Atomic bond | (2-Me)Pyridin 3-yl |
| NHCO(4) | 0 | Atomic bond | (2-Cl)Pyridin 3-yl |
| NHCO(4) | 1 | O | Pyridin 2-yl |
| NHCO(4) | 0 | Atomic bond | (4-CF₃)Pyridin 3-yl |
| NHCO(4) | 0 | Atomic bond | (2-iPr)Phenyl |

### <Typical Compound Example B-200>

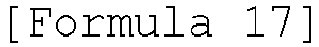

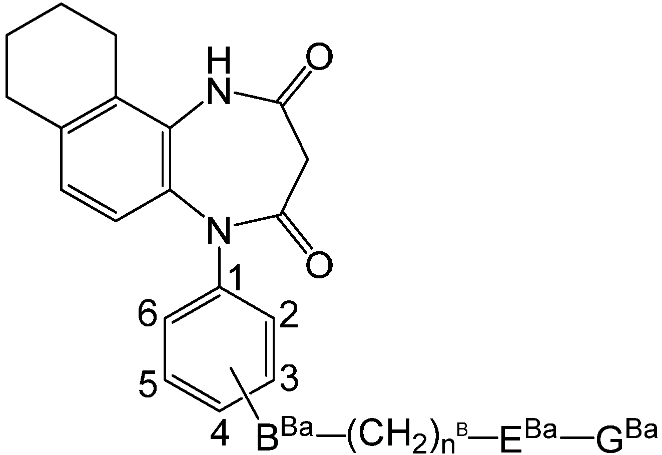

(In the formula B^{Ba} (substitution position), n^{B}, E^{Ba}, and G^{Ba} in the formula are indicated in Tables 18 and 19)

**[Table 18]**

| B^{Ba}(substitution position) | n^{B} | E^{Ba} | G^{Ba} |
|---|---|---|---|
| NHCO(4) | 0 | Atomic bond | Cyclohexyl |
| NHCO(4) | 0 | Atomic bond | (6-Me) pyridin-2-yl |
| NHCO(4) | 0 | Atomic bond | (2-Me) pyridin-3-yl |
| NHCO(4) | 0 | Atomic bond | (2-OMe,3-Me)Phenyl |
| NHCO(4) | 0 | Atomic bond | (2,3-Cl)Phenyl |
| NHCO(4) | 0 | Atomic bond | (2-OH,3-Me)Phenyl |
| NHCO(4) | 0 | Atomic bond | (2-I)Phenyl |
| NHCO(4) | 1 | Atomic bond | (1-Me)pyrrol 2-yl |
| NHCO(4) | 1 | Atomic bond | (2-tBu)Phenyl |
| NHCO(4) | 0 | Atomic bond | (2-Isopropenyl) phenyl |
| NHCO(4) | 0 | Atomic bond | (2-iPr)Phenyl |
| NHCO(4) | 1 | Atomic bond | morpholin 2-yl |
| NHCO(4) | 0 | Atomic bond | (2-Cl)pyridin 2-yl |

**[Table 19]**

| B^{Ba}(substitution position) | n^{B} | E^{Ba} | G^{Ba} |
|---|---|---|---|
| NHSO₂(4) | 0 | Atomic bond | (2-NO₂)Phenyl |
| NMeSO₂(4) | 0 | Atomic bond | (2-NO₂)Phenyl |
| SO₂NH(4) | 0 | Atomic bond | Phenyl |
| OSO₂(4) | 0 | Atomic bond | (3-Br) Phenyl |
| NHSO₂(4) | 1 | Atomic bond | (2-Cl)Phenyl |
| NHSO₂(4) | 0 | Atomic bond | (3-Br) Phenyl |
| NHSO₂(4) | 0 | Atomic bond | (3-OMe)Phenyl |
| NHSO₂(4) | 1 | Atomic bond | (2,3-Cl)Phenyl |
| NHSO₂(4) | 1 | Atomic bond | (2,6-Cl)Phenyl |
| NHSO₂(4) | 1 | Atomic bond | (2-I)Phenyl |
| NMeSO₂(4) | 1 | Atomic bond | (2-Cl)Phenyl |

### <Typical Compound Example B-300>

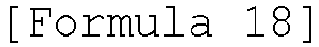

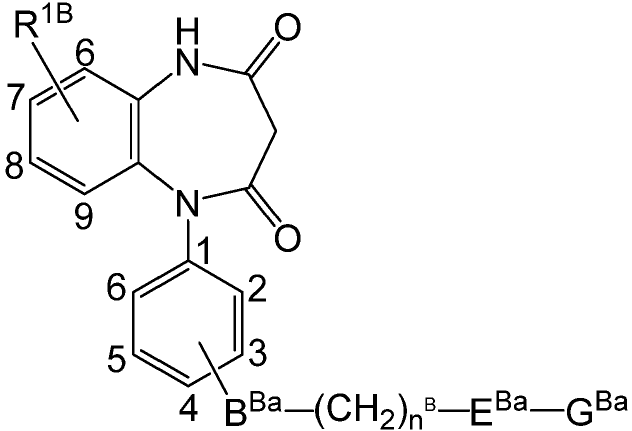

(In the formula R^{1B}, B^{Ba} (substitution position), n^{B}, E^{Ba}, and G^{Ba} in the formula are indicated in Table 20)

**[Table 20]**

| R^{1B} | B^{Ba}(substitution position) | n^{B} | E^{Ba} | G^{Ba} |
|---|---|---|---|---|
| 7-OMe | NHCO(4) | 0 | Atomic bond | (2,3-Me)Phenyl |
| 7-OH | NHCO(4) | 0 | Atomic bond | (2,3-Me)Phenyl |
| 6-Me | NHCO(4) | 0 | Atomic bond | (2,3-Me)Phenyl |
| 6,7-Me | NHCO(4) | 0 | Atomic bond | (2-I)Phenyl |
| 6-Et | NHCO(4) | 0 | Atomic bond | (2-I)Phenyl |
| 7-Ph | NHCO(4) | 0 | Atomic bond | (2-Isopropyl))Phenyl |
| 7-(Pyridin-3yl) | NHCO(4) | 0 | Atomic bond | (2-Isopropyl))Phenyl |
| 7-(Pyridin-2yl) | NHCO(4) | 0 | Atomic bond | (2-Isopropyl)Phenyl |
| 7-Cl | NHSO₂(4) | 0 | Atomic bond | (2-Isopropyl)Phenyl |
| 7-Br | NHSO₂(4) | 0 | Atomic bond | (2-Isopropyl)Phenyl |
| 7-CF₃ | NHSO₂(4) | 0 | Atomic bond | (2-Isopropyl)Phenyl |
| H | NHSO₂(4) | 0 | Atomic bond | (2-Isopropyl)Phenyl |
| 6-Me,7-Br | NHSO₂(4) | 0 | Atomic bond | (2-Isopropyl)Phenyl |
| 7-OMe | NHSO₂(4) | 1 | Atomic bond | (2-Cl)Phenyl |
| 7-OH | NHSO₂(4) | 1 | Atomic bond | (2-Cl)Phenyl |
| 6-Me | NHSO₂(4) | 1 | Atomic bond | (2-Cl)Phenyl |

### <Typical Compound Example B-400>

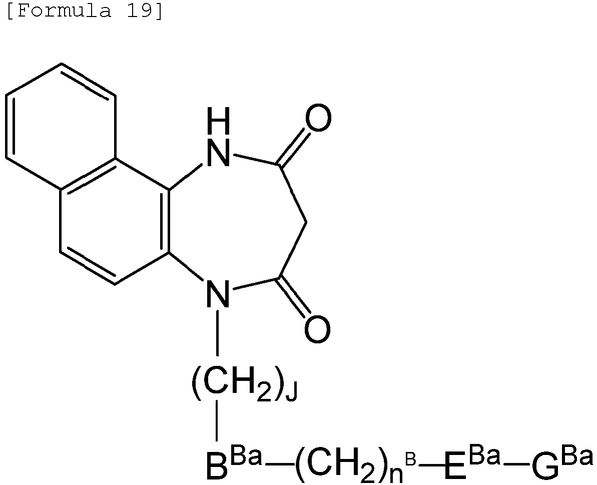

(In the formula B^{Ba} (substitution position), n^{B}, E^{Ba}, and G^{Ba} in the formula are indicated in Table 21)

**[Table 21]**

| B^{Ba}(substitution position) | n^{B} | E^{Ba} | G^{Ba} |
|---|---|---|---|
| NHCO | 0 | Atomic bond | (2-Cl,3-OMe)Phenyl |
| NHCO | 0 | Atomic bond | (2-I)Phenyl |
| NHSO₂ | 1 | Atomic bond | (2-Cl)Phenyl |
| NHSO₂ | 1 | Atomic bond | (2-Cl)Phenyl |

### <Typical Compound Example B-500>

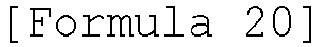

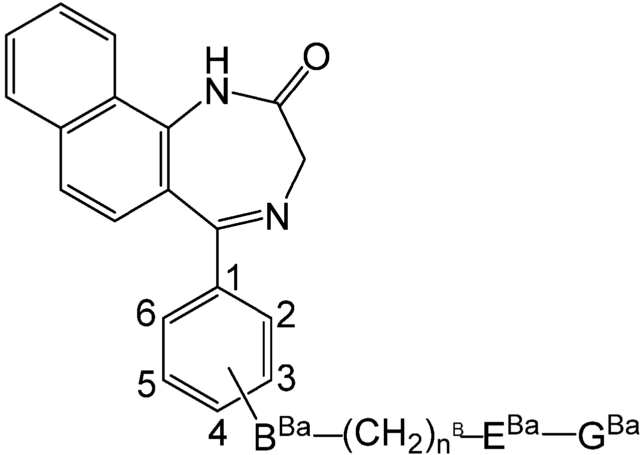

(In the formula B^{Ba} (substitution position), n^{B}, E^{Ba}, and G^{Ba} in the formula are indicated in Table 22)

**[Table 22]**

| B^{Ba}(substitution position) | n^{B} | E^{Ba} | G^{Ba} |
|---|---|---|---|
| NHCO(4) | 0 | Atomic bond | (2-Cl,3-OMe)Phenyl |
| NHCO(4) | 0 | Atomic bond | (2-Cl,3-OH)Phenyl |
| NHCO(4) | 0 | Atomic bond | (2-tBu)Phenyl |
| NHCO(4) | 0 | Atomic bond | (2-Cl,6-OMe)Phenyl |
| NHCO(4) | 0 | Atomic bond | (2-Cl,6-OH)Phenyl |
| NHSO2(3) | 0 | Atomic bond | Phenyl |
| NHSO2(4) | 0 | Atomic bond | (2-Cl)Phenyl |

### <Typical Compound Example B-600>

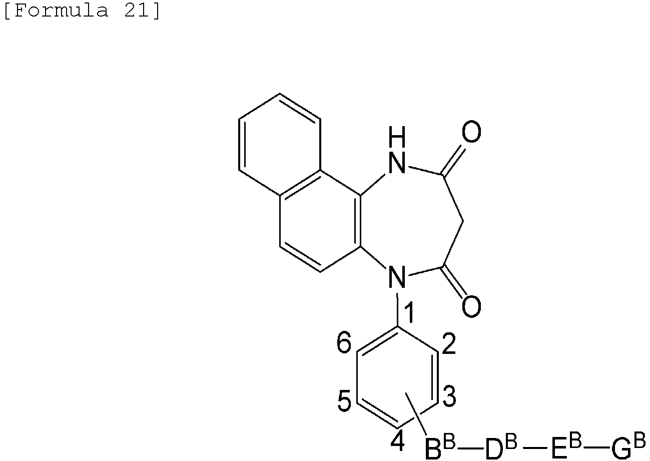

(In the formula B^{B} (substitution position), D^{B}, E^{B}, and G^{B} are indicated in Table 23)

**[Table 23]**

| B^{B} (substitution position) | D^{B} | E^{B} | G^{B} |
|---|---|---|---|
| NHCO(4) | C(Me)H | Atomic bond | Phenyl |
| NHCO(4) | C(Me)₂ | Atomic bond | Phenyl |
| NHCO(4) | CH=CH | Atomic bond | Phenyl |
| NHCO(4) | C(Me)H | O | Phenyl |
| NHCO(4) | C(Me)₂ | O | Phenyl |
| NHCO(4) | CH=CH | Atomic bond | (2-Me) Phenyl |
| NHCO(4) | CH=CH | Atomic bond | (2-Cl)Phenyl |

### <Typical Compound Example B-700>

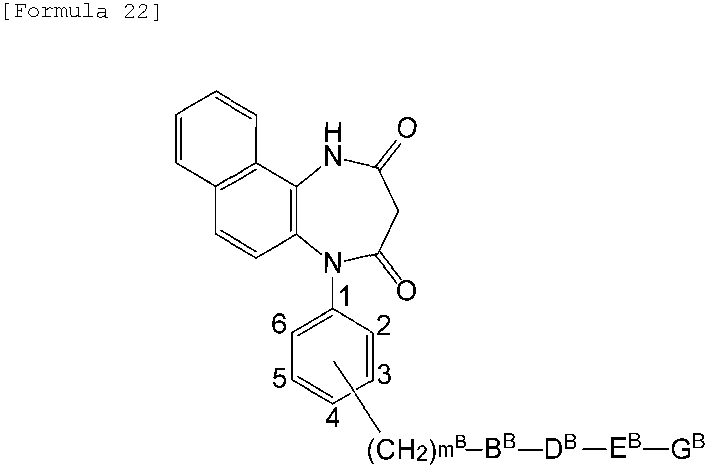

(In the formula m^{B} (substitution position), B^{B}, D^{B}, E^{B}, and G^{B} are indicated in Table 24)

**[Table 24]**

| m^{B} (substitution position) | B^{B} | D^{B} | E^{B} | G^{B} |
|---|---|---|---|---|
| 1(4) | NHCO | Atomic bond | Atomic bond | Phenyl |
| 1(4) | NHCO | Atomic bond | Atomic bond | (2-Cl)Phenyl |
| 1(4) | NHSO₂ | CH₂ | Atomic bond | (2-Cl)Phenyl |

### <Typical Compound Example B-800>

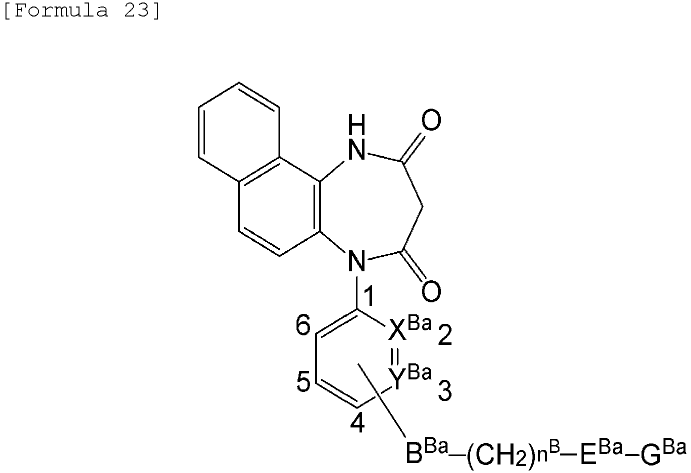

(In the formula X^{Ba}, Y^{Ba}, B^{Ba} (substitution position), n^{B}, E^{Ba}, and G^{Ba} in the formula are indicated in Table 25)

**[Table 25]**

| X^{Ba} | Y^{Ba} | B^{Ba}(substitution position) | n^{B} | E^{Ba} | G^{Ba} |
|---|---|---|---|---|---|
| CH | C-F | NHCO(4) | 0 | Atomic bond | (2,3-Me)Phenyl |
| CH | C-OH | NHCO(4) | 0 | Atomic bond | (2,3-Me)Phenyl |
| CH | C-F | NHCO(4) | 0 | Atomic bond | (2-I)Phenyl |
| CH | N | NHCO(4) | 0 | Atomic bond | (2-I)Phenyl |
| CH | N | NHCO(4) | 0 | Atomic bond | Phenyl |
| N | CH | NHCO(4) | 0 | Atomic bond | (2-I)Phenyl |
| CH | N | NHCO(4) | 0 | Atomic bond | (2-Cl)Phenyl |
| CH | N | NHCO(4) | 0 | Atomic bond | (2-OH)Phenyl |
| CH | N | NHC(=O)NH(4) | 0 | Atomic bond | (2-OH)Phenyl |
| CH | N | NHCO(4) | 0 | Atomic bond | (2-OH,6-Me)Phenyl |
| CH | N | NHCO(4) | 0 | Atomic bond | (2-OH,6-Cl)Phenyl |
| CH | N | NHCO(3) | 0 | Atomic bond | (2-OH,6-Cl)Phenyl |
| CH | N | NHCO(4) | 0 | Atomic bond | (2-Cl)pyridin 2-yl |
| CH | N | NHCO(4) | 1 | Atomic bond | (2-Cl)pyridin 2-yl |
| CH | N | NHCO(4) | 0 | Atomic bond | (2-Me) pyridin 2-yl |
| CH | C-OMe | NHSO₂(4) | 1 | Atomic bond | (2-Cl)Phenyl |
| CH | C-OH | NHSO₂(4) | 1 | Atomic bond | (2-Cl)Phenyl |

### <Typical Compound Example B-900>

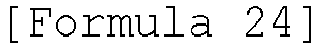

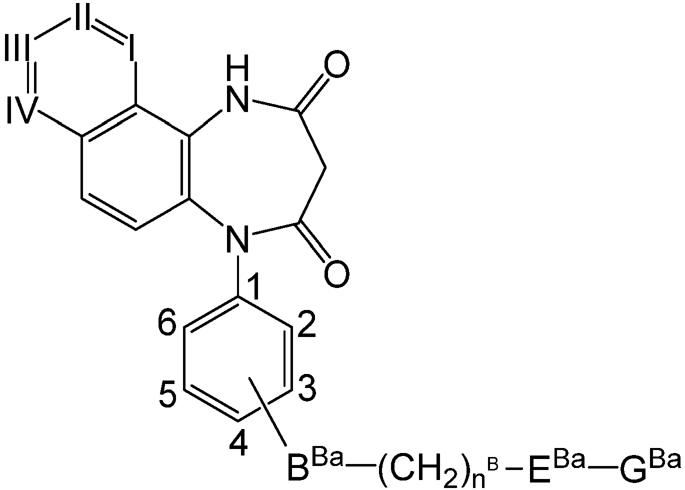

(In the formula I=II-III=IV, B^{Ba} (substitution position), n^{B}, E^{Ba}, and G^{Ba} in the formula are indicated in Table 26)

**[Table 26]**

| I=II-III=IV | B^{Ba}(substitution position) | n^{B} | E^{Ba} | G^{Ba} |
|---|---|---|---|---|
| N=CH-CH=CH | NHCO(4) | 0 | Atomic bond | (2-I)Phenyl |
| CH=N-CH=CH | NHCO(4) | 0 | Atomic bond | (2-I)Phenyl |
| CH=CH-N=CH | NHCO(4) | 0 | Atomic bond | (2-I)Phenyl |
| CH=CH-CH=N | NHCO(4) | 0 | Atomic bond | (2-I)Phenyl |
| N=CH-CH=CH | NHCO(4) | 1 | O | Phenyl |
| N=CH-CH=CH | NHCO(3) | 0 | Atomic bond | (2-I)Phenyl |
| N=CH-CH=CH | NHCO(4) | 0 | Atomic bond | (2-Cl)Phenyl |
| N=CH-CH=CH | NHCO(4) | 0 | Atomic bond | (2-OH)Phenyl |
| N=CH-CH=CH | NHC(=O)NH(4) | 0 | Atomic bond | (2-OH)Phenyl |
| N=CH-CH=CH | NHCO(4) | 1 | O | (2-OH, 6-Me)Phenyl |
| N=CH-CH=CH | NHCO(4) | 0 | Atomic bond | (2-OH, 6-Cl)Phenyl |
| N=CH-CH=CH | NHCO(3) | 0 | Atomic bond | (2-OH, 6-Cl)Phenyl |
| N=CH-CH=CH | NHCO(4) | 0 | Atomic bond | (2-Cl)pyridin 2-yl |
| N=CH-CH=CH | NHCO(4) | 1 | Atomic bond | (2-Cl)pyridin 2-yl |
| N=CH-CH=CH | NHCO(4) | 0 | Atomic bond | (2-Me) pyridin 2-yl |
| CH=CH-N=CH | NHCO(4) | 0 | Atomic bond | (2-Cl)pyridin 3-yl |

### <Typical Compound Example B-1000>

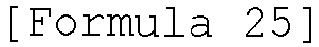

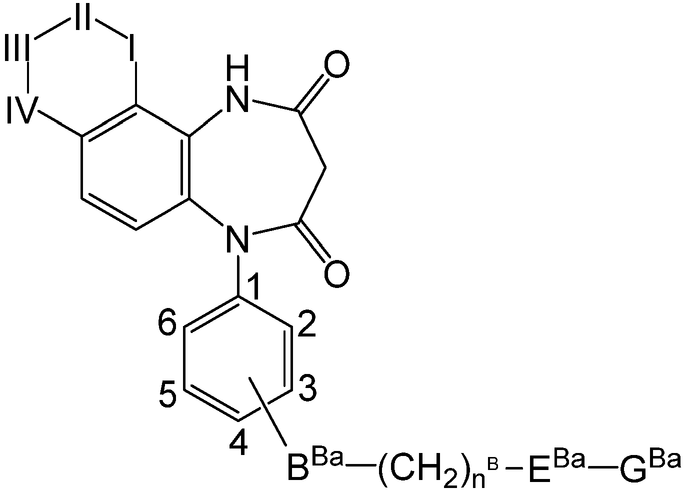

(In the formula I-II-III-IV, B^{Ba} (substitution position), n^{B}, E^{Ba}, and G^{Ba} in the formula are indicated in Table 27)

**[Table 27]**

| I-II-III-IV | B^{Ba} (subst itution position) | n^{B} | E^{Ba} | G^{Ba} |
|---|---|---|---|---|
| NH-CH₂-CH₂-CH₂ | NHCO(4) | 0 | Atomic bond | (2-I)Phenyl |
| CH₂-NH-CH₂-CH₂ | NHCO(4) | 0 | Atomic bond | (2-I)Phenyl |
| CH₂-CH₂-NH-CH₂ | NHCO(4) | 0 | Atomic bond | (2-I)Phenyl |
| CH₂-CH₂-CH₂-NH | NHCO(4) | 0 | Atomic bond | (2-I)Phenyl |
| CH₂-CH₂-NH-CH₂ | NHCO(4) | 1 | O | Phenyl |
| CH₂-CH₂-NH-CH₂ | NHCO(3) | 0 | Atomic bond | (2-I)Phenyl |
| CH₂-CH₂-NH-CH₂ | NHCO(4) | 0 | Atomic bond | (2-Cl)Phenyl |
| CH₂-CH₂-NH-CH₂ | NHCO(4) | 0 | Atomic bond | (2-Cl)pyridin 3-yl |
| CH₂-CH₂-NH-CH₂ | NHCO(4) | 0 | Atomic bond | (2-OH)Phenyl |
| CH₂-CH₂-NH-CH₂ | NHC(=O)NH (4) | 0 | Atomic bond | (2-OH)Phenyl |
| CH₂-CH₂-NH-CH₂ | NHCO(4) | 1 | O | (2-OH, 6-Me)Phenyl |
| CH₂-CH₂-NH-CH₂ | NHCO(4) | 0 | Atomic bond | (2-OH, 6-Cl)Phenyl |
| CH₂-CH₂-NH-CH₂ | NHCO(3) | 0 | Atomic bond | (2-OH, 6-Cl)Phenyl |
| CH₂-CH₂-NH-CH₂ | NHCO(4) | 0 | Atomic bond | (2-Cl)pyridin 2-yl |
| CH₂-CH₂-NH-CH₂ | NHCO(4) | 1 | Atomic bond | (2-Cl)pyridin 2-yl |
| CH₂-CH₂-NH-CH₂ | NHCO(4) | 0 | Atomic bond | (2-Me) pyridin 2-yl |
| CH₂-CH₂-NH-CH₂ | NHCO(4) | 0 | Atomic bond | (2-Cl)pyridin 3-yl |

### <Typical Compound Example B-1100>

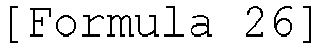

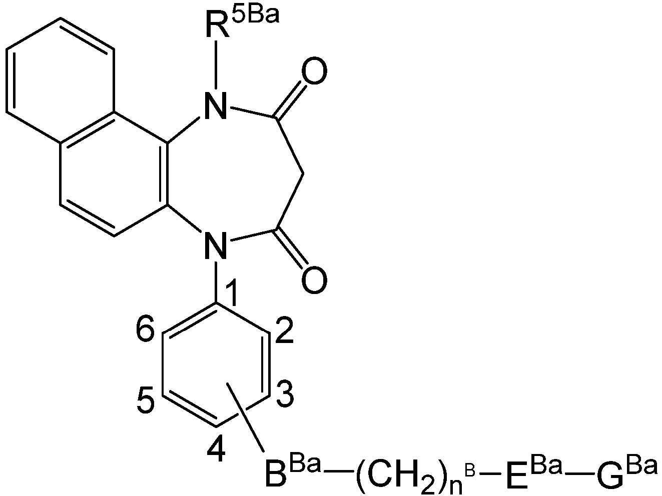

(R^{5Ba}, B^{Ba} (substitution position), n^{B}, E^{Ba}, and G^{Ba} in the formula are indicated in Table 28)

**[Table 28]**

| R^{5Ba} | B^{Ba}(substitution position) | n^{B} | E^{Ba} | G^{Ba} |
|---|---|---|---|---|
| Bn | NBnSO₂(4) | 0 | Atomic bond | (2-NO₂)Phenyl |
| Me | NBnSO₂(4) | 0 | Atomic bond | (2-NO₂)Phenyl |
| Et | NBnSO₂(4) | 0 | Atomic bond | (2-NO₂)Phenyl |

Since the compounds represented by the general formula (A) are disclosed in WO 2010/093061 A, all of the compounds can be easily obtained by referring to this international publication. The disclosure of this international publication is incorporated herein by reference in its entirety.

Since the compounds represented by the general formulas (BI) and (BII) are disclosed in WO 2013/105608 A, all of the compounds can be easily obtained by referring to this international publication. The disclosure of this international publication is incorporated herein by reference in its entirety.

Furthermore, WO 2010/093061 A, WO 2013/105608 A and WO 2015/005467 A disclose that the compounds represented by the general formulas (A) to (BII) have a P2X4 receptor antagonistic action.

Specific examples of a preferable compound comprised in the compounds represented by the general formulas (A) to (BII), or a pharmaceutically acceptable salt thereof, are shown below, however, the compound or a pharmaceutically acceptable salt thereof that can be used as the active ingredient of the pharmaceutical composition of the present invention is not limited thereto.
(Compound A1) 5-[3-(1H-tetrazol-5-yl)phenyl]-1H-naphtho[1,2-b][1,4]diazepine-2,4(3H,5H)-dione;
(Compound A2) 5-[3-(1H-tetrazol-5-yl)phenyl]-1H-naphtho[1,2-b][1,4]diazepine-2,4(3H,5H)-dione sodium salt;
(Compound A3) 5-[3-(1H-tetrazol-5-yl)phenyl]-1H-naphtho[1,2-b][1,4]diazepine-2,4(3H,5H)-dione potassium salt;
(Compound A4) 5-[4-(1H-tetrazol-5-yl)phenyl]-1H-naphtho[1,2-b][1,4]diazepine-2,4(3H,5H)-dione;
(Compound A5) 5-[4-(1H-tetrazol-5-yl)phenyl]-1H-naphtho[1,2-b][1,4]diazepine-2,4(3H,5H)-dione sodium salt;
(Compound A6) 1-methyl-5-[3-(1H-tetrazol-5-yl)phenyl]-1H-naphtho[1,2-b][1,4]diazepine-2,4(3H,5H)-dione;
(Compound A7) 1,3-dimethyl-5-[3-(1H-tetrazol-5-yl)phenyl]-1H-naphtho[1,2-b][1,4]diazepine-2,4(3H,5H)-dione;
(Compound A8) 5-[2-chloro-5-(1H-tetrazol-5-yl)phenyl]-1H-naphtho[1,2-b][1,4]diazepine-2,4(3H,5H)-dione;
(Compound A9) 5-[2-chloro-5-(1H-tetrazol-5-yl)phenyl]-1H-naphtho[1,2-b][1,4]diazepine-2,4(3H,5H)-dione sodium salt;
(Compound A10) 5-[2-methyl-5-(1H-tetrazol-5-yl)phenyl]-1H-naphtho[1,2-b][1,4]diazepine-2,4(3H,5H)-dione;
(Compound A11) 5-[2-methyl-5-(1H-tetrazol-5-yl)phenyl]-1H-naphtho[1,2-b][1,4]diazepine-2,4(3H,5H)-dione sodium salt;
(Compound A12) 5-[2-bromo-5-(1H-tetrazol-5-yl)phenyl]-1H-naphtho[1,2-b][1,4]diazepine-2,4(3H,5H)-dione;
(Compound A13) 5-[3-(2-methyl-2H-tetrazol-5-yl)phenyl]-1H-naphtho[1,2-b][1,4]diazepine-2,4(3H,5H)-dione;
(Compound A14) 5-[3-(1-methyl-1H-tetrazol-5-yl)phenyl]-1H-naphtho[1,2-b][1,4]diazepine-2,4(3H,5H)-dione;
(Compound A15) 5-[3-(5-oxo-4H-[1,2,4]oxadiazol-3-yl)phenyl]-1H-naphtho[1,2-b][1,4]diazepine-2,4(3H,5H)-dione;
(Compound A16) 5-[3-(5-thioxo-4H-[1,2,4]oxadiazol-3-yl)phenyl]-1H-naphtho[1,2-b][1,4]diazepine-2,4(3H,5H)-dione;
(Compound A17) 5-[3-(5-thioxo-4H-[1,2,4]oxadiazol-3-yl)phenyl]-1H-naphtho[1,2-b][1,4]diazepine-2,4(3H,5H)-dione sodium salt;
(Compound A18) 5-[3-(oxazol-2-yl)phenyl]-1H-naphtho[1,2-b][1,4]diazepine-2,4(3H,5H)-dione;
(Compound A19) 5-[3-(1H-pyrazol-4-yl)phenyl]-1H-naphtho[1,2-b][1,4]diazepine-2,4(3H,5H)-dione;
(Compound A20) 5-[4-fluoro-3-(1H-tetrazol-5-yl)phenyl]-1H-naphtho[1,2-b][1,4]diazepine-2,4(3H,5H)-dione;
(Compound A21) 5-[4-fluoro-3-(1H-tetrazol-5-yl)phenyl]-1H-naphtho[1,2-b][1,4]diazepine-2,4(3H,5H)-dione sodium salt;
(Compound B1) 5-(4-benzoylaminophenyl)-1H-naphtho[1,2-b][1,4]diazepine-2,4(3H,5H)-dione;
(Compound B2) 5-[4-[2-(trifluoromethyl)benzoyl]aminophenyl]-1H-naphtho[1,2-b][1,4]diazepine-2,4(3H,5H)-dione;
(Compound B3) 5-[4-(3-bromobenzoyl)aminophenyl]-1H-naphtho[1,2-b][1,4]diazepine-2,4(3H,5H)-dione;
(Compound B4) 5-[4-[4-(trifluoromethyl)benzoyl]aminophenyl]-1H-naphtho[1,2-b][1,4]diazepine-2,4(3H,5H)-dione;
(Compound B5) 5-[4-(2-methylbenzoyl)aminophenyl]-1H-naphtho[1,2-b][1,4]diazepine-2,4(3H,5H)-dione;
(Compound B6) 5-[4-(2,6-dimethylbenzoylamino)phenyl]-1H-naphtho[1,2-b][1,4]diazepine-2,4(3H,5H)-dione;
(Compound B7) 5-[4-(2,6-dichlorobenzoyl)aminophenyl]-1H-naphtho[1,2-b][1,4]diazepine-2,4(3H,5H)-dione;
(Compound B8) 5-[4-(3-chlorobenzoyl)aminophenyl]-1H-naphtho[1,2-b][1,4]diazepine-2,4(3H,5H)-dione;
(Compound B9) 5-[4-(2-phenylacetylamino)phenyl]-1H-naphtho[1,2-b][1,4]diazepine-2,4(3H,5H)-dione;
(Compound B10) 1-[4-(2,4-dioxo-1,2,3,4-tetrahydronaphtho[1,2-b][1,4]diazepin-5-yl)phenyl]-3-phenylthiourea;
(Compound B11) 5-[4-(2,3-dimethoxybenzoylamino)phenyl]-1H-naphtho[1,2-b][1,4]diazepine-2,4(3H,5H)-dione;
(Compound B12) 5-[4-(2-methoxybenzoylamino)phenyl]-1H-naphtho[1,2-b][1,4]diazepine-2,4(3H,5H)-dione;
(Compound B13) 5-[4-[(2-chlorophenylacetyl)amino]phenyl]-1H-naphtho[1,2-b][1,4]diazepine-2,4(3H,5H)-dione;
(Compound B14) 5-[4-(2,3-dimethylbenzoylamino)phenyl]-1H-naphtho[1,2-b][1,4]diazepine-2,4(3H,5H)-dione;
(Compound B15) 5-[4-(2,5-dimethylbenzoylamino)phenyl]-1H-naphtho[1,2-b][1,4]diazepine-2,4(3H,5H)-dione;
(Compound B16) 5-[4-(5-bromo-2-chlorobenzoylamino)phenyl]-1H-naphtho[1,2-b][1,4]diazepine-2,4(3H,5H)-dione;
(Compound B17) 5-[4-(2,4-dichlorobenzoylamino)phenyl]-1H-naphtho[1,2-b][1,4]diazepine-2,4(3H,5H)-dione;
(Compound B18) 5-[4-(2-hydroxybenzoylamino)phenyl]-1H-naphtho[1,2-b][1,4]diazepine-2,4(3H,5H)-dione;
(Compound B19) 5-[4-(2,3-dihydroxybenzoylamino)phenyl]-1H-naphtho[1,2-b][1,4]diazepine-2,4(3H,5H)-dione;
(Compound B20) 1-[4-(2,4-dioxo-1,2,3,4-tetrahydronaphtho[1,2-b][1,4]diazepin-5-yl)phenyl]-3-phenylurea;
(Compound B21) 5-[4-[(2,6-dichlorophenylacetyl)amino]phenyl]-1H-naphtho[1,2-b][1,4]diazepine-2,4(3H,5H)-dione;
(Compound B22) 5-[4-[(2-methoxyphenylacetyl)amino]phenyl]-1H-naphtho[1,2-b][1,4]diazepine-2,4(3H,5H)-dione;
(Compound B23) 5-[4-[(2-hydroxyphenylacetyl)amino]phenyl]-1H-naphtho[1,2-b][1,4]diazepine-2,4(3H,5H)-dione;
(Compound B24) 1-(2-chlorophenyl)-3-[4-(2,4-dioxo-1,2,3,4-tetrahydronaphtho[1,2-b][1,4]diazepin-5-yl)phenyl]thiourea;
(Compound B25) 5-[4-[3-(trifluoromethyl)benzoylamino]phenyl]-1H-naphtho[1,2-b][1,4]diazepine-2,4(3H,5H)-dione;
(Compound B26) 5-[4-[2-[2-(trifluoromethyl)phenyl]acetylamino]phenyl]-1H-naphtho[1,2-b][1,4]diazepine-2,4(3H,5H)-dione;
(Compound B27) 1-(2-chlorophenyl)-3-[4-(2,4-dioxo-1,2,3,4-tetrahydronaphtho[1,2-b][1,4]diazepin-5-yl)phenyl]urea;
(Compound B28) 5-[4-[(2-phenylpropionyl)amino]phenyl]-1H-naphtho[1,2-b][1,4]diazepine-2,4(3H,5H)-dione;
(Compound B29) 5-[4-(2-chloro-3-methoxybenzoylamino)phenyl]-1H-naphtho[1,2-b][1,4]diazepine-2,4(3H,5H)-dione;
(Compound B30) 5-[4-(3-phenylpropionylamino)phenyl]-1H-naphtho[1,2-b][1,4]diazepine-2,4(3H,5H)-dione;
(Compound B31) 5-[4-[(1H-indole-3-carbonyl)amino]phenyl]-1H-naphtho[1,2-b][1,4]diazepine-2,4(3H,5H)-dione;
(Compound B32) 5-[4-(2-chloro-3-hydroxybenzoylamino)phenyl]-1H-naphtho[1,2-b][1,4]diazepine-2,4(3H,5H)-dione;
(Compound B33) 5-[4-[(2-methyl-2-phenylpropionyl)amino]phenyl]-1H-naphtho[1,2-b][1,4]diazepine-2,4(3H,5H)-dione;
(Compound B34) 5-[4-(2-phenoxyacetylamino)phenyl]-1H-naphtho[1,2-b][1,4]diazepine-2,4(3H,5H)-dione;
(Compound B35) 5-[4-[2-(2-chloro-4-methoxyphenyl)acetylamino]phenyl]-1H-naphtho[1,2-b][1,4]diazepine-2,4(3H,5H)-dione;
(Compound B36) 5-[4-[(1-methyl-1H-imidazole-2-carbonyl)amino]phenyl]-1H-naphtho[1,2-b][1,4]diazepine-2,4(3H,5H)-dione;
(Compound B37) 5-[4-[2-(2,4-dichlorophenyl)acetylamino]phenyl]-1H-naphtho[1,2-b][1,4]diazepine-2,4(3H,5H)-dione;
(Compound B38) 5-[4-[2-(2-chloro-4-hydroxyphenyl)acetylamino]phenyl]-1H-naphtho[1,2-b][1,4]diazepine-2,4(3H,5H)-dione;
(Compound B39) 5-[4-(3-phenylpropenylamino)phenyl]-1H-naphtho[1,2-b][1,4]diazepine-2,4(3H,5H)-dione;
(Compound B40) 5-[4-[(3-pyridylacetyl)amino]phenyl]-1H-naphtho[1,2-b][1,4]diazepine-2,4(3H,5H)-dione hydrochloride;
(Compound B41) 5-[4-(1H-benzimidazole-2-carbonylamino)phenyl]-1H-naphtho[1,2-b][1,4]diazepine-2,4(3H,5H)-dione;
(Compound B42) 1-[4-(2,3-dimethylbenzoylamino)phenyl]-7-methoxy-1H-1,5-benzodiazepine-2,4(3H,5H)-dione;
(Compound B43) 5-[4-[(benzoylamino)methyl]phenyl]-1H-naphtho[1,2-b][1,4]diazepine-2,4(3H,5H)-dione;
(Compound B44) 5-[4-[(2-chlorobenzoylamino)methyl]phenyl]-1H-naphtho[1,2-b][1,4]diazepine-2,4(3H,5H)-dione;
(Compound B45) 1-[4-(2,3-dimethylbenzoylamino)phenyl]-7-hydroxy-1H-1,5-benzodiazepine-2,4(3H,5H)-dione;
(Compound B46) 5-[4-(2-chlorobenzoylamino)phenyl]-1H-naphtho[1,2-b][1,4]diazepine-2,4(3H,5H)-dione;
(Compound B47) 5-[4-(2-bromobenzoylamino)phenyl]-1H-naphtho[1,2-b][1,4]diazepine-2,4(3H,5H)-dione;
(Compound B48) 5-[4-(2-iodobenzoylamino)phenyl]-1H-naphtho[1,2-b][1,4]diazepine-2,4(3H,5H)-dione;
(Compound B49) 5-[4-(2,3-dimethylbenzoylamino)-3-fluorophenyl]-1H-naphtho[1,2-b][1,4]diazepine-2,4(3H,5H)-dione;
(Compound B50) 5-[4-[2-(2-methylphenyl)acetylamino]phenyl]-1H-naphtho[1,2-b][1,4]diazepine-2,4(3H,5H)-dione;
(Compound B51) 5-[4-[(quinoxalin-2-yl)carbonylamino]phenyl]-1H-naphtho[1,2-b][1,4]diazepine-2,4(3H,5H)-dione;
(Compound B52) 5-[4-[(5-methylthiophen-2-yl)carbonylamino]phenyl]-1H-naphtho[1,2-b][1,4]diazepine-2,4(3H,5H)-dione;
(Compound B53) 5-[3-[(2-chlorophenylacetyl)amino]phenyl]-1H-naphtho[1,2-b][1,4]diazepine-2,4(3H,5H)-dione;
(Compound B54) 5-[4-[(2,4,6-trimethylbenzoyl)amino]phenyl]-1H-naphtho[1,2-b][1,4]diazepine-2,4(3H,5H)-dione;
(Compound B55) 5-[4-(cyclohexylcarbonylamino)phenyl]-1,2,3,4,8,9,10,11-octahydronaphtho[1,2-b][1,4]diazepine-2,4(3H,5H)-dione;
(Compound B56) 1-[4-(2,3-dimethylbenzoyl)aminophenyl]-6-methyl-1H-1,5-benzodiazepine-2,4(3H,5H)-dione;
(Compound B57) 5-[4-[(2-ethylbenzoyl)amino]phenyl]-1H-naphtho[1,2-b][1,4]diazepine-2,4(3H,5H)-dione;
(Compound B58) 5-[4-[(6-methylpyridin-2-yl)carbonylamino]phenyl]-1,2,3,4,8,9,10,11-octahydronaphtho[1,2-b][1,4]diazepine-2,4(3H,5H)-dione;
(Compound B59) 5-[4-[(2-methylpyridin-3-yl)carbonylamino]phenyl]-1,2,3,4,8,9,10,11-octahydronaphtho[1,2-b][1,4]diazepine-2,4(3H,5H)-dione;
(Compound B60) 1-[4-(2,4-dioxo-1,2,3,4-tetrahydronaphtho[1,2-b][1,4]diazepin-5-yl)phenyl]-3-(2-methylphenyl)thiourea;
(Compound B61) 5-[4-(2-methoxy-3-methylbenzoyl)aminophenyl]-1,2,3,4,8,9,10,11-octahydronaphtho[1,2-b][1,4]diazepine-2,4(3H,5H)-dione;
(Compound B62) 5-[4-(2,3-dichlorobenzoyl)aminophenyl]-1,2,3,4,8,9,10,11-octahydronaphtho[1,2-b][1,4]diazepine-2,4(3H,5H)-dione;
(Compound B63) 5-[4-(2,3-dimethylbenzoylamino)-3-hydroxyphenyl]-1H-naphtho[1,2-b][1,4]diazepine-2,4(3H,5H)-dione;
(Compound B64) 5-[4-(2-chloro-3-methoxybenzoylamino)phenyl]-1,3-dihydronaphtho[1,2-e]-1,4-diazepine-2-one;
(Compound B65) 5-[4-[(4-dimethylaminobenzoyl)amino]phenyl]-1H-naphtho[1,2-b][1,4]diazepine-2,4(3H,5H)-dione;
(Compound B66) 5-[4-[2-(2,4-dichlorophenoxy)acetylamino]phenyl]-1H-naphtho[1,2-b][1,4]diazepine-2,4(3H,5H)-dione;
(Compound B67) 5-[4-[2-(2-methylphenoxy)acetylamino]phenyl]-1H-naphtho[1,2-b][1,4]diazepine-2,4(3H,5H)-dione;
(Compound B68) N-[4-(2,4-dioxo-1,2,3,4-tetrahydronaphtho[1,2-b][1,4]diazepin-5-yl)butyl]-2-chloro-3-methoxybenzamide;
(Compound B69) 5-[4-(2-chloro-3-hydroxybenzoylamino)phenyl]-1,3-dihydronaphtho[1,2-e]-1,4-diazepine-2-one;
(Compound B70) 5-[4-(2-acetylbenzoylamino)phenyl]-1H-naphtho[1,2-b][1,4]diazepine-2,4(3H,5H)-dione;
(Compound B71) 5-[4-(2-tert-butylbenzoylamino)phenyl]-1H-naphtho[1,2-b][1,4]diazepine-2,4(3H,5H)-dione;
(Compound B72) 5-[2-(2-iodobenzoyl)aminoethyl]-1H-naphtho[1,2-b][1,4]diazepine-2,4(3H,5H)-dione;
(Compound B73) 5-[3-[(2-iodobenzoyl)amino]phenyl]-1H-naphtho[1,2-b][1,4]diazepine-2,4(3H,5H)-dione;
(Compound B74) 6,7-dimethyl-1-[4-(2-iodobenzoyl)aminophenyl]-1H-1,5-benzodiazepine-2,4(3H,5H)-dione;
(Compound B75) 5-[4-[(1-methylpiperidin-4-yl)carbonylamino]phenyl]-1H-naphtho[1,2-b][1,4]diazepine-2,4(3H,5H)-dione hydrochloride;
(Compound B76) 5[4-[(benzofuran-2-yl)carbonylamino]phenyl]-1H-naphtho[1,2-b][1,4]diazepine-2,4(3H,5H)-dione;
(Compound B77) 5-[4-[(1-methyl-1H-indol-3-yl)carbonylamino]phenyl]-1H-naphtho[1,2-b][1,4]diazepine-2,4(3H,5H)-dione;
(Compound B78) 5-[4-(2-propenylbenzoylamino)phenyl]-1H-naphtho[1,2-b][1,4]diazepine-2,4(3H,5H)-dione;
(Compound B79) 5-[4-(2-propylbenzoylamino)phenyl]-1H-naphtho[1,2-b] [1,4]diazepine-2,4(3H,5H)-dione;
(Compound B80) 5-[3-fluoro-4-(2-iodobenzoylamino)phenyl]-1H-naphtho[1,2-b] [1,4]diazepine-2,4(3H,5H)-dione;
(Compound B81) 5-[4-(2-hydroxy-3-methylbenzoyl)aminophenyl]-1,2,3,4,8,9,10,11-octahydronaphtho[1,2-b][1,4]diazepine-2,4(3H,5H)-dione;
(Compound B82) 5-[4-[(2-isopropoxybenzoyl)amino]phenyl]-1H-naphtho[1,2-b][1,4]diazepine-2,4(3H,5H)-dione;
(Compound B83) 5-[4-[(3-methylthiophen-2-yl)carbonylamino]phenyl]-1H-naphtho[1,2-b][1,4]diazepine-2,4(3H,5H)-dione;
(Compound B84) 5-[4-(2-phenoxypropionylamino)phenyl]-1H-naphtho[1,2-b][1,4]diazepine-2,4(3H,5H)-dione;
(Compound B85) 5-[4-[2-(4-chloro-2-methylphenoxy)acetylamino]phenyl]-1H-naphtho[1,2-b][1,4]diazepine-2,4(3H,5H)-dione;
(Compound B86) 5-[4-[(4-fluoro-2-trifluoromethyl)benzoyl]aminophenyl]-1H-naphtho[1,2-b][1,4]diazepine-2,4(3H,5H)-dione;
(Compound B87) 5-[4-(4-fluoro-2-methoxybenzoyl)aminophenyl]-1H-naphtho[1,2-b][1,4]diazepine-2,4(3H,5H)-dione;
(Compound B88) 5-[4-(4-fluoro-2-hydroxybenzoyl)aminophenyl]-1H-naphtho[1,2-b][1,4]diazepine-2,4(3H,5H)-dione;
(Compound B89) 5-[3-[(2-iodophenylacetyl)amino]phenyl]-1H-naphtho[1,2-b][1,4]diazepine-2,4(3H,5H)-dione;
(Compound B90) 5-[4-(2-methyl-2-phenoxypropionylamino)phenyl]-1H-naphtho[1,2-b][1,4]diazepine-2,4(3H,5H)-dione;
(Compound B91) 5-[4-(2-tert-butylbenzoylamino)phenyl]-1,3-dihydronaphtho[1,2-e]-1,4-diazepine-2-one;
(Compound B92) 5-[4-[(3-dimethylaminobenzoyl)amino]phenyl]-1H-naphtho[1,2-b][1,4]diazepine-2,4(3H,5H)-dione;
(Compound B93) 5-[4-(4-iodo-2-methoxybenzoylamino)phenyl]-1H-naphtho[1,2-b][1,4]diazepine-2,4(3H,5H)-dione;
(Compound B94) 5-[4-(6-fluoro-2-methoxybenzoylamino)phenyl]-1H-naphtho[1,2-b][1,4]diazepine-2,4(3H,5H)-dione;
(Compound B95) 5-[4-(2-hydroxy-4-iodobenzoylamino)phenyl]-1H-naphtho[1,2-b][1,4]diazepine-2,4(3H,5H)-dione;
(Compound B96) 5-[4-(6-fluoro-2-hydroxybenzoylamino)phenyl]-1H-naphtho[1,2-b][1,4]diazepine-2,4(3H,5H)-dione;
(Compound B97) 5-[4-(2-fluorobenzoyl)aminophenyl]-1H-naphtho[1,2-b][1,4]diazepine-2,4(3H,5H)-dione;
(Compound B98) 5-[4-[(2-dimethylaminobenzoyl)amino]phenyl]-1H-naphtho[1,2-b][1,4]diazepine-2,4(3H,5H)-dione;
(Compound B99) 5-[4-(2-methoxy-6-methylbenzoylamino)phenyl]-1H-naphtho[1,2-b][1,4]diazepine-2,4(3H,5H)-dione;
(Compound B100) 5-[4-(2-hydroxy-6-methylbenzoylamino)phenyl]-1H-naphtho[1,2-b][1,4]diazepine-2,4(3H,5H)-dione;
(Compound B101) 5-[4-[3-(2-methylphenyl)propionylamino]phenyl]-1H-naphtho[1,2-b][1,4]diazepine-2,4(3H,5H)-dione;
(Compound B102) 5-(4-phenylcarbamoylphenyl)-1H-naphtho[1,2-b][1,4]diazepine-2,4(3H,5H)-dione;
(Compound B103) 5-(4-benzylcarbamoylphenyl)-1H-naphtho[1,2-b][1,4]diazepine-2,4(3H,5H)-dione;
(Compound B104) 5-[4-[3-(2-methylphenyl)propenoylamino]phenyl]-1H-naphtho[1,2-b][1,4]diazepine-2,4(3H,5H)-dione;
(Compound B105) 5-[4-[3-(2-chlorophenyl)propionylamino]phenyl]-1H-naphtho[1,2-b][1,4]diazepine-2,4(3H,5H)-dione;
(Compound B106) 5-[4-(2-iodobenzoyl)aminophenyl]-1,2,3,4,8,9,10,11-octahydronaphtho[1,2-b][1,4]diazepine-2,4(3H,5H)-dione;
(Compound B107) 5-[4-[(1-methyl-1H-pyrrol-2-ylacetyl)amino]phenyl]-1,2,3,4,8,9,10,11-octahydronaphtho[1,2-b][1,4]diazepine-2,4(3H,5H)-dione;
(Compound B108) 5-[4-(2-chlorobenzyl)carbamoylphenyl]-1H-naphtho[1,2-b][1,4]diazepine-2,4(3H,5H)-dione;
(Compound B109) 5-[4-[3-(2-chlorophenyl)propenoylamino]phenyl]-1H-naphtho[1,2-b][1,4]diazepine-2,4(3H,5H)-dione;
(Compound B110) 5-[4-(2-chlorophenyl)carbamoylphenyl]-1H-naphtho[1,2-b][1,4]diazepine-2,4(3H,5H)-dione;
(Compound B111) 5-[4-(6-bromo-2,3-methylenedioxybenzoylamino)phenyl]-1H-naphtho[1,2-b][1,4]diazepine-2,4(3H,5H)-dione;
(Compound B112) 5-[4-(6-bromo-2-methoxybenzoylamino)phenyl]-1H-naphtho[1,2-b][1,4]diazepine-2,4(3H,5H)-dione;
(Compound B113) 5-[4-[(2-tert-butylbenzoyl)amino]phenyl]-1,2,3,4,8,9,10,11-octahydronaphtho[1,2-b][1,4]diazepine-2,4(3H,5H)-dione;
(Compound B114) 5-[2-(2-iodobenzoyl)aminopyridin-5-yl]-1H-naphtho[1,2-b][1,4]diazepine-2,4(3H,5H)-dione;
(Compound B115) 5-[4-(6-bromo-2-hydroxybenzoylamino)phenyl]-1H-naphtho[1,2-b][1,4]diazepine-2,4(3H,5H)-dione;
(Compound B116) 5-[4-(6-chloro-2-methoxybenzoylamino)phenyl]-1H-naphtho[1,2-b][1,4]diazepine-2,4(3H,5H)-dione;
(Compound B117) 5-[4-(2-iodobenzoylamino)phenyl]-1H-[1,4]diazepino[2,3-h]quinoline-2,4(3H,5H)-dione;
(Compound B118) 5-[4-(6-chloro-2-hydroxybenzoylamino)phenyl]-1H-naphtho[1,2-b][1,4]diazepine-2,4(3H,5H)-dione;
(Compound B119) 5-[4-(2-hydroxy-6-methoxybenzoylamino)phenyl]-1H-naphtho[1,2-b][1,4]diazepine-2,4(3H,5H)-dione;
(Compound B120) 5-[4-[2-methoxy-6-(trifluoromethyl)benzoylamino]phenyl]-1H-naphtho[1,2-b][1,4]diazepine-2,4(3H,5H)-dione;
(Compound B121) 5-[4-[2-hydroxy-6-(trifluoromethyl)benzoylamino]phenyl]-1H-naphtho[1,2-b][1,4]diazepine-2,4(3H,5H)-dione;
(Compound B122) 5-[4-[(2-isopropenylbenzoyl)amino]phenyl]-1,2,3,4,8,9,10,11-octahydronaphtho[1,2-b][1,4]diazepine-2,4(3H,5H)-dione;
(Compound B123) 5-[4-[(2-isopropylbenzoyl)amino]phenyl]-1,2,3,4,8,9,10,11-octahydronaphtho[1,2-b][1,4]diazepine-2,4(3H,5H)-dione;
(Compound B124) 5-[4-[2-chloro-5-(methylthio)benzoylamino]phenyl]-1H-naphtho[1,2-b][1,4]diazepine-2,4(3H,5H)-dione;
(Compound B125) 5-[4-[2-(methylthio)benzoylamino]phenyl]-1H-naphtho[1,2-b][1,4]diazepine-2,4(3H,5H)-dione;
(Compound B126) 5-[4-[3-(methylthio)benzoylamino]phenyl]-1H-naphtho[1,2-b][1,4]diazepine-2,4(3H,5H)-dione;
(Compound B127) 5-[4-[2-ethyl-6-methoxybenzoylamino]phenyl]-1H-naphtho[1,2-b][1,4]diazepine-2,4(3H,5H)-dione;
(Compound B128) 5-[4-(3-methanesulfonylbenzoylamino)phenyl]-1H-naphtho[1,2-b][1,4]diazepine-2,4(3H,5H)-dione;
(Compound B129) 6-ethyl-1-[4-(2-iodobenzoyl)aminophenyl]-1H-1,5-benzodiazepine-2,4(3H,5H)-dione;
(Compound B130) 5-[4-[2-ethyl-6-hydroxybenzoylamino]phenyl]-1H-naphtho[1,2-b][1,4]diazepine-2,4(3H,5H)-dione;
(Compound B131) 5-[4-(3-methanesulfinylbenzoylamino)phenyl]-1H-naphtho[1,2-b][1,4]diazepine-2,4(3H,5H)-dione;
(Compound B132) 5-[4-(2-chloro-5-methanesulfinylbenzoylamino)phenyl]-1H-naphtho[1,2-b][1,4]diazepine-2,4(3H,5H)-dione;
(Compound B133) 5-[4-(2-methanesulfinylbenzoylamino)phenyl]-1H-naphtho[1,2-b][1,4]diazepine-2,4(3H,5H)-dione;
(Compound B134) 5-[4-[[2-(4-morpholinyl)acetyl]amino]phenyl]-1,2,3,4,8,9,10,11-octahydronaphtho[1,2-b][1,4]diazepine-2,4(3H,5H)-dione hydrochloride;
(Compound B135) 5-[4-(2-chloro-6-methoxybenzoylamino)phenyl]-1,3-dihydronaphtho[1,2-e]-1,4-diazepine-2-one;
(Compound B136) 5-[4-[[(3-chloropyridin-2-yl)carbonyl]amino]phenyl]-1H-naphtho[1,2-b][1,4]diazepine-2,4(3H,5H)-dione;
(Compound B137) 5-[4-(2-chloro-6-hydroxybenzoylamino)phenyl]-1,3-dihydronaphtho[1,2-e]-1,4-diazepine-2-one;
(Compound B138) 5-[4-(3-chloro-2-methoxybenzoylamino)phenyl]-1H-naphtho[1,2-b][1,4]diazepine-2,4(3H,5H)-dione;
(Compound B139) 5-[4-[(3-methylpyridin-2-yl)carbonylamino]phenyl]-1H-naphtho[1,2-b][1,4]diazepine-2,4(3H,5H)-dione;
(Compound B140) 5-[4-[[(3-chloropyridin-2-yl)carbonyl]amino]phenyl]-1,2,3,4,8,9,10,11-octahydronaphtho[1,2-b][1,4]diazepine-2,4(3H,5H)-dione;
(Compound B141) 5-[4-(3-chloro-2-hydroxybenzoylamino)phenyl]-1H-naphtho[1,2-b][1,4]diazepine-2,4(3H,5H)-dione;
(Compound B142) 5-[4-[[(3-hydroxypyridin-2-yl)carbonyl]amino]phenyl]-1H-naphtho[1,2-b][1,4]diazepine-2,4(3H,5H)-dione;
(Compound B143) 5-[4-[(3-vinylpyridin-2-yl)carbonylamino]phenyl]-1H-naphtho[1,2-b][1,4]diazepine-2,4(3H,5H)-dione;
(Compound B144) 5-[4-[(3-ethylpyridin-2-yl)carbonylamino]phenyl]-1H-naphtho[1,2-b][1,4]diazepine-2,4(3H,5H)-dione;
(Compound B145) N-[4-(2,4-dioxo-1,2,3,4-tetrahydronaphtho-[1,2-b][1,4]-diazepin-5-yl)phenyl]-2-nitrobenzenesulfonamide;
(Compound B146) N-[4-(2,4-dioxo-1,2,3,4-tetrahydronaphtho[1,2-b][1,4]diazepin-5-yl)phenyl]benzenesulfonamide;
(Compound B147) 3-bromo-N-[4-(2,4-dioxo-1,2,3,4-tetrahydronaphtho[1,2-b][1,4]diazepin-5-yl)phenyl]benzenesulfonamide;
(Compound B148) N-[4-(2,4-dioxo-1,2,3,4-tetrahydronaphtho[1,2-b][1,4]diazepin-5-yl)phenyl]-3-methoxybenzenesulfonamide;
(Compound B149) N-[3-(2-oxo-2,3-dihydro-1H-naphtho[1,2-e][1,4]diazepin-5-yl)phenyl]benzenesulfonamide;
(Compound B150) N-[3-(2,4-dioxo-1,2,3,4-tetrahydronaphtho-[1,2-b][1,4]-diazepin-5-yl)phenyl]-2-nitrobenzenesulfonamide;
(Compound B151) N-[4-(2,4-dioxo-1,2,3,4,8,9,10,11-octahydro-naphtho[1,2-b][1,4]-diazepin-5-yl)phenyl]-2-nitrobenzenesulfonamide;
(Compound B152) N-[4-(2,4-dioxo-1,2,3,4,8,9,10,11-octahydro-naphtho[1,2-b][1,4]-diazepin-5-yl)phenyl]-N-methyl-2-nitrobenzenesulfonamide;
(Compound B153) N-[3-(2,4-dioxo-1,2,3,4-tetrahydronaphtho-[1,2-b][1,4]-diazepin-5-yl)phenyl]-N-methyl-2-nitrobenzenesulfonamide;
(Compound B154) 4-(2,4-dioxo-1,2,3,4,8,9,10,11-octahydronaphtho[1,2-b][1,4]diazepin-5-yl)-N-phenylbenzenesulfonamide;
(Compound B155) N-[3-(2,4-dioxo-1,2,3,4-tetrahydronaphtho[1,2-b]-[1,4]diazepin-5-yl)phenyl]-2-naphthalenesulfonamide;
(Compound B156) N-[3-(2,4-dioxo-1,2,3,4-tetrahydronaphtho[1,2-b]-[1,4]diazepin-5-yl)phenyl]-1-naphthalenesulfonamide;
(Compound B157) N-[4-(2,4-dioxo-1,2,3,4-tetrahydronaphtho[1,2-b][1,4]diazepin-5-yl)phenyl]cyclohexanesulfonamide;
(Compound B158) N-[4-(2,4-dioxo-1,2,3,4-tetrahydronaphtho[1,2-b][1,4]diazepin-5-yl)phenyl]-3-pyridinesulfonamide hydrochloride;
(Compound B159) N-[4-(2,4-dioxo-1,2,3,4-tetrahydronaphtho[1,2-b][1,4]diazepin-5-yl)phenyl]-4-isopropylbenzenesulfonamide;
(Compound B160) N-[4-(2,4-dioxo-1,2,3,4-tetrahydronaphtho[1,2-b][1,4]diazepin-5-yl)phenyl]phenylmethanesulfonamide;
(Compound B161) N-[4-(2,4-dioxo-1,2,3,4-tetrahydronaphtho[1,2-b][1,4]diazepin-5-yl)phenyl]-2-thiophenesulfonamide;
(Compound B162) N-[4-(2,4-dioxo-1,2,3,4-tetrahydronaphtho[1,2-b][1,4]diazepin-5-yl)phenyl]-2-naphthalenesulfonamide;
(Compound B163) 4-(2,4-dioxo-1,2,3,4,8,9,10,11-octahydronaphtho-[1,2-b][1,4]diazepin-5-yl)phenyl 3-bromobenzene-sulfonate;
(Compound B164) N-benzyl-N-[4-(1-benzyl-2,4-dioxo-1,2,3,4-tetrahydronaphtho[1,2-b][1,4]diazepin-5-yl)phenyl]-2-nitrobenzenesulfonamide;
(Compound B165) N-benzyl-N-[4-(2,4-dioxo-1,2,3,4-tetrahydronaphtho[1,2-b][1,4]diazepin-5-yl)phenyl]-2-nitrobenzenesulfonamide;
(Compound B166) 3-bromo-N-[4-(2,4-dioxo-1,2,3,4-tetrahydronaphtho[1,2-b][1,4]diazepin-5-yl)phenyl]-N-methylbenzenesulfonamide;
(Compound B167) N-[4-(2,4-dioxo-1,2,3,4-tetrahydronaphtho-[1,2-b][1,4]-diazepin-5-yl)phenyl]-N-methyl-2-nitrobenzenesulfonamide;
(Compound B168) N-[4-(2,4-dioxo-1,2,3,4-tetrahydronaphtho-[1,2-b][1,4]-diazepin-5-yl)phenyl]-N-(2-hydroxyethyl)-2-nitrobenzenesulfonamide;
(Compound B169) N-[4-(7-chloro-2,4-dioxo-2,3,4,5-tetrahydro-1H-benzo[b][1,4]diazepin-1-yl)phenyl]benzenesulfonamide;
(Compound B170) N-[4-(7-bromo-2,4-dioxo-2,3,4,5-tetrahydro-1H-benzo[b][1,4]diazepin-1-yl)phenyl]benzenesulfonamide;
(Compound B171) N-[4-[(2,4-dioxo-7-(trifluoromethyl)-2,3,4,5-tetrahydro-1H-benzo[b][1,4]diazepin-1-yl)]phenyl]benzenesulfonamide;
(Compound B172) N-[4-(2,4-dioxo-2,3,4,5-tetrahydro-1H-benzo[b][1,4]diazepin-1-yl)phenyl]benzenesulfonamide;
(Compound B173) 1-(2-chlorophenyl)-N-[4-(2,4-dioxo-1,2,3,4-tetrahydronaphtho[1,2-b][1,4]diazepin-5-yl)phenyl]methanesulfonamide;
(Compound B174) 1-(3-bromophenyl)-N-[4-(2,4-dioxo-1,2,3,4-tetrahydronaphtho[1,2-b][1,4]diazepin-5-yl)phenyl]methanesulfonamide;
(Compound B175) N-[4-(2,4-dioxo-1,2,3,4-tetrahydronaphtho-[1,2-b][1,4]-diazepin-5-yl)phenyl]-2-trifluoromethylbenzenesulfonamide;
(Compound B176) N-[4-(7-bromo-6-methyl-2,4-dioxo-2,3,4,5-tetrahydro-1H-benzo[b][1,4]diazepin-1-yl)phenyl]benzenesulfonamide;
(Compound B177) 1-(2-chlorophenyl)-N-[4-(2,4-dioxo-1,2,3,4,8,9,10,11-octahydronaphtho[1,2-b][1,4]diazepin-5-yl)phenyl]methanesulfonamide;
(Compound B178) 3-bromo-N-[4-(2,4-dioxo-1,2,3,4,8,9,10,11-octahydronaphtho[1,2-b][1,4]diazepin-5-yl)phenyl]benzenesulfonamide;
(Compound B179) N-[4-(2,4-dioxo-1,2,3,4,8,9,10,11-octahydronaphtho[1,2-b][1,4]diazepin-5-yl)phenyl]-3-methoxybenzenesulfonamide;
(Compound B180) 1-(2-bromophenyl)-N-[4-(2,4-dioxo-1,2,3,4-tetrahydronaphtho[1,2-b][1,4]diazepin-5-yl)phenyl]methanesulfonamide;
(Compound B181) N-[4-(2,4-dioxo-1,2,3,4-tetrahydronaphtho[1,2-b][1,4]diazepin-5-yl)phenyl]-1-(2-methylphenyl)methanesulfonamide;
(Compound B182) N-[4-(2,4-dioxo-1,2,3,4-tetrahydronaphtho[1,2-b][1,4]diazepin-5-yl)phenyl]-1-(2-nitrophenyl)methanesulfonamide;
(Compound B183) N-[4-(2,4-dioxo-1,2,3,4-tetrahydronaphtho[1,2-b][1,4]diazepin-5-yl)phenyl]-2-phenylethanesulfonamide;
(Compound B184) 1-(2,3-dichlorophenyl)-N-[4-(2,4-dioxo-1,2,3,4,8,9,10,11-octahydronaphtho[1,2-b][1,4]diazepin-5-yl)phenyl]methanesulfonamide;
(Compound B185) 1-(2-chlorophenyl)-N-[4-(2,4-dioxo-7-methoxy-1H-benzo[1,2-b][1,4]diazepin-1-yl)phenyl]methanesulfonamide;
(Compound B186) 1-(2-chlorophenyl)-N-[4-(2,4-dioxo-7-hydroxy-1H-benzo[1,2-b][1,4]diazepin-1-yl)phenyl]methanesulfonamide;
(Compound B187) 1-(4-chlorophenyl)-N-[4-(2,4-dioxo-1,2,3,4-tetrahydronaphtho[1,2-b][1,4]diazepin-5-yl)phenyl]methanesulfonamide;
(Compound B188) 1-(2-chlorophenyl)-N-[4-(2,4-dioxo-1,2,3,4-tetrahydronaphtho[1,2-b][1,4]diazepin-5-yl)benzyl]methanesulfonamide;
(Compound B189) 1-(2-chlorophenyl)-N-[4-(2,4-dioxo-1,2,3,4-tetrahydronaphtho[1,2-b][1,4]diazepin-5-yl)-2-methoxyphenyl]methanesulfonamide;
(Compound B190) 1-(2-chlorophenyl)-N-[4-(2,4-dioxo-1,2,3,4-tetrahydronaphtho[1,2-b][1,4]diazepin-5-yl)-2-hydroxyphenyl]methanesulfonamide;
(Compound B191) 1-(2,6-dichlorophenyl)-N-[4-(2,4-dioxo-1,2,3,4,8,9,10,11-octahydronaphtho[1,2-b][1,4]diazepin-5-yl)phenyl]methanesulfonamide;
(Compound B192) 1-(2-chlorophenyl)-N-[4-(2,4-dioxo-6-methyl-1H-benzo[1,2-b][1,4]diazepin-1-yl)phenyl]methanesulfonamide;
(Compound B193) 1-(2-chlorophenyl)-N-[3-(2,4-dioxy-1,2,3,4-tetrahydronaphtho[1,2-b][1,4]diazepin-5-yl)propyl]methanesulfonamide;
(Compound B194) 1-(2-chlorophenyl)-N-[2-(2,4-dioxo-1,2,3,4-tetrahydronaphtho[1,2-b][1,4]diazepin-5-yl)ethyl]methanesulfonamide;
(Compound B195) N-[4-(2,4-dioxo-1,2,3,4,8,9,10,11-octahydronaphtho[1,2-b][1,4]diazepin-5-yl)phenyl]-1-(2-iodophenyl)methanesulfonamide;
(Compound B196) 1-(2-chlorophenyl)-N-[4-(2,4-dioxo-1,2,3,4,8,9,10,11-octahydronaphtho[1,2-b][1,4]diazepin-5-yl)phenyl]-N-methylmethanesulfonamide;
(Compound B197) 1-(2-chlorophenyl)-N-[4-(2-oxo-2,3-dihydro-1H-naphtho[1,2-e][1,4]diazepin-5-yl)phenyl]methanesulfonamide;
(Compound B198) 1-[2-(trifluoromethyl)phenyl]-N-[4-(2,4-dioxo-1,2,3,4-tetrahydronaphtho[1,2-b][1,4]diazepin-5-yl)phenyl]phenyl-N-methylmethanesulfonamide;
(Compound B199) 1-(2-ethylphenyl)-N-[4-(2,4-dioxo-1,2,3,4-tetrahydronaphtho[1,2-b][1,4]diazepin-5-yl)phenyl]phenyl-N-methylmethanesulfonamide;
(Compound B200) 1-(2,3-dimethylphenyl)-N-[4-(2,4-dioxo-1,2,3,4-tetrahydronaphtho[1,2-b][1,4]diazepin-5-yl)phenyl]phenyl-N-methylmethanesulfonamide;
(Compound B201) 2-(2-chlorophenyl)-N-[4-(2,4-dioxo-1,2,3,4-tetrahydronaphtho[1,2-b][1,4]diazepin-5-yl)phenyl]phenyl-N-methylethanesulfonamide;
(Compound B202) 1-(2-nitrophenyl)-N-[4-(2,4-dioxo-1,2,3,4-tetrahydronaphtho[1,2-b][1,4]diazepin-5-yl)phenyl]phenyl-N-methylmethanesulfonamide;
(Compound B203) 1-(2-aminophenyl)-N-[4-(2,4-dioxo-1,2,3,4-tetrahydronaphtho[1,2-b][1,4]diazepin-5-yl)phenyl]phenyl-N-methylmethanesulfonamide;
(Compound B204) 1-(2-dimethylaminophenyl)-N-[4-(2,4-dioxo-1,2,3,4-tetrahydronaphtho[1,2-b][1,4]diazepin-5-yl)phenyl]phenyl-N-methylmethanesulfonamide;
(Compound B205) 5-[4-[(pyridin-4-yl)carbonylamino]phenyl]-1H-naphtho[1,2-b][1,4]diazepine-2,4(3H,5H)-dione hydrochloride;
(Compound B206) 5-[4-[2-[(pyridin-3-yl)oxy]acetylamino]phenyl]-1H-naphtho[1,2-b][1,4]diazepine-2,4(3H,5H)-dione hydrochloride;
(Compound B207) 5-[4-[(pyridin-3-yl)carbonylamino]phenyl]-1H-naphtho[1,2-b][1,4]diazepine-2,4(3H,5H)-dione hydrochloride;
(Compound B208) 5-[4-[(2-methylpyridin-3-yl)carbonylamino]phenyl]-1H-naphtho[1,2-b][1,4]diazepine-2,4(3H,5H)-dione hydrochloride;
(Compound B209) 5-[4-[(2-chloropyridin-3-yl)carbonylamino]phenyl]-1H-naphtho[1,2-b][1,4]diazepine-2,4(3H,5H)-dione;
(Compound B210) 5-[4-[2-[(pyridin-2-yl)oxy]acetylamino]phenyl]-1H-naphtho[1,2-b][1,4]diazepine-2,4(3H,5H)-dione;
(Compound B211) 5-[4-[[4-(trifluoromethyl)pyridin-3-yl]carbonylamino]phenyl]-1H-naphtho[1,2-b][1,4]diazepine-2,4(3H,5H)-dione;
(Compound B212) 5-[4-[(2-chloropyridin-3-yl)carbonylamino]phenyl]-1H-[1,4]diazepino[2,3-f]isoquinoline-2,4(3H,5H)-dione;
(Compound B213) 5-[4-[(2-chloropyridin-3-yl)carbonylamino]phenyl]-8,9,10,11-tetrahydro-1H-[1,4]diazepino[2,3-f]isoquinoline-2,4(3H,5H)-dione; and
(Compound B214) 5-[4-[(2-isopropylbenzoyl)amino]phenyl]-1H-naphtho[1,2-b][1,4]diazepine-2,4(3H,5H)-dione

A more preferable compound or a pharmaceutically acceptable salt thereof as the active ingredient of the pharmaceutical composition of the present invention is comprised in the compounds represented by the general formulas (A) to (BII), or a pharmaceutically acceptable salt thereof. Specific examples of the more preferable compound or a pharmaceutically acceptable salt thereof can comprise 5-[3-(1H-tetrazol-5-yl)phenyl]-1H-naphtho[1,2-b][1,4]diazepine-2,4(3H,5H)-dione; a 5-[3-(1H-tetrazol-5-yl)phenyl]-1H-naphtho[1,2-b][1,4]diazepine-2,4(3H,5H)-dione sodium salt; a 5-[3-(1H-tetrazol-5-yl)phenyl]-1H-naphtho[1,2-b][1,4]diazepine-2,4(3H,5H)-dione potassium salt; 5-[3-(5-thioxo-4H-[1,2,4]oxadiazol-3-yl)phenyl]-1H-naphtho[1,2-b][1,4]diazepine-2,4(3H,5H)-dione; a 5-[3-(5-thioxo-4H-[1,2,4]oxadiazol-3-yl)phenyl]-1H-naphtho[1,2-b][1,4]diazepine-2,4(3H,5H)-dione sodium salt; 5-[4-fluoro-3-(1H-tetrazol-5-yl)phenyl]-1H-naphtho[1,2-b][1,4]diazepine-2,4(3H,5H)-dione; a 5-[4-fluoro-3-(1H-tetrazol-5-yl)phenyl]-1H-naphtho[1,2-b][1,4]diazepine-2,4(3H,5H)-dione sodium salt; 5-[4-[2-(trifluoromethyl)benzoyl]aminophenyl]-1H-naphtho[1,2-b] [1,4]diazepine-2,4(3H,5H)-dione; 5-[4-[(2-chlorophenylacetyl)amino]phenyl]-1H-naphtho[1,2-b] [1,4]diazepine-2,4(3H,5H)-dione; 1-[4-(2,4-dioxo-1,2,3,4-tetrahydronaphtho[1,2-b] [1,4]diazepin-5-yl)phenyl]-3-phenylurea; 5-[4-(2-iodobenzoylamino)phenyl]-1H-naphtho[1,2-b] [1,4]diazepine-2,4(3H,5H)-dione; 5-[4-[(2-ethylbenzoyl)amino]phenyl]-1H-naphtho[1,2-b] [1,4]diazepine-2,4(3H,5H)-dione; 5-[4-(2-tert-butylbenzoylamino)phenyl]-1H-naphtho[1,2-b] [1,4]diazepine-2,4(3H,5H)-dione; 5-[4-(2-iodobenzoyl)aminophenyl]-1,2,3,4,8,9,10,11-octahydronaphtho[1,2-b] [1,4]diazepine-2,4(3H,5H)-dione; 5-[4-(6-chloro-2-hydroxybenzoylamino)phenyl]-1H-naphtho[1,2-b] [1,4]diazepine-2,4(3H,5H)-dione; N-[4-(2,4-dioxo-1,2,3,4-tetrahydronaphtho[1,2-b] [1,4]diazepin-5-yl)phenyl]benzenesulfonamide; 1-(2-chlorophenyl)-N-[4-(2,4-dioxo-1,2,3,4-tetrahydronaphtho[1,2-b] [1,4]diazepin-5-yl)phenyl]methanesulfonamide; 1-(2-chlorophenyl)-N-[4-(2,4-dioxo-1,2,3,4,8,9,10,11-octahydronaphtho[1,2-b] [1,4]diazepin-5-yl)phenyl]methanesulfonamide; 1-(2-bromophenyl)-N-[4-(2,4-dioxo-1,2,3,4-tetrahydronaphtho[1,2-b][1,4]diazepin-5-yl)phenyl]methanesulfonamide; N-[4-(2,4-dioxo-1,2,3,4-tetrahydronaphtho[1,2-b] [1,4]diazepin-5-yl)phenyl]-1-(2-methylphenyl)methanesulfonamide; N-[4-(2,4-dioxo-1,2,3,4-tetrahydronaphtho[1,2-b][1,4]diazepin-5-yl)phenyl]-2-phenylethanesulfonamide; 1-(2-chlorophenyl)-N-[4-(2,4-dioxo-1,2,3,4,8,9,10,11-octahydronaphtho[1,2-b][1,4]diazepin-5-yl)phenyl]-N-methylmethanesulfonamide; 1-(2-chlorophenyl)-N-[4-(2-oxo-2,3-dihydro-1H-naphtho[1,2-e][1,4]diazepin-5-yl)phenyl]methanesulfonamide; 5-[4-[(2-methylpyridin-3-yl)carbonylamino]phenyl]-1H-naphtho[1,2-b][1,4]diazepine-2,4(3H,5H)-dione hydrochloride; 5-[4-[(2-chloropyridin-3-yl)carbonylamino]phenyl]-1H-naphtho[1,2-b][1,4]diazepine-2,4(3H,5H)-dione; 5-[4-[2-[(pyridin-2-yl)oxy]acetylamino]phenyl]-1H-naphtho[1,2-b][1,4]diazepine-2,4(3H,5H)-dione; and 5-[4-[(2-isopropylbenzoyl)amino]phenyl]-1H-naphtho[1,2-b][1,4]diazepine-2,4(3H,5H)-dione. Note that the active ingredient of the pharmaceutical composition of the present invention is not limited to the above specific compounds or a pharmaceutically acceptable salt thereof.

An even more preferable compound or a pharmaceutically acceptable salt thereof as the active ingredient of the pharmaceutical composition of the present invention is comprised in the compounds represented by the general formulas (A) to (BII), or a pharmaceutically acceptable salt thereof. Specific examples of the even more preferable compound or a pharmaceutically acceptable salt thereof can comprise 5-[3-(1H-tetrazol-5-yl)phenyl]-1H-naphtho[1,2-b][1,4]diazepine-2,4(3H,5H)-dione; a 5-[3-(1H-tetrazol-5-yl)phenyl]-1H-naphtho[1,2-b][1,4]diazepine-2,4(3H,5H)-dione sodium salt; 5-[3-(5-thioxo-4H-[1,2,4]oxadiazol-3-yl)phenyl]-1H-naphtho[1,2-b][1,4]diazepine-2,4(3H,5H)-dione; a 5-[3-(5-thioxo-4H-[1,2,4]oxadiazol-3-yl)phenyl]-1H-naphtho[1,2-b][1,4]diazepine-2,4(3H,5H)-dione sodium salt; 5-[4-[2-(trifluoromethyl)benzoyl]aminophenyl]-1H-naphtho[1,2-b][1,4]diazepine-2,4(3H,5H)-dione; 5-[4-(2-iodobenzoylamino)phenyl]-1H-naphtho[1,2-b][1,4]diazepine-2,4(3H,5H)-dione; and 5-[4-(6-chloro-2-hydroxybenzoylamino)phenyl]-1H-naphtho[1,2-b][1,4]diazepine-2,4(3H,5H)-dione. Note that the active ingredient of the pharmaceutical composition of the present invention is not limited to the above specific compounds or a pharmaceutically acceptable salt thereof.

As an active ingredient of the pharmaceutical composition of the present invention, a compound represented by the following general formula (C), or a pharmaceutically acceptable salt thereof, can be used. These compounds can be produced by a known method (Patent Literature 4). 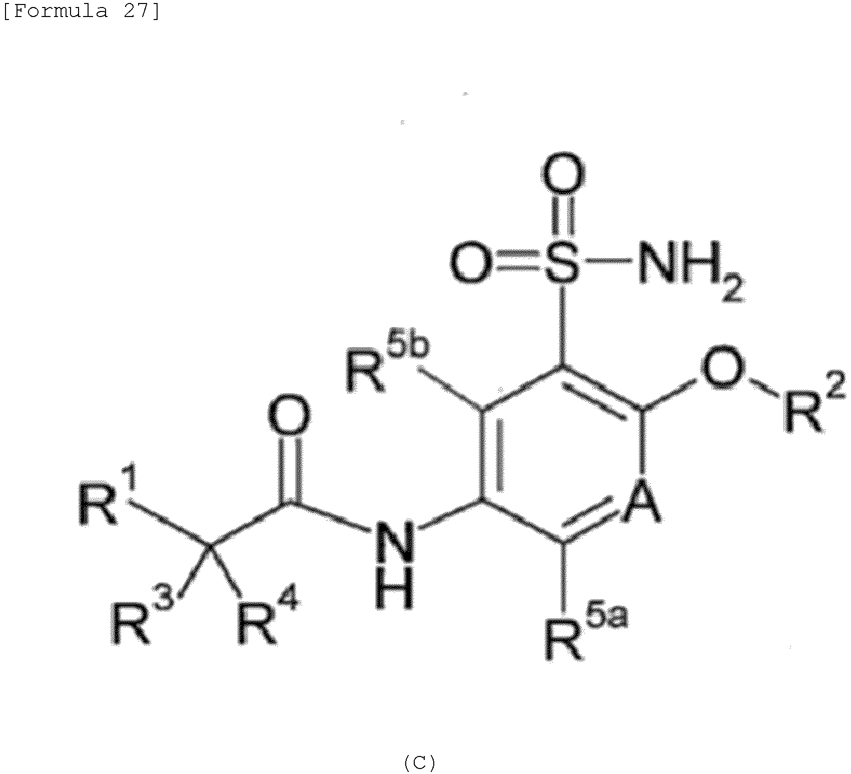 In the formula
A represents CR⁵ or N;
R¹ represents a group selected from 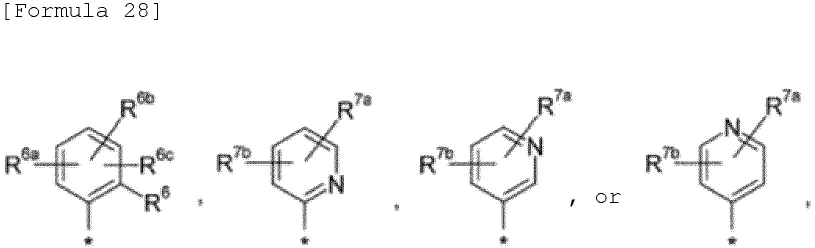 (In the formula, * represents the point of attachment of the group to the remainder of the molecule);
   R² represents cycloalkyl having 3 to 6 carbon atoms, cycloalkyl having 3 to 6 carbon atoms-alkyl having 1 to 4 carbon atoms, four to six membered heterocycloalkyl, four to six membered heterocycloalkyl-alkyl having 1 to 4 carbon atoms, phenyl, phenyl-alkyl having 1 to 4 carbon atoms, heteroaryl, or heteroaryl-alkyl having 1 to 4 carbon atoms,
   in which the groups are optionally substituted 1 to 4 times, independently of one another, with the same or different R¹¹(s),
   optionally substituted 1 time with R^{11a} and 1 or 2 times, independently of one another, with the same or different R¹¹(s),
   substituted together with two adjacent substituents R¹¹s representing a methylenedioxy group to form a five membered ring, or
   substituted with one to five deuterium atoms and optionally substituted 1 or 2 times, independently of one another, with the same or different R¹¹(s);
   R² represents a branched (alkyl having 1 to 4 carbon atoms)-alkyl having 1 to 4 carbon atoms;
   R³ represents hydrogen, deuterium, fluoro, or methyl;
   R⁴ represents hydrogen, deuterium, or fluoro;
   R⁵, R^{5a}, and R^{5b} are the same or different and independently represent hydrogen, halogen, alkyl having 1 to 4 carbon atoms, haloalkyl having 1 to 4 carbon atoms, alkoxy having 1 to 4 carbon atoms, or haloalkoxy having 1 to 4 carbon atoms;
   R⁶, R^{6a}, R^{6b}, and R^{6c} are the same or different and are independently of one another,
   in which R⁶ represents hydrogen, halogen, cyano, alkyl having 1 to 4 carbon atoms, haloalkyl having 1 to 4 carbon atoms, alkoxy having 1 to 4 carbon atoms, haloalkoxy having 1 to 4 carbon atoms, HO- (alkoxy having 2 to 4 carbon atoms)-, (alkoxy having 1 to 4 carbon atoms)-(alkoxy having 2 to 4 carbon atoms)-, or F₃C-S-;
   R^{6a} represents hydrogen, halogen, hydroxy, nitro, cyano, alkyl having 1 to 4 carbon atoms, cycloalkyl having 3 to 6 carbon atoms, haloalkyl having 1 to 4 carbon atoms, alkoxy having 1 to 4 carbon atoms, haloalkoxy having 1 to 4 carbon atoms, HO- (alkoxy having 2 to 4 carbon atoms)-, (alkoxy having 1 to 4 carbon atoms)-(alkoxy having 2 to 4 carbon atoms)-, R⁹R¹⁰N-, R⁸-C(O)-NH-, R⁸-C(O)-, R⁸-O-C(O)-, R⁹R¹⁰N-C(O)-, or (alkyl having 1 to 4 carbon atoms)-SO₂-;
   R^{6b} represents hydrogen, halogen, hydroxy, nitro, cyano, alkyl having 1 to 4 carbon atoms, cycloalkyl having 3 to 6 carbon atoms, haloalkyl having 1 to 4 carbon atoms, haloalkoxy having 1 to 4 carbon atoms, HO- (alkoxy having 2 to 4 carbon atoms)-, (alkoxy having 1 to 4 carbon atoms)-(alkoxy having 2 to 4 carbon atoms)-, R⁹R¹⁰N-, R⁸-C(O)-NH-, R⁸-C(O)-, R⁸-O-C(O)-, R⁹R¹⁰N-C(O)-, or (alkyl having 1 to 4 carbon atoms)-SO₂-; or
   R^{6a} and R^{6b} adjacent to each other together represent a group selected from -O-CH₂-CH₂-, -O-CH₂-O-, or -O-CH₂-CH₂-O-;
   R^{6c} represents hydrogen or halogen;
   R^{7a} and R^{7b} are the same or different and independently represent hydrogen, hydroxy, halogen, alkyl having 1 to 4 carbon atoms, or haloalkyl having 1 to 4 carbon atoms;
   R⁸, independently from each respective occurrence, represents alkyl having 1 to 6 carbon atoms, alkoxy having 1 to 4 carbon atoms-alkyl having 1 to 4 carbon atoms, cycloalkyl having 3 to 6 carbon atoms, or haloalkyl having 1 to 4 carbon atoms;
   R⁹ and R¹⁰ are the same or different and independently represent hydrogen, alkyl having 1 to 4 carbon atoms, cycloalkyl having 3 to 6 carbon atoms, haloalkyl having 1 to 4 carbon atoms, (alkoxy having 1 to 4 carbon atoms)-(alkyl having 2 to 4 carbon atoms), phenyl, or a heteroaryl group, in which the phenyl and heteroaryl group are optionally substituted 1 to 3 times, independently of one another, with hydrogen, halogen, alkyl having 1 to 4 carbon atoms, haloalkyl having 1 to 4 carbon atoms, alkoxy having 1 to 4 carbon atoms, or haloalkoxy having 1 to 4 carbon atoms,
   R^{9a} and R^{10a}, together with the nitrogen atom to which they are bonded, form a four to six membered nitrogen-containing heterocyclic ring, in which the ring optionally contains one further heteroatom selected from O, NH, NR^{a} (In the formula R^{a} represents an alkyl-group having 1 to 6 carbon atoms or a haloalkyl-group having 1 to 6 carbon atoms), or S and is optionally substituted 1 to 3 times, independently of one another, with halogen or alkyl having 1 to 4 carbon atoms;
   R¹¹ independently represents halogen, hydroxy, nitro, cyano, alkyl having 1 to 4 carbon atoms, alkenyl having 2 to 4 carbon atoms, haloalkyl having 1 to 4 carbon atoms, hydroxyalkyl having 1 to 4 carbon atoms, alkoxy having 1 to 4 carbon atoms, haloalkoxy having 1 to 4 carbon atoms, HO- (alkoxy having 2 to 4 carbon atoms)-, (alkoxy having 1 to 4 carbon atoms)-(alkoxy having 2 to 4 carbon atoms)-, (haloalkyl having 1 to 4 carbon atoms)-S-, R⁹R¹⁰N-, R⁸-C(O)-NH-, R⁸-C(O)-, R⁸-O-C(O)-, R⁹R¹⁰N-C(O)-, or (alkyl having 1 to 4 carbon atoms)-SO₂-; and
   R^{11a} represents cycloalkyl having 3 to 6 carbon atoms, morpholino, R^{9a}R^{10a}N-, or R^{9a}R^{10a} N-C(O)-; or represents a group selected from five or six membered heteroaryl optionally substituted with methyl, or represents

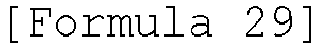

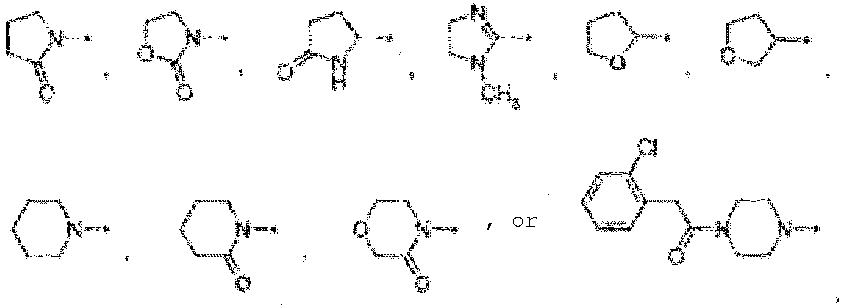

(In the formula * represents the point of attachment of the group to the remainder of the molecule).

Examples of the compound of the general formula (C) include the following compounds. That is, examples of the compound suitable as the active ingredient of the pharmaceutical composition of the present invention include the following compounds (C1) to (C297) or a pharmaceutically acceptable salt thereof. These compounds can be produced by a known method (Patent Literature 4).
(C1) N-[4-(3-chloro-5-cyanophenoxy)-3-sulfamoylphenyl]-2-(2-chlorophenyl)acetamide
(C2) 2-(2-chlorophenyl)-N-4-[3-(dimethylamino)phenoxy]-3-sulfamoylphenylacetamide (C3) 2-(2-chlorophenyl)-N-4-[(2-chloropyridin-4-yl)oxy]-3-sulfamoylphenylacetamide
(C4) 2-(2-chlorophenyl)-N-[4-(3-isopropylphenoxy)-3-sulfamoylphenyl]acetamide
(C5) 2-(2-chlorophenyl)-N-3-sulfamoyl-4-[3-(trifluoromethyl)phenoxy]phenylacetamide
(C6) 2-(2-chlorophenyl)-N-3-sulfamoyl-4-[3-(trifluoromethoxy)phenoxy]phenylacetamide
(C7) N-[4-(3-acetylphenoxy)-3-sulfamoylphenyl]-2-(2-chlorophenyl) acetamide
(C8) N-[4-(1,3-benzodioxol-5-yloxy)-3-sulfamoylphenyl]-2-(2-chlorophenyl)acetamide
(C9) N-[4-(3-acetamidophenoxy)-3-sulfamoylphenyl]-2-(2-chlorophenyl) acetamide
(C10) 2-(2-chlorophenyl)-N-[4-(2-fluorophenoxy)-3-sulfamoylphenyl]acetamide
(C11) 2-(2-chlorophenyl)-N-[4-(3-fluorophenoxy)-3-sulfamoylphenyl]acetamide
(C12) 2-(2-chlorophenyl)-N-[4-(4-fluorophenoxy)-3-sulfamoylphenyl]acetamide
(C13) 2-(2-chlorophenyl)-N-[4-(pyridin-2-yloxy)-3-sulfamoylphenyl]acetamide
(C14) 2-(2-chlorophenyl)-N-(4-phenoxy-3-sulfamoylphenyl)acetamide
(C15) 2-(2-chlorophenyl)-N-[4-(3-cyanophenoxy)-3-sulfamoylphenyl]acetamide
(C16) 2-(2-chlorophenyl)-N-4-[3-(methylsulfonyl)phenoxy]-3-sulfamoylphenylacetamide
(C17) 3-(4-[(2-chlorophenyl)acetyl]amino-2-sulfamoylphenoxy)benzamide
(C18) 2-(2-chlorophenyl)-N-[4-(3-methylphenoxy)-3-sulfamoylphenyl]acetamide
(C19) 2-(2-chlorophenyl)-N-[4-(pyrimidin-5-yloxy)-3-sulfamoylphenyl]acetamide
(C20) 2-(2-chlorophenyl)-N-3-sulfamoyl-4-[3-(4H-1,2,4-triazol-4-yl)phenoxy]phenylacetamide
(C21) 2-(2-chlorophenyl)-N-3-sulfamoyl-4-[3-(1H-tetrazol-5-yl)phenoxy]phenylacetamide
(C22) 2-(2-chlorophenyl)-N-[4-(3-methoxyphenoxy)-3-sulfamoylphenyl]acetamide
(C23) 2-(2-chlorophenyl)-N-[4-(4-methoxyphenoxy)-3-sulfamoylphenyl]acetamide
(C24) 2-(2-chlorophenyl)-N-4-[3-(difluoromethoxy)phenoxy]-3-sulfamoylphenylacetamide
(C25) 2-(2-chlorophenyl)-N-[4-(3,4-dicyanophenoxy)-3-sulfamoylphenyl]acetamide
(C26) 2-(2-chlorophenyl)-N-4-[3-(morpholin-4-yl)phenoxy]-3-sulfamoylphenylacetamide
(C27) 2-(2-chlorophenyl)-N-[4-(3-4-[(2-chlorophenyl)acetyl]piperazin-1-ylphenoxy)-3-sulfamoylphenyl]acetamide
(C28) 2-(2-chlorophenyl)-N-[4-(pyridin-3-yloxy)-3-sulfamoylphenyl]acetamide
(C29) 2-(2-chlorophenyl)-N-4-[(5-chloropyridin-3-yl)oxy]-3-sulfamoylphenylacetamide
(C30) 2-(2-chlorophenyl)-N-[4-(4-cyanophenoxy)-3-sulfamoylphenyl]acetamide
(C31) 2-(2-chlorophenyl)-N-4-[3-(difluoromethyl)phenoxy]-3-sulfamoylphenylacetamide
(C32) 2-(2-chlorophenyl)-N-[4-(2-methoxyphenoxy)-3-sulfamoylphenyl]acetamide
(C33) 2-(2-chlorophenyl)-N-[4-(3,5-dicyanophenoxy)-3-sulfamoylphenyl]acetamide
(C34) 2-(2-chlorophenyl)-N-[4-(5-cyano-2-methoxyphenoxy)-3-sulfamoylphenyl]acetamide
(C35) 2-(2-chlorophenyl)-N-4-[(2,5-dichloropyridin-3-yl)oxy]-3-sulfamoylphenylacetamide
(C36) 2-(2-chlorophenyl)-N-4-[(5,6-dichloropyridin-3-yl)oxy]-3-sulfamoylphenylacetamide
(C37) 3-(4-[(2-chlorophenyl)acetyl]amino-2-sulfamoylphenoxy)-N-cyclopropylbenzamide
(C38) 2-(2-chlorophenyl)-N-4-[(3-chloropyridin-2-yl)oxy]-3-sulfamoylphenylacetamide
(C39) 2-(2-chlorophenyl)-N-4-[(4-chloropyridin-2-yl)oxy]-3-sulfamoylphenylacetamide
(C40) 2-(2-chlorophenyl)-N-4-[(6-chloropyridin-2-yl)oxy]-3-sulfamoylphenylacetamide
(C41) 2-(2-chlorophenyl)-N-4-[3-(1-methyl-4,5-dihydro-1H-imidazol-2-yl)phenoxy]-3-sulfamoylphenylacetamide
(C42) 2-(2-chlorophenyl)-N-4-[4-(1H-imidazol-1-yl)phenoxy]-3-sulfamoylphenylacetamide
(C43) 2-(2-chlorophenyl)-N-4-[4-(2-oxopyrrolidin-1-yl)phenoxy]-3-sulfamoylphenylacetamide
(C44) 2-(2-chlorophenyl)-N-4-[4-(morpholin-4-yl)phenoxy]-3-sulfamoylphenylacetamide
(C45) 2-(2-chlorophenyl)-N-[4-(5-cyano-2-methylphenoxy)-3-sulfamoylphenyl]acetamide
(C46) 2-(2-chlorophenyl)-N-[4-(3-cyano-2-methylphenoxy)-3-sulfamoylphenyl]acetamide
(C47) 2-(2-chlorophenyl)-N-[4-(3-cyano-4-fluorophenoxy)-3-sulfamoylphenyl]acetamide
(C48) N-4-[(5-chloro-2-cyanopyridin-3-yl)oxy]-3-sulfamoylphenyl-2-(2-chlorophenyl)acetamide
(C49) 2-(2-chlorophenyl)-N-4-[3-(piperidin-1-yl)phenoxy]-3-sulfamoylphenylacetamide
(C50) 2-(2-chlorophenyl)-N-4-[3-(2-oxopyrrolidin-1-yl)phenoxy]-3-sulfamoylphenylacetamide
(C51) 2-(2-chlorophenyl)-N-4-[3-(2-oxo-1,3-oxazolidin-3-yl)phenoxy]-3-sulfamoylphenylacetamide
(C52) 2-(2-chlorophenyl)-N-4-[3-(morpholin-4-ylcarbonyl)phenoxy]-3-sulfamoylphenylacetamide
(C53) 2-(2-chlorophenyl)-N-4-[(4-methyltetrahydro-2H-pyran-4-yl)methoxy]-3-sulfamoylphenylacetamide
(C54) 2-(2-chlorophenyl)-N-4-[(4-fluorotetrahydro-2H-pyran-4-yl)methoxy]-3-sulfamoylphenylacetamide
(C55) 2-(2-chlorophenyl)-N-4-[(4-cyanotetrahydro-2H-pyran-4-yl)methoxy]-3-sulfamoylphenylacetamide
(C56) 2-(2-chlorophenyl)-N-(3-sulfamoyl-4-[2-(trifluoromethyl)pyrimidin-5-yl]oxyphenyl)acetamide
(C57) 2-(2-chlorophenyl)-N-4-[(2-isopropylpyrimidin-5-yl)oxy]-3-sulfamoylphenylacetamide
(C58) 2-(2-chlorophenyl)-N-4-[(2-cyclopropyl-4-methylpyrimidin-5-yl)oxy]-3-sulfamoylphenylacetamide
(C59) N-[4-(3-bromophenoxy)-3-sulfamoylphenyl]-2-(2-chlorophenyl) acetamide
(C60) N-[4-(4-bromophenoxy)-3-sulfamoylphenyl]-2-(2-chlorophenyl) acetamide
(C61) 2-(2-chlorophenyl)-N-4-[3-(2-methyl-1,3-thiazol-4-yl)phenoxy]-3-sulfamoylphenylacetamide
(C62) 2-(2-chlorophenyl)-N-4-[4-(5-oxopyrrolidin-2-yl)phenoxy]-3-sulfamoylphenylacetamide
(C63) 2-(2-chlorophenyl)-N-4-[4-(2-oxo-1,3-oxazolidin-3-yl)phenoxy]-3-sulfamoylphenylacetamide
(C64) 2-(2-chlorophenyl)-N-3-sulfamoyl-4-[4-(1,3-thiazol-2-yl)phenoxy]phenylacetamide
(C65) N-[4-(2-chlorophenoxy)-3-sulfamoylphenyl]-2-(2-chlorophenyl) acetamide
(C66) N-[4-(4-chlorophenoxy)-3-sulfamoylphenyl]-2-(2-chlorophenyl) acetamide
(C67) 2-(2-chlorophenyl)-N-4-[3-(piperidin-1-ylcarbonyl)phenoxy]-3-sulfamoylphenylacetamide
(C68) 2-(2-chlorophenyl)-N-3-sulfamoyl-4-[4-(tetrahydrofuran-3-yl)phenoxy]phenylacetamide
(C69) 2-(2-chlorophenyl)-N-[4-(3-cyano-5-fluorophenoxy)-3-sulfamoylphenyl]acetamide
(C70) N-[4-(2-methoxyphenoxy)-3-sulfamoylphenyl]-2-phenylacetamide
(C71) N-[4-(2-methoxyphenoxy)-3-sulfamoylphenyl]-2-[4-(trifluoromethyl)phenyl]acetamide
(C72) N-3-sulfamoyl-4-[2-(trifluoromethoxy)phenoxy]phenyl-2-[4-(trifluoromethyl)phenyl]acetamide
(C73) N-[4-(2-chlorophenoxy)-3-sulfamoylphenyl]-2-[4-(trifluoromethyl)phenyl]acetamide
(C74) 2-phenyl-N-3-sulfamoyl-4-[2-(trifluoromethoxy)phenoxy]phenylacetamide
(C75) 2-(2-chlorophenyl)-N-4-[(2-oxo-1,2-dihydropyridin-3-yl)oxy]-3-sulfamoylphenylacetamide
(C76) N-[4-(2-chlorophenoxy)-3-sulfamoylphenyl]-2-phenylacetamide
(C77) N-[4-(4-chlorophenoxy)-3-sulfamoylphenyl]-2-phenylacetamide
(C78) N-4-[(5-chloropyridin-3-yl)oxy]-3-sulfamoylphenyl-2-phenylacetamide
(C79) 2-(2-chlorophenyl)-N-4-[(2-chloropyrimidin-5-yl)oxy]-3-sulfamoylphenylacetamide
(C80) 2-(2-chlorophenyl)-N-4-[(5-fluoropyridin-3-yl)oxy]-3-sulfamoylphenylacetamide
(C81) 2-(2-chlorophenyl)-N-4-[(6-chloropyridin-3-yl)oxy]-3-sulfamoylphenylacetamide
(C82) N-[2-chloro-4-(3-chlorophenoxy)-5-sulfamoylphenyl]-2-(2-chlorophenyl)acetamide
(C83) N-[2-chloro-4-(3-chlorophenoxy)-5-sulfamoylphenyl]-2-(2-chloro-3-fluorophenyl)acetamide
(C84) N-[4-(3-chlorophenoxy)-3-sulfamoylphenyl]-2-(3-fluorophenyl) acetamide
(C85) N-[4-(3-chlorophenoxy)-3-sulfamoylphenyl]-2-(4-fluorophenyl) acetamide
(C86) N-[4-(3-chlorophenoxy)-3-sulfamoylphenyl]-2-[2-(trifluoromethyl)phenyl]acetamide
(C87) N-[4-(3-chlorophenoxy)-3-sulfamoylphenyl]-2-(2-isopropylphenyl) acetamide
(C88) N-[4-(3-chlorophenoxy)-3-sulfamoylphenyl]-2-(2-ethoxyphenyl) acetamide
(C89) N-[4-(3-chlorophenoxy)-3-sulfamoylphenyl]-2-[2-(difluoromethyl)phenyl]acetamide
(C90) N-[4-(3-chlorophenoxy)-3-sulfamoylphenyl]-2-2-[(trifluoromethyl)sulfanyl]phenylacetamide
(C91) 2-(2-Bromophenyl)-N-[4-(3-chlorophenoxy)-3-sulfamoylphenyl]acetamide
(C92) N-[4-(3-chlorophenoxy)-3-sulfamoylphenyl]-2-(4-methylpyridin-3-yl)acetamide
(C93) N-[4-(3-chlorophenoxy)-3-sulfamoylphenyl]-2-(2-chloropyridin-3-yl)acetamide
(C94) N-[4-(3-chlorophenoxy)-3-sulfamoylphenyl]-2-(2-chlorophenyl)-2,2-difluoroacetamide
(C95) 2-(2-chloro-4-methylphenyl)-N-[4-(3-chlorophenoxy)-3-sulfamoylphenyl]acetamide
(C96) 2-(2-chloro-6-methylphenyl)-N-[4-(3-chlorophenoxy)-3-sulfamoylphenyl]acetamide
(C97) 2-(2-chloro-5-methylphenyl)-N-[4-(3-chlorophenoxy)-3-sulfamoylphenyl]acetamide
(C98) 2-(2-chloro-3-fluorophenyl)-N-[4-(3-chlorophenoxy)-3-sulfamoylphenyl]acetamide
(C99) 2-(2-chloro-5-fluorophenyl)-N-[4-(3-chlorophenoxy)-3-sulfamoylphenyl]acetamide
(C100) 2-(2-chloro-6-fluorophenyl)-N-[4-(3-chlorophenoxy)-3-sulfamoylphenyl]acetamide
(C101) 2-(2-chloro-6-methoxyphenyl)-N-[4-(3-chlorophenoxy)-3-sulfamoylphenyl]acetamide
(C102) 2-(2-chloro-5-methoxyphenyl)-N-[4-(3-chlorophenoxy)-3-sulfamoylphenyl]acetamide
(C103) N-[4-(3-chlorophenoxy)-3-sulfamoylphenyl]-2-(2,3-dichlorophenyl) acetamide
(C104) N-[4-(3-chlorophenoxy)-3-sulfamoylphenyl]-2-(2,6-dichlorophenyl) acetamide
(C105) N-[4-(3-chlorophenoxy)-3-sulfamoylphenyl]-2-[2-(trifluoromethoxy)phenyl]acetamide
(C106) N-[4-(3-chlorophenoxy)-3-sulfamoylphenyl]-2,2-difluoro-2-phenylacetamide
(C107) N-[4-(3-chlorophenoxy)-3-sulfamoylphenyl]-2-[2-chloro-3-(trifluoromethyl)phenyl]acetamide
(C108) N-[4-(3-chlorophenoxy)-3-sulfamoylphenyl]-2-[2-chloro-6-(trifluoromethyl)phenyl]acetamide
(C109) N-[4-(3-chlorophenoxy)-3-sulfamoylphenyl]-2-[2-chloro-5-(trifluoromethyl)phenyl]acetamide
(C110) N-[4-(3-chlorophenoxy)-3-sulfamoylphenyl]-2-(2,4-dichlorophenyl)acetamide
(C111) N-[4-(3-chlorophenoxy)-3-sulfamoylphenyl]-2-(4,6-dichloropyridin-3-yl)acetamide
(C112) N-[4-(3-chlorophenoxy)-3-sulfamoylphenyl]-2-(3-chloropyridin-2-yl)acetamide
(C113) N-[4-(3-chlorophenoxy)-3-sulfamoylphenyl]-2-[2-(difluoromethoxy)phenyl]acetamide
(C114) N-[4-(3-chlorophenoxy)-3-sulfamoylphenyl]-2-(2,5-dichlorophenyl) acetamide
(C115) 2-[6-chloro-2,3-difluoro-4-(trifluoromethyl)phenyl]-N-[4-(3-chlorophenoxy)-3-sulfamoylphenyl]acetamide
(C116) N-[4-(3-chlorophenoxy)-3-sulfamoylphenyl]-2-[4-(trifluoromethyl)phenyl]acetamide
(C117) 2-(5-bromo-2-chlorophenyl)-N-[4-(3-chlorophenoxy)-3-sulfamoylphenyl]acetamide
(C118) 2-(4-bromo-2-chloro-5-methylphenyl)-N-[4-(3-chlorophenoxy)-3-sulfamoylphenyl]acetamide
(C119) N-[4-(3-chlorophenoxy)-3-sulfamoylphenyl]-2-(3-chloropyridin-4-yl)acetamide
(C120) 2-(2-chloro-6-fluoro-3-methylphenyl)-N-[4-(3-chlorophenoxy)-3-sulfamoylphenyl]acetamide
(C121) 2-(6-chloro-2-fluoro-3-methylphenyl)-N-[4-(3-chlorophenoxy)-3-sulfamoylphenyl]acetamide
(C122) 2-(2-chloro-3,6-difluorophenyl)-N-[4-(3-chlorophenoxy)-3-sulfamoylphenyl]acetamide
(C123) 2-(2-chloro-4,5-difluorophenyl)-N-[4-(3-chlorophenoxy)-3-sulfamoylphenyl]acetamide
(C124) N-[4-(3-chlorophenoxy)-3-sulfamoylphenyl]-2-(2,3-dichloro-6-fluorophenyl)acetamide
(C125) N-[4-(3-chlorophenoxy)-3-sulfamoylphenyl]-2-(2,3,6-trichlorophenyl)acetamide
(C126) N-[4-(3-chlorophenoxy)-3-sulfamoylphenyl]-2-(2,6-dichloro-4-methylphenyl)acetamide
(C127) N-[4-(3-chlorophenoxy)-3-sulfamoylphenyl]-2-[2,3-dichloro-6-(trifluoromethyl)phenyl]acetamide
(C128) N-[4-(3-chlorophenoxy)-3-sulfamoylphenyl]-2-(2,6-dichloro-3-methylphenyl)acetamide
(C129) N-[4-(3-chlorophenoxy)-3-sulfamoylphenyl]-2-(2,6-dichloro-3-cyclopropylphenyl)acetamide
(C130) N-[4-(3-chlorophenoxy)-3-sulfamoylphenyl]-2-[2,6-dichloro-3-(trifluoromethyl)phenyl]acetamide
(C131) 2-(3-bromo-2,6-dichlorophenyl)-N-[4-(3-chlorophenoxy)-3-sulfamoylphenyl]acetamide
(C132) 2-(3-bromo-2-chloro-6-methylphenyl)-N-[4-(3-chlorophenoxy)-3-sulfamoylphenyl]acetamide
(C133) 2-(3-bromo-6-chloro-2-methylphenyl)-N-[4-(3-chlorophenoxy)-3-sulfamoylphenyl]acetamide
(C134) N-[4-(3-chlorophenoxy)-3-sulfamoylphenyl]-2-[2-chloro-5-(1,1,2,2-tetrafluoroethoxy)phenyl]acetamide
(C135) N-[4-(3-chlorophenoxy)-3-sulfamoylphenyl]-2-[2-chloro-4-(trifluoromethyl)phenyl]acetamide
(C136) 2-(2-chlorophenyl)-N-(4-[3-(methylsulfonyl)benzyl]oxy-3-sulfamoylphenyl)acetamide
(C137) 2-(2-chlorophenyl)-N-4-[(2-fluorobenzyl)oxy]-3-sulfamoylphenylacetamide
(C138) 2-(2-chlorophenyl)-N-4-[(4-cyanobenzyl)oxy]-3-sulfamoylphenylacetamide
(C139) N-4-[(3-chlorobenzyl)oxy]-3-sulfamoylphenyl-2-(2-chlorophenyl) acetamide
(C140) 2-(2-chlorophenyl)-N-4-[(3-methoxybenzyl)oxy]-3-sulfamoylphenylacetamide
(C141) N-[4-(benzyloxy)-3-sulfamoylphenyl]-2-(2-chlorophenyl) acetamide
(C142) 2-(2-chlorophenyl)-N-4-[(3-cyanobenzyl)oxy]-3-sulfamoylphenylacetamide
(C143) 2-(2-chlorophenyl)-N-4-[(4-fluorobenzyl)oxy]-3-sulfamoylphenylacetamide
(C144) N-4-[(2-chlorobenzyl)oxy]-3-sulfamoylphenyl-2-(2-chlorophenyl) acetamide
(C145) 2-(2-chlorophenyl)-N-4-[(2-cyanobenzyl)oxy]-3-sulfamoylphenylacetamide
(C146) N-[4-(benzyloxy)-3-sulfamoylphenyl]-2-phenylacetamide
(C147) 2-(2-chlorophenyl)-N-(4-[4-(methylsulfonyl)benzyl]oxy-3-sulfamoylphenyl)acetamide
(C148) 2-(2-chlorophenyl)-N-[4-(1-phenylethoxy)-3-sulfamoylphenyl]acetamide
(C149) 2-(2-chlorophenyl)-N-[4-(1-phenylethoxy)-3-sulfamoylphenyl]acetamide
(C150) 2-(2-chlorophenyl)-N-[4-(pyridin-3-ylmethoxy)-3-sulfamoylphenyl]acetamide
(C151) 2-(2-chlorophenyl)-N-[4-(pyridin-2-ylmethoxy)-3-sulfamoylphenyl]acetamide
(C152) 2-(2-chlorophenyl)-N-[4-(pyridin-4-ylmethoxy)-3-sulfamoylphenyl]acetamide
(C153) N-[4-(pyridin-2-ylmethoxy)-3-sulfamoylphenyl]-2-[4-(trifluoromethyl)phenyl]acetamide
(C154) 2-(2-chlorophenyl)-N-[4-(pyrimidin-4-ylmethoxy)-3-sulfamoylphenyl]acetamide
(C155) 2-(2-chlorophenyl)-N-[4-(pyrimidin-2-ylmethoxy)-3-sulfamoylphenyl]acetamide
(C156) 2-(2-chlorophenyl)-N-[4-(2-phenylethoxy)-3-sulfamoylphenyl]acetamide
(C157) 2-(2-chlorophenyl)-N-4-[2-(3-chlorophenyl)ethoxy]-3-sulfamoylphenylacetamide
(C158) 2-(2-chlorophenyl)-N-[4-(cyclobutylmethoxy)-3-sulfamoylphenyl]acetamide
(C159) 2-(2-chlorophenyl)-N-[4-(oxetan-2-ylmethoxy)-3-sulfamoylphenyl]acetamide
(C160) 2-(2-chlorophenyl)-N-[4-(oxetan-3-ylmethoxy)-3-sulfamoylphenyl]acetamide
(C161) 2-(2-chlorophenyl)-N-[4-(cyclopentylmethoxy)-3-sulfamoylphenyl]acetamide
(C162) 2-(2-chlorophenyl)-N-[3-sulfamoyl-4-(tetrahydrofuran-2-ylmethoxy)phenyl]acetamide
(C163) 2-(2-chlorophenyl)-N-[3-sulfamoyl-4-(tetrahydrofuran-3-ylmethoxy)phenyl]acetamide
(C164) 2-(2-chloro-5-fluorophenyl)-N-[3-sulfamoyl-4-(tetrahydro-2H-pyran-4-ylmethoxy)phenyl]acetamide
(C165) 2-(2-chlorophenyl)-N-[3-sulfamoyl-4-(tetrahydro-2H-pyran-4-ylmethoxy)phenyl]acetamide
(C166) 2-(2-chlorophenyl)-N-[3-sulfamoyl-4-(tetrahydro-2H-pyran-3-ylmethoxy)phenyl]acetamide
(C167) 2-(2-chloro-6-fluorophenyl)-N-[3-sulfamoyl-4-(tetrahydro-2H-pyran-4-ylmethoxy)phenyl]acetamide
(C168) 2-(2-chloro-3-fluorophenyl)-N-[3-sulfamoyl-4-(tetrahydro-2H-pyran-4-ylmethoxy)phenyl]acetamide
(C169) 2-(2-Chlorophenyl)-N-5-sulfamoyl-6-[3-(trifluoromethyl)phenoxy]pyridin-3-ylacetamide
(C170) 2-phenyl-N-5-sulfamoyl-6-[3-(trifluoromethyl)phenoxy]pyridin-3-ylacetamide
(C171) N-[4-(3-chlorophenoxy)-3-fluoro-5-sulfamoylphenyl]-2-phenylacetamide
(C172) N-[4-(3-chlorophenoxy)-3-fluoro-5-sulfamoylphenyl]-2-(2-methylphenyl)acetamide
(C173) N-[4-(3-chlorophenoxy)-3-fluoro-5-sulfamoylphenyl]-2-(3-methylphenyl)acetamide
(C174) 2-(2-chlorophenyl)-N-4-[3-(3-oxomorpholin-4-yl)phenoxy]-3-sulfamoylphenylacetamide
(C175) 2-(2-chlorophenyl)-N-4-[4-(3-oxomorpholin-4-yl)phenoxy]-3-sulfamoylphenylacetamide
(C176) 2-(2-chlorophenyl)-N-4-[4-(2-oxopiperidin-1-yl)phenoxy]-3-sulfamoylphenylacetamide
(C177) 2-(2-chlorophenyl)-N-4-[3-(2-oxopiperidin-1-yl)phenoxy]-3-sulfamoylphenylacetamide
(C178) 2-(2-chlorophenyl)-N-4-[3-(prop-1-en-2-yl)phenoxy]-3-sulfamoylphenylacetamide
(C179) 2-(2-chlorophenyl)-N-4-[2-(prop-1-en-2-yl)phenoxy]-3-sulfamoylphenylacetamide
(C180) N-[4-(3-chlorophenoxy)-3-sulfamoylphenyl]-2-(4-methylphenyl)acetamide
(C181) N-[4-(3-chlorophenoxy)-3-sulfamoylphenyl]-2-(4-chlorophenyl) acetamide
(C182) N-[4-(3-chlorophenoxy)-3-sulfamoylphenyl]-2-(pyridin-3-yl)acetamide
(C183) N-[4-(3-chlorophenoxy)-3-sulfamoylphenyl]-2-(2-methylphenyl) acetamide
(C184) N-[4-(3-chlorophenoxy)-3-sulfamoylphenyl]-2-(3-methylphenyl) acetamide
(C185) N-[4-(3-chlorophenoxy)-3-sulfamoylphenyl]-2-phenylpropanamide
(C186) N-[4-(3-chlorophenoxy)-3-sulfamoylphenyl]-2-(pyridin-2-yl)acetamide
(C187) N-[4-(3-chlorophenoxy)-3-sulfamoylphenyl]-2-(3-chlorophenyl) acetamide
(C188) N-[4-(3-chlorophenoxy)-3-sulfamoylphenyl]-2-(2-chlorophenyl) acetamide
(C189) N-[4-(3-chlorophenoxy)-3-sulfamoylphenyl]-2-(pyridin-4-yl)acetamide
(C190) N-[4-(3-chlorophenoxy)-3-sulfamoylphenyl]-2-(6-methylpyridin-2-yl)acetamide
(C191) N-[4-(3-chlorophenoxy)-3-sulfamoylphenyl]-2-(4-methoxyphenyl) acetamide
(C192) N-[4-(3-chlorophenoxy)-3-sulfamoylphenyl]-2-(3-methoxyphenyl) acetamide
(C193) N-[4-(3-chlorophenoxy)-3-sulfamoylphenyl]-2-(2-methoxyphenyl) acetamide
(C194) N-[4-(3-chlorophenoxy)-3-sulfamoylphenyl]-2-(5-methylpyridin-2-yl)acetamide
(C195) (2S)-N-[4-(3-chlorophenoxy)-3-sulfamoylphenyl]-2-phenylpropanamide
(C196) (2R)-N-[4-(3-chlorophenoxy)-3-sulfamoylphenyl]-2-phenylpropanamide
(C197) N-[4-(3-chlorophenoxy)-3-sulfamoylphenyl]-2-(2-chlorophenyl)propanamide
(C198) 2-(2-[4-(3-chlorophenoxy)-3-sulfamoylphenyl]amino-2-oxoethyl)-N-(2-methoxyethyl)-N-methylbenzamide
(C199) 2-(2-[4-(3-chlorophenoxy)-3-sulfamoylphenyl]amino-2-oxoethyl)-N,N-dimethylbenzamide
(C200) N-[4-(cyclohexyloxy)-3-sulfamoylphenyl]-2-phenylacetamide
(C201) 2-(2-chlorophenyl)-N-[4-(cyclohexyloxy)-3-sulfamoylphenyl]acetamide
(C202) 3-(2-[4-(3-chlorophenoxy)-3-sulfamoylphenyl]amino-2-oxoethyl)-N-(2-methoxyethyl)benzamide
(C203) 3-(2-[4-(3-chlorophenoxy)-3-sulfamoylphenyl]amino-2-oxoethyl)-N,N-dimethylbenzamide
(C204) 3-(2-[4-(3-chlorophenoxy)-3-sulfamoylphenyl]amino-2-oxoethyl)-N-methylbenzamide
(C205) N-[4-(cyclobutyloxy)-3-sulfamoylphenyl]-2-phenylacetamide
(C206) 2-(2-chlorophenyl)-N-[4-(cyclobutyloxy)-3-sulfamoylphenyl]acetamide
(C207) 2-phenyl-N-[3-sulfamoyl-4-(tetrahydro-2H-pyran-4-yloxy)phenyl]acetamide
(C208) 2-(2-chlorophenyl)-N-[3-sulfamoyl-4-(tetrahydro-2H-pyran-4-yloxy)phenyl]acetamide
(C209) 3-(2-[4-(3-chlorophenoxy)-3-sulfamoylphenyl]amino-2-oxoethyl)-N-(2-methoxyethyl)-N-methylbenzamide
(C210) N-[4-(3-chlorophenoxy)-3-sulfamoylphenyl]-2-(5-chloropyridin-2-yl)acetamide
(C211) N-[4-(3-chlorophenoxy)-3-sulfamoylphenyl]-2-[3-(2-methoxyethoxy)phenyl]acetamide
(C212) N-[4-(3-chlorophenoxy)-3-sulfamoylphenyl]-2-[2-(2-methoxyethoxy)phenyl]acetamide
(C213) N-[4-(3-chlorophenoxy)-3-sulfamoylphenyl]-2-[3-(2-hydroxyethoxy)phenyl]acetamide
(C214) N-[4-(3-chlorophenoxy)-3-sulfamoylphenyl]-2-[2-(2-hydroxyethoxy)phenyl]acetamide
(C215) N-[4-(3-chlorophenoxy)-3-sulfamoylphenyl]-2-(2-fluorophenyl) acetamide
(C216) N-[4-(oxetan-3-yloxy)-3-sulfamoylphenyl]-2-phenylacetamide
(C217) 2-(2-chlorophenyl)-N-[4-(oxetan-3-yloxy)-3-sulfamoylphenyl]acetamide
(C218) N-[4-(cyclopentyloxy)-3-sulfamoylphenyl]-2-phenylacetamide
(C219) 2-(2-chlorophenyl)-N-[4-(cyclopentyloxy)-3-sulfamoylphenyl]acetamide
(C220) N-4-[(1-methylpiperidin-3-yl)oxy]-3-sulfamoylphenyl-2-phenylacetamide
(C221) 2-(2-chlorophenyl)-N-4-[(1-methylpiperidin-3-yl)oxy]-3-sulfamoylphenylacetamide
(C222) N-4-[(1-methylpyrrolidin-3-yl)oxy]-3-sulfamoylphenyl-2-phenylacetamide
(C223) 2-(2-chlorophenyl)-N-4-[(1-methylpyrrolidin-3-yl)oxy]-3-sulfamoylphenylacetamide
(C224) N-[4-(4-chlorophenoxy)-3-sulfamoylphenyl]-2-(4-fluorophenyl) acetamide
(C225) N-[4-(3-chlorophenoxy)-3-sulfamoylphenyl]-2-(4-cyanophenyl)acetamide
(C226) N-[4-(3-chlorophenoxy)-3-sulfamoylphenyl]-2-(2-cyanophenyl)acetamide
(C227) N-[4-(3-chlorophenoxy)-3-sulfamoylphenyl]-2-(3-cyanophenyl)acetamide
(C228) N-[4-(4-chlorophenoxy)-3-sulfamoylphenyl]-2-[4-(trifluoromethyl)phenyl]acetamide
(C229) N-[4-(4-chlorophenoxy)-3-sulfamoylphenyl]-2-(4-chlorophenyl) acetamide
(C230) N-[4-(4-chlorophenoxy)-3-sulfamoylphenyl]-2-(4-methoxyphenyl) acetamide
(C231) N-[4-(4-chlorophenoxy)-3-sulfamoylphenyl]-2-(2-fluorophenyl) acetamide
(C232) 2-(2-chloro-4-fluorophenyl)-N-[4-(4-chlorophenoxy)-3-sulfamoylphenyl]acetamide
(C233) 2-(2-chlorophenyl)-N-4-[(1,1-dioxidetetrahydrothiophen-3-yl)oxy]-3-sulfamoylphenylacetamide
(C234) 2-(2-chlorophenyl)-N-4-[(1-methyl-1H-pyrazol-4-yl)oxy]-3-sulfamoylphenylacetamide
(C235) N-[4-(4-chlorophenoxy)-3-sulfamoylphenyl]-2-[4-(difluoromethyl)phenyl]acetamide
(C236) 2-(2-chloro-4-methoxyphenyl)-N-[4-(4-chlorophenoxy)-3-sulfamoylphenyl]acetamide
(C237) 2-(2-chlorophenyl)-N-4-[(1-methyl-1H-pyrazol-3-yl)oxy]-3-sulfamoylphenylacetamide
(C238) 2-(2-chlorophenyl)-N-4-[(1-methyl-1H-pyrazol-5-yl)oxy]-3-sulfamoylphenylacetamide
(C239) 2-(2-chlorophenyl)-N-4-[(1-methylpiperidin-4-yl)oxy]-3-sulfamoylphenylacetamide
(C240) 2-(2-chlorophenyl)-N-(4-[5-methyl-2-(pyridin-3-yl)-1,3-thiazol-4-yl]oxy-3-sulfamoylphenyl)acetamide
(C241) N-[4-(3-chlorophenoxy)-2-methyl-5-sulfamoylphenyl]-2-(2-chlorophenyl)acetamide
(C242) 2-(2-Chlorophenyl)-N-{4-[(1-oxidetetrahydrothiophen-3-yl)oxy]-3-sulfamoylphenyl}acetamide
(C243) N-[4-(4-chlorophenoxy)-3-sulfamoylphenyl]-2-[2,6-dichloro-4-(trifluoromethyl)phenyl]acetamide
(C244) N-[4-(4-chlorophenoxy)-3-sulfamoylphenyl]-2-(2,5-dichloro-4-cyanophenyl)acetamide
(C245) N-[4-(3-chlorophenoxy)-3-sulfamoylphenyl]-2-phenylacetamide
(C246) N-[4-(cyclopropylmethoxy)-3-sulfamoylphenyl]-2-phenylacetamide
(C247) N-[4-(3,5-dimethylphenoxy)-3-sulfamoylphenyl]-2-phenylacetamide
(C248) N-[4-(2,4-difluorophenoxy)-3-sulfamoylphenyl]-2-phenylacetamide
(C249) N-[4-(4-fluorophenoxy)-3-sulfamoylphenyl]-2-phenylacetamide
(C250) N-[4-(3-fluorophenoxy)-3-sulfamoylphenyl]-2-phenylacetamide
(C251) N-[4-(3-methoxyphenoxy)-3-sulfamoylphenyl]-2-phenylacetamide
(C252) N-[4-(2-fluoro-5-methylphenoxy)-3-sulfamoylphenyl]-2-phenylacetamide
(C253) 2-phenyl-N-3-sulfamoyl-4-[4-(trifluoromethoxy)phenoxy]phenylacetamide
(C254) 2-phenyl-N-3-sulfamoyl-4-[3-(trifluoromethyl)phenoxy]phenylacetamide
(C255) N-[4-(3,5-dimethoxyphenoxy)-3-sulfamoylphenyl]-2-phenylacetamide
(C256) N-[4-(3-cyanophenoxy)-3-sulfamoylphenyl]-2-phenylacetamide
(C257) N-[4-(3-chlorophenoxy)-3-sulfamoylphenyl]-2-(4-hydroxyphenyl) acetamide
(C258) 2-(2-chloro-6-methoxy-4-methylphenyl)-N-[4-(3-chlorophenoxy)-3-sulfamoylphenyl]acetamide
(C259) 2-(2-chloro-6-fluorophenyl)-N-[4-(3-chlorophenoxy)-3-sulfamoylphenyl]propanamide
(C260) 2-(2-chloro-4,6-difluorophenyl)-N-[4-(3-chlorophenoxy)-3-sulfamoylphenyl]acetamide
(C261) N-[4-(3-chlorophenoxy)-3-sulfamoylphenyl]-2-(2,6-dichlorophenyl)propanamide
(C262) 2-(2-chlorophenyl)-N-4-[(2H5)phenyloxy]-3-sulfamoylphenylacetamide
(C263) 2-(2-chlorophenyl)-N-(4-{[4-chloro(2H4)phenyl]oxy}-3-sulfamoylphenyl)acetamide
(C264) 2-(2-chlorophenyl)-N-(4-{[2-chloro(2H4)phenyl]oxy}-3-sulfamoylphenyl)acetamide
(C265) 2-(2-chlorophenyl)-N-4-[4-(2-hydroxypropan-2-yl)phenoxy]-3-sulfamoylphenylacetamide
(C266) 2-(2-chlorophenyl)-N-4-[(2,2-dimethyltetrahydro-2H-pyran-4-yl)methoxy]-3-sulfamoylphenylacetamide
(C267) 2-(2-chlorophenyl)-N-{4-[(1R,5S,6r)-3-oxabicyclo[3.1.0]hex-6-ylmethoxy]-3-sulfamoylphenyl}acetamide
(C268) 2-(2-chlorophenyl)-N-4-[(4-chlorotetrahydro-2H-pyran-4-yl)methoxy]-3-sulfamoylphenylacetamide
(C269) 2-(2-chlorophenyl)-N-[4-(1,4-dioxane-2-ylmethoxy)-3-sulfamoylphenyl]acetamide
(C270) 2-(2-chlorophenyl)-N-3-sulfamoyl-4-[(2,2,6,6-tetramethyltetrahydro-2H-pyran-4-yl)oxy]phenylacetamide
(C271) N-[4-(3-chlorophenoxy)-3-methyl-5-sulfamoylphenyl]-2-(2-chlorophenyl)acetamide
(C272) N-[4-(3-chlorophenoxy)-3-methyl-5-sulfamoylphenyl]-2-phenylacetamide
(C273) methyl 2-(4-[(2-chlorophenyl)acetyl]amino-2-sulfamoylphenoxy)benzoate
(C274) methyl 4-(4-[(2-chlorophenyl)acetyl]amino-2-sulfamoylphenoxy)benzoate
(C275) 2-(2-chlorophenyl)-N-4-[3-(2-hydroxypropan-2-yl)phenoxy]-3-sulfamoylphenylacetamide
(C276) 2-(2-chlorophenyl)-N-4-[2-(2-hydroxypropan-2-yl)phenoxy]-3-sulfamoylphenylacetamide
(C277) N-[4-(4-chlorophenoxy)-3-sulfamoylphenyl]-2-(2,3-dihydro-1,4-benzodioxin-6-yl)acetamide
(C278) 2-(7-chloro-2,3-dihydro-1,4-benzodioxin-6-yl)-N-[4-(3-chlorophenoxy)-3-sulfamoylphenyl]acetamide
(C279) 2-(5-chloro-2,3-dihydro-1-benzofuran-4-yl)-N-[4-(3-chlorophenoxy)-3-sulfamoylphenyl]acetamide
(C280) 2-(2-fluorophenyl)-N-[3-sulfamoyl-4-(tetrahydro-2H-pyran-4-ylmethoxy)phenyl]acetamide
(C281) N-[3-sulfamoyl-4-(tetrahydro-2H-pyran-4-ylmethoxy)phenyl]-2-[2-(trifluoromethyl)phenyl]acetamide
(C282) 2-[2-(difluoromethyl)phenyl]-N-[3-sulfamoyl-4-(tetrahydro-2H-pyran-4-ylmethoxy)phenyl]acetamide
(C283) 2-(2-chloro-4-fluorophenyl)-N-[3-sulfamoyl-4-(tetrahydro-2H-pyran-4-ylmethoxy)phenyl]acetamide
(C284) 2-(2-chlorophenyl)-N-(3-sulfamoyl-4-{[6-(trifluoromethyl)pyridin-3-yl]oxy}phenyl)acetamide
(C285) 2-(2-chlorophenyl)-N-(4-{[5-chloro-4-(trifluoromethyl)pyridin-2-yl]oxy}-3-sulfamoyl-phenyl)acetamide
(C286) N-[4-(3-chlorophenoxy)-3-sulfamoylphenyl]-2-phenyl(2H2)acetamide
(C287) N-{4-[(6-chloro-5-fluoropyridin-3-yl)oxy]-3-sulfamoylphenyl}-2-(2-chlorophenyl)acetamide
(C288) 2-(2-chlorophenyl)-N-{4-[(4,4-difluoro-1-hydroxycyclohexyl)methoxy]-3-sulfamoyl-phenyl}acetamide
(C289) 2-(2-chlorophenyl)-N-{4-[(1-hydroxycyclohexyl)methoxy]-3-sulfamoylphenyl}acetamide
(C290) N-[4-(3-chlorophenoxy)-3-fluoro-5-sulfamoylphenyl]-2-(2-fluorophenyl)acetamide
(C291) N-[4-(3-chlorophenoxy)-3-fluoro-5-sulfamoylphenyl]-2-[2-(difluoromethyl)phenyl]acetamide
(C292) N-[4-(3-chlorophenoxy)-3-fluoro-5-sulfamoylphenyl]-2-(2-chlorophenyl)acetamide
(C293) 2-(2-chloro-5-fluorophenyl)-N-[4-(3-chlorophenoxy)-3-fluoro-5-sulfamoylphenyl]acetamide
(C294) N-[6-(3-chlorophenoxy)-5-sulfamoylpyridin-3-yl]-2-(2-fluorophenyl)acetamide
(C295) N-[6-(3-chlorophenoxy)-5-sulfamoylpyridin-3-yl]-2-[2-(trifluoromethyl)phenyl]acetamide
(C296) N-[6-(3-chlorophenoxy)-5-sulfamoylpyridin-3-yl]-2-[2-(difluoromethyl)phenyl]acetamide, and
(C297) 2-(2-chloro-5-fluorophenyl)-N-[6-(3-chlorophenoxy)-5-sulfamoylpyridin-3-yl]acetamide

As an active ingredient of the pharmaceutical composition of the present invention, a compound represented by the following general formula (D) or a pharmaceutically acceptable salt thereof can be used. These compounds can be produced by a known method (Patent Literature 5). 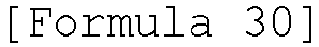 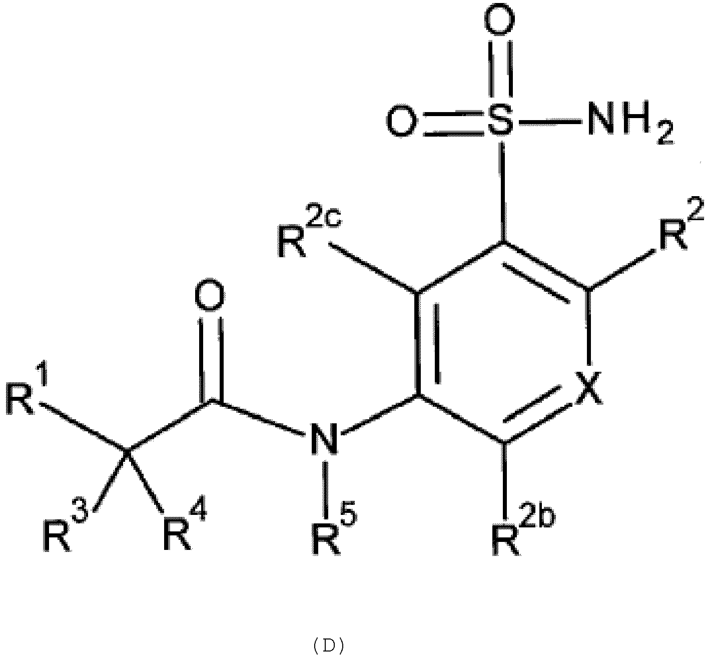 In the formula X represents C-R^{2a} or N;
R¹ represents a group selected from 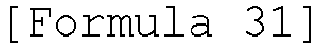 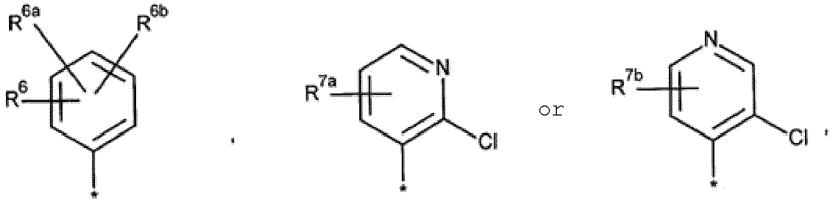 In the formula * represents the point of attachment of the group to the remainder of the molecule;
R² represents phenyl or heteroaryl,
in which the phenyl or heteroaryl group is optionally substituted 1 to 3 times, independently of one another, with the same or different R¹¹s, or
substituted 1 time, with R^{11a}, and optionally substituted 1 or 2 times, independently of one another, with the same or different R¹¹(s);
R^{2a} represents hydrogen, cyano, nitro, halogen, alkyl having 1 to 2 carbon atoms, or haloalkyl having 1 to 2 carbon atoms;
R^{2a} represents hydrogen, halogen, alkyl having 1 to 2 carbon atoms, or haloalkyl having 1 to 2 carbon atoms;
R^{2c} represents hydrogen, halogen, alkyl having 1 to 2 carbon atoms, or haloalkyl having 1 to 2 carbon atoms,
one or more of R^{2a}, R^{2a}, and R^{2c} represent hydrogen;
R³ represents hydrogen or fluoro;
R⁴ represents hydrogen, fluoro, methyl, or OH;
R⁵ represents hydrogen or alkyl having 1 to 3 carbon atoms;
R⁶ represents halogen, cyano, nitro, OH, alkyl having 1 to 4 carbon atoms, haloalkyl having 1 to 4 carbon atoms, alkoxy having 1 to 4 carbon atoms, haloalkoxy having 1 to 4 carbon atoms, or F3CS-;
R^{6a} and R^{6b} are the same or different and are independently of one another, in which R^{6a} represents hydrogen, halogen, hydroxy, nitro, cyano, alkyl having 1 to 4 carbon atoms, cycloalkyl having 3 to 6 carbon atoms, haloalkyl having 1 to 4 carbon atoms, alkoxy having 1 to 4 carbon atoms, haloalkoxy having 1 to 4 carbon atoms, HO- (alkoxy having 2 to 4 carbon atoms)-, (alkoxy having 1 to 4 carbon atoms)-(alkoxy having 2 to 4 carbon atoms)-, R⁹R¹⁰N-, R⁸-C(O)-NH-, R⁸-C(O)-, R⁸-O-C(O)-, R⁹R¹⁰N-C(O)-, or (alkyl having 1 to 4 carbon atoms)-SO2-;
R^{6b} represents hydrogen, halogen, hydroxy, nitro, cyano, alkyl having 1 to 4 carbon atoms, cycloalkyl having 3 to 6 carbon atoms, haloalkyl having 1 to 4 carbon atoms, haloalkoxy having 1 to 4 carbon atoms, HO- (alkoxy having 2 to 4 carbon atoms)-, (alkoxy having 1 to 4 carbon atoms)-(alkoxy having 2 to 4 carbon atoms)-, R⁹R¹⁰N-,R⁸-C(O)-NH-, R⁸-C(O)-, R⁸-O-C(O)-, R⁹R¹⁰N-C(O)-, or (alkyl having 1 to 4 carbon atoms)-SO2-; or
R^{6a} and R^{6b} adjacent to each other together represent a group selected from -O-CH2-CH2-, -O-CH2-O-, or -O-CH2-CH2-O-;
R^{7a} and R^{7b} are the same or different and independently represent hydrogen, hydroxy, halogen, alkyl having 1 to 4 carbon atoms, or haloalkyl having 1 to 4 carbon atoms;
R⁸, independently at each occurrence, represents alkyl having 1 to 6 carbon atoms, alkoxy having 1 to 4 carbon atoms-alkyl having 1 to 4 carbon atoms, cycloalkyl having 3 to 6 carbon atoms, or haloalkyl having 1 to 4 carbon atoms;
R⁹ and R¹⁰ are the same or different and independently represent hydrogen, alkyl having 1 to 4 carbon atoms, cycloalkyl having 3 to 6 carbon atoms, haloalkyl having 1 to 4 carbon atoms, or (CH3)₂N-alkyl having 1 to 4 carbon atoms, or R⁹ and R¹⁰, together with the nitrogen atom to which they are bonded, form a four to six membered nitrogen-containing heterocyclic ring, in which the ring optionally contains one further heteroatom selected from O, S, NH, and NR^{a} (In the formula R^{a} represents an alkyl-group having 1 to 6 carbon atoms or a haloalkyl-group having 1 to 6 carbon atoms, and is optionally substituted 1 to 3 times, independently of one another, with halogen or alkyl having 1 to 4 carbon atoms);
R¹¹ independently represents halogen, hydroxy, nitro, cyano, alkyl having 1 to 4 carbon atoms, alkenyl having 2 to 4 carbon atoms, haloalkyl having 1 to 4 carbon atoms, hydroxyalkyl having 1 to 6 carbon atoms, alkoxy having 1 to 4 carbon atoms, haloalkoxy having 1 to 4 carbon atoms, (alkoxy having 1 to 4 carbon atoms)-(alkyl having 1 to 4 carbon atoms)-, (haloalkoxy having 1 to 4 carbon atoms)-(alkyl having 1 to 4 carbon atoms)-, R⁹R¹⁰N-(alkyl having 1 to 4 carbon atoms)-, R⁹R¹⁰N-, R⁸-C(O)-NH-, R⁸-C(O)-, R⁸-O-C(O)-, R⁹R¹⁰N-C(O)-, (alkyl having 1 to 4 carbon atoms)-S-, or (alkyl having 1 to 4 carbon atoms)-SO2-;
R^{11a} represents cycloalkyl having 3 to 6 carbon atoms, morpholino, or represents a group selected from 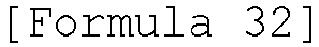 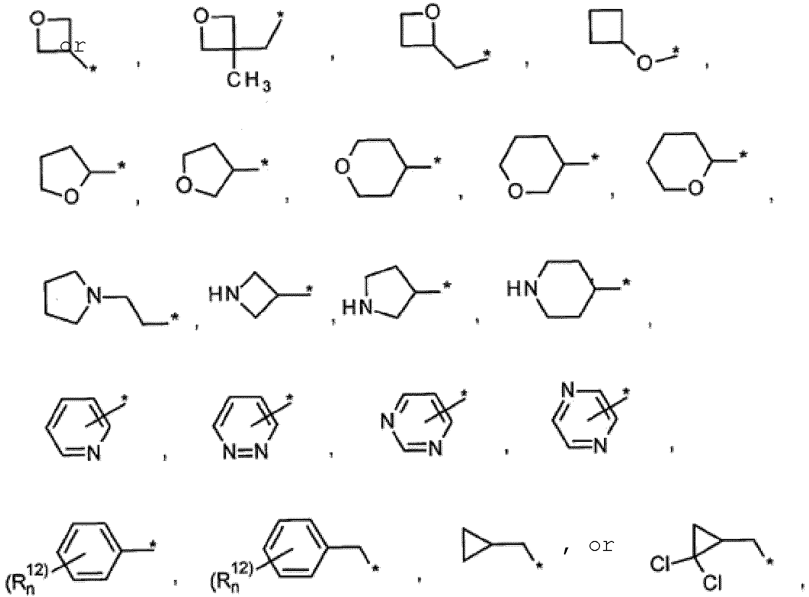 In the formula * represents the point of attachment of the group to the remainder of the molecule;
   R¹² independently represents halogen, hydroxy, nitro, cyano, alkyl having 1 to 4 carbon atoms, alkenyl having 2 to 4 carbon atoms, haloalkyl having 1 to 4 carbon atoms, hydroxyalkyl having 1 to 4 carbon atoms, alkoxy having 1 to 4 carbon atoms, haloalkoxy having 1 to 4 carbon atoms, (alkoxy having 1 to 4 carbon atoms)-(alkyl having 2 to 4 carbon atoms)-, (haloalkoxy having 1 to 4 carbon atoms)-(alkyl having 2 to 4 carbon atoms)-, R⁹R¹⁰N-, R⁸-C(O)-NH-, R⁸-C(O)-, R⁸-O-C(O)-, R⁹R¹⁰N-C(O)-, or (alkyl having 1 to 4 carbon atoms)-SO2-; and
   n represents 0, 1, 2, or 3.

Examples of the compound of the general formula (D) include the following compounds. That is, examples of the compound suitable as the active ingredient of the pharmaceutical composition of the present invention include the following compounds (D1) to (D558) or a pharmaceutically acceptable salt thereof. These compounds can be produced by a known method (Patent Literature 5).
(D1) 2-(2-chlorophenyl)-N-[4-(2-oxopyridin-1(2H)-yl)-3-sulfamoylphenyl]acetamide
(D2) N-[4-(4-chloro-2-oxopyridin-1(2H)-yl)-3-sulfamoylphenyl]-2-(2-chlorophenyl)acetamide
(D3) 2-(2-chlorophenyl)-N-[4-(3,5-dichloro-2-oxopyridin-1(2H)-yl)-3-sulfamoylphenyl]acetamide
(D4) N-[4-(3-chloro-2-oxopyridin-1(2H)-yl)-3-sulfamoylphenyl]-2-(2-chlorophenyl)acetamide
(D5) 2-(2-chlorophenyl)-N-[4-(3-methyl-1H-1,2,4-triazol-1-yl)-3-sulfamoylphenyl]acetamide
(D6) 2-(2-chlorophenyl)-N-[4-(5-methyl-1H-1,2,4-triazol-1-yl)-3-sulfamoylphenyl]acetamide
(D7) 2-(2-chlorophenyl)-N-{3-sulfamoyl-4-[3-(trifluoromethyl)-1H-1,2,4-triazol-1-yl]phenyl}acetamide
(D8) 2-(2-chlorophenyl)-N-{4-[5-methyl-3-(trifluoromethyl)-1H-1,2,4-triazol-1-yl]-3-sulfamoylphenyl}acetamide
(D9) 2-(2-chlorophenyl)-N-[4-(1H-imidazol-1-yl)-3-sulfamoylphenyl]acetamide
(D10) 2-(2-chlorophenyl)-N-{3-sulfamoyl-4-[4-(trifluoromethyl)-1H-imidazol-1-yl]phenyl}acetamide
(D11) 2-(2-chlorophenyl)-N-{3-sulfamoyl-4-[3-(trifluoromethyl)-1H-pyrazol-1-yl]phenyl}acetamide
(D12) 2-(2-chlorophenyl)-N-{4-[3-(difluoromethyl)-1H-pyrazol-1-yl]-3-sulfamoylphenyl}acetamide
(D13) 2-(2-chlorophenyl)-N-{4-[5-cyclopropyl-3-(difluoromethyl)-1H-pyrazol-1-yl]-3-sulfamoylphenyl}acetamide
(D14) 2-(2-chlorophenyl)-N-{4-[4-methyl-3-(trifluoromethyl)-1H-pyrazol-1-yl]-3-sulfamoylphenyl}acetamide
(D15) 2-(2-chlorophenyl)-N-{4-[5-methyl-3-(trifluoromethyl)-1H-pyrazol-1-yl]-3-sulfamoylphenyl}acetamide
(D16) 2-(2-chlorophenyl)-N-[3-sulfamoyl-4-(1H-1,2,4-triazol-1-yl)phenyl]acetamide
(D17) 2-(2-chlorophenyl)-N-{4-[3-(difluoromethyl)-1H-1,2,4-triazol-1-yl]-3-sulfamoylphenyl}acetamide
(D18) N-{4-[3-(difluoromethyl)-1H-1,2,4-triazol-1-yl]-3-sulfamoylphenyl}-2-(2-fluorophenyl)acetamide
(D19) 2-(2-chlorophenyl)-N-{3-sulfamoyl-4-[4-(trifluoromethyl)-1H-pyrazol-1-yl]phenyl}acetamide
(D20) 2-(2-fluorophenyl)-N-{3-sulfamoyl-4-[4-(trifluoromethyl)-1H-pyrazol-1-yl]phenyl}acetamide
(D21) 2-[2-(difluoromethyl)phenyl]-N-{3-sulfamoyl-4-[4-(trifluoromethyl)-1H-pyrazol-1-yl]phenyl}acetamide
(D22) N-{3-sulfamoyl-4-[4-(trifluoromethyl)-1H-pyrazol-1-yl]phenyl}-2-[2-(trifluoromethyl)phenyl]acetamide
(D23) N-[4-(3-tert-butyl-1H-1,2,4-triazol-1-yl)-3-sulfamoylphenyl]-2-(2-chlorophenyl)acetamide
(D24) 2-(2-chlorophenyl)-N-[4-(3-chloro-1H-1,2,4-triazol-1-yl)-3-sulfamoylphenyl]acetamide
(D25) 2-(2-chlorophenyl)-N-[4-(4-chloro-1H-pyrazol-1-yl)-3-sulfamoylphenyl]acetamide
(D26) 2-(2-chlorophenyl)-N-[4-(4-fluoro-1H-pyrazol-1-yl)-3-sulfamoylphenyl]acetamide
(D27) 2-(2-chlorophenyl)-N-[4-(4-isopropoxy-1H-pyrazol-1-yl)-3-sulfamoylphenyl]acetamide
(D28) N-[4-(4-bromo-1H-pyrazol-1-yl)-3-sulfamoylphenyl]-2-(2-chlorophenyl)acetamide
(D29) 2-(2-chlorophenyl)-N-[4-(3-isobutyl-1H-1,2,4-triazol-1-yl)-3-sulfamoylphenyl]acetamide
(D30) 2-(2-chlorophenyl)-N-{4-[4-(methylsulfanyl)-1H-pyrazol-1-yl]-3-sulfamoylphenyl}acetamide
(D31) 2-(2-chlorophenyl)-N-[4-(4-methoxy-1H-pyrazol-1-yl)-3-sulfamoylphenyl]acetamide
(D32) N-[4-(1H-benzoimidazol-1-yl)-3-sulfamoylphenyl]-2-(2-chlorophenyl)acetamide
(D33) N-[4-(4-chloro-1H-imidazol-1-yl)-3-sulfamoylphenyl]-2-(2-chlorophenyl)acetamide
(D34) 2-(2-chlorophenyl)-N-{4-[3-(dimethylamino)-1H-1,2,4-triazol-1-yl]-3-sulfamoylphenyl}acetamide
(D35) 2-(2-chlorophenyl)-N-[4-(3-ethyl-1H-1,2,4-triazol-1-yl)-3-sulfamoylphenyl]acetamide
(D36) (2-chlorophenyl)-N-[4-(3-cyclopropyl-1H-1,2,4-triazol-1-yl)-3-sulfamoylphenyl]acetamide
(D37) 2-(2-chlorophenyl)-N-[4-(5-cyclopropyl-1H-1,2,4-triazol-1-yl)-3-sulfamoylphenyl]acetamide
(D38) 2-(2-chlorophenyl)-N-{4-[3-(methoxymethyl)-1H-1,2,4-triazol-1-yl]-3-sulfamoylphenyl}acetamide
(D39) 2-(2-chlorophenyl)-N-[4-(4-cyano-1H-pyrazol-1-yl)-3-sulfamoylphenyl]acetamide
(D40) N-[4-(4-tert-butyl-1H-imidazol-1-yl)-3-sulfamoylphenyl]-2-(2-chlorophenyl)acetamide
(D41) 2-(2-chlorophenyl)-N-[4-(3-cyano-1H-1,2,4-triazol-1-yl)-3-sulfamoylphenyl]acetamide
(D42) N-[4-(4-bromo-1H-imidazol-1-yl)-3-sulfamoylphenyl]-2-(2-chlorophenyl)acetamide
(D43) 2-(2-chlorophenyl)-N-[4-(3H-imidazo[4,5-b]pyridin-3-yl)-3-sulfamoylphenyl]acetamide
(D44) 2-(2-chlorophenyl)-N-[4-(1H-imidazo[4,5-b]pyridin-1-yl)-3-sulfamoylphenyl]acetamide
(D45) 2-(2-chlorophenyl)-N-[4-(1H-imidazo[4,5-c]pyridin-1-yl)-3-sulfamoylphenyl]acetamide
(D46) 2-(2-chlorophenyl)-N-[4-(3H-imidazo[4,5-c]pyridin-3-yl)-3-sulfamoylphenyl]acetamide
(D47) 2-(2-chlorophenyl)-N-[4-(2,4-dimethyl-1H-imidazol-1-yl)-3-sulfamoylphenyl]acetamide
(D48) 2-(2-fluorophenyl)-N-[4-(4-fluoro-1H-pyrazol-1-yl)-3-sulfamoylphenyl]acetamide
(D49) N-[4-(4-chloro-1H-pyrazol-1-yl)-3-sulfamoylphenyl]-2-[2-(difluoromethyl)phenyl]acetamide
(D50) N-[4-(4-chloro-1H-pyrazol-1-yl)-3-sulfamoylphenyl]-2-(2-fluorophenyl)acetamide
(D51) 2-[2-(difluoromethyl)phenyl]-N-[4-(4-fluoro-1H-pyrazol-1-yl)-3-sulfamoylphenyl]acetamide
(D52) N-[4-(4-cyano-1H-pyrazol-1-yl)-3-sulfamoylphenyl]-2-(2-fluorophenyl)acetamide
(D53) 2-(2-methoxyphenyl)-N-{3-sulfamoyl-4-[3-(trifluoromethyl)-1H-1,2,4-triazol-1-yl]phenyl}acetamide
(D54) 2-(2-fluorophenyl)-N-{3-sulfamoyl-4-[3-(trifluoromethyl)-1H-1,2,4-triazol-1-yl]phenyl}acetamide
(D55) 2-(3-fluorophenyl)-N-{3-sulfamoyl-4-[3-(trifluoromethyl)-1H-1,2,4-triazol-1-yl]phenyl}acetamide
(D56) 2-(2-chloro-4-fluorophenyl)-N-{3-sulfamoyl-4-[3-(trifluoromethyl)-1H-1,2,4-triazol-1-yl]phenyl}acetamide
(D57) 2-[2-(difluoromethoxy)phenyl]-N-{3-sulfamoyl-4-[3-(trifluoromethyl)-1H-1,2,4-triazol-1-yl]phenyl}acetamide
(D58) 2-(2-chloro-5-fluorophenyl)-N-{3-sulfamoyl-4-[3-(trifluoromethyl)-1H-1,2,4-triazol-1-yl]phenyl}acetamide
(D59) 2-(3-chloropyridin-4-yl)-N-{3-sulfamoyl-4-[3-(trifluoromethyl)-1H-1,2,4-triazol-1-yl]phenyl}acetamide
(D60) 2-[4-(difluoromethyl)phenyl]-N-{3-sulfamoyl-4-[3-(trifluoromethyl)-1H-1,2,4-triazol-1-yl]phenyl}acetamide
(D61) 2-(2-chlorophenyl)-2,2-difluoro-N-{3-sulfamoyl-4-[3-(trifluoromethyl)-1H-1,2,4-triazol-1-yl]phenyl}acetamide
(D62) N-[4-(4-bromo-1H-pyrazol-1-yl)-3-sulfamoylphenyl]-2-(2-chlorophenyl)-2,2-difluoroacetamide
(D63) N-[4-(4-bromo-1H-pyrazol-1-yl)-3-sulfamoylphenyl]-2-(2-fluorophenyl)acetamide
(D64) N-[4-(4-bromo-1H-pyrazol-1-yl)-3-sulfamoylphenyl]-2-[2-(trifluoromethyl)phenyl]acetamide
(D65) N-[4-(4-bromo-1H-pyrazol-1-yl)-3-sulfamoylphenyl]-2-(3-fluorophenyl)acetamide
(D66) N-[4-(4-bromo-1H-pyrazol-1-yl)-3-sulfamoylphenyl]-2-(2-methylphenyl)acetamide
(D67) N-[4-(4-bromo-1H-pyrazol-1-yl)-3-sulfamoylphenyl]-2-(2-chloropyridin-3-yl)acetamide
(D68) N-[4-(4-bromo-1H-pyrazol-1-yl)-3-sulfamoylphenyl]-2-(2-chloro-4-fluorophenyl)acetamide
(D69) N-[4-(4-bromo-1H-pyrazol-1-yl)-3-sulfamoylphenyl]-2-[2-(difluoromethoxy)phenyl]acetamide
(D70) N-[4-(4-bromo-1H-pyrazol-1-yl)-3-sulfamoylphenyl]-2-[2-(trifluoromethoxy)phenyl]acetamide
(D71) N-[4-(4-bromo-1H-pyrazol-1-yl)-3-sulfamoylphenyl]-2-(2-chloro-4,5-difluorophenyl)acetamide
(D72) N-[4-(4-bromo-1H-pyrazol-1-yl)-3-sulfamoylphenyl]-2-(3-chloropyridin-4-yl)acetamide
(D73) N-[4-(4-bromo-1H-pyrazol-1-yl)-3-sulfamoylphenyl]-2-[4-(difluoromethyl)phenyl]acetamide
(D74) 2-(2-bromophenyl)-N-[4-(4-chloro-1H-pyrazol-1-yl)-3-sulfamoylphenyl]acetamide
(D75) N-[4-(4-chloro-1H-pyrazol-1-yl)-3-sulfamoylphenyl]-2-(2-methoxyphenyl)acetamide
(D76) 2-(4-chlorophenyl)-N-[4-(4-chloro-1H-pyrazol-1-yl)-3-sulfamoylphenyl]acetamide
(D77) 2-(2-chloro-6-fluorophenyl)-N-[4-(4-chloro-1H-pyrazol-1-yl)-3-sulfamoylphenyl]acetamide
(D78) N-[4-(4-chloro-1H-pyrazol-1-yl)-3-sulfamoylphenyl]-2-(2-nitrophenyl)acetamide
(D79) N-[4-(4-chloro-1H-pyrazol-1-yl)-3-sulfamoylphenyl]-2-(2,4-dichlorophenyl)acetamide
(D80) N-[4-(4-chloro-1H-pyrazol-1-yl)-3-sulfamoylphenyl]-2-(2,6-dichlorophenyl)acetamide
(D81) N-[4-(4-chloro-1H-pyrazol-1-yl)-3-sulfamoylphenyl]-2-(3,4-difluorophenyl)acetamide
(D82) 2-(3-chlorophenyl)-N-[4-(4-chloro-1H-pyrazol-1-yl)-3-sulfamoylphenyl]acetamide
(D83) N-[4-(4-chloro-1H-pyrazol-1-yl)-3-sulfamoylphenyl]-2-(3,5-difluorophenyl)acetamide
(D84) N-[4-(4-chloro-1H-pyrazol-1-yl)-3-sulfamoylphenyl]-2-(4-fluorophenyl)acetamide
(D85) N-[4-(4-chloro-1H-pyrazol-1-yl)-3-sulfamoylphenyl]-2-(2-hydroxyphenyl)acetamide
(D86) N-[4-(4-chloro-1H-pyrazol-1-yl)-3-sulfamoylphenyl]-2-(4-hydroxyphenyl)acetamide
(D87) N-[4-(4-chloro-1H-pyrazol-1-yl)-3-sulfamoylphenyl]-2-(2,3-difluorophenyl)acetamide
(D88) 2-(2-chloro-4-fluorophenyl)-N-[4-(4-chloro-1H-pyrazol-1-yl)-3-sulfamoylphenyl]acetamide
(D89) N-[4-(4-chloro-1H-pyrazol-1-yl)-3-sulfamoylphenyl]-2-(2-chloropyridin-3-yl)acetamide
(D90) N-[4-(4-chloro-1H-pyrazol-1-yl)-3-sulfamoylphenyl]-2-[2-chloro-3-(trifluoromethyl)phenyl]acetamide
(D91) N-[4-(4-chloro-1H-pyrazol-1-yl)-3-sulfamoylphenyl]-2-[2-(difluoromethoxy)phenyl]acetamide
(D92) N-[4-(4-chloro-1H-pyrazol-1-yl)-3-sulfamoylphenyl]-2-[2-(trifluoromethoxy)phenyl]acetamide
(D93) 2-(2-chloro-4,5-difluorophenyl)-N-[4-(4-chloro-1H-pyrazol-1-yl)-3-sulfamoylphenyl]acetamide
(D94) 2-(2-chloro-4-methoxyphenyl)-N-[4-(4-chloro-1H-pyrazol-1-yl)-3-sulfamoylphenyl]acetamide
(D95) 2-(2-chloro-6-fluoro-3-methylphenyl)-N-[4-(4-chloro-1H-pyrazol-1-yl)-3-sulfamoylphenyl]acetamide
(D96) 2-(2-chloro-3,6-difluorophenyl)-N-[4-(4-chloro-1H-pyrazol-1-yl)-3-sulfamoylphenyl]acetamide
(D97) 2-(2-chloro-5-methylphenyl)-N-[4-(4-chloro-1H-pyrazol-1-yl)-3-sulfamoylphenyl]acetamide
(D98) 2-(2-chloro-5-fluorophenyl)-N-[4-(4-chloro-1H-pyrazol-1-yl)-3-sulfamoylphenyl]acetamide
(D99) N-[4-(4-chloro-1H-pyrazol-1-yl)-3-sulfamoylphenyl]-2-(2,5-dichlorophenyl)acetamide
(D100) N-[4-(4-chloro-1H-pyrazol-1-yl)-3-sulfamoylphenyl]-2-(2-isopropylphenyl)acetamide
(D101) 2-(2-chloro-5-methoxyphenyl)-N-[4-(4-chloro-1H-pyrazol-1-yl)-3-sulfamoylphenyl]acetamide
(D102) 2-(2-chloro-4,6-difluorophenyl)-N-[4-(4-chloro-1H-pyrazol-1-yl)-3-sulfamoylphenyl]acetamide
(D103) N-[4-(4-chloro-1H-pyrazol-1-yl)-3-sulfamoylphenyl]-2-[2-chloro-6-(trifluoromethyl)phenyl]acetamide
(D104) 2-(5-bromo-4-fluoro-2-methylphenyl)-N-[4-(4-chloro-1H-pyrazol-1-yl)-3-sulfamoylphenyl]acetamide
(D105) 2-(2-chloro-6-methoxyphenyl)-N-[4-(4-chloro-1H-pyrazol-1-yl)-3-sulfamoylphenyl]acetamide
(D106) N-[4-(4-chloro-1H-pyrazol-1-yl)-3-sulfamoylphenyl]-2-(2,6-difluorophenyl)propanamide
(D107) N-[4-(4-chloro-1H-pyrazol-1-yl)-3-sulfamoylphenyl]-2-[4-(difluoromethyl)phenyl]acetamide
(D108) 2-(4-chlorophenyl)-N-{3-sulfamoyl-4-[4-(trifluoromethyl)-1H-pyrazol-1-yl]phenyl}acetamide
(D109) 2-(2-chlorophenyl)-N-[4-(4-chloro-1H-pyrazol-1-yl)-3-sulfamoylphenyl]-2,2-difluoroacetamide
(D110) 2-(2-nitrophenyl)-N-{3-sulfamoyl-4-[4-(trifluoromethyl)-1H-pyrazol-1-yl]phenyl}acetamide
(D111) 2-(2,4-dichlorophenyl)-N-{3-sulfamoyl-4-[4-(trifluoromethyl)-1H-pyrazol-1-yl]phenyl}acetamide
(D112) 2-(2-methoxyphenyl)-N-{3-sulfamoyl-4-[4-(trifluoromethyl)-1H-pyrazol-1-yl]phenyl}acetamide;
(D113) 2-(2-chloro-6-fluorophenyl)-N-{3-sulfamoyl-4-[4-(trifluoromethyl)-1H-pyrazol-1-yl]phenyl}acetamide
(D114) 2-(3-fluorophenyl)-N-{3-sulfamoyl-4-[4-(trifluoromethyl)-1H-pyrazol-1-yl]phenyl}acetamide
(D115) 2-(3,5-difluorophenyl)-N-{3-sulfamoyl-4-[4-(trifluoromethyl)-1H-pyrazol-1-yl]phenyl}acetamide
(D116) 2-(2,6-dichlorophenyl)-N-{3-sulfamoyl-4-[4-(trifluoromethyl)-1H-pyrazol-1-yl]phenyl}acetamide
(D117) 2-(2-bromophenyl)-N-{3-sulfamoyl-4-[4-(trifluoromethyl)-1H-pyrazol-1-yl]phenyl}acetamide
(D118) 2-(3,4-difluorophenyl)-N-{3-sulfamoyl-4-[4-(trifluoromethyl)-1H-pyrazol-1-yl]phenyl}acetamide
(D119) 2-(4-fluorophenyl)-N-{3-sulfamoyl-4-[4-(trifluoromethyl)-1H-pyrazol-1-yl]phenyl}acetamide
(D120) 2-(3-chlorophenyl)-N-{3-sulfamoyl-4-[4-(trifluoromethyl)-1H-pyrazol-1-yl]phenyl}acetamide
(D121) 2-(4-methoxyphenyl)-N-{3-sulfamoyl-4-[4-(trifluoromethyl)-1H-pyrazol-1-yl]phenyl}acetamide
(D122) 2-(2,3-difluorophenyl)-N-{3-sulfamoyl-4-[4-(trifluoromethyl)-1H-pyrazol-1-yl]phenyl}acetamide
(D123) 2-(2-methylphenyl)-N-{3-sulfamoyl-4-[4-(trifluoromethyl)-1H-pyrazol-1-yl]phenyl}acetamide
(D124) 2-(3-methylphenyl)-N-{3-sulfamoyl-4-[4-(trifluoromethyl)-1H-pyrazol-1-yl]phenyl}acetamide
(D125) 2-[2-(difluoromethoxy)phenyl]-N-{3-sulfamoyl-4-[4-(trifluoromethyl)-1H-pyrazol-1-yl]phenyl}acetamide
(D126) 2-(2-chloropyridin-3-yl)-N-{3-sulfamoyl-4-[4-(trifluoromethyl)-1H-pyrazol-1-yl]phenyl}acetamide
(D127) 2-(2-chloro-4-fluorophenyl)-N-{3-sulfamoyl-4-[4-(trifluoromethyl)-1H-pyrazol-1-yl]phenyl}acetamide
(D128) 2-(2-chloro-5-methylphenyl)-N-{3-sulfamoyl-4-[4-(trifluoromethyl)-1H-pyrazol-1-yl]phenyl}acetamide
(D129) N-{3-sulfamoyl-4-[4-(trifluoromethyl)-1H-pyrazol-1-yl]phenyl}-2-[2-(trifluoromethoxy)phenyl]acetamide
(D130) 2-(2-chloro-4,5-difluorophenyl)-N-{3-sulfamoyl-4-[4-(trifluoromethyl)-1H-pyrazol-1-yl]phenyl}acetamide
(D131) 2-[2-chloro-3-(trifluoromethyl)phenyl]-N-{3-sulfamoyl-4-[4-(trifluoromethyl)-1H-pyrazol-1-yl]phenyl}acetamide
(D132) 2-(2-chloro-6-fluoro-3-methylphenyl)-N-{3-sulfamoyl-4-[4-(trifluoromethyl)-1H-pyrazol-1-yl]phenyl}acetamide
(D133) 2-(2-chloro-3,6-difluorophenyl)-N-{3-sulfamoyl-4-[4-(trifluoromethyl)-1H-pyrazol-1-yl]phenyl}acetamide
(D134) 2-[2-chloro-6-(trifluoromethyl)phenyl]-N-{3-sulfamoyl-4-[4-(trifluoromethyl)-1H-pyrazol-1-yl]phenyl}acetamide
(D135) 2-(2-chloro-4-methoxyphenyl)-N-{3-sulfamoyl-4-[4-(trifluoromethyl)-1H-pyrazol-1-yl]phenyl}acetamide
(D136) 2-(2,5-dichlorophenyl)-N-{3-sulfamoyl-4-[4-(trifluoromethyl)-1H-pyrazol-1-yl]phenyl}acetamide
(D137) 2-(2-isopropylphenyl)-N-{3-sulfamoyl-4-[4-(trifluoromethyl)-1H-pyrazol-1-yl]phenyl}acetamide
(D138) 2-(2-chloro-5-methoxyphenyl)-N-{3-sulfamoyl-4-[4-(trifluoromethyl)-1H-pyrazol-1-yl]phenyl}acetamide
(D139) 2-(2-chloro-5-fluorophenyl)-N-{3-sulfamoyl-4-[4-(trifluoromethyl)-1H-pyrazol-1-yl]phenyl}acetamide
(D140) N-[4-(4-fluoro-1H-pyrazol-1-yl)-3-sulfamoylphenyl]-2-[2-(trifluoromethyl)phenyl]acetamide
(D141) 2-(5-bromo-4-fluoro-2-methylphenyl)-N-{3-sulfamoyl-4-[4-(trifluoromethyl)-1H-pyrazol-1-yl]phenyl}acetamide
(D142) 2-(2-chloro-6-methoxyphenyl)-N-{3-sulfamoyl-4-[4-(trifluoromethyl)-1H-pyrazol-1-yl]phenyl}acetamide
(D143) 2-(2-chloro-4,6-difluorophenyl)-N-{3-sulfamoyl-4-[4-(trifluoromethyl)-1H-pyrazol-1-yl]phenyl}acetamide
(D144) 2-(3-fluorophenyl)-N-[4-(4-fluoro-1H-pyrazol-1-yl)-3-sulfamoylphenyl]acetamide
(D145) 2-(3,4-difluorophenyl)-N-[4-(4-fluoro-1H-pyrazol-1-yl)-3-sulfamoylphenyl]acetamide
(D146) 2-(3,5-difluorophenyl)-N-[4-(4-fluoro-1H-pyrazol-1-yl)-3-sulfamoylphenyl]acetamide
(D147) N-[4-(4-fluoro-1H-pyrazol-1-yl)-3-sulfamoylphenyl]-2-(2-hydroxyphenyl)acetamide
(D148) 2-(3-chlorophenyl)-N-[4-(4-fluoro-1H-pyrazol-1-yl)-3-sulfamoylphenyl]acetamide
(D149) 2-(4-fluorophenyl)-N-[4-(4-fluoro-1H-pyrazol-1-yl)-3-sulfamoylphenyl]acetamide
(D150) N-[4-(4-fluoro-1H-pyrazol-1-yl)-3-sulfamoylphenyl]-2-(4-hydroxyphenyl)acetamide
(D151) N-[4-(4-fluoro-1H-pyrazol-1-yl)-3-sulfamoylphenyl]-2-(3-methylphenyl)acetamide
(D152) N-[4-(4-fluoro-1H-pyrazol-1-yl)-3-sulfamoylphenyl]-2-(4-methylphenyl)acetamide
(D153) N-[4-(4-fluoro-1H-pyrazol-1-yl)-3-sulfamoylphenyl]-2-(4-methoxyphenyl)acetamide
(D154) N-[4-(4-fluoro-1H-pyrazol-1-yl)-3-sulfamoylphenyl]-2-(2-methylphenyl)acetamide
(D155) 2-(2,3-difluorophenyl)-N-[4-(4-fluoro-1H-pyrazol-1-yl)-3-sulfamoylphenyl]acetamide
(D156) 2-(2-ethoxyphenyl)-N-[4-(4-fluoro-1H-pyrazol-1-yl)-3-sulfamoylphenyl]acetamide
(D157) 2-[2-(difluoromethoxy)phenyl]-N-[4-(4-fluoro-1H-pyrazol-1-yl)-3-sulfamoylphenyl]acetamide
(D158) 2-(2-chloropyridin-3-yl)-N-[4-(4-fluoro-1H-pyrazol-1-yl)-3-sulfamoylphenyl]acetamide
(D159) 2-(2-chloro-6-fluoro-3-methylphenyl)-N-[4-(4-fluoro-1H-pyrazol-1-yl)-3-sulfamoylphenyl]acetamide
(D160) N-[4-(4-fluoro-1H-pyrazol-1-yl)-3-sulfamoylphenyl]-2-[2-(trifluoromethoxy)phenyl]acetamide
(D161) 2-(2-chloro-4,5-difluorophenyl)-N-[4-(4-fluoro-1H-pyrazol-1-yl)-3-sulfamoylphenyl]acetamide
(D162) 2-[2-chloro-3-(trifluoromethyl)phenyl]-N-[4-(4-fluoro-1H-pyrazol-1-yl)-3-sulfamoylphenyl]acetamide
(D163) 2-(2,5-dichlorophenyl)-N-[4-(4-fluoro-1H-pyrazol-1-yl)-3-sulfamoylphenyl]acetamide
(D164) 2-(2-chloro-3,6-difluorophenyl)-N-[4-(4-fluoro-1H-pyrazol-1-yl)-3-sulfamoylphenyl]acetamide
(D165) 2-(2-chloro-5-methylphenyl)-N-[4-(4-fluoro-1H-pyrazol-1-yl)-3-sulfamoylphenyl]acetamide
(D166) 2-(5-bromo-4-fluoro-2-methylphenyl)-N-[4-(4-fluoro-1H-pyrazol-1-yl)-3-sulfamoylphenyl]acetamide
(D167) N-[4-(4-fluoro-1H-pyrazol-1-yl)-3-sulfamoylphenyl]-2-(2-isopropylphenyl)acetamide
(D168) 2-(2-chloro-5-fluorophenyl)-N-[4-(4-fluoro-1H-pyrazol-1-yl)-3-sulfamoylphenyl]acetamide
(D169) 2-[2-chloro-6-(trifluoromethyl)phenyl]-N-[4-(4-fluoro-1H-pyrazol-1-yl)-3-sulfamoylphenyl]acetamide
(D170) N-[4-(4-chloro-1H-pyrazol-1-yl)-3-sulfamoylphenyl]-2-(4-methoxyphenyl)acetamide
(D171) 2-(2,6-difluorophenyl)-N-[4-(4-fluoro-1H-pyrazol-1-yl)-3-sulfamoylphenyl]propanamide
(D172) N-[4-(4-chloro-1H-pyrazol-1-yl)-3-sulfamoylphenyl]-2-(4-methylphenyl)acetamide
(D173) N-[4-(4-chloro-1H-pyrazol-1-yl)-3-sulfamoylphenyl]-2-(2-methylphenyl)acetamide
(D174) N-[4-(4-chloro-1H-pyrazol-1-yl)-3-sulfamoylphenyl]-2-(3-methylphenyl)acetamide
(D175) N-[4-(4-chloro-1H-pyrazol-1-yl)-3-sulfamoylphenyl]-2-(2-ethoxyphenyl)acetamide
(D176) 2-(2-ethoxyphenyl)-N-{3-sulfamoyl-4-[4-(trifluoromethyl)-1H-pyrazol-1-yl]phenyl}acetamide
(D177) 2-(2-bromophenyl)-N-[4-(4-fluoro-1H-pyrazol-1-yl)-3-sulfamoylphenyl]acetamide
(D178) 2-(2,4-dichlorophenyl)-N-[4-(4-fluoro-1H-pyrazol-1-yl)-3-sulfamoylphenyl]acetamide
(D179) 2-(2,6-dichlorophenyl)-N-[4-(4-fluoro-1H-pyrazol-1-yl)-3-sulfamoylphenyl]acetamide
(D180) N-[4-(4-cyano-1H-pyrazol-1-yl)-3-sulfamoylphenyl]-2-(2-nitrophenyl)acetamide
(D181) 2-(4-chlorophenyl)-N-[4-(4-cyano-1H-pyrazol-1-yl)-3-sulfamoylphenyl]acetamide
(D182) N-[4-(4-cyano-1H-pyrazol-1-yl)-3-sulfamoylphenyl]-2-(2-methoxyphenyl)acetamide
(D183) 2-(2-chloro-6-fluorophenyl)-N-[4-(4-cyano-1H-pyrazol-1-yl)-3-sulfamoylphenyl]acetamide
(D184) N-[4-(4-cyano-1H-pyrazol-1-yl)-3-sulfamoylphenyl]-2-[2-(trifluoromethyl)phenyl]acetamide
(D185) N-[4-(4-cyano-1H-pyrazol-1-yl)-3-sulfamoylphenyl]-2-(3-fluorophenyl)acetamide
(D186) N-[4-(4-cyano-1H-pyrazol-1-yl)-3-sulfamoylphenyl]-2-(2,4-dichlorophenyl)acetamide
(D187) N-[4-(4-cyano-1H-pyrazol-1-yl)-3-sulfamoylphenyl]-2-(2,6-dichlorophenyl)acetamide
(D188) 2-(2-bromophenyl)-N-[4-(4-cyano-1H-pyrazol-1-yl)-3-sulfamoylphenyl]acetamide
(D189) N-[4-(4-cyano-1H-pyrazol-1-yl)-3-sulfamoylphenyl]-2-(3,4-difluorophenyl)acetamide
(D190) N-[4-(4-cyano-1H-pyrazol-1-yl)-3-sulfamoylphenyl]-2-(3,5-difluorophenyl)acetamide
(D191) 2-(3-chlorophenyl)-N-[4-(4-cyano-1H-pyrazol-1-yl)-3-sulfamoylphenyl]acetamide
(D192) N-[4-(4-cyano-1H-pyrazol-1-yl)-3-sulfamoylphenyl]-2-(4-fluorophenyl)acetamide
(D193) N-[4-(4-cyano-1H-pyrazol-1-yl)-3-sulfamoylphenyl]-2-(2-hydroxyphenyl)acetamide
(D194) N-[4-(4-cyano-1H-pyrazol-1-yl)-3-sulfamoylphenyl]-2-(4-hydroxyphenyl)acetamide
(D195) N-[4-(4-cyano-1H-pyrazol-1-yl)-3-sulfamoylphenyl]-2-(4-methylphenyl)acetamide
(D196) N-[4-(4-cyano-1H-pyrazol-1-yl)-3-sulfamoylphenyl]-2-(4-methoxyphenyl)acetamide
(D197) N-[4-(4-cyano-1H-pyrazol-1-yl)-3-sulfamoylphenyl]-2-(2-methylphenyl)acetamide
(D198) N-[4-(4-cyano-1H-pyrazol-1-yl)-3-sulfamoylphenyl]-2-(3-methylphenyl)acetamide
(D199) N-[4-(4-cyano-1H-pyrazol-1-yl)-3-sulfamoylphenyl]-2-(2-ethoxyphenyl)acetamide
(D200) N-[4-(4-cyano-1H-pyrazol-1-yl)-3-sulfamoylphenyl]-2-(2,3-difluorophenyl)acetamide
(D201) 2-(2-chloropyridin-3-yl)-N-[4-(4-cyano-1H-pyrazol-1-yl)-3-sulfamoylphenyl]acetamide
(D202) 2-(2-chloro-4-fluorophenyl)-N-[4-(4-cyano-1H-pyrazol-1-yl)-3-sulfamoylphenyl]acetamide
(D203) N-[4-(4-cyano-1H-pyrazol-1-yl)-3-sulfamoylphenyl]-2-[2-(difluoromethoxy)phenyl]acetamide
(D204) N-[4-(4-cyano-1H-pyrazol-1-yl)-3-sulfamoylphenyl]-2-[2-(trifluoromethoxy)phenyl]acetamide
(D205) 2-(2-chloro-4,5-difluorophenyl)-N-[4-(4-cyano-1H-pyrazol-1-yl)-3-sulfamoylphenyl]acetamide
(D206) 2-[2-chloro-3-(trifluoromethyl)phenyl]-N-[4-(4-cyano-1H-pyrazol-1-yl)-3-sulfamoylphenyl]acetamide
(D207) 2-(2-chloro-6-fluoro-3-methylphenyl)-N-[4-(4-cyano-1H-pyrazol-1-yl)-3-sulfamoylphenyl]acetamide
(D208) 2-(2-chloro-3,6-difluorophenyl)-N-[4-(4-cyano-1H-pyrazol-1-yl)-3-sulfamoylphenyl]acetamide
(D209) 2-(2-chloro-5-methylphenyl)-N-[4-(4-cyano-1H-pyrazol-1-yl)-3-sulfamoylphenyl]acetamide
(D210) 2-(2-chloro-4-methoxyphenyl)-N-[4-(4-cyano-1H-pyrazol-1-yl)-3-sulfamoylphenyl]acetamide
(D211) N-[4-(4-cyano-1H-pyrazol-1-yl)-3-sulfamoylphenyl]-2-(2,5-dichlorophenyl)acetamide
(D212) 2-(5-chloro-2-methoxyphenyl)-N-[4-(4-cyano-1H-pyrazol-1-yl)-3-sulfamoylphenyl]acetamide
(D213) N-[4-(4-cyano-1H-pyrazol-1-yl)-3-sulfamoylphenyl]-2-[2-(propan-2-yl)phenyl]acetamide
(D214) 2-(2-chloro-5-fluorophenyl)-N-[4-(4-cyano-1H-pyrazol-1-yl)-3-sulfamoylphenyl]acetamide
(D215) 2-[2-chloro-6-(trifluoromethyl)phenyl]-N-[4-(4-cyano-1H-pyrazol-1-yl)-3-sulfamoylphenyl]acetamide
(D216) 2-(2-chloro-6-methoxyphenyl)-N-[4-(4-cyano-1H-pyrazol-1-yl)-3-sulfamoylphenyl]acetamide
(D217) 2-(2-chloro-4,6-difluorophenyl)-N-[4-(4-cyano-1H-pyrazol-1-yl)-3-sulfamoylphenyl]acetamide
(D218) 2-(3-chloropyridin-4-yl)-N-[4-(4-cyano-1H-pyrazol-1-yl)-3-sulfamoylphenyl]acetamide
(D219) N-[4-(4-cyano-1H-pyrazol-1-yl)-3-sulfamoylphenyl]-2-[4-(difluoromethyl)phenyl]acetamide
(D220) 2-(2-chlorophenyl)-N-[4-(4-cyano-1H-pyrazol-1-yl)-3-sulfamoylphenyl]-2,2-difluoroacetamide
(D221) 2-(2-fluorophenyl)-N-{3-sulfamoyl-4-[5-(trifluoromethyl)pyridin-3-yl]phenyl}acetamide
(D222) N-{3-sulfamoyl-4-[5-(trifluoromethyl)pyridin-3-yl]phenyl}-2-[2-(trifluoromethoxy)phenyl]acetamide
(D223) N-{4-[1-(difluoromethyl)-1H-pyrazol-4-yl]-3-sulfamoylphenyl}-2-(2-methylphenyl)acetamide
(D224) N-{4-[1-(difluoromethyl)-1H-pyrazol-4-yl]-3-sulfamoylphenyl}-2-[2-(trifluoromethyl)phenyl]acetamide
(D225) 2-[2-(difluoromethyl)phenyl]-N-{4-[1-(difluoromethyl)-1H-pyrazol-4-yl]-3-sulfamoylphenyl}acetamide
(D226) N-{4-[1-(difluoromethyl)-1H-pyrazol-4-yl]-3-sulfamoylphenyl}-2-(2-methoxyphenyl)acetamide
(D227) N-{4-[1-(difluoromethyl)-1H-pyrazol-4-yl]-3-sulfamoylphenyl}-2-(4-fluorophenyl)acetamide
(D228) 2-(2-chloro-5-fluorophenyl)-N-{4-[1-(difluoromethyl)-1H-pyrazol-4-yl]-3-sulfamoylphenyl}acetamide
(D229) 2-(2,3-dichlorophenyl)-N-{4-[1-(difluoromethyl)-1H-pyrazol-4-yl]-3-sulfamoylphenyl}acetamide
(D230) 2-(3-chloropyridin-4-yl)-N-{4-[1-(difluoromethyl)-1H-pyrazol-4-yl]-3-sulfamoylphenyl}acetamide
(D231) 2-(2-chlorophenyl)-N-[4-(3-cyclobutyl-1H-1,2,4-triazol-1-yl)-3-sulfamoylphenyl]acetamide
(D232) N-[4-(4-acetyl-1H-pyrazol-1-yl)-3-sulfamoylphenyl]-2-(2-chlorophenyl)acetamide
(D233) 2-(2-chlorophenyl)-N-[4-(3-isopropyl-1H-1,2,4-triazol-1-yl)-3-sulfamoylphenyl]acetamide
(D234) N-[4-(4-chloro-1H-pyrazol-1-yl)-3-sulfamoylphenyl]-2-[2-(trifluoromethyl)phenyl]acetamide
(D235) ethyl 1-(4-{[(2-chlorophenyl)acetyl]amino}-2-sulfamoylphenyl)-1H-pyrazole-4-carboxylate
(D236) ethyl 1-(4-{[(2-fluorophenyl)acetyl]amino}-2-sulfamoylphenyl)-1H-pyrazole-4-carboxylate
(D237) 2-(2-fluorophenyl)-N-{4-[4-(2-hydroxypropan-2-yl)-1H-pyrazol-1-yl]-3-sulfamoylphenyl}acetamide
(D238) 2-(2-chlorophenyl)-N-{4-[4-(2-hydroxypropan-2-yl)-1H-pyrazol-1-yl]-3-sulfamoylphenyl}acetamide
(D239) 2-(2-chloropyridin-3-yl)-N-{3-sulfamoyl-4-[3-(trifluoromethyl)-1H-1,2,4-triazol-1-yl]phenyl}acetamide
(D240) 2-(2-chlorophenyl)-N-methyl-N-{3-sulfamoyl-4-[4-(trifluoromethyl)-1H-pyrazol-1-yl]phenyl}acetamide
(D241) 2-(2-chlorophenyl)-N-{3-sulfamoyl-4-[4-(trifluoromethyl)-2H-1,2,3-triazol-2-yl]phenyl}acetamide
(D242) 2-(2-chlorophenyl)-N-{3-sulfamoyl-4-[4-(trifluoromethyl)-1H-1,2,3-triazol-1-yl]phenyl}acetamide
(D243) 2-(2-chloro-5-cyanophenyl)-N-{3-sulfamoyl-4-[4-(trifluoromethyl)-1H-pyrazol-1-yl]phenyl}acetamide
(D244) 2-(2-chlorophenyl)-N-{3-cyano-5-sulfamoyl-4-[4-(trifluoromethyl)-1H-pyrazol-1-yl]phenyl}acetamide
(D245) 2-(5-bromo-2-chlorophenyl)-N-{3-sulfamoyl-4-[4-(trifluoromethyl)-1H-pyrazol-1-yl]phenyl}acetamide
(D246) N-[4-(3-chloro-1H-1,2,4-triazol-1-yl)-3-sulfamoylphenyl]-2-(2-fluorophenyl)acetamide
(D247) 2-(2-chlorophenyl)-N-[3-cyano-4-(4-fluoro-1H-pyrazol-1-yl)-5-sulfamoylphenyl]acetamide
(D248) 2-(2-chlorophenyl)-N-[4-(4-methyl-1H-pyrazol-1-yl)-3-sulfamoylphenyl]acetamide
(D249) 2-(2-chlorophenyl)-N-[4-(3-methoxy-1H-1,2,4-triazol-1-yl)-3-sulfamoylphenyl]acetamide
(D250) 2-(2-chlorophenyl)-N-[4-(4-cyclopropyl-1H-imidazol-1-yl)-3-sulfamoylphenyl]acetamide
(D251) 2-(2-chlorophenyl)-N-[4-(4-methyl-1H-imidazol-1-yl)-3-sulfamoylphenyl]acetamide
(D252) 2-(2-chlorophenyl)-N-[4-(3-cyclopropyl-1H-pyrazol-1-yl)-3-sulfamoylphenyl]acetamide
(D253) 2-(2-chlorophenyl)-N-[4-(2H-pyrazolo[3,4-b]pyridin-2-yl)-3-sulfamoylphenyl]acetamide
(D254) 2-(2-chlorophenyl)-N-[4-(2H-pyrazolo[3,4-c]pyridin-2-yl)-3-sulfamoylphenyl]acetamide
(D255) 2-(2-chlorophenyl)-N-[4-(2H-pyrazolo[4,3-b]pyridin-2-yl)-3-sulfamoylphenyl]acetamide
(D256) 2-(2-chlorophenyl)-N-{4-[4-(2-methoxyethyl)-1H-pyrazol-1-yl]-3-sulfamoylphenyl}acetamide
(D257) 2-(2-chlorophenyl)-N-[4-(3-fluoro-1H-pyrazol-1-yl)-3-sulfamoylphenyl]acetamide
(D258) 2-(2-chlorophenyl)-N-(4-{4-[(2,2-difluoroethyl)amino]-1H-pyrazol-1-yl}-3-sulfamoylphenyl) acetamide
(D259) 2-(2-chlorophenyl)-N-{4-[4-(2,2-difluoroethyl)-1H-pyrazol-1-yl]-3-sulfamoylphenyl}acetamide
(D260) 2-(2-chlorophenyl)-N-[4-(4-{[(2,2-difluoroethyl)amino]methyl}-1H-pyrazol-1-yl)-3-sulfamoylphenyl]acetamide
(D261) 2-(2-chlorophenyl)-N-{3-sulfamoyl-4-[5-(trifluoromethyl)-1,2,4-oxadiazol-3-yl]phenyl}acetamide
(D262) 2-(2-fluorophenyl)-N-[4-(4-methyl-1H-pyrazol-1-yl)-3-sulfamoylphenyl]acetamide
(D263) N-[4-(4-cyclopropyl-1H-imidazol-1-yl)-3-sulfamoylphenyl]-2-(2-fluorophenyl)acetamide
(D264) N-[4-(3-cyclopropyl-1H-pyrazol-1-yl)-3-sulfamoylphenyl]-2-(2-fluorophenyl)acetamide
(D265) 2-(2-fluorophenyl)-N-{4-[4-(2-methoxyethyl)-1H-pyrazol-1-yl]-3-sulfamoylphenyl}acetamide
(D266) 2-(2-chlorophenyl)-N-(2-sulfamoylbiphenyl-4-yl)acetamide
(D267) 2-(2-chlorophenyl)-N-{4-[1-(cyclopropylmethyl)-1H-pyrazol-4-yl]-3-sulfamoylphenyl}acetamide
(D268) 2-(2-chlorophenyl)-N-{4-[1-(2-methoxyethyl)-1H-pyrazol-4-yl]-3-sulfamoylphenyl}acetamide
(D269) 2-(2-chlorophenyl)-N-{4-[1-(piperidin-4-yl)-1H-pyrazol-4-yl]-3-sulfamoylphenyl}acetamide
(D270) 2-(2-fluorophenyl)-N-{4-[1-(piperidin-4-yl)-1H-pyrazol-4-yl]-3-sulfamoylphenyl}acetamide
(D271) N-{4-[1-(azetidin-3-yl)-1H-pyrazol-4-yl]-3-sulfamoylphenyl}-2-(2-chlorophenyl)acetamide
(D272) 2-(3-chlorophenyl)-N-(2-sulfamoylbiphenyl-4-yl)acetamide
(D273) 2-(2-chlorophenyl)-N-[4-(3-methyl-1H-pyrazol-1-yl)-3-sulfamoylphenyl]acetamide
(D274) 2-(2-chlorophenyl)-N-[4-(6-chloropyridin-3-yl)-3-sulfamoylphenyl]acetamide
(D275) 2-(2-chlorophenyl)-N-[4-(3,5-dimethyl-1,2-oxazol-4-yl)-3-sulfamoylphenyl]acetamide
(D276) 2-(2-chlorophenyl)-N-(4-{1-[(3-methyloxetan-3-yl)methyl]-1H-pyrazol-4-yl}-3-sulfamoylphenyl)acetamide
(D277) 2-(2-chloro-4-fluorophenyl)-N-{4-[1-(2-methoxyethyl)-1H-pyrazol-4-yl]-3-sulfamoylphenyl}acetamide
(D278) 2-(2-chloro-4-fluorophenyl)-N-[4-(1-cyclopropyl-1H-pyrazol-4-yl)-3-sulfamoylphenyl]acetamide
(D279) 2-(2-chlorophenyl)-N-(3-sulfamoyl-4-{1-[2-(trifluoromethoxy)ethyl]-1H-pyrazol-4-yl}phenyl)acetamide
(D280) 2-(2-chlorophenyl)-N-[4-(1-cyclobutyl-1H-pyrazol-4-yl)-3-sulfamoylphenyl]acetamide
(D281) 2-(2-chlorophenyl)-N-(4-{1-[2-(pyrrolidin-1-yl)ethyl]-1H-pyrazol-4-yl}-3-sulfamoylphenyl)acetamide
(D282) 2-(2-chlorophenyl)-N-[4-(5-cyanopyridin-3-yl)-3-sulfamoylphenyl]acetamide
(D283) 2-(2-chlorophenyl)-N-(4-{1-[oxetan-2-ylmethyl]-1H-pyrazol-4-yl}-3-sulfamoylphenyl) acetamide
(D284) 2-(2-chlorophenyl)-N-{3-sulfamoyl-4-[2-(2,2,2-trifluoroethoxy)pyrimidin-5-yl]phenyl}acetamide
(D285) 2-(2-chlorophenyl)-N-[4-(2-methoxypyrimidin-5-yl)-3-sulfamoylphenyl]acetamide
(D286) 2-(2-chlorophenyl)-N-(4-{1-[2-(propan-2-yloxy)ethyl]-1H-pyrazol-4-yl}-3-sulfamoylphenyl)acetamide
(D287) 2-(2-chlorophenyl)-N-{4-[1-(3-hydroxy-3-methylbutyl)-1H-pyrazol-4-yl]-3-sulfamoylphenyl}acetamide
(D288) 2-(2-chlorophenyl)-N-{4-[5-(2-hydroxypropan-2-yl)-1-methyl-1H-pyrazol-3-yl]-3-sulfamoylphenyl}acetamide
(D289) 2-(2-chlorophenyl)-N-{4-[5-(difluoromethoxy)pyridin-3-yl]-3-sulfamoylphenyl}acetamide
(D290) N-[4-(2-chloro-5-methoxypyridin-3-yl)-3-sulfamoylphenyl]-2-(2-chlorophenyl)acetamide
(D291) 2-(2-chlorophenyl)-N-{4-[1-(2-hydroxyethyl)-1H-pyrazol-4-yl]-3-sulfamoylphenyl}acetamide
(D292) N-[4-(5-tert-butyl-1H-pyrazol-3-yl)-3-sulfamoylphenyl]-2-(2-chlorophenyl)acetamide
(D293) N-[4-(1-benzyl-1H-pyrazol-4-yl)-3-sulfamoylphenyl]-2-(2-chlorophenyl)acetamide
(D294) 2-(2-chlorophenyl)-N-[4-(6-methylpyridazin-4-yl)-3-sulfamoylphenyl]acetamide
(D295) 2-(2-chlorophenyl)-N-{3-sulfamoyl-4-[6-(trifluoromethyl)pyridin-2-yl]phenyl}acetamide
(D296) N-{4-[1-(difluoromethyl)-1H-pyrazol-4-yl]-3-sulfamoylphenyl}-2-(2-fluorophenyl)acetamide
(D297) 2-(2-chlorophenyl)-N-{6-[1-(difluoromethyl)-1H-pyrazol-4-yl]-5-sulfamoylpyridin-3-yl}acetamide
(D298) N-[4-(6-chloro-5-methylpyridin-3-yl)-3-sulfamoylphenyl]-2-(2-chlorophenyl)acetamide
(D299) 2-(2-chlorophenyl)-N-{4-[2-(cyclopropylamino)pyrimidin-5-yl]-3-sulfamoylphenyl}acetamide
(D300) 2-(2-chlorophenyl)-N-{3-sulfamoyl-4-[1-(tetrahydrofuran-3-yl)-1H-pyrazol-4-yl]phenyl}acetamide
(D301) 2-(2-chlorophenyl)-N-{4-[2-(methylamino)pyrimidin-5-yl]-3-sulfamoylphenyl}acetamide
(D302) 2-(2-chlorophenyl)-N-[6-(1-methyl-1H-pyrazol-4-yl)-5-sulfamoylpyridin-3-yl]acetamide
(D303) 2-(2-chlorophenyl)-N-{4-[1-(2,2-difluoroethyl)-1H-pyrazol-4-yl]-3-sulfamoylphenyl}acetamide
(D304) N-{3-sulfamoyl-4-[5-(trifluoromethyl)pyridin-3-yl]phenyl}-2-[2-(trifluoromethyl)phenyl]acetamide
(D305) 2-(2-chlorophenyl)-N-[3-sulfamoyl-5'-(trifluoromethyl)-2,3'-bipyridin-5-yl]acetamide
(D306) 2-(2-chlorophenyl)-N-{3-sulfamoyl-4-[2-(trifluoromethyl)pyrimidin-5-yl]phenyl}acetamide
(D307) 2-(2-chlorophenyl)-N-{3-sulfamoyl-4-[5-(trifluoromethoxy)pyridin-3-yl]phenyl}acetamide
(D308) 2-(2-chlorophenyl)-N-[4-(2-cyclopropylpyrimidin-5-yl)-3-sulfamoylphenyl]acetamide
(D309) 2-(2-chlorophenyl)-N-[4-(2-ethoxypyrimidin-5-yl)-3-sulfamoylphenyl]acetamide
(D310) 2-(2-chlorophenyl)-N-{4-[2-(propan-2-ylamino)pyrimidin-5-yl]-3-sulfamoylphenyl}acetamide
(D311) 2-(2-chlorophenyl)-N-{4-[2-(propan-2-yloxy)pyrimidin-5-yl]-3-sulfamoylphenyl}acetamide
(D312) 2-(2-chlorophenyl)-N-{4-[2-(ethylamino)pyrimidin-5-yl]-3-sulfamoylphenyl}acetamide
(D313) 2-(2-chlorophenyl)-N-[4-(2-methylpyrimidin-5-yl)-3-sulfamoylphenyl]acetamide
(D314) 2-(2-chlorophenyl)-N-{4-[2-(propylamino)pyrimidin-5-yl]-3-sulfamoylphenyl}acetamide
(D315) 2-(2-chlorophenyl)-N-(3-sulfamoyl-4-{2-[(2,2,2-trifluoroethyl)amino]pyrimidin-5-yl}phenyl)acetamide
(D316) 2-(2-chlorophenyl)-N-{4-[2-(cyclobutyloxy)pyrimidin-5-yl]-3-sulfamoylphenyl}acetamide
(D317) N-[4-(2-chloro-4-methylpyrimidin-5-yl)-3-sulfamoylphenyl]-2-(2-chlorophenyl)acetamide
(D318) 2-(2-Chlorophenyl)-N-{3-sulfamoyl-4-[4-(trifluoromethyl)pyridin-2-yl]phenyl}acetamide
(D319) 2-(2-chlorophenyl)-N-[4-(5-chloropyridin-2-yl)-3-sulfamoylphenyl]acetamide
(D320) 2-(2-chlorophenyl)-N-[4-(1,2-dimethyl-1H-imidazol-4-yl)-3-sulfamoylphenyl]acetamide
(D321) N-{6-[1-(difluoromethyl)-1H-pyrazol-4-yl]-5-sulfamoylpyridin-3-yl}-2-(2-fluorophenyl)acetamide
(D322) 2-(2-chlorophenyl)-N-{4-[5-(pyrrolidin-1-yl)pyridin-3-yl]-3-sulfamoylphenyl}acetamide
(D323) 2-(2-chlorophenyl)-N-[4-(1-cyclopropyl-1H-pyrazol-4-yl)-3-sulfamoylphenyl]-2-hydroxyethanamide
(D324) 2-(2-chlorophenyl)-N-{4-[1-(difluoromethyl)-1H-pyrazol-4-yl]-3-sulfamoylphenyl}-2-hydroxyethanamide
(D325) 2-(2-chlorophenyl)-N-[4-(5-chloropyridin-3-yl)-3-sulfamoylphenyl]acetamide
(D326) 2-(2-chlorophenyl)-N-{4-[1-(difluoromethyl)-1H-pyrazol-4-yl]-3-sulfamoylphenyl}acetamide
(D327) 2-(2-chlorophenyl)-N-[4-(5-fluoropyridin-3-yl)-3-sulfamoylphenyl]acetamide
(D328) 2-(2-chlorophenyl)-N-{4-[1-(2-methylpropyl)-1H-pyrazol-4-yl]-3-sulfamoylphenyl}acetamide
(D329) 2-(2-chlorophenyl)-N-[4-(1-cyclopentyl-1H-pyrazol-4-yl)-3-sulfamoylphenyl]acetamide
(D330) 2-(2-chlorophenyl)-N-[2'-fluoro-3'-(propan-2-yloxy)-2-sulfamoylbiphenyl-4-yl]acetamide
(D331) 2-(2-chlorophenyl)-N-{3-sulfamoyl-4-[5-(trifluoromethyl)pyridin-3-yl]phenyl}acetamide
(D332) N-[4-(6-chloro-5-methoxypyridin-3-yl)-3-sulfamoylphenyl]-2-(2-chlorophenyl)acetamide
(D333) 2-(2-chloro-6-fluorophenyl)-N-[4-(1-cyclopropyl-1H-pyrazol-4-yl)-3-sulfamoylphenyl]acetamide
(D334) 2-(2-chloro-3-fluorophenyl)-N-[4-(1-methyl-1H-pyrazol-4-yl)-3-sulfamoylphenyl]acetamide
(D335) N-[4-(1-tert-butyl-1H-pyrazol-4-yl)-3-sulfamoylphenyl]-2-(2-chlorophenyl)acetamide
(D336) 2-(2-chlorophenyl)-N-{4-[2-(propan-2-yloxy)pyridin-3-yl]-3-sulfamoylphenyl}acetamide
(D337) 2-(2-chloro-3-fluorophenyl)-N-[4-(1-cyclopropyl-1H-pyrazol-4-yl)-3-sulfamoylphenyl]acetamide
(D338) 2-(2-fluorophenyl)-N-[4-(pyridin-3-yl)-3-sulfamoylphenyl]acetamide
(D339) 2-(2-chloro-6-fluorophenyl)-N-[4-(1-methyl-1H-pyrazol-4-yl)-3-sulfamoylphenyl]acetamide
(D340) 2-(2-chlorophenyl)-N-[3'-fluoro-5'-(2-hydroxypropan-2-yl)-2-sulfamoylbiphenyl-4-yl]acetamide
(D341) 2-(2-chlorophenyl)-N-[4-(5-methoxypyridin-3-yl)-3-sulfamoylphenyl]acetamide
(D342) 2-(2-chlorophenyl)-N-[3'-(2-hydroxypropan-2-yl)-2-sulfamoylbiphenyl-4-yl]acetamide
(D343) 2-(2-fluorophenyl)-N-{4-[1-(propan-2-yl)-1H-pyrazol-4-yl]-3-sulfamoylphenyl}acetamide
(D344) N-[4-(1-cyclopropyl-1H-pyrazol-4-yl)-3-sulfamoylphenyl]-2-(2-fluorophenyl)acetamide
(D345) 2-(2-chlorophenyl)-N-{3-sulfamoyl-4-[1-(tetrahydro-2H-pyran-2-yl)-1H-pyrazol-4-yl]phenyl}acetamide
(D346) N-[4-(1-tert-butyl-1H-pyrazol-4-yl)-3-sulfamoylphenyl]-2-(2-fluorophenyl)acetamide
(D347) N-[3'-fluoro-5'-(2-hydroxypropan-2-yl)-2-sulfamoylbiphenyl-4-yl]-2-(2-fluorophenyl)acetamide
(D348) N-[4-(1-cyclopentyl-1H-pyrazol-4-yl)-3-sulfamoylphenyl]-2-(2-fluorophenyl)acetamide
(D349) 2-(2-chlorophenyl)-N-[4-(1-cyclopropyl-1H-pyrazol-4-yl)-3-sulfamoylphenyl]acetamide
(D350) 2-(2-chloro-6-fluorophenyl)-N-{4-[1-(2-methoxyethyl)-1H-pyrazol-4-yl]-3-sulfamoylphenyl}acetamide
(D351) 2-(2-chlorophenyl)-N-[4-(1,3-dimethyl-1H-pyrazol-5-yl)-3-sulfamoylphenyl]acetamide
(D352) 2-(2-chlorophenyl)-N-(4'-chloro-2-sulfamoylbiphenyl-4-yl)acetamide
(D353) 2-(2-chlorophenyl)-N-{4-[1-methyl-3-(trifluoromethyl)-1H-pyrazol-5-yl]-3-sulfamoylphenyl}acetamide
(D354) 2-(2-chlorophenyl)-N-[4-(pyridin-3-yl)-3-sulfamoylphenyl]acetamide
(D355) 2-(2-chlorophenyl)-N-{4-[1-methyl-3-(trifluoromethyl)-1H-pyrazol-4-yl]-3-sulfamoylphenyl}acetamide
(D356) 2-(2-chlorophenyl)-N-(3'-chloro-2-sulfamoylbiphenyl-4-yl)acetamide
(D357) 2-(2-chlorophenyl)-N-(4-{1-[(2,2-dichlorocyclopropyl)methyl]-1H-pyrazol-4-yl}-3-sulfamoylphenyl)acetamide
(D358) 2-(2-chlorophenyl)-N-[4-(1,3-dimethyl-1H-pyrazol-4-yl)-3-sulfamoylphenyl]acetamide
(D359) 2-(2-chlorophenyl)-N-{4-[1-(propan-2-yl)-1H-pyrazol-4-yl]-3-sulfamoylphenyl}acetamide
(D360) 2-(2-chlorophenyl)-N-[4-(1-methyl-1H-pyrazol-4-yl)-3-sulfamoylphenyl]acetamide
(D361) 2-(2-chlorophenyl)-N-{4-[1-(2-hydroxy-3,3-dimethylbutyl)-1H-pyrazol-4-yl]-3-sulfamoylphenyl}acetamide
(D362) 2-(2-fluorophenyl)-N-[4-(5-fluoropyridin-3-yl)-3-sulfamoylphenyl]acetamide
(D363) 2-(2-chlorophenyl)-N-[4-(5-methyl-1-phenyl-1H-pyrazol-4-yl)-3-sulfamoylphenyl]acetamide
(D364) 4'-{[(2-chlorophenyl)acetyl]amino}-N-[2-(dimethylamino)ethyl]-2'-sulfamoylbiphenyl-3-carboxamide
(D365) 2-(2-chlorophenyl)-N-[4-(pyrazolo[1,5-a]pyrimidin-3-yl)-3-sulfamoylphenyl]acetamide
(D366) 2-(2-chlorophenyl)-N-{4-[5-(pyrrolidin-1-ylcarbonyl)pyridin-3-yl]-3-sulfamoylphenyl}acetamide
(D367) 2-(2-chlorophenyl)-N-[4-(1H-pyrazol-4-yl)-3-sulfamoylphenyl]acetamide
(D368) 2-(2-fluorophenyl)-N-{4-[1-methyl-3-(trifluoromethyl)-1H-pyrazol-5-yl]-3-sulfamoylphenyl}acetamide
(D369) 2-(2-fluorophenyl)-N-{4-[1-(2-methoxyethyl)-1H-pyrazol-4-yl]-3-sulfamoylphenyl}acetamide
(D370) 2-(2-chlorophenyl)-N-{4-[1-(2-hydroxy-2-methylpropyl)-1H-pyrazol-4-yl]-3-sulfamoylphenyl}acetamide
(D371) 2-(5-chloro-2-fluorophenyl)-N-[4-(1-cyclopropyl-1H-pyrazol-4-yl)-3-sulfamoylphenyl]acetamide
(D372) 2-(2-chlorophenyl)-N-[4-(6-methylpyridin-3-yl)-3-sulfamoylphenyl]acetamide
(D373) 2-(2-chlorophenyl)-N-{4-[1-(oxetan-3-yl)-1H-pyrazol-4-yl]-3-sulfamoylphenyl}acetamide
(D374) 2-(2-fluorophenyl)-N-[4-(1-methyl-1H-pyrazol-4-yl)-3-sulfamoylphenyl]acetamide
(D375) N-[4-(1,3-dimethyl-1H-pyrazol-4-yl)-3-sulfamoylphenyl]-2-(2-fluorophenyl)acetamide
(D376) N-[4-(5-cyclopropyl-1,3,4-oxadiazol-2-yl)-3-sulfamoylphenyl]-2-(2-fluorophenyl)acetamide
(D377) 2-(2-chlorophenyl)-N-[4-(5-cyclopropyl-1,3,4-oxadiazol-2-yl)-3-sulfamoylphenyl]acetamide
(D378) N-{4-[4-(difluoromethyl)-1H-pyrazol-1-yl]-3-sulfamoylphenyl}-2-(2-fluorophenyl)acetamide
(D379) 2-(2-chlorophenyl)-N-{4-[4-(difluoromethyl)-1H-pyrazol-1-yl]-3-sulfamoylphenyl}acetamide
(D380) 2-(2-chlorophenyl)-N-{5-sulfamoyl-6-[4-(trifluoromethyl)-1H-pyrazol-1-yl]pyridin-3-yl}acetamide
(D381) 2-(2-fluorophenyl)-N-{5-sulfamoyl-6-[4-(trifluoromethyl)-1H-pyrazol-1-yl]pyridin-3-yl}acetamide
(D382) N-[6-(4-cyano-1H-pyrazol-1-yl)-5-sulfamoylpyridin-3-yl]-2-(2-fluorophenyl)acetamide
(D383) 2-(2-chlorophenyl)-N-[6-(4-cyano-1H-pyrazol-1-yl)-5-sulfamoylpyridin-3-yl]acetamide
(D384) 2-(2-fluorophenyl)-N-[6-(4-fluoro-1H-pyrazol-1-yl)-5-sulfamoylpyridin-3-yl]acetamide
(D385) 2-(2-chlorophenyl)-N-[6-(4-fluoro-1H-pyrazol-1-yl)-5-sulfamoylpyridin-3-yl]acetamide
(D386) N-[6-(4-bromo-1H-pyrazol-1-yl)-5-sulfamoylpyridin-3-yl]-2-(2-fluorophenyl)acetamide
(D387) 2-(2-chlorophenyl)-N-[6-(4-chloro-1H-pyrazol-1-yl)-5-sulfamoylpyridin-3-yl]acetamide
(D388) N-[6-(4-chloro-1H-pyrazol-1-yl)-5-sulfamoylpyridin-3-yl]-2-(2-fluorophenyl)acetamide
(D389) N-[6-(4-bromo-1H-pyrazol-1-yl)-5-sulfamoylpyridin-3-yl]-2-(2-chlorophenyl)acetamide
(D390) 2-(2-fluorophenyl)-N-{3-sulfamoyl-4-[5-(trifluoromethyl)-1,3,4-oxadiazol-2-yl]phenyl}acetamide
(D391) 2-(2-chlorophenyl)-N-{3-sulfamoyl-4-[5-(trifluoromethyl)-1,3,4-oxadiazol-2-yl]phenyl}acetamide
(D392) 2-(2-chlorophenyl)-N-{4-[3-(pyridin-3-yl)-1H-1,2,4-triazol-1-yl]-3-sulfamoylphenyl}acetamide
(D393) 2-(2-fluorophenyl)-N-[4-(1H-pyrazol-1-yl)-3-sulfamoylphenyl]acetamide
(D394) N-[4-(4-tert-butyl-1H-pyrazol-1-yl)-3-sulfamoylphenyl]-2-(2-chlorophenyl)acetamide
(D395) 2-(2-chlorophenyl)-N-[4-(1H-indazol-1-yl)-3-sulfamoylphenyl]acetamide
(D396) 2-(2-chlorophenyl)-N-[4-(2H-indazol-2-yl)-3-sulfamoylphenyl]acetamide
(D397) 2-(2-fluorophenyl)-N-[4-(2H-indazol-2-yl)-3-sulfamoylphenyl]acetamide
(D398) 2-(2-fluorophenyl)-N-[4-(1H-indazol-1-yl)-3-sulfamoylphenyl]acetamide
(D399) 1-(4-{[(2-chlorophenyl)acetyl]amino}-2-sulfamoylphenyl)-N,N-dimethyl-1H-pyrazole-4-carboxamide
(D400) N-[1-(4-{[(2-chlorophenyl)acetyl]amino}-2-sulfamoylphenyl)-1H-pyrazol-4-yl]cyclopropanecarboxamide
(D401) 2-(2-chlorophenyl)-N-{4-[4-(pyrazin-2-yl)-1H-pyrazol-1-yl]-3-sulfamoylphenyl}acetamide
(D402) 2-(2-chlorophenyl)-N-{4-[4-(pyridin-2-yl)-1H-pyrazol-1-yl]-3-sulfamoylphenyl}acetamide
(D403) 2-(2-chlorophenyl)-N-{4-[4-(pyridin-3-yl)-1H-pyrazol-1-yl]-3-sulfamoylphenyl}acetamide
(D404) 2-(2-chlorophenyl)-N-{4-[4-(pyrimidin-4-yl)-1H-pyrazol-1-yl]-3-sulfamoylphenyl}acetamide
(D405) 2-(2-fluorophenyl)-N-{4-[4-(pyrimidin-4-yl)-1H-pyrazol-1-yl]-3-sulfamoylphenyl}acetamide
(D406) 2-(4-methoxyphenyl)-N-{3-sulfamoyl-4-[4-(trifluoromethyl)-2H-1,2,3-triazol-2-yl]phenyl}acetamide
(D407) 2-(4-methoxyphenyl)-N-{3-sulfamoyl-4-[4-(trifluoromethyl)-1H-1,2,3-triazol-1-yl]phenyl}acetamide
(D408) 2-(4-methylphenyl)-N-{3-sulfamoyl-4-[4-(trifluoromethyl)-2H-1,2,3-triazol-(D)2-yl]phenyl}acetamide
(D409) 2-(4-methylphenyl)-N-{3-sulfamoyl-4-[4-(trifluoromethyl)-1H-1,2,3-triazol-1-yl]phenyl}acetamide
(D410) 2-[4-(difluoromethyl)phenyl]-N-{3-sulfamoyl-4-[4-(trifluoromethyl)-2H-1,2,3-triazol-2-yl]phenyl}acetamide
(D411) 2-[4-(difluoromethyl)phenyl]-N-{3-sulfamoyl-4-[4-(trifluoromethyl)-1H-1,2,3-triazol-1-yl]phenyl}acetamide
(D412) 2-(2-fluorophenyl)-N-{3-sulfamoyl-4-[4-(trifluoromethyl)-2H-1,2,3-triazol-2-yl]phenyl}acetamide
(D413) 2-(2-fluorophenyl)-N-{3-sulfamoyl-4-[4-(trifluoromethyl)-1H-1,2,3-triazol-1-yl]phenyl}acetamide
(D414) 2-[2-chloro-4-(trifluoromethyl)phenyl]-N-{3-sulfamoyl-4-[4-(trifluoromethyl)-2H-1,2,3-triazol-2-yl]phenyl}acetamide
(D415) 2-[2-chloro-4-(trifluoromethyl)phenyl]-N-{3-sulfamoyl-4-[4-(trifluoromethyl)-1H-1,2,3-triazol-1-yl]phenyl}acetamide
(D416) 2-(2,4-dichlorophenyl)-N-{3-sulfamoyl-4-[4-(trifluoromethyl)-2H-1,2,3-triazol-2-yl]phenyl}acetamide
(D417) 2-(2,4-dichlorophenyl)-N-{3-sulfamoyl-4-[4-(trifluoromethyl)-1H-1,2,3-triazol-1-yl]phenyl}acetamide
(D418) 2-(2-chlorophenyl)-N-[3-sulfamoyl-4-(1,3-thiazol-5-yl)phenyl]acetamide
(D419) 2-(2-chlorophenyl)-N-[3-sulfamoyl-4-(1,2-thiazol-3-yl)phenyl]acetamide
(D420) 2-(2-chlorophenyl)-N-[4-(2-methyl-1,3-thiazol-4-yl)-3-sulfamoylphenyl]acetamide
(D421) 2-(2-chlorophenyl)-N-[4-(2-methoxy-1,3-thiazol-4-yl)-3-sulfamoylphenyl]acetamide
(D422) 2-(2-chlorophenyl)-N-[4-(2-methoxy-1,3-thiazol-5-yl)-3-sulfamoylphenyl]acetamide
(D423) 2-(2-chlorophenyl)-N-[4-(2-methyl-1,3-thiazol-5-yl)-3-sulfamoylphenyl]acetamide
(D424) 2-(2-chlorophenyl)-N-[4-(3-methyl-1,2-thiazol-5-yl)-3-sulfamoylphenyl]acetamide
(D425) 2-(2-chlorophenyl)-N-[4-(4-methyl-1,3-thiazol-2-yl)-3-sulfamoylphenyl]acetamide
(D426) 2-(2-chlorophenyl)-N-[3-sulfamoyl-4-(1,2-thiazol-4-yl)phenyl]acetamide
(D427) 2-(2-chlorophenyl)-N-[3-sulfamoyl-4-(1,3-thiazol-2-yl)phenyl]acetamide
(D428) 2-(2-chlorophenyl)-N-[4-(4-cyano-1,3-thiazol-2-yl)-3-sulfamoylphenyl]acetamide
(D429) 2-(2-chlorophenyl)-N-{4-[2-(difluoromethyl)-1,3-thiazol-5-yl]-3-sulfamoylphenyl}acetamide
(D430) 2-(2-chlorophenyl)-N-[4-(2-cyclopropyl-1,3-thiazol-5-yl)-3-sulfamoylphenyl]acetamide
(D431) 2-(2-chlorophenyl)-N-[4-(2-cyclopropyl-1,3-thiazol-4-yl)-3-sulfamoylphenyl]acetamide
(D432) 2-(2-chlorophenyl)-N-[4-(4-methyl-1,3-oxazol-2-yl)-3-sulfamoylphenyl]acetamide
(D433) 2-(2-chlorophenyl)-N-{3-sulfamoyl-4-[2-(trifluoromethyl)-1,3-thiazol-4-yl]phenyl}acetamide
(D434) 2-(2-chlorophenyl)-N-{3-sulfamoyl-4-[4-(trifluoromethyl)-1,3-thiazol-2-yl]phenyl}acetamide
(D435) 2-(2-chlorophenyl)-N-{4-[2-(2-hydroxypropan-2-yl)-1,3-thiazol-5-yl]-3-sulfamoylphenyl}acetamide
(D436) 2-(2-chlorophenyl)-N-{4-[2-(2-hydroxypropan-2-yl)-1,3-thiazol-4-yl]-3-sulfamoylphenyl}acetamide
(D437) 2-(2-chlorophenyl)-N-[3-sulfamoyl-4-(1,3-thiazol-4-yl)phenyl]acetamide
(D438) 2-(2-chlorophenyl)-N-[4-(5-cyclopropyl-1,2-oxazol-3-yl)-3-sulfamoylphenyl]acetamide
(D439) 2-(2-chlorophenyl)-N-[4-(2-cyclopropyl-1,3-oxazol-5-yl)-3-sulfamoylphenyl]acetamide
(D440) 2-(2-chlorophenyl)-N-(4-{4-[(3,3-difluoroazetidin-1-yl)carbonyl]-1H-pyrazol-1-yl}-3-sulfamoylphenyl)acetamide
(D441) N-{4-[4-(azetidin-1-ylcarbonyl)-1H-pyrazol-1-yl]-3-sulfamoylphenyl}-2-(2-chlorophenyl)acetamide
(D442) 2-(2-chlorophenyl)-N-{4-[4-(pyrrolidin-1-ylcarbonyl)-1H-pyrazol-1-yl]-3-sulfamoylphenyl}acetamide
(D443) 2-(2-chlorophenyl)-N-(4-{4-[(3,3-difluoropyrrolidin-1-yl)carbonyl]-1H-pyrazol-1-yl}-3-sulfamoylphenyl)acetamide
(D444) 2-(4-chlorophenyl)-N-{3-sulfamoyl-4-[5-(trifluoromethyl)pyridin-3-yl]phenyl}acetamide
(D445) 2-(2-chloro-6-fluorophenyl)-N-{3-sulfamoyl-4-[5-(trifluoromethyl)pyridin-3-yl]phenyl}acetamide
(D446) N-{3-sulfamoyl-4-[5-(trifluoromethyl)pyridin-3-yl]phenyl}-2-[4-(trifluoromethyl)phenyl]acetamide
(D447) 2-(3-fluorophenyl)-N-{3-sulfamoyl-4-[5-(trifluoromethyl)pyridin-3-yl]phenyl}acetamide
(D448) 2-(2,4-dichlorophenyl)-N-{3-sulfamoyl-4-[5-(trifluoromethyl)pyridin-3-yl]phenyl}acetamide
(D449) 2-(2-bromophenyl)-N-{3-sulfamoyl-4-[5-(trifluoromethyl)pyridin-3-yl]phenyl}acetamide
(D450) 2-(2,4-difluorophenyl)-N-{3-sulfamoyl-4-[5-(trifluoromethyl)pyridin-3-yl]phenyl}acetamide
(D451) 2-(3,4-difluorophenyl)-N-{3-sulfamoyl-4-[5-(trifluoromethyl)pyridin-3-yl]phenyl}acetamide
(D452) 2-(3,5-difluorophenyl)-N-{3-sulfamoyl-4-[5-(trifluoromethyl)pyridin-3-yl]phenyl}acetamide
(D453) 2-(3-chlorophenyl)-N-{3-sulfamoyl-4-[5-(trifluoromethyl)pyridin-3-yl]phenyl}acetamide
(D454) 2-(4-methylphenyl)-N-{3-sulfamoyl-4-[5-(trifluoromethyl)pyridin-3-yl]phenyl}acetamide
(D455) 2-(4-methoxyphenyl)-N-{3-sulfamoyl-4-[5-(trifluoromethyl)pyridin-3-yl]phenyl}acetamide
(D456) 2-(2-fluoro-4-methylphenyl)-N-{3-sulfamoyl-4-[5-(trifluoromethyl)pyridin-3-yl]phenyl}acetamide
(D457) 2-(2-fluoro-4-methoxyphenyl)-N-{3-sulfamoyl-4-[5-(trifluoromethyl)pyridin-3-yl]phenyl}acetamide
(D458) 2-[4-(difluoromethyl)phenyl]-N-{3-sulfamoyl-4-[5-(trifluoromethyl)pyridin-3-yl]phenyl}acetamide
(D459) 2-(4-chlorophenyl)-N-[4-(6-chloropyridin-3-yl)-3-sulfamoylphenyl]acetamide
(D460) N-[4-(6-chloropyridin-3-yl)-3-sulfamoylphenyl]-2-(2-fluorophenyl)acetamide
(D461) 2-(2-chloro-6-fluorophenyl)-N-[4-(6-chloropyridin-3-yl)-3-sulfamoylphenyl]acetamide
(D462) N-[4-(6-chloropyridin-3-yl)-3-sulfamoylphenyl]-2-[4-(trifluoromethyl)phenyl]acetamide
(D463) N-[4-(6-chloropyridin-3-yl)-3-sulfamoylphenyl]-2-[2-(trifluoromethyl)phenyl]acetamide
(D464) N-[4-(6-chloropyridin-3-yl)-3-sulfamoylphenyl]-2-(3-fluorophenyl)acetamide
(D465) N-[4-(6-chloropyridin-3-yl)-3-sulfamoylphenyl]-2-(2,4-dichlorophenyl)acetamide
(D466) N-[4-(6-chloropyridin-3-yl)-3-sulfamoylphenyl]-2-(2,4-difluorophenyl)acetamide
(D467) 2-(2-bromophenyl)-N-[4-(6-chloropyridin-3-yl)-3-sulfamoylphenyl]acetamide
(D468) N-[4-(6-chloropyridin-3-yl)-3-sulfamoylphenyl]-2-(3,4-difluorophenyl)acetamide
(D469) N-[4-(6-chloropyridin-3-yl)-3-sulfamoylphenyl]-2-(4-methylphenyl)acetamide
(D470) N-[4-(6-chloropyridin-3-yl)-3-sulfamoylphenyl]-2-(4-methoxyphenyl)acetamide
(D471) N-[4-(6-chloropyridin-3-yl)-3-sulfamoylphenyl]-2-(2-fluoro-4-methylphenyl)acetamide
(D472) N-[4-(6-chloropyridin-3-yl)-3-sulfamoylphenyl]-2-(2-fluoro-4-methoxyphenyl)acetamide
(D473) N-[4-(6-chloropyridin-3-yl)-3-sulfamoylphenyl]-2-[4-(difluoromethyl)phenyl]acetamide
(D474) N-[4-(6-chloropyridin-3-yl)-3-sulfamoylphenyl]-2-[2-chloro-4-(trifluoromethyl)phenyl]acetamide
(D475) 2-(2,4-dichlorophenyl)-N-[4-(5-fluoropyridin-3-yl)-3-sulfamoylphenyl]acetamide
(D476) 2-(2-fluoro-4-methylphenyl)-N-[4-(5-fluoropyridin-3-yl)-3-sulfamoylphenyl]acetamide
(D477) 2-[4-(difluoromethyl)phenyl]-N-[4-(5-fluoropyridin-3-yl)-3-sulfamoylphenyl]acetamide
(D478) 2-(2-chlorophenyl)-N-[4-(4-cyclopropyl-1H-pyrazol-1-yl)-3-sulfamoylphenyl]acetamide
(D479) N-[4-(4,6-difluoro-2H-benzotriazol-2-yl)-3-sulfamoylphenyl]-2-(2-fluorophenyl)acetamide
(D480) N-[4-(4,6-difluoro-1H-benzotriazol-1-yl)-3-sulfamoylphenyl]-2-(2-fluorophenyl)acetamide
(D481) 2-(2-chlorophenyl)-N-[4-(4,6-difluoro-1H-benzotriazol-1-yl)-3-sulfamoylphenyl]acetamide
(D482) 2-(2-chlorophenyl)-N-[4-(4,6-difluoro-2H-benzotriazol-2-yl)-3-sulfamoylphenyl]acetamide
(D483) 2-(2-chlorophenyl)-N-{3-sulfamoyl-4-[2-(trifluoromethyl)-1,3-thiazol-5-yl]phenyl}acetamide
(D484) 2-(2-chlorophenyl)-N-[4-(4-cyano-1H-pyrazol-1-yl)-5-sulfamoyl-2-(trifluoromethyl)phenyl]acetamide
(D485) 2-[2-chloro-5-(trifluoromethyl)phenyl]-N-[4-(4-cyano-1H-pyrazol-1-yl)-3-sulfamoylphenyl]acetamide
(D486) 2-[2-chloro-5-(trifluoromethyl)phenyl]-N-[4-(4-cyano-1H-pyrazol-1-yl)-5-sulfamoyl-2-(trifluoromethyl)phenyl]acetamide
(D487) 2-(2-chlorophenyl)-N-[4-(4-cyano-1H-pyrazol-1-yl)-3-sulfamoyl-2-(trifluoromethyl)phenyl]acetamide
(D488) 2-[2-chloro-3-(trifluoromethyl)phenyl]-N-[4-(4-cyano-1H-pyrazol-1-yl)-5-sulfamoyl-2-(trifluoromethyl)phenyl]acetamide
(D489) 2-(2-chlorophenyl)-N-{4-[1-(difluoromethyl)-1H-pyrazol-4-yl]-3-sulfamoyl-2-(trifluoromethyl)phenyl}acetamide (D490) 2-[2-chloro-3-(trifluoromethyl)phenyl]-N-{4-[1-(difluoromethyl)-1H-pyrazol-4-yl]-3-sulfamoylphenyl}acetamide
(D491) 2-(2-chlorophenyl)-N-{4-[1-(difluoromethyl)-1H-pyrazol-4-yl]-5-sulfamoyl-2-(trifluoromethyl)phenyl}acetamide
(D492) 2-(2-chlorophenyl)-N-{4-[1-(difluoromethyl)-5-(trifluoromethyl)-1H-pyrazol-4-yl]-3-sulfamoylphenyl}acetamide
(D493) 2-(2-chlorophenyl)-N-{4-[1-(difluoromethyl)-1H-pyrazol-4-yl]-2-fluoro-3-sulfamoylphenyl}acetamide
(D494) 2-(2-chlorophenyl)-N-{4-[1-(difluoromethyl)-1H-pyrazol-4-yl]-2-fluoro-5-sulfamoylphenyl}acetamide
(D495) 2-(2-chlorophenyl)-N-{4-[2-(dimethylamino)-1,3-thiazol-4-yl]-3-sulfamoylphenyl}acetamide
(D496) 2-(2-chlorophenyl)-N-[3-sulfamoyl-4-(1,2-thiazol-5-yl)phenyl]acetamide
(D497) 1-(4-{[(2-chlorophenyl)acetyl]amino}-2-sulfamoylphenyl)-N-cyclopropyl-N-methyl-1H-pyrazol-4-carboxamide
(D498) 2-(2-chlorophenyl)-2-hydroxy-N-{3-sulfamoyl-4-[4-(trifluoromethyl)-1H-pyrazol-1-yl]phenyl}ethanamide
(D499) 2-(2-chlorophenyl)-N-{3-chloro-5-sulfamoyl-4-[4-(trifluoromethyl)-1H-pyrazol-1-yl]phenyl}acetamide
(D500) 2-(2-chlorophenyl)-N-[4-(4-cyano-3-hydroxy-1H-pyrazol-1-yl)-3-sulfamoylphenyl]acetamide
(D501) 2-(2-chlorophenyl)-N-[4-(1H-pyrazolo[4,3-c]pyridin-1-yl)-3-sulfamoylphenyl]acetamide
(D502) 2-(2-chlorophenyl)-N-[4-(4,5-dimethyl-1,3-thiazol-2-yl)-3-sulfamoylphenyl]acetamide
(D503) 2-(2-chlorophenyl)-N-[4-(2,4-dimethyl-1,3-thiazol-5-yl)-3-sulfamoylphenyl]acetamide
(D504) 2-(2-chlorophenyl)-N-[4-(4-methyl-1,3-thiazol-5-yl)-3-sulfamoylphenyl]acetamide
(D505) N-{4-(4-amino-1H-pyrazol-1-yl)-3-[(2,4-dimethoxybenzyl)sulfamoyl]phenyl}-2-(2-chlorophenyl)acetamide
(D506) N-[1-(4-{[(2-chlorophenyl)acetyl]amino}-2-sulfamoylphenyl)-1H-pyrazol-4-yl]-2,2-difluoroacetamide
(D507) N-[1-(4-{[(2-chlorophenyl)acetyl]amino}-2-sulfamoylphenyl)-1H-pyrazol-4-yl]-3,3,3-trifluoropropanamide
(D508) N-[1-(4-{[(2-chlorophenyl)acetyl]amino}-2-sulfamoylphenyl)-1H-pyrazol-4-yl]-3,3,3-trifluoro-2-methylpropanamide (racemate)
(D509) N-[1-(4-{[(2-chlorophenyl)acetyl]amino}-2-sulfamoylphenyl)-1H-pyrazol-4-yl]-3,3,3-trifluoro-2-methylpropanamide (enantiomer A)
(D510) N-[1-(4-{[(2-chlorophenyl)acetyl]amino}-2-sulfamoylphenyl)-1H-pyrazol-4-yl]-3,3,3-trifluoro-2-methylpropanamide (enantiomer B)
(D511) 2-(2-chlorophenyl)-N-(4-{4-[(cis)-2,5-dimethylpyrrolidin-1-yl]-1H-pyrazol-1-yl}-3-sulfamoylphenyl)acetamide
(D512) 2-(2-chlorophenyl)-N-(4-{4-[(trans)-2,5-dimethylpyrrolidin-1-yl]-1H-pyrazol-1-yl}-3-sulfamoylphenyl)acetamide (enantiomer A)
(D513) 2-(2-chlorophenyl)-N-(4-{4-[(trans)-2,5-dimethylpyrrolidin-1-yl]-1H-pyrazol-1-yl}-3-sulfamoylphenyl)acetamide (enantiomer B)
(D514) N-(4-{4-[(2,2-difluoroethyl)amino]-1H-pyrazol-1-yl}-3-sulfamoylphenyl)-2-(2-fluorophenyl)acetamide
(D515) 2-(2-chlorophenyl)-N-(3-sulfamoyl-4-{4-[(2,2,2-trifluoroethyl)amino]-1H-pyrazol-1-yl}phenyl)acetamide
(D516) 2-(2-chlorophenyl)-N-[4-(4-isopropyl-1H-pyrazol-1-yl)-3-sulfamoylphenyl]acetamide
(D517) 2-(2-fluorophenyl)-N-[4-(4-isopropyl-1H-pyrazol-1-yl)-3-sulfamoylphenyl]acetamide
(D518) 2-(2-chlorophenyl)-N-{3-sulfamoyl-4-[4-(2,2,2-trifluoroethyl)-1H-pyrazol-1-yl]phenyl}acetamide
(D519) 2-(2-fluorophenyl)-N-{3-sulfamoyl-4-[4-(2,2,2-trifluoroethyl)-1H-pyrazol-1-yl]phenyl}acetamide
(D520) 2-(2-chlorophenyl)-N-[4-(5-cyclopropyl-1,2,4-oxadiazol-3-yl)-3-sulfamoylphenyl]acetamide
(D521) 2-(2-chlorophenyl)-N-{3-sulfamoyl-4-[3-(trifluoromethyl)-1,2,4-oxadiazol-5-yl]phenyl}acetamide
(D522) 2-(2-chlorophenyl)-N-{4-[5-(difluoromethyl)-1,3,4-oxadiazol-2-yl]-3-sulfamoylphenyl}acetamide
(D523) N-{4-[5-(difluoromethyl)-1,3,4-oxadiazol-2-yl]-3-sulfamoylphenyl}-2-(2-fluorophenyl)acetamide
(D524) 2-(2-chlorophenyl)-N-[4-(5-methyl-1,3,4-oxadiazol-2-yl)-3-sulfamoylphenyl]acetamide
(D525) 2-(2-fluorophenyl)-N-[4-(5-methyl-1,3,4-oxadiazol-2-yl)-3-sulfamoylphenyl]acetamide
(D526) N-[4-(5-methyl-1,3,4-oxadiazol-2-yl)-3-sulfamoylphenyl]-2-(4-methylphenyl)acetamide
(D527) 2-(2-chlorophenyl)-N-[4-(1H-pyrrol-3-yl)-3-sulfamoylphenyl]acetamide
(D528) 2-(2-chlorophenyl)-N-{4-[5-(difluoroacetyl)-1H-pyrrol-3-yl]-3-sulfamoylphenyl}acetamide
(D529) 2-(2-chlorophenyl)-N-[4-(1-methyl-1H-pyrrol-3-yl)-3-sulfamoylphenyl]acetamide
(D530) 2-(2-chlorophenyl)-N-[4-(5-cyano-1-methyl-1H-pyrrol-2-yl)-3-sulfamoylphenyl]acetamide
(D531) 2-(2-chlorophenyl)-N-{3-sulfamoyl-2-(trifluoromethyl)-4-[4-(trifluoromethyl)-1H-pyrazol-1-yl]phenyl}acetamide
(D532) 2-(2-chlorophenyl)-N-{5-sulfamoyl-2-(trifluoromethyl)-4-[4-(trifluoromethyl)-1H-pyrazol-1-yl]phenyl}acetamide
(D533) 2-[2-chloro-3-(trifluoromethyl)phenyl]-N-{5-sulfamoyl-2-(trifluoromethyl)-4-[4-(trifluoromethyl)-1H-pyrazol-1-yl]phenyl}acetamide
(D534) 2-[2-chloro-5-(trifluoromethyl)phenyl]-N-{5-sulfamoyl-2-(trifluoromethyl)-4-[4-(trifluoromethyl)-1H-pyrazol-1-yl]phenyl}acetamide
(D535) 2-[2-chloro-6-(trifluoromethyl)phenyl]-N-[4-(4-fluoro-1H-pyrazol-1-yl)-5-sulfamoyl-2-(trifluoromethyl)phenyl]acetamide
(D536) 2-[2-chloro-3-(trifluoromethyl)phenyl]-N-[4-(4-fluoro-1H-pyrazol-1-yl)-5-sulfamoyl-2-(trifluoromethyl)phenyl]acetamide
(D537) 2-[2-chloro-5-(trifluoromethyl)phenyl]-N-[4-(4-fluoro-1H-pyrazol-1-yl)-5-sulfamoyl-2-(trifluoromethyl)phenyl]acetamide
(D538) 2-[2-chloro-4-(trifluoromethyl)phenyl]-N-[4-(4-fluoro-1H-pyrazol-1-yl)-5-sulfamoyl-2-(trifluoromethyl)phenyl]acetamide
(D539) N-[4-(3-tert-butyl-4-cyano-1H-pyrazol-1-yl)-3-sulfamoylphenyl]-2-(2-chlorophenyl)acetamide
(D540) N-[4-(3-bromo-4-cyano-1H-pyrazol-1-yl)-3-sulfamoylphenyl]-2-(2-chlorophenyl)acetamide
(D541) 2-(2-chlorophenyl)-N-{4-[4-chloro-3-(trifluoromethyl)-1H-pyrazol-1-yl]-3-sulfamoylphenyl}acetamide
(D542) 2-(2-chlorophenyl)-N-[4-(3,4-dimethyl-1H-pyrazol-1-yl)-3-sulfamoylphenyl]acetamide
(D543) N-[4-(4-chloro-3,5-dimethyl-1H-pyrazol-1-yl)-3-sulfamoylphenyl]-2-(2-chlorophenyl)acetamide
(D544) 2-(2-chlorophenyl)-N-[4-(1H-pyrazol-1-yl)-3-sulfamoylphenyl]acetamide
(D545) 2-(2-chlorophenyl)-N-[4-(3-cyano-5-methyl-1H-pyrazol-1-yl)-3-sulfamoylphenyl]acetamide
(D546) 2-(2-chlorophenyl)-N-[4-(3-hydroxy-5-methyl-1H-pyrazol-1-yl)-3-sulfamoylphenyl]acetamide
(D547) 2-(2-chlorophenyl)-N-[4-(4-cyano-5-methyl-1H-pyrazol-1-yl)-3-sulfamoylphenyl]acetamide
(D548) 2-(2-chlorophenyl)-N-[4-(4-cyano-3-methyl-1H-pyrazol-1-yl)-3-sulfamoylphenyl]acetamide
(D549) 2-(2-chlorophenyl)-N-{4-[4-(morpholin-4-yl)-1,3-thiazol-2-yl]-3-sulfamoylphenyl}acetamide
(D550) 2-(2-chlorophenyl)-N-{4-[5-(morpholin-4-yl)-1,3-thiazol-2-yl]-3-sulfamoylphenyl}acetamide
(D551) 2-(2-chlorophenyl)-N-[4-(5-methyl-1,3-thiazol-2-yl)-3-sulfamoylphenyl]acetamide
(D552) 2-(2-chlorophenyl)-N-[4-(pyridin-4-yl)-3-sulfamoylphenyl]acetamide
(D553) 1-(4-{[(2-chlorophenyl)acetyl]amino}-2-sulfamoylphenyl)-1H-pyrazol-4-carboxamide
(D554) 2-(2-chloro-3-hydroxyphenyl)-N-[4-(4-cyano-1H-pyrazol-1-yl)-3-sulfamoylphenyl]acetamide
(D555) 2-(2-chloro-4-hydroxyphenyl)-N-[4-(4-cyano-1H-pyrazol-1-yl)-3-sulfamoylphenyl]acetamide
(D556) 2-(2-chloro-5-hydroxyphenyl)-N-[4-(4-cyano-1H-pyrazol-1-yl)-3-sulfamoylphenyl]acetamide
(D557) 2-(2-chloro-6-hydroxyphenyl)-N-[4-(4-cyano-1H-pyrazol-1-yl)-3-sulfamoylphenyl]acetamide
(D558) 2-(2-chlorophenyl)-N-[4-(4-cyano-1H-pyrazol-1-yl)-3-sulfamoylphenyl]-2-hydroxyacetamide

Stereoisomers of the compounds represented by the general formulas (A) to (D), such as a cis-trans isomer, an optically active form, or a racemic form thereof, may exist as well, all of which are included in the present invention.

Tautomers of the compounds represented by the general formulas (A) to (D) may exist as well, and the tautomers show the same activities as those of the compounds and are included in the present invention.

Furthermore, the compounds represented by the general formulas (A) to (D) may have one or two or more asymmetric carbons depending on the type of substituent, and any optical isomer or any mixture or a racemic form of optical isomers, which is based on the asymmetric carbon, or a diastereoisomer or any mixture of diastereoisomers, which is based on two or more asymmetric carbons, may also be used as the active ingredient of the pharmaceutical composition of the present invention. In a case where the compounds represented by the general formulas (A) to (D) have a double bond or a cyclic structure, a geometric isomer may exist, and in addition to a pure form of the geometric isomer, a mixture of geometric isomers, which are contained in the mixture at any ratio, may also be used as the active ingredient of the pharmaceutical composition of the present invention.

In addition to the compounds represented by the general formulas (A) to (D), any solvate of a free form of the compound or a salt form of the compound may also be used as active ingredient of the pharmaceutical composition of the present invention. The solvate also includes a hydrate.

In the present specification, unless otherwise specified, the compounds represented by general formulas (A) to (D) can includes the stereoisomer of the compound, such as the cis-trans isomer, the optically active form, or the racemic form thereof; the tautomer of the compound; any optical isomer or racemic form of the compound, which is based on an asymmetric carbon; and the diastereoisomer of the compound and a mixture thereof, which is based on two or more asymmetric carbons.

In addition to the compounds represented by the general formulas (A) to (D), acid addition salts or base addition salts of these compounds may also be used as the active ingredient of the pharmaceutical composition of the present invention. As the acid addition salt, for example, a mineral acid salt such as hydrochloride, sulfate, and nitrate; an organic acid salt such as methanesulfonate, p-toluenesulfonate, oxalate, and malate; and the like can be used, however, the acid addition salt is not limited thereto. Examples of the base addition salt can include a metal salt such as a lithium salt, a sodium salt, a potassium salt, a magnesium salt, and a calcium salt; an ammonium salt; an organic amine salt such as a triethylamine salt and an ethanolamine salt; and the like, however, the base addition salt is not limited thereto. Among these salts, a pharmaceutically acceptable salt is preferably used as the active ingredient of the pharmaceutical composition of the present invention.

The pharmaceutical composition of the present invention can be administered orally or parenterally. The pharmaceutical composition of the present invention can be produced in a suitable dosage form such as a tablet, granules, a powder, a capsule, a suspension, an inhalation, a dry powder inhaler, an inhalation liquid, an aerosol inhaler, an ointment, a gel, a cream, a poultice, a patch, a liniment, a tape, a plaster, an injection, and a suppository, according to a general method in the technical field of preparations. In one embodiment, the "pharmaceutical composition" comprises the active ingredient and a pharmaceutically acceptable additive.

These pharmaceutical preparations can be manufactured using a general technique. For example, in a case of the tablet, a general pharmaceutically acceptable additive such as a diluent, a disintegrant, a binder, a lubricant, and a coloring agent can be used. Examples of the diluent can include lactose, D-mannitol, microcrystalline cellulose, glucose, and the like. Examples of the disintegrant can include starch, carboxymethylcellulose calcium (CMC-Ca), and the like. Examples of the lubricant can include magnesium stearate, talc, and the like. Examples of the binder can include hydroxypropyl cellulose (HPC), gelatin, polyvinyl pyrrolidone (PVP), and the like.

A pharmaceutically acceptable additive such as a solvent, a stabilizer, a solubilizing agent, a suspension, an emulsifier, an analgesic agent, a buffer, and a preservative is used in preparation of the injection. The pharmaceutically acceptable additive and a method of preparing a pharmaceutical preparation can be suitably selected by those skilled in the art.

In other words, the pharmaceutical composition of the present invention can be provided as a pharmaceutical composition comprising the active ingredient and the pharmaceutically acceptable additive.

Types of the inhalation for the parenteral administration include an aerosol, a dry powder for inhalation, a liquid for inhalation (for example, a solution for inhalation, a suspension for inhalation, and the like), and a capsule inhalation, and the liquid for inhalation may be in a form that is used by being dissolved or suspended in water or another suitable medium at the time of use. The inhalation can be applied using a suitable inhalation container. For example, when administering the liquid for inhalation, a sprayer (an atomizer or a nebulizer) or the like can be used, and when administering the dry powder for inhalation, an inhaler for a dry powder drug or the like can be used.

The inhalation can be manufactured according to a known method. For example, the inhalation is manufactured by obtaining a dry powder or a liquid of the compounds represented by the general formulas (A) to (D), blending the dry powder or the liquid of the compounds into an inhalation propellant or a carrier, and filling a suitable inhalation container with the blended product. In a case of powderizing the compounds represented by the general formulas (A) to (D), the powderization is performed according to a general method. For example, a dry powder is prepared by obtaining a fine powder of the compounds and lactose, starch, magnesium stearate, or the like, thereby obtaining a homogeneous mixture, or by granulating the compounds. In addition, in a case where the compounds represented by the general formulas (A) to (D) are liquefied, for example, the compounds may be dissolved in a liquid carrier such as water, physiological saline, and an organic solvent. As the propellant, a conventionally known propellant, for example, alternative chlorofluorocarbon, a liquefied gas propellant (for example, fluorohydrocarbon, liquefied petroleum, diethyl ether, dimethyl ether, and the like), a compressed gas (for example, a soluble gas (for example, carbon dioxide gas, nitrous oxide gas, and the like), an insoluble gas (for example, nitrogen gas and the like), and the like are used.

A pharmaceutically acceptable additive may be suitably blended into the inhalation as necessary. The additive may be any additive that is generally used. For example, a solid diluent (for example, sucrose, lactose, glucose, mannitol, sorbitol, maltose, cellulose, and the like), a liquid diluent (for example, propylene glycol and the like), a binder (starch, dextrin, methylcellulose, hydroxypropylcellulose, polyvinylpyrrolidone, polyethylene glycol, sucrose, and the like), a lubricant (for example, magnesium stearate, light anhydrous silicic acid, talc, sodium lauryl sulfate, and the like), a corrigent (for example, citric acid, menthol, glycyrrhizin ammonium salt, glycine, an orange powder, and the like), a preservative (for example, sodium benzoate, sodium bisulfite, methylparaben, propylparaben, and the like), a stabilizer (for example, citric acid, sodium citrate, and the like), a suspending agent or an emulsifier (for example, methyl cellulose, polyvinyl pyrrolidone, polyvinyl alcohol, lecithin, sorbitan trioleate, and the like), a dispersing agent (for example, a surfactant and the like), a solvent (for example, water and the like), a tonicity agent (for example, sodium chloride, concentrated glycerin, and the like), a pH regulator (for example, hydrochloric acid, sulfuric acid, and the like), a solubilizer (for example, ethanol and the like), an antiseptic agent (benzalkonium chloride, paraben, and the like), a colorant, a buffer agent (sodium phosphate, sodium acetate, and the like), a thickening agent (cariboxyvinyl polymer and the like), an absorption promoter, and the like are used. The liquid for inhalation is prepared by, for example, suitably selecting the antiseptic agent, the colorant, the buffer agent, the tonicity agent, the thickening agent, the absorption promoter, or the like as necessary. Furthermore, the dry powder for inhalation is prepared by, for example, suitably selecting the lubricant, the binder, the diluent, the colorant, the antiseptic agent, the absorption promoter (bile salt, chitosan, and the like), or the like as necessary.

Furthermore, in order to prepare the compounds represented by the general formulas (A) to (D) in a sustained release form, the inhalation may contain a biodegradable polymer. Examples of the biodegradable polymer can include a fatty acid ester polymer or a copolymer thereof, polyacrylic acid esters, polyhydroxybutyric acids, polyalkylene oxalates, polyorthoester, polycarbonate, and polyamino acids, and one polymer can be used alone, or two or more thereof can be used in combination. Furthermore, a phospholipid such as egg yolk lecithin, chitosan, or the like may also be used. Examples of the fatty acid ester polymer or a copolymer thereof can include polylactic acid, polyglycolic acid, polycitric acid, polymalic acid, and a lactic acid-glycolic acid copolymer, and one polymer or copolymer can be used alone, or two or more thereof can be used in combination. In addition, one of poly-α-cyanoacrylic acid ester, poly-β-hydroxybutyric acid, polytrimethylene oxide, polyorthoester, polyorthocarbonate, polyethylenecarbonate, poly-γ-benzyl-L-glutamic acid, and poly-L-alanine can be used alone, or two or more thereof can be used in combination. The fatty acid ester polymer or a copolymer thereof is preferably polylactic acid, polyglycolic acid, or a lactic acid-glycolic acid copolymer, and is more preferably a lactic acid-glycolic acid copolymer. Furthermore, a microsphere or a nanosphere may be prepared by encapsulating a drug using the biodegradable polymer such as a lactic acid-glycolic acid copolymer.

An ointment is produced by known or generally used formulation. For example, the ointment is produced and prepared by triturating or melting one or more active substances in a base. The ointment base is selected from known or generally used bases. For example, a single base or a mixture of two or more bases selected from a higher fatty acid or a higher fatty acid ester (adipic acid, myristic acid, palmitic acid, stearic acid, oleic acid, adipic acid ester, myristic acid ester, palmitic acid ester, stearic acid ester, oleic acid ester, or the like), waxes (beeswax, spermaceti, ceresin, or the like), a surfactant (polyoxyethylene alkyl ether phosphoric acid ester or the like), a higher alcohol (cetanol, stearyl alcohol, cetostearyl alcohol, or the like), silicone oil (dimethylpolysiloxane or the like), hydrocarbons (hydrophilic petrolatum, white petrolatum, purified lanolin, liquid paraffin, or the like), glycols (ethylene glycol, diethylene glycol, propylene glycol, polyethylene glycol, macrogol, or the like), plant oil (castor oil, olive oil, sesame oil, turpentine oil, or the like), animal oil (mink oil, egg-yolk oil, squalane, squalene, or the like), water, an absorption promoter, and an irritation preventing agent is used. The ointment may further contain a moisturizing agent, a preservative, a stabilizing agent, an antioxidant, a fragrance, or the like.

A gel is produced by known or generally used formulation. For example, the gel is produced and prepared by melting one or more active substances in a base. The gel base is selected from known or generally used bases. For example, a single base or a mixture of two or more bases selected from a lower alcohol (ethanol, isopropyl alcohol, or the like), a gelling agent (carboxymethyl cellulose, hydroxyethyl cellulose, hydroxypropyl cellulose, ethyl cellulose, or the like), a neutralizing agent (triethanolamine, diisopropanolamine, or the like), a surfactant (polyethylene glycol monostearate or the like), gums, water, an absorption promoter, and an irritation preventing agent is used. The gel may further contain a preservative, an antioxidant, a fragrance, or the like.

A cream is produced by known or generally used formulation. For example, the cream is produced and prepared by melting or emulsifying one or more active substances in a base. The cream base is selected from known or generally used bases. For example, a single base or a mixture of two or more bases selected from a higher fatty acid ester, a lower alcohol, hydrocarbons, polyhydric alcohol (propylene glycol, 1,3-butylene glycol, or the like), a higher alcohol (2-hexyldecanol, cetanol, or the like), an emulsifying agent (polyoxyethylene alkyl ethers, fatty acid esters, or the like), water, an absorption promoter, and an irritation preventing agent is used. The cream may further contain a preservative, an antioxidant, a fragrance, or the like.

A poultice is produced by known or generally used formulation. For example, the poultice is produced by melting one or more active substances in a base, forming a kneaded product, and spreading and coating the kneaded product onto a support. The poultice base is selected from known or generally used bases. For example, a single base or a mixture of two or more bases selected from a thickening agent (polyacrylic acid, polyvinylpyrrolidone, gum arabic, starch, gelatin, methylcellulose, or the like), a wetting agent (urea, glycerin, propylene glycol, or the like), a filler (kaolin, zinc oxide, talc, calcium, magnesium, or the like), water, a solubilizing agent, a tackifier, and an irritation preventing agent is used. The poultice may further contain a preservative, an antioxidant, a fragrance, or the like.

A patch is produced by known or generally used formulation. For example, the patch is produced by melting one or more active substances in a base and spreading and coating the base containing the active substances onto a support. The base for the patch is selected from known or generally used bases. For example, a single base or a mixture of two or more bases selected from a polymer base, a fat, a higher fatty acid, a tackifier, and an irritation preventing agent is used. The patch may further contain a preservative, an antioxidant, a fragrance, or the like.

A liniment is produced by known or generally used formulation. For example, the liniment is produced and prepared by dissolving, suspending, or emulsifying one or more active substances in one or two or more selected from water, an alcohol (ethanol, polyethylene glycol, or the like), a higher fatty acid, glycerin, a soap, an emulsifying agent, a suspending agent, and the like. The liniment may further contain a preservative, an antioxidant, a fragrance, or the like.

The dose of the pharmaceutical composition of the present invention is not particularly limited, and generally, to an adult, approximately 0.01 µg to 100 mg and preferably 0.3 µg to 10 mg can be administered per day as an active ingredient amount in the case of administration by an inhalation, a dry powder inhaler, an inhalation liquid, or an aerosol inhaler, approximately 0.01mg to 1,000 mg can be administered per day as an active ingredient amount in the case of administration by an ointment, a gel, a cream, a poultice, a patch, a liniment, a tape, or a plaster, approximately 0.01mg to 100 mg can be administered per day as an active ingredient amount in the case of administration by an injection, and approximately 0.01mg to 2,000 mg can be administered per day in the case of oral administration. Note that the dose is not limited to the above doses and is suitably determined in accordance with the administration method, the age, body weight, sex, and symptom of the administration subject, and sensitivity to a drug. The dose may be adjusted according to the improvement of the symptom and can be increased or decreased according to the age, the symptom, and the like.

The pharmaceutical composition of the present invention can be used in combination with another drug that is useful in treating or preventing various symptoms associated with a pseudo-allergic reaction. Individual components in such combination can be administered in divided preparations or in a single preparation separately at different times or at the same time, during the period of the treatment or prevention. Therefore, it should be understood that the present invention includes both administration at the same time or administrations at different times, and the administration in the present invention should be understood in such a way. The range of the combination of the pharmaceutical composition of the present invention with another drug that is useful in treating or preventing the various symptoms associated with a pseudo-allergic reaction includes, in principle, a combination with a certain pharmaceutical preparation that is useful in treating or preventing the various symptoms associated with a pseudo-allergic reaction.

Various forms can be selected for each preparation in the combined preparation according to the present invention, and each preparation can be produced in the same manner as the above-described preparations. Furthermore, a drug combination which includes the pharmaceutical composition of the present invention and another drug for treating or preventing various symptoms associated with a pseudo-allergic reaction can also be easily produced by those skilled in the art according to a conventional method or a conventional art.

The combination includes not only a combination of the pharmaceutical composition of the present invention with one other active substance, but also a combination of the pharmaceutical composition of the present invention with two or more other active substances. There are many examples of the combination of the pharmaceutical composition of the present invention with one or two or more active substances selected from the drugs for treating or preventing the various symptoms associated with a pseudo-allergic reaction.

Examples of the drug that is combined with the pharmaceutical composition of the present invention include a steroid, a β2 agonist, muscarinic receptor antagonist, an antihistamine, an antiallergic drug, a bronchodilator, a leukotriene synthesis inhibitor, prostaglandins, a leukotriene receptor antagonist, an antipruritic agent, and other antitussive drugs and expectorants. Among these drugs, a combination with an antihistamine or an antiallergic drug is preferred. It is also possible to combine the pharmaceutical composition of the present invention with a Chinese herbal medicine.

Examples of the steroid include a topical agent such as clobetasol propionate, diflorasone diacetate, fluocinonide, mometasone furoate, betamethasone dipropionate, betamethasone butyrate propionate, betamethasone valerate, difluprednate, pudesonide, diflucortolone valerate, amcinonide, halcinonide, dexamethasone, dexamethasone propionate, dexamethasone valerate, dexamethasone acetate, hydrocortisone acetate, hydrocortisone butyrate, hydrocortisone butyrate propionate, deprodone propionate, prednisolone valerate acetate, fluocinolone acetonide, beclomethasone dipropionate, triamcinolone acetonide, flumethasone pivalate, alclometasone dipropionate, clobetasone butyrate, prednisolone, peclomethasone dipropionate, and fludroxycortide; an orally administered drug or an injection such as cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone diacetate, triamcinolone acetonide, dexamethasone, dexamethasone acetate, dexamethasone sodium phosphate, dexamethasone palmitate, paramethasone acetate, and betamethasone; and an inhalation such as beclomethasone dipropionate, fluticasone propionate, budesonide, flunisolide, triamcinolone, ST-126P, ciclesonide, dexamethasone palomitionate, mometasone furancarbonate, prasterone sulfonate, deflazacort, methylprednisolone suleptanate, and methylprednisolone sodium succinate.

Examples of the β2 agonist include formoterol, salmeterol, carmoterol, indacaterol, vilanterol, arformoterol, bambuterol, isoproterenol, milveterol, clenbuterol, olodaterol, fenoterol, salbutamol, levalbuterol, procaterol, terbutaline, pirbuterol, procaterol, metaproterenol, bitolterol, ritodrine, and albuterol.

Examples of the muscarinic receptor antagonist include tiotropium, ipratropium, flutropium, oxitropium, aclidinium, darotropium, glycopyrrolate, and umeclidinium.

Examples of the antihistamine include diphenhydramine, diphenylpyraline hydrochloride, diphenylpyraline teoclate, clemastine fumarate, dimenhydrinate, dl-chlorpheniramine maleate, d-chlorpheniramine maleate, triprolidine hydrochloride, promethazine hydrochloride, alimemazine tartrate, isothipendyl hydrochloride, homochlorcyclizine hydrochloride, hydroxyzine, cyproheptadine hydrochloride, levocabastine hydrochloride, astemizole, bepotastine, desloratadine, TAK-427, ZCR-2060, NIP-530, mometasone furoate, mizolastine, BP-294, andolast, auranofin, and acrivastine.

Examples of the antiallergic drug include a chemical mediator release inhibitor such as sodium cromoglicate, tranilast, amlexanox, repirinast, ibudilast, pemirolast potassium, dazanolast, nedocromil, cromoglicate, and israpafant; a histamine antagonist such as ketotifen fumarate, azelastine hydrochloride, oxatomide, mequitazine, terfenadine, emedastine difumarate, epinastine hydrochloride, ebastine, cetirizine hydrochloride, olopatadine hydrochloride, loratadin, and fexofenadine; a thromboxane synthase inhibitor such as ozagrel hydrochloride and imitrodast sodium; a thromboxane antagonist such as seratrodast, ramatroban, domitroban calcium hydrate, and KT-2-962; and a Th2 cytokine inhibitor such as suplatast tosilate.

Examples of the bronchodilator include a xanthine derivative such as aminophylline, theophylline, doxofylline, cipamfylline, diprophylline, proxyphylline, and choline theophylline; a sympathomimetic agent such as epinephrine, ephedrine hydrochloride, dl-methylephedrine hydrochloride, methoxyphenamine hydrochloride, isoproterenol sulfate, isoproterenol hydrochloride, orciprenaline sulfate, clorprenaline hydrochloride, trimetoquinol hydrochloride, salbutamol sulfate, terbutaline sulfate, hexoprenaline sulfate, tulobuterol hydrochloride, procaterol hydrochloride, fenoterol hydrobromide, formoterol fumarate, clenbuterol hydrochloride, mabuterol hydrochloride, salmeterol xinafoate, R,R-formoterol, tulobuterol, pirbuterol hydrochloride, ritodrine hydrochloride, bambuterol, dopexamine hydrochloride, meluadrine tartrate, ARC68397, levosalbutamol, KUR-1246, KUL-7211, AR-C89855, and S-1319; and a parasympatholytic drug such as ipratropium bromide, flutropium bromide, oxitropium bromide, cimetropium bromide, temiverine, tiotropium bromide, and revatropate.

Examples of the leukotriene synthesis inhibitor include auranofin, proglumetacin maleate, L-674636, A-81834, UPA-780, A-93178, MK-886, REV-5901A, SCH-40120, MK-591, Bay-x-1005, Bay-y-1015, DTI-0026, amlexanox, and E-6700.

Examples of the prostaglandins include a PGE2EP1 receptor, PGE2EP2 receptor, PGE2EP3 receptor, or PGE2EP4 receptor agonist or antagonist; a PGD2 receptor or CRTH2 receptor agonist or antagonist; a PGFFP receptor agonist or antagonist; a PGIIP receptor agonist or antagonist; and a TX receptor agonist and antagonist.

Examples of the leukotriene receptor antagonist include pranlukast hydrate, montelukast, zafirlukast, seratrodast, MCC-847, KCA-757, CS-615, YM-158, L-740515, CP-195494, LM-1484, RS-635, A-93178, S-36496, BIIL-284, and ONO-4057.

Examples of other antitussive drugs include codeine phosphate, dihydrocodeine phosphate, oxymethebanol, dextromethorphan hydrobromide, pentoxyverine citrate, dimemorfan phosphate, oxeladin citrate, cloperastine, benproperine phosphate, clofedanol hydrochloride, fominoben hydrochloride, noscapine, tipemidine hibenzate, eprazinone hydrochloride, and a Plantago herb extract.

Examples of the expectorant include foeniculated ammonia spirit, sodium bicarbonate, potassium iodide, bromhexine hydrochloride, a cherry bark extract, carbocysteine, fudosteine, ambroxol hydrochloride, an ambroxol hydrochloride sustained release preparation, methylcysteine hydrochloride, acetylcysteine, ethyl L-cysteine hydrochloride, and tyloxapol.

Examples of the antipruritic agent include diphenhydramine hydrochloride, crotamiton, and lidocaine.

As another aspect, the present invention is a method of preventing, suppressing, or treating a symptom associated with a pseudo-allergic reaction, the method including administering a compound having a P2X4 receptor antagonizing action (for example, the compounds represented by the general formulas (A) to (D), or a pharmaceutically acceptable salt thereof, to a subject in need of the method (for example, mammals including human) at a dose effective for preventing, suppressing, or treating the symptom associated with the pseudo-allergic reaction. The pseudo-allergic reaction may be caused by an allergen having a cationic group, and the allergen may be an MrgprX2 agonist.

As still another aspect, the present invention is a compound having a P2X4 receptor antagonizing action (for example, the compounds represented by the general formulas (A) to (D)), or a pharmaceutically acceptable salt thereof, which is for use in prevention, suppression, or treatment of a symptom associated with a pseudo-allergic reaction. The pseudo-allergic reaction may be caused by an allergen having a cationic group, and the allergen may be an MrgprX2 agonist.

As still another aspect, the present invention is use of a compound having a P2X4 receptor antagonizing action (for example, the compounds represented by the general formulas (A) to (D)), or a pharmaceutically acceptable salt thereof, for producing a pharmaceutical composition for preventing, suppressing, or treating a symptom associated with a pseudo-allergic reaction. The pseudo-allergic reaction may be caused by an allergen having a cationic group, and the allergen may be an MrgprX2 agonist.

### Examples

Hereinafter, the present invention is more specifically described with Examples, however, the scope of the present invention is not limited to the following Examples. In addition, all of the literature referred to in the present specification is incorporated herein by reference in their entirety.

In the present examples, the following animals were used.

7- to 10-week-old C57BL/6 mice were purchased from Japan SLC, Inc. Mast cell-deficient KitW-sh/W-sh mice (RBRC01888) were obtained from RIKEN BioResource Research Center. P2X4 receptor deficiency (P2rx4^{-/-}) (Yamamoto K. et al., Nat Med 2006,12:133) was generated on a C57BL/6 background. These animals were kept under specific pathogen-removing conditions in the animal facility of Takasaki University of Health and Welfare. All experiments were performed according to the regulations of the Animal Research Committee of Takasaki University of Health and Welfare.

### Example 1 When the P2X4 receptor is deficient, pseudo-allergy is reduced.

Administration of C48/80 to mice causes a pseudo-allergic reaction via Mrgprb2-mediated mast cell activation (Meixiong J. et al. Immunity 2019, 50:1163). Therefore, the role of the P2X4 receptor in the pseudo-allergic reaction was investigated using P2X4 receptor knockout mice.

### 1-1) Passive Systemic Anaphylaxis

Wild-type C57BL/6 mice (9-week-old, male; n = 8) and P2X4 receptor-deficient mice (10-week-old, male; n = 7) were intravenously administered with 100 uL of physiological saline containing 50 ug of C48/80 (manufactured by Sigma-Aldrich, Inc.). Thereafter, the rectal temperatures of the mice were measured with a digital thermometer (manufactured by Physitemp Instruments, LLC.) every 5 minutes for a period of 60 minutes.

There was no difference in rectal temperature between the wild-type mice and the P2X4 receptor-deficient mice. In the wild-type mice, a pseudo-allergic reaction in which the rectal temperature was rapidly reduced by intravenous injection of C48/80 and then was slowly recovered was observed. In the P2X4 receptor-deficient mice, the decrease in rectal temperature was much smaller and recovered quickly compared to the wild-type mice (Fig. 1(A)). A difference between two groups was significant (Student's t-test: p < 0.01) from 15 minutes to 60 minutes after administration. From this, it was considered that the symptom of the pseudo-allergy can be reduced by inhibiting the function of the P2X4 receptor.

### 1-2) Passive Cutaneous Anaphylaxis

Wild-type C57BL/6 mice (10-week-old, male; n = 5) and P2X4 receptor-deficient mice (10-week-old, male; n = 5) were anesthetized with isoflurane (manufactured by FUJIFILM Wako Pure Chemical Corporation), 200 µL of a 0.5% Evans Blue (manufactured by FUJIFILM Wako Pure Chemical Corporation) solution diluted with PBS was intravenously injected, a physiological saline solution of C48/80 (100 ng/20 µL) (manufactured by Sigma-Aldrich, Inc.) was intradermally injected into the right ear, and a vehicle (0.1% DMSO (manufactured by FUJIFILM Wako Pure Chemical Corporation) physiological saline solution) was injected into the left ear. After 30 minutes, the mice were sacrificed and auricles were collected and weighed. The collected auricles were chopped into small pieces, and Evans Blue was extracted by heating (55°C) the chopped auricles in formamide (1 mL) for 24 hours. Absorbance (620 nm) of the extracted Evans Blue and the calibration curve was measured using the absorbance microplate reader Sunrise (Tecan Trading AG), and the concentrations (Evans Blue amount (µg)/auricles (mg)) were calculated.

Intradermal injection of C48/80 into the auricles of the wild-type mice caused significant Evans Blue extravasation compared to the vehicle injected auricles, and a pseudo-allergic reaction was observed. Evans Blue extravasation of control ears treated with the vehicle was slightly weaker but not significant in the P2X4 receptor-deficient mice than in the wild-type mice. On the other hand, Evans Blue extravasation induced by C48/80 was significantly lower in the P2X4 receptor-deficient mice (Fig. 1(B)) (Student's t-test: p < 0.05). From this, it was considered that the symptom of the pseudo-allergy can be reduced by inhibiting the function of the P2X4 receptor.

### 1-3) Involvement of Mast Cell

It is considered that C48/80 binds to Mrgprb2 expressed in mouse mast cells to cause a pseudo-allergic reaction. Therefore, whether or not the reduction of the pseudo-allergy in the P2X4 receptor-deficient mice was caused by the deficiency of the P2X4 receptor in mast cells was examined using mouse Kit^{W-sh/W-sh} deficient in mast cells.

Bone marrow-derived mast cells (BMMCs) were prepared from the wild-type C57BL/6 mice and the P2X4 receptor-deficient mice according to a conventional method (Patent Literature 4). Bone marrow was collected from the femur of male mice (7- to 8-week-old). Red blood cells were hemolyzed, and culture was performed in RPMI1640 medium containing 10% fetal bovine serum (FBS), penicillin (100 unit/ml), streptomycin (100 µg/ml), and recombinant mouse IL-3 (10 ng/ml, manufactured by PeproTech, Inc.). After 2 weeks, stem cell factor (SCF) (10 ng/ml, manufactured by PeproTech, Inc.) was added to the medium, and the culture was performed for 4 to 6 weeks to be used for the experiment. 95% or more of the prepared BMMCs were double positive for c-KIT and FcεRI (FACSCant II, manufactured by BD Biosciences).

Mast cell-deficient mice (male, 5-week-old) were intravenously administered with 5 × 10⁶ wild-type mouse-derived BMMCs or P2X4 receptor-deficient mouse-derived BMMCs and reared for 16 weeks to reconstitute mast cells. When a passive systemic anaphylaxis test was performed according to Example 1-1), the decrease in rectal temperature was significantly reduced in mice reconstituted with the P2X4 receptor-deficient mouse-derived BMMCs compared with mice reconstituted with the wild-type mouse-derived BMMCs (Fig. 2: Student's t-test: p < 0.05). From the above, it was considered that the symptom of the pseudo-allergy can be reduced by inhibiting the function of the P2X4 receptor with mast cells.

### Example 2 Analysis of Mast Cells Deficient in P2X4 Receptor

The P2X4 receptor antagonist inhibited degranulation associated with IgE receptor stimulation of BMMCs (Patent Literature 4). As a result of confirming degranulation of BMMCs to C48/80 stimulation, the response was significantly low. Peritoneal mast cells (PMCs) are considered to be more differentiated mast cells than BMMCs, and are sometimes classified as connective tissue-type mast cells with respect to mucosal mast cells of BMMCs. Therefore, the action of C48/80 on PMCs was confirmed.

### 2-1) Preparation of PMCs

The abdominal cavity of wild-type C57BL/6 mice (7- to 8-week-old, male; n = 4) or P2X4 receptor-deficient mice (7- to 8-week-old, male; n = 4) was washed with 4 mL of RPMI1640 medium to prepare peritoneal cells. The cells were cultured in RPMI1640 medium containing 10% FBS, penicillin (100 unit/ml), streptomycin (100 µg/ml), recombinant mouse IL-3 (10 ng/ml, manufactured by PeproTech, Inc.), and 10 ng/mL of SCF (manufactured by PeproTech, Inc.) for 14 days to be used for the experiment. 95% or more of the prepared PMCs were double positive for c-KIT and FcεRI (FACSCant II, manufactured by BD Biosciences).

### 2-2) Gene Expression of PMCs

Total RNA was prepared from PMCs (manufactured by MACHEREY-NAGEL GmbH & Co. KG: NucleoSpin RNA Kit). cDNA was synthesized using a 6-mer random primer and Moloney-murine leukemia virus reverse transcriptase (manufactured by Takara Bio Inc.), and quantitative PCR was performed using the following primers (manufactured by Takara Bio Inc.: TB Green Premix Ex TaqII). The expression level of each gene was corrected using the expression level of the GAPDH gene as a standard.

**[Table 29]**

| Gene name | Primer sequence (Forward (F), Reverse (R): 5'→3') | SEQ ID NO: |
|---|---|---|
| P2X1 | F: gaaggtggcatatgccaaga | SEQ ID NO: 4 |
| | R: tgtcatccacctctacagga | SEQ ID NO: 5 |
| P2X2 | F: acttcgtgtggtacgtcttc | SEQ ID NO: 6 |
| | R: tgatccccttgactttggtg | SEQ ID NO: 7 |
| P2X3 | F: ggattctgtccagagaatgagg | SEQ ID NO: 8 |
| | R: ggcttccatcatgataggca | SEQ ID NO: 9 |
| P2X4 | F: aacatgatcgtcaccgtgaa | SEQ ID NO: 10 |
| | R: aatggaacacaccttccagt | SEQ ID NO: 11 |
| P2X5 | F: gaaaactggtcgctgtctac | SEQ ID NO: 12 |
| | R: gtagagattggtggagctga | SEQ ID NO: 13 |
| P2X6 | F: ttgcaacctggacacgaa | SEQ ID NO: 14 |
| | R: tttctgctgcagctggaa | SEQ ID NO: 15 |
| P2X7 | F: taagctgtaccagcggaaag | SEQ ID NO: 16 |
| | R: tgcaaagggaaggtgtagtc | SEQ ID NO: 17 |
| P2Y1 | F: cagaccccagaaatgtgtga | SEQ ID NO: 18 |
| | R: ctcgtgtctccattctgctt | SEQ ID NO: 19 |
| P2Y2 | F: gcctgtgcatatgtgagtgaag | SEQ ID NO: 20 |
| | R: cacgtacttgaagtcctcgttg | SEQ ID NO: 21 |
| P2Y4 | F: gtcttctctgcctaggtgtt | SEQ ID NO: 22 |
| | R: ccagagtgatcaagaaggga | SEQ ID NO: 23 |
| P2Y6 | F: tgcctttccacatcacca | SEQ ID NO: 24 |
| | R: gagcctctgtaagagatcgt | SEQ ID NO: 25 |
| P2Y 12 | F: tcagccaataccaccttctc | SEQ ID NO: 26 |
| | R: tcctcattgccaagctgt | SEQ ID NO: 27 |
| P2Y13 | F: ccatgtgtgagatggggaaa | SEQ ID NO: 28 |
| | R: gctagggtgatgttgtctgt | SEQ ID NO: 29 |
| P2Y14 | F**:** tccagccgcaatatcttcag | SEQ ID NO: 30 |
| | R: ataatggggtccagacacac | SEQ ID NO: 31 |
| MrgprB2 | F: acgtccaagacacacatcag | SEQ ID NO: 32 |
| | R: tagacccacagagaacacca | SEQ ID NO: 33 |
| GAPDH | F: tgctgagtatgtcgtggagt | SEQ ID NO: 34 |
| | R: catacttggcaggtttctcc | SEQ ID NO: 35 |

As shown in Fig. 3, PMCs derived from the wild-type mice expressed the P2X4 receptor gene, but the expression of the gene in PMCs derived from the P2X4 receptor-deficient mice was significantly lower than that in the wild-type mice, and was not substantially detected (n = 4, p < 0.01 in Sudent's-t test). In addition, the expression of MrgprB2 was not affected by the deficiency of the P2X4 receptor gene. When the gene expression of each subtype of the P2 receptor family (P2X receptor and P2Y receptor) having a purine nucleotide as an endogenous ligand like the P2X4 receptor was examined, PMCs expressed P2X1, P2X4, P2X7, P2Y1, and P2Y14, and the expression other than the P2X4 receptor was not affected by the deficiency of the P2X4 receptor gene. From these results, it was suggested that the reduction of pseudo-allergy due to P2X4 receptor deficiency was not due to the decrease in expression of MrgprB2.

2-3) ATP Promotes Degranulation of PMCs Associated with Pseudo-allergic Reaction via P2X4 Receptor.

The influence of P2X4 gene deficiency on degranulation of PMCs by various pseudo-allergic substances stimulating MrgprB2 was examined.

Anti-DNP-IgE antibody (clone: SPE-7, manufactured by Sigma-Aldrich, Inc.) was added to a culture solution containing PMCs derived from wild-type C57BL/6 mice or P2X4 receptor-deficient mice so that the concentration reached 50 ng/ml, and the PMCs were sensitized overnight. The PMCs were washed with PBS(-) and suspended in Krebs Ringer-HEPES Buffer containing 0.1% BSA (NaCl 130 mM, KCl 4.7 mM, NaHCO₃ 4.0 mM, KH₂P₄ 1.2 mM, Glucose 11.5 mM, HEPES 10 mM, and CaCl₂-H₂O 1.8 mM) so that the concentration reached 1 × 10⁶ cell/mL. Next, to the PMCs sensitized with IgE,
(1) 2,4-dinitrophenyl-conjugated human serum albumin (DNP-HSA) (manufactured by Sigma-Aldrich, Inc.) having a final concentration of 1 ng/mL,
(2) DNP-HSA at a final concentration of 10 ng/mL and 100 uM ATP,
(3) C48/80 at a final concentration of 10 uM, or
(4) C48/80 at a final concentration of 10 uM and 100 uM ATP were added, and incubation was performed at 37°C for 10 minutes to degranulate. Thereafter, the mixture was rapidly cooled on ice and centrifuged to stop the reaction. After collecting the supernatant, precipitated PMCs were lysed with 0.1 % triton X-100. The supernatant and the cell lysis solution (50 µL) were mixed with 10 mM 4-Nitrophenyl N-acetyl-β-D-glucosaminide (manufactured by Sigma-Aldrich, Inc.) which was a β-hexosaminidase substrate/50 mM citrate buffer (pH 4.5, 50 µL), and the mixture was incubated at 37°C for 30 minutes. 50 mM carbonate buffer (pH 10.0, 100 µL) was added to stop the enzyme reaction of β-hexosaminidase, and absorbance was measured at 405/655 nm using an absorbance microplate reader Sunrise (manufactured by Tecan Trading AG). The β-hexosaminidase release rate was obtained from the following formula.

### β-hexosaminidase release rate (%) =

Absorbance of supernatant/(Absorbance of supernatant + absorbance of cell lysis station) × 100

Similar to the degranulation of BMMCs reported in Non Patent Literature 4, the degranulation of PMCs was induced by IgE receptor stimulation by DNP-HSA, and the coexistence of ATP significantly promoted the degranulation (Fig. 4(A), Student's t-test: p < 0.01). C48/80 inducing pseudo-allergy also induced degranulation of PMCs, and degranulation was significantly promoted by ATP (Fig. 4(B), Student's t-test: p < 0.01). On the other hand, the effect of promoting degranulation of ATP was not observed in PMCs derived from P2X4 receptor-deficient mice, and it was found that ATP promoted degranulation of PMCs by IgE receptor stimulation or MrgPrB2 stimulation via the P2X4 receptor (Figs. 4(A) and 4(B)).

Note that, unlike ATP, α,β-methyl mATP (manufactured by Sigma-Aldorich, Inc., final concentration: 10 uM) as a P2X1 receptor agonist, ADP (manufactured by Sigma-Aldorich, Inc., final concentration: 100 uM) as a P2Y1 receptor agonist, or UDP-Glucose (manufactured by Sigma-Aldorich, Inc., final concentration: 100 uM) as a P2Y14 receptor agonist did not affect IgE receptor stimulation or degranulation by C48/80. On the other hand, BzATP (manufactured by Sigma-Aldorich, Inc.; final concentration: 10 uM) as a P2X7 receptor agonist alone induced degranulation of PMCs, unlike other purine nucleotide receptor agonists. Therefore, it was found that the P2X4 receptor is a specific receptor that promotes MrgPrB2 stimulation among purine nucleotide receptors expressed in mast cells.

Degranulation of PMCs was examined using Substance P (manufactured by Peptide Institute, Inc.: final concentration of 100 uM), Proadrenomedullin N-terminal 20 peptide (PAMP-12) (manufactured by Peptide Institute, Inc.: final concentration of 10 uM), and vancomycin (manufactured by Nacalai Tesque, Inc.: final concentration of 1 mM), which stimulate MrgprB2 to cause pseudo-allergy like C48/80. As a result, degranulation was all induced, and degranulation was enhanced by ATP in a P2X4 dependent manner (Fig. 5). From this, it was suggested that a wide range of pseudo-allergic symptoms can be reduced, regardless of the pseudo-allergy-causing substance, by inhibiting the P2X4 receptor.

### 2-4) ATP Enhances Maximum Response of Degranulation Caused by Pseudo-allergic Reaction

The degranulation of PMCs was examined for IgE receptor stimulation and dose dependency of C48/80. That is, the release rate (%) of β-hexosaminidase was measured when PMCs were stimulated using 0, 1, 5, or 10 ug/mL DNP-HSA, or 0, 1, 10, or 100 uM C48/80.

Regarding degranulation by IgE receptor stimulation, it was considered that ATP promoted degranulation when DNP-HSA as an antigen was at a low concentration, and the promoting effect was not observed when DNP-HSA was at a high concentration, such that the sensitivity to IgE receptor stimulation was increased (Fig. 6(A)). On the other hand, regarding degranulation by MrgprB2 stimulation with C48/80, it was found that ATP significantly increased the degree of degranulation regardless of the concentration of C48/80, and enhanced the maximum response (Fig. 6 (B)). From this, it was considered that a wide range of pseudo-allergic reactions can be reduced, regardless of the degree of the pseudo-allergic symptom, by inhibiting the function of the P2X4 receptor.

### Example 3 Treatments of Pseudo-allergy with P2X4 Receptor Antagonists

### 3-1) P2X4 Receptor Antagonist Compounds

The compounds listed below as described in the present specification were prepared as examples of compounds of P2X4 receptor antagonists. The inhibitory activity (IC₅₀) on the P2X4 receptor was measured by the following method using recombinant cells into which human P2X4 was introduced.

### (Test Method)

A P2X4 receptor antagonizing action of a compound which is the active ingredient of the present invention was measured. 1321N1 cells into which ATP receptors (human P2X4) were introduced were used as a P2X4 receptor stable expression system. Among these compounds, Compound A17 described in the specification is referred to as Compound A, and Compound B48 is referred to as Compound B.
The P2X4-receptor-expressing cells were seeded into a 96-well plate and cultured for 24 hours under a condition of 37°C and 5% CO₂ to be used for calcium measurement. Fura-2AM, a calcium fluorescent indicator, was dissolved in an extracellular fluid for calcium imaging, and the seeded cells were treated therewith and left to stand at room temperature for 45 minutes, thereby incorporating Fura-2AM into the cells. EnVision (PerkinElmer) which is a microplate reader was used for the measurement. 510 nm fluorescences F340 and F380 that are emitted when the cells are irradiated with light radiating from a xenon lamp and transmitted through 340 nm and 380 nm filters, respectively, were observed, and change in a ratio value F340/F380 was used as an index of intracellular calcium change. Measurement was performed by adding ATP to each well such that the final concentration of ATP becomes 1 µM and observing an ATP-induced intracellular calcium response over time. Inhibitory activity of a test substance was measured by pre-treating the test substance for 15 minutes after addition of ATP, and calculation was performed by comparison with a case where the test substance was not present. The results are shown in Table 30.

### (Test Result)

**[Table 30]**

| Test substance | IC₅₀ (µM) |
|---|---|
| Compound A2 | 0.53 |
| Compound A17 (Compound A) | 0.27 |
| Compound B2 | 0.75 |
| Compound B13 | 0.54 |
| Compound B20 | 1.2 |
| Compound B48 (Compound B) | 0.30 |
| Compound B57 | 0.72 |
| Compound B71 | 0.4 |
| Compound B106 | 1.8 |
| Compound B118 | 1.10 |
| Compound B146 | 0.67 |
| Compound B173 | 0.064 |
| Compound B177 | 0.056 |
| Compound B180 | 0.05 |
| Compound B181 | 0.068 |
| Compound B183 | 0.42 |
| Compound B196 | 0.97 |
| Compound B197 | 0.44 |
| Compound B208 | 1.3 |
| Compound B209 | 0.94 |
| Compound B210 | 1.4 |
| Compound B214 | 0.62 |

### 3-2) Suppression of Degranulation of PMCs by P2X4 Receptor Antagonist Compounds

Compound B, a P2X4 receptor antagonist, was used after being dissolved in DMSO at a concentration of 10 mM. In the degranulation test of PMCs in (3) or (4) of Example 2-3), PMCs were treated with Compound B or a solvent thereof (vehicle) at a final concentration of 10 uM before the degranulation reaction. As shown in Fig. 7(A), Compound B significantly suppressed the promotion of degranulation of PMCs by ATP (Student's t-test: p < 0.01). From this, it was considered that the degranulation of mast cells can be suppressed by the P2X4 receptor antagonist.

### 3-3) Treatment of Pseudo-allergy with P2X4 Receptor Antagonist Compounds

Compound B, a P2X4 receptor antagonist, was used after being dissolved in physiological saline at a concentration of 10 mg/ml. In a passive systemic anaphylaxis test using the wild-type C57BL/6 mice of Example 1-1), 10 mg/kg of Compound B or a solvent thereof (vehicle) was intraperitoneally administered 15 minutes before administration of C48/80. As shown in Fig. 7(B), Compound B significantly suppressed the decrease in rectal temperature by C48/80 (Student's t-test: p < 0.05). From this, it was considered that the symptom of the pseudo-allergy can be reduced by the P2X4 receptor antagonist.

From the above, it was found that the P2X4 receptor antagonist can treat, prevent, or reduce a symptom of pseudo-allergy. The pseudo-allergy is not limited to a specific causative substance as long as it is a drug or the like that stimulates MrgprX2, and is not limited to the degree of symptom, and it is considered that the pseudo-allergy can be widely treated.

### Industrial Applicability

The present invention is useful as a pharmaceutical composition for preventing, suppressing, or treating a symptom associated with a pseudo-allergic reaction, the pharmaceutical composition including a compound having a P2X4 receptor antagonizing action, or a pharmaceutically acceptable salt thereof, as an active ingredient.

### [Sequence Listing]

NJ145_ST26

## Claims

1. A pharmaceutical composition for preventing, suppressing, or treating a symptom associated with a pseudo-allergic reaction, the pharmaceutical composition comprising: a compound having a P2X4 receptor antagonizing action, or a pharmaceutically acceptable salt thereof, as an active ingredient.

2. The pharmaceutical composition according to claim 1, wherein the symptom associated with the pseudo-allergic reaction is one or more selected from the group consisting of a skin and mucous membrane symptom (including wheals, erythema punctatum, urticaria, angioedema, eyelid edema, bulbar chemosis, and pruritus), a digestive symptom (including pharyngeal and laryngeal edema, vomiting, abdominal pain, and diarrhea), a respiratory symptom (including wheezing and dyspnea), a cardiovascular symptom (including arrhythmia, paleness, a drop in body temperature, and a drop in blood pressure), and a central nervous system symptom (including headache and loss of consciousness).

3. The pharmaceutical composition according to claim 1 or claim 2, wherein the pseudo-allergic reaction is caused by an allergen having a cationic group.

4. The pharmaceutical composition according to claim 3, wherein the allergen is amorolfine hydrochloride, atracurium, beta-defensin, bleomycin, cetrorelix, C48/80 (monomer or cyclized monomer), ciprofloxacin, cisatracurium, codeine, colistimethate, complanadine A, cortistatin-14, degarelix, enfuvirtide, exenatide, fluoroquinolone, ganirelix, gentamicin sulfate, glatiramer acetate, glucagon, goserelin, histrelin, icatibant, ketoconazole, lanreotide, leuprolide, levofloxacin, liraglutide, LL-37, mafenide acetate, mastoparan, micronomicin sulfate, mivacurium, morphine, moxifloxacin, octreotide, ofloxacin, PAMP-12, PAMP-20, pasireotide, PMX-53, pramlintide, rocuronium, sermorelin, sisomicin sulfate, substance P, succinylcholine, sulfadoxine, sulfamethoxazole, terbinafine hydrochloride, teriparatide, tesamorelin, triptorelin, tubocurarine, or vancomycin.

5. The pharmaceutical composition according to any one of claims 1 to 4, wherein the compound is a compound represented by the following general formula (A): 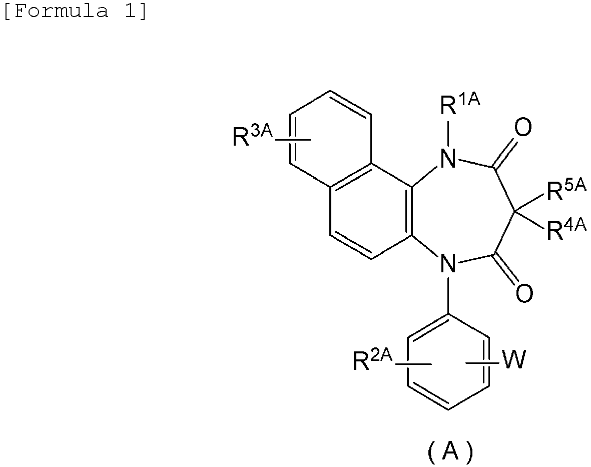 (In the formula R^{1A} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, or an alkyl group having 1 to 3 carbon atoms and substituted with a phenyl group,
R^{2A} and R^{3A} may be the same or different, and represent a hydrogen atom, an alkyl group having 1 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, an acylamino group having 2 to 8 carbon atoms and substituted with 1 to 3 halogen atoms, an alkylsulfonylamino group having 1 to 8 carbon atoms, a carboxyl group, an acyl group having 2 to 8 carbon atoms, an alkoxycarbonyl group (the alkoxy moiety has 1 to 8 carbon atoms), a carbamoyl group, an alkylthio group having 1 to 8 carbon atoms, an alkylsulfinyl group having 1 to 8 carbon atoms, an alkylsulfonyl group having 1 to 8 carbon atoms, or a sulfamoyl group,
R^{4A} and R^{5A} may be the same or different, and represent a hydrogen atom, an alkyl group having 1 to 8 carbon atoms, an alkyl group having 1 to 8 carbon atoms and substituted with 1 to 3 halogen atoms, or an alkyl group having 1 to 3 carbon atoms substituted with a phenyl group, and
W^{A} represents a five or six membered heterocyclic ring including 1 to 4 nitrogen atoms as the members of the ring, which may have a substituent).

6. The pharmaceutical composition according to any one of claims 1 to 4, wherein the compound is a compound represented by the following general formula (BI): 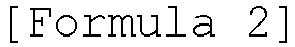 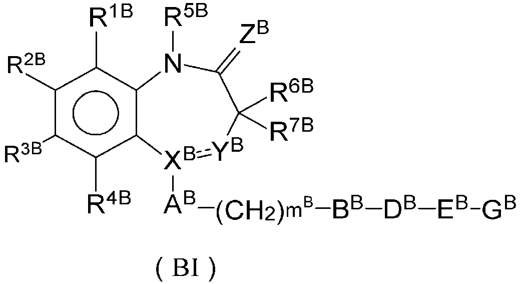 (In the formula R^{1B} and R^{2B} may be the same or different, and represent a hydrogen atom, an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), a phenyl group which may be substituted, a pyridyl group which may be substituted, or an aralkyl group (the aryl moiety has 6 to 10 carbon atoms, and the alkylene moiety has 1 to 8 carbon atoms), or
R^{1B} and R^{2B} may bind together to form a condensed ring selected from a naphthalene ring, a quinoline ring, an isoquinoline ring, a tetrahydronaphthalene ring, an indane ring, a tetrahydroquinoline ring, and a tetrahydroisoquinoline ring together with the benzene ring to which they bind, and the ring constituted by R^{1B} and R^{2B} bound to each other, together with the carbon atoms to which R^{1B} and R^{2B} bind may be substituted with 1 to 4 of the same or different substituents selected from an alkyl group having 1 to 8 carbon atoms, a cycloalkyl group having 3 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), and an aralkyl group (the aryl moiety has 6 to 10 carbon atoms, and the alkylene moiety has 1 to 8 carbon atoms),
R^{3B} and R^{4B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acylamino group having 2 to 8 carbon atoms, a carboxyl group, an acyl group having 2 to 8 carbon atoms, an alkoxycarbonyl group (the alkoxy moiety has 1 to 8 carbon atoms), or an aralkyl group (the aryl moiety has 6 to 10 carbon atoms, and the alkylene moiety has 1 to 8 carbon atoms),
R^{5B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
R^{6B} and R^{7B} may be the same or different, and represent a hydrogen atom, an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, or an amino group,
X^{B} represents C, CH, or N,
Y^{B} represents N, NH, or C(=O),
provided that when X^{B} is N, Y^{B} is not N or NH, and
when X^{B} is C or CH, Y^{B} is not C(=O),
the double line consisting of the solid line and the broken line represents a single bond or a double bond,
Z^{B} represents an oxygen atom or a sulfur atom,
A^{B} represents a benzene ring, a pyridine ring, a thiophene ring, a pyrimidine ring, a naphthalene ring, a quinoline ring, or an indole ring, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an aralkyl group (the aryl moiety has 6 to 10 carbon atoms, and the alkylene moiety has 1 to 8 carbon atoms), a phenyl group, and a pyridyl group, as a substituent, or represents an atomic bond,
B^{B} represents N(R^{8B})C(=O), NHCONH, CON(R^{9B}), NHC (=S) NH, N (R^{10B}) SO₂, SO₂N(R^{11B}), or OSO₂, in the formula
R^{8B}, R^{9B}, R^{10B}, and R^{11B} represent a hydrogen atom, an alkyl group having 1 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
D^{B} represents an alkylene chain having 1 to 6 carbon atoms, which may have 1 to 4 of the same or different substituents selected from an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, and an aralkyl group (the aryl moiety has 6 to 10 carbon atoms, and the alkylene moiety has 1 to 8 carbon atoms), as a substituent, and may further have a double bond, or represents an atomic bond,
E^{B} represents O, S, NR^{12B}, or an atomic bond, in the formula
R^{12B} represents a hydrogen atom, an alkyl group having 1 to 8 carbon atoms, an alkenyl group having 2 to 8 carbon atoms, an alkyl group having 1 to 8 carbon atoms and substituted with 1 to 3 halogen atoms, an alkyl group having 1 to 8 carbon atoms and substituted with a hydroxyl group, or an aralkyl group (the aryl moiety has 6 to 10 carbon atoms, and the alkylene moiety has 1 to 8 carbon atoms),
G^{B} represents piperazine, piperidine, morpholine, cyclohexane, benzene, naphthalene, quinoline, quinoxaline, benzimidazole, thiophene, imidazole, thiazole, oxazole, indole, benzofuran, pyrrole, pyridine, or pyrimidine, which may have 1 to 4 of the same or different substituents selected from an alkyl group having 1 to 8 carbon atoms, an alkenyl group having 2 to 8 carbon atoms, an alkoxy group having 1 to 8 carbon atoms, an alkyl group having 1 to 8 carbon atoms and substituted with 1 to 3 halogen atoms, an alkoxy group having 1 to 8 carbon atoms and substituted with 1 to 3 halogen atoms, a halogen atom, a hydroxyl group, a nitro group, a cyano group, an amino group, an alkylamino group having 1 to 8 carbon atoms, a dialkylamino group having 2 to 8 carbon atoms, an acyl group having 2 to 8 carbon atoms, a methylenedioxy group, a carboxyl group, an alkylsulfinyl group having 1 to 6 carbon atoms, an alkylthio group having 1 to 6 carbon atoms, an alkylsulfonyl group having 1 to 6 carbon atoms, an aralkyl group (the aryl moiety has 6 to 10 carbon atoms, and the alkylene moiety has 1 to 8 carbon atoms), a phenyl group which may be substituted, a pyridyl group which may be substituted, an imidazolyl group which may be substituted, an oxazolyl group which may be substituted, and a thiazolyl group which may be substituted, as a substituent, and
m^{B} represents an integer of 0 to 5,
provided that when R^{1B} and R^{2B} do not bind together to form a ring, those compounds are excluded in the formula, X^{B} is C, Y^{B} is N, the double line consisting of the solid line and the broken line is a double bond, Z^{B} is an oxygen atom, A^{B} is a benzene ring, m^{B} is 0, B^{B} is C(=O)NH, E^{B} is an atomic bond, and G^{B} is a phenyl group).

7. The pharmaceutical composition according to any one of claims 1 to 4, wherein the compound or a pharmaceutically acceptable salt thereof is a compound selected from the group consisting of the following (A1) to (A21) and (B1) to (B214), or a pharmaceutically acceptable salt thereof:
(A1) 5-[3-(1H-tetrazol-5-yl)phenyl]-1H-naphtho[1,2-b][1,4]diazepine-2,4(3H,5H)-dione;
(A2) 5-[3-(1H-tetrazol-5-yl)phenyl]-1H-naphtho[1,2-b][1,4]diazepine-2,4(3H,5H)-dione sodium salt;
(A3) 5-[3-(1H-tetrazol-5-yl)phenyl]-1H-naphtho[1,2-b][1,4]diazepine-2,4(3H,5H)-dione potassium salt;
(A4) 5-[4-(1H-tetrazol-5-yl)phenyl]-1H-naphtho[1,2-b][1,4]diazepine-2,4(3H,5H)-dione;
(A5) 5-[4-(1H-tetrazol-5-yl)phenyl]-1H-naphtho[1,2-b][1,4]diazepine-2,4(3H,5H)-dione sodium salt;
(A6) 1-methyl-5-[3-(1H-tetrazol-5-yl)phenyl]-1H-naphtho[1,2-b][1,4]diazepine-2,4(3H,5H)-dione;
(A7) 1,3-dimethyl-5-[3-(1H-tetrazol-5-yl)phenyl]-1H-naphtho[1,2-b][1,4]diazepine-2,4(3H,5H)-dione;
(A8) 5-[2-chloro-5-(1H-tetrazol-5-yl)phenyl]-1H-naphtho[1,2-b][1,4]diazepine-2,4(3H,5H)-dione;
(A9) 5-[2-chloro-5-(1H-tetrazol-5-yl)phenyl]-1H-naphtho[1,2-b][1,4]diazepine-2,4(3H,5H)-dione sodium salt;
(A10) 5-[2-methyl-5-(1H-tetrazol-5-yl)phenyl]-1H-naphtho[1,2-b][1,4]diazepine-2,4(3H,5H)-dione;
(A11) 5-[2-methyl-5-(1H-tetrazol-5-yl)phenyl]-1H-naphtho[1,2-b][1,4]diazepine-2,4(3H,5H)-dione sodium salt;
(A12) 5-[2-bromo-5-(1H-tetrazol-5-yl)phenyl]-1H-naphtho[1,2-b][1,4]diazepine-2,4(3H,5H)-dione;
(A13) 5-[3-(2-methyl-2H-tetrazol-5-yl)phenyl]-1H-naphtho[1,2-b][1,4]diazepine-2,4(3H,5H)-dione;
(A14) 5-[3-(1-methyl-1H-tetrazol-5-yl)phenyl]-1H-naphtho[1,2-b][1,4]diazepine-2,4(3H,5H)-dione;
(A15) 5-[3-(5-oxo-4H-[1,2,4]oxadiazol-3-yl)phenyl]-1H-naphtho[1,2-b][1,4]diazepine-2,4(3H,5H)-dione;
(A16) 5-[3-(5-thioxo-4H-[1,2,4]oxadiazol-3-yl)phenyl]-1H-naphtho[1,2-b][1,4]diazepine-2,4(3H,5H)-dione;
(A17) 5-[3-(5-thioxo-4H-[1,2,4]oxadiazol-3-yl)phenyl]-1H-naphtho[1,2-b][1,4]diazepine-2,4(3H,5H)-dione sodium salt;
(A18) 5-[3-(oxazol-2-yl)phenyl]-1H-naphtho[1,2-b][1,4]diazepine-2,4(3H,5H)-dione;
(A19) 5-[3-(1H-pyrazol-4-yl)phenyl]-1H-naphtho[1,2-b][1,4]diazepine-2,4(3H,5H)-dione;
(A20) 5-[4-fluoro-3-(1H-tetrazol-5-yl)phenyl]-1H-naphtho[1,2-b][1,4]diazepine-2,4(3H,5H)-dione; and
(A21) 5-[4-fluoro-3-(1H-tetrazol-5-yl)phenyl]-1H-naphtho[1,2-b][1,4]diazepine-2,4(3H,5H)-dione sodium salt; and
(B1) 5-(4-benzoylaminophenyl)-1H-naphtho[1,2-b][1,4]diazepine-2,4(3H,5H)-dione;
(B2) 5-[4-[2-(trifluoromethyl)benzoyl]aminophenyl]-1H-naphtho[1,2-b][1,4]diazepine-2,4(3H,5H)-dione;
(B3) 5-[4-(3-bromobenzoyl)aminophenyl]-1H-naphtho[1,2-b][1,4]diazepine-2,4(3H,5H)-dione;
(B4) 5-[4-[4-(trifluoromethyl)benzoyl]aminophenyl]-1H-naphtho[1,2-b][1,4]diazepine-2,4(3H,5H)-dione;
(B5) 5-[4-(2-methylbenzoyl)aminophenyl]-1H-naphtho[1,2-b][1,4]diazepine-2,4(3H,5H)-dione;
(B6) 5-[4-(2,6-dimethylbenzoylamino)phenyl]-1H-naphtho[1,2-b][1,4]diazepine-2,4(3H,5H)-dione;
(B7) 5-[4-(2,6-dichlorobenzoyl)aminophenyl]-1H-naphtho[1,2-b][1,4]diazepine-2,4(3H,5H)-dione;
(B8) 5-[4-(3-chlorobenzoyl)aminophenyl]-1H-naphtho[1,2-b][1,4]diazepine-2,4(3H,5H)-dione;
(B9) 5-[4-(2-phenylacetylamino)phenyl]-1H-naphtho[1,2-b][1,4]diazepine-2,4(3H,5H)-dione;
(B10) 1-[4-(2,4-dioxo-1,2,3,4-tetrahydronaphtho[1,2-b][1,4]diazepin-5-yl)phenyl]-3-phenylthiourea;
(B11) 5-[4-(2,3-dimethoxybenzoylamino)phenyl]-1H-naphtho[1,2-b][1,4]diazepine-2,4(3H,5H)-dione;
(B12) 5-[4-(2-methoxybenzoylamino)phenyl]-1H-naphtho[1,2-b][1,4]diazepine-2,4(3H,5H)-dione;
(B13) 5-[4-[(2-chlorophenylacetyl)amino]phenyl]-1H-naphtho[1,2-b][1,4]diazepine-2,4(3H,5H)-dione;
(B14) 5-[4-(2,3-dimethylbenzoylamino)phenyl]-1H-naphtho[1,2-b][1,4]diazepine-2,4(3H,5H)-dione;
(B15) 5-[4-(2,5-dimethylbenzoylamino)phenyl]-1H-naphtho[1,2-b][1,4]diazepine-2,4(3H,5H)-dione;
(B16) 5-[4-(5-bromo-2-chlorobenzoylamino)phenyl]-1H-naphtho[1,2-b][1,4]diazepine-2,4(3H,5H)-dione;
(B17) 5-[4-(2,4-dichlorobenzoylamino)phenyl]-1H-naphtho[1,2-b][1,4]diazepine-2,4(3H,5H)-dione;
(B18) 5-[4-(2-hydroxybenzoylamino)phenyl]-1H-naphtho[1,2-b][1,4]diazepine-2,4(3H,5H)-dione;
(B19) 5-[4-(2,3-dihydroxybenzoylamino)phenyl]-1H-naphtho[1,2-b][1,4]diazepine-2,4(3H,5H)-dione;
(B20) 1-[4-(2,4-dioxo-1,2,3,4-tetrahydronaphtho[1,2-b][1,4]diazepin-5-yl)phenyl]-3-phenylurea;
(B21) 5-[4-[(2,6-dichlorophenylacetyl)amino]phenyl]-1H-naphtho[1,2-b][1,4]diazepine-2,4(3H,5H)-dione;
(B22) 5-[4-[(2-methoxyphenylacetyl)amino]phenyl]-1H-naphtho[1,2-b][1,4]diazepine-2,4(3H,5H)-dione;
(B23) 5-[4-[(2-hydroxyphenylacetyl)amino]phenyl]-1H-naphtho[1,2-b][1,4]diazepine-2,4(3H,5H)-dione;
(B24) 1-(2-chlorophenyl)-3-[4-(2,4-dioxo-1,2,3,4-tetrahydronaphtho[1,2-b][1,4]diazepin-5-yl)phenyl]thiourea;
(B25) 5-[4-[3-(trifluoromethyl)benzoylamino]phenyl]-1H-naphtho[1,2-b][1,4]diazepine-2,4(3H,5H)-dione;
(B26) 5-[4-[2-[2-(trifluoromethyl)phenyl]acetylamino]phenyl]-1H-naphtho[1,2-b][1,4]diazepine-2,4(3H,5H)-dione;
(B27) 1-(2-chlorophenyl)-3-[4-(2,4-dioxo-1,2,3,4-tetrahydronaphtho[1,2-b][1,4]diazepin-5-yl)phenyl]urea;
(B28) 5-[4-[(2-phenylpropionyl)amino]phenyl]-1H-naphtho[1,2-b][1,4]diazepine-2,4(3H,5H)-dione;
(B29) 5-[4-(2-chloro-3-methoxybenzoylamino)phenyl]-1H-naphtho[1,2-b][1,4]diazepine-2,4(3H,5H)-dione;
(B30) 5-[4-(3-phenylpropionylamino)phenyl]-1H-naphtho[1,2-b][1,4]diazepine-2,4(3H,5H)-dione;
(B31) 5-[4-[(1H-indol-3-carbonyl)amino]phenyl]-1H-naphtho[1,2-b][1,4]diazepine-2,4(3H,5H)-dione;
(B32) 5-[4-(2-chloro-3-hydroxybenzoylamino)phenyl]-1H-naphtho[1,2-b][1,4]diazepine-2,4(3H,5H)-dione;
(B33) 5-[4-[(2-methyl-2-phenylpropionyl)amino]phenyl]-1H-naphtho[1,2-b][1,4]diazepine-2,4(3H,5H)-dione;
(B34) 5-[4-(2-phenoxyacetylamino)phenyl]-1H-naphtho[1,2-b][1,4]diazepine-2,4(3H,5H)-dione;
(B35) 5-[4-[2-(2-chloro-4-methoxyphenyl)acetylamino]phenyl]-1H-naphtho[1,2-b] [1,4]diazepine-2,4(3H,5H)-dione;
(B36) 5-[4-[(1-methyl-1H-imidazol-2-carbonyl)amino]phenyl]-1H-naphtho[1,2-b] [1,4]diazepine-2,4(3H,5H)-dione;
(B37) 5-[4-[2-(2,4-dichlorophenyl)acetylamino]phenyl]-1H-naphtho [1,2-b] [1,4]diazepine-2,4(3H,5H)-dione;
(B38) 5-[4-[2-(2-chloro-4-hydroxyphenyl)acetylamino]phenyl]-1H-naphtho[1,2-b] [1,4]diazepine-2,4(3H,5H)-dione;
(B39) 5-[4-(3-phenylpropenylamino)phenyl]-1H-naphtho[1,2-b] [1,4]diazepine-2,4(3H,5H)-dione;
(B40) 5-[4-[(3-pyridylacetyl)amino]phenyl]-1H-naphtho[1,2-b] [1,4]diazepine-2,4(3H,5H)-dione hydrochloride;
(B41) 5-[4-(1H-benzimidazol-2-carbonylamino)phenyl]-1H-naphtho [1,2-b] [1,4]diazepine-2,4(3H,5H)-dione;
(B42) 1-[4-(2,3-dimethylbenzoylamino)phenyl]-7-methoxy-1H-1,5-benzodiazepine-2,4(3H,5H)-dione;
(B43) 5-[4-[(benzoylamino)methyl]phenyl]-1H-naphtho[1,2-b][1,4]diazepine-2,4(3H,5H)-dione;
(B44) 5-[4-[(2-chlorobenzoylamino)methyl]phenyl]-1H-naphtho[1,2-b][1,4]diazepine-2,4(3H,5H)-dione;
(B45) 1-[4-(2,3-dimethylbenzoylamino)phenyl]-7-hydroxy-1H-1,5-benzodiazepine-2,4(3H,5H)-dione;
(B46) 5-[4-(2-chlorobenzoylamino)phenyl]-1H-naphtho[1,2-b][1,4]diazepine-2,4(3H,5H)-dione;
(B47) 5-[4-(2-bromobenzoylamino)phenyl]-1H-naphtho[1,2-b][1,4]diazepine-2,4(3H,5H)-dione;
(B48) 5-[4-(2-iodobenzoylamino)phenyl]-1H-naphtho[1,2-b][1,4]diazepine-2,4(3H,5H)-dione;
(B49) 5-[4-(2,3-dimethylbenzoylamino)-3-fluorophenyl]-1H-naphtho[1,2-b][1,4]diazepine-2,4(3H,5H)-dione;
(B50) 5-[4-[2-(2-methylphenyl)acetylamino]phenyl]-1H-naphtho[1,2-b][1,4]diazepine-2,4(3H,5H)-dione;
(B51) 5-[4-[(quinoxalin-2-yl)carbonylamino]phenyl]-1H-naphtho[1,2-b][1,4]diazepine-2,4(3H,5H)-dione;
(B52) 5-[4-[(5-methylthiophen-2-yl)carbonylamino]phenyl]-1H-naphtho[1,2-b][1,4]diazepine-2,4(3H,5H)-dione;
(B53) 5-[3-[(2-chlorophenylacetyl)amino]phenyl]-1H-naphtho[1,2-b][1,4]diazepine-2,4(3H,5H)-dione;
(B54) 5-[4-[(2,4,6-trimethylbenzoyl)amino]phenyl]-1H-naphtho[1,2-b][1,4]diazepine-2,4(3H,5H)-dione;
(B55) 5-[4-(cyclohexylcarbonylamino)phenyl]-1,2,3,4,8,9,10,11-octahydronaphtho[1,2-b][1,4]diazepine-2,4(3H,5H)-dione;
(B56) 1-[4-(2,3-dimethylbenzoyl)aminophenyl]-6-methyl-1H-1,5-benzodiazepine-2,4(3H,5H)-dione;
(B57) 5-[4-[(2-ethylbenzoyl)amino]phenyl]-1H-naphtho[1,2-b][1,4]diazepine-2,4(3H,5H)-dione;
(B58) 5-[4-[(6-methylpyridin-2-yl)carbonylamino]phenyl]-1,2,3,4,8,9,10,11-octahydronaphtho[1,2-b][1,4]diazepine-2,4(3H,5H)-dione;
(B59) 5-[4-[(2-methylpyridin-3-yl)carbonylamino]phenyl]-1,2,3,4,8,9,10,11-octahydronaphtho[1,2-b][1,4]diazepine-2,4(3H,5H)-dione;
(B60) 1-[4-(2,4-dioxo-1,2,3,4-tetrahydronaphtho[1,2-b][1,4]diazepin-5-yl)phenyl]-3-(2-methylphenyl)thiourea;
(B61) 5-[4-(2-methoxy-3-methylbenzoyl)aminophenyl]-1,2,3,4,8,9,10,11-octahydronaphtho[1,2-b][1,4]diazepine-2,4(3H,5H)-dione;
(B62) 5-[4-(2,3-dichlorobenzoyl)aminophenyl]-1,2,3,4,8,9,10,11-octahydronaphtho[1,2-b][1,4]diazepine-2,4(3H,5H)-dione;
(B63) 5-[4-(2,3-dimethylbenzoylamino)-3-hydroxyphenyl]-1H-naphtho[1,2-b][1,4]diazepine-2,4(3H,5H)-dione;
(B64) 5-[4-(2-chloro-3-methoxybenzoylamino)phenyl]-1,3-dihydronaphtho[1,2-e]-1,4-diazepine-2-one;
(B65) 5-[4-[(4-dimethylaminobenzoyl)amino]phenyl]-1H-naphtho[1,2-b][1,4]diazepine-2,4(3H,5H)-dione;
(B66) 5-[4-[2-(2,4-dichlorophenoxy)acetylamino]phenyl]-1H-naphtho[1,2-b][1,4]diazepine-2,4(3H,5H)-dione;
(B67) 5-[4-[2-(2-methylphenoxy)acetylamino]phenyl]-1H-naphtho[1,2-b][1,4]diazepine-2,4(3H,5H)-dione;
(B68) N-[4-(2,4-dioxo-1,2,3,4-tetrahydronaphtho[1,2-b][1,4]diazepin-5-yl)butyl]-2-chloro-3-methoxybenzamide;
(B69) 5-[4-(2-chloro-3-hydroxybenzoylamino)phenyl]-1,3-dihydronaphtho[1,2-e]-1,4-diazepine-2-one;
(B70) 5-[4-(2-acetylbenzoylamino)phenyl]-1H-naphtho[1,2-b][1,4]diazepine-2,4(3H,5H)-dione;
(B71) 5-[4-(2-tert-butylbenzoylamino)phenyl]-1H-naphtho[1,2-b][1,4]diazepine-2,4(3H,5H)-dione;
(B72) 5-[2-(2-iodobenzoyl)aminoethyl]-1H-naphtho[1,2-b][1,4]diazepine-2,4(3H,5H)-dione;
(B73) 5-[3-[(2-iodobenzoyl)amino]phenyl]-1H-naphtho[1,2-b][1,4]diazepine-2,4(3H,5H)-dione;
(B74) 6,7-dimethyl-1-[4-(2-iodobenzoyl)aminophenyl]-1H-1,5-benzodiazepine-2,4(3H,5H)-dione;
(B75) 5-[4-[(1-methylpiperidin-4-yl)carbonylamino]phenyl]-1H-naphtho[1,2-b][1,4]diazepine-2,4(3H,5H)-dione hydrochloride;
(B76) 5[4-[(benzofuran-2-yl)carbonylamino]phenyl]-1H-naphtho[1,2-b][1,4]diazepine-2,4(3H,5H)-dione;
(B77) 5-[4-[(1-methyl-1H-indol-3-yl)carbonylamino]phenyl]-1H-naphtho[1,2-b][1,4]diazepine-2,4(3H,5H)-dione;
(B78) 5-[4-(2-propenylbenzoylamino)phenyl]-1H-naphtho[1,2-b][1,4]diazepine-2,4(3H,5H)-dione;
(B79) 5-[4-(2-propylbenzoylamino)phenyl]-1H-naphtho[1,2-b][1,4]diazepine-2,4(3H,5H)-dione;
(B80) 5-[3-fluoro-4-(2-iodobenzoylamino)phenyl]-1H-naphtho[1,2-b][1,4]diazepine-2,4(3H,5H)-dione;
(B81) 5-[4-(2-hydroxy-3-methylbenzoyl)aminophenyl]-1,2,3,4,8,9,10,11-octahydronaphtho[1,2-b][1,4]diazepine-2,4(3H,5H)-dione;
(B82) 5-[4-[(2-isopropoxybenzoyl)amino]phenyl]-1H-naphtho[1,2-b][1,4]diazepine-2,4(3H,5H)-dione;
(B83) 5-[4-[(3-methylthiophen-2-yl)carbonylamino]phenyl]-1H-naphtho[1,2-b][1,4]diazepine-2,4(3H,5H)-dione;
(B84) 5-[4-(2-phenoxypropionylamino)phenyl]-1H-naphtho[1,2-b][1,4]diazepine-2,4(3H,5H)-dione;
(B85) 5-[4-[2-(4-chloro-2-methylphenoxy)acetylamino]phenyl]-1H-naphtho[1,2-b][1,4]diazepine-2,4(3H,5H)-dione;
(B86) 5-[4-[(4-fluoro-2-trifluoromethyl)benzoyl]aminophenyl]-1H-naphtho[1,2-b][1,4]diazepine-2,4(3H,5H)-dione;
(B87) 5-[4-(4-fluoro-2-methoxybenzoyl)aminophenyl]-1H-naphtho[1,2-b][1,4]diazepine-2,4(3H,5H)-dione;
(B88) 5-[4-(4-fluoro-2-hydroxybenzoyl)aminophenyl]-1H-naphtho[1,2-b][1,4]diazepine-2,4(3H,5H)-dione;
(B89) 5-[3-[(2-iodophenylacetyl)amino]phenyl]-1H-naphtho[1,2-b][1,4]diazepine-2,4(3H,5H)-dione;
(B90) 5-[4-(2-methyl-2-phenoxypropionylamino)phenyl]-1H-naphtho[1,2-b][1,4]diazepine-2,4(3H,5H)-dione;
(B91) 5-[4-(2-tert-butylbenzoylamino)phenyl]-1,3-dihydronaphtho[1,2-e]-1,4-diazepine-2-one;
(B92) 5-[4-[(3-dimethylaminobenzoyl)amino]phenyl]-1H-naphtho[1,2-b][1,4]diazepine-2,4(3H,5H)-dione;
(B93) 5-[4-(4-iodo-2-methoxybenzoylamino)phenyl]-1H-naphtho[1,2-b][1,4]diazepine-2,4(3H,5H)-dione;
(B94) 5-[4-(6-fluoro-2-methoxybenzoylamino)phenyl]-1H-naphtho[1,2-b][1,4]diazepine-2,4(3H,5H)-dione;
(B95) 5-[4-(2-hydroxy-4-iodobenzoylamino)phenyl]-1H-naphtho[1,2-b][1,4]diazepine-2,4(3H,5H)-dione;
(B96) 5-[4-(6-fluoro-2-hydroxybenzoylamino)phenyl]-1H-naphtho[1,2-b][1,4]diazepine-2,4(3H,5H)-dione;
(B97) 5-[4-(2-fluorobenzoyl)aminophenyl]-1H-naphtho[1,2-b][1,4]diazepine-2,4(3H,5H)-dione;
(B98) 5-[4-[(2-dimethylaminobenzoyl)amino]phenyl]-1H-naphtho[1,2-b][1,4]diazepine-2,4(3H,5H)-dione;
(B99) 5-[4-(2-methoxy-6-methylbenzoylamino)phenyl]-1H-naphtho[1,2-b][1,4]diazepine-2,4(3H,5H)-dione;
(B100) 5-[4-(2-hydroxy-6-methylbenzoylamino)phenyl]-1H-naphtho[1,2-b][1,4]diazepine-2,4(3H,5H)-dione;
(B101) 5-[4-[3-(2-methylphenyl)propionylamino]phenyl]-1H-naphtho[1,2-b][1,4]diazepine-2,4(3H,5H)-dione;
(B102) 5-(4-phenylcarbamoylphenyl)-1H-naphtho[1,2-b][1,4]diazepine-2,4(3H,5H)-dione;
(B103) 5-(4-benzylcarbamoylphenyl)-1H-naphtho[1,2-b][1,4]diazepine-2,4(3H,5H)-dione;
(B104) 5-[4-[3-(2-methylphenyl)propenoylamino]phenyl]-1H-naphtho[1,2-b][1,4]diazepine-2,4(3H,5H)-dione;
(B105) 5-[4-[3-(2-chlorophenyl)propionylamino]phenyl]-1H-naphtho[1,2-b][1,4]diazepine-2,4(3H,5H)-dione;
(B106) 5-[4-(2-iodobenzoyl)aminophenyl]-1,2,3,4,8,9,10,11-octahydronaphtho[1,2-b][1,4]diazepine-2,4(3H,5H)-dione;
(B107) 5-[4-[(1-methyl-1H-pyrrol-2-ylacetyl)amino]phenyl]-1,2,3,4,8,9,10,11-octahydronaphtho[1,2-b][1,4]diazepine-2,4(3H,5H)-dione;
(B108) 5-[4-(2-chlorobenzyl)carbamoylphenyl]-1H-naphtho[1,2-b][1,4]diazepine-2,4(3H,5H)-dione;
(B109) 5-[4-[3-(2-chlorophenyl)propenoylamino]phenyl]-1H-naphtho[1,2-b][1,4]diazepine-2,4(3H,5H)-dione;
(B110) 5-[4-(2-chlorophenyl)carbamoylphenyl]-1H-naphtho[1,2-b][1,4]diazepine-2,4(3H,5H)-dione;
(B111) 5-[4-(6-bromo-2,3-methylenedioxybenzoylamino)phenyl]-1H-naphtho[1,2-b][1,4]diazepine-2,4(3H,5H)-dione;
(B112) 5-[4-(6-bromo-2-methoxybenzoylamino)phenyl]-1H-naphtho[1,2-b][1,4]diazepine-2,4(3H,5H)-dione;
(B113) 5-[4-[(2-tert-butylbenzoyl)amino]phenyl]-1,2,3,4,8,9,10,11-octahydronaphtho[1,2-b][1,4]diazepine-2,4(3H,5H)-dione;
(B114) 5-[2-(2-iodobenzoyl)aminopyridin-5-yl]-1H-naphtho[1,2-b][1,4]diazepine-2,4(3H,5H)-dione;
(B115) 5-[4-(6-bromo-2-hydroxybenzoylamino)phenyl]-1H-naphtho[1,2-b][1,4]diazepine-2,4(3H,5H)-dione;
(B116) 5-[4-(6-chloro-2-methoxybenzoylamino)phenyl]-1H-naphtho[1,2-b][1,4]diazepine-2,4(3H,5H)-dione;
(B117) 5-[4-(2-iodobenzoylamino)phenyl]-1H-[1,4]diazepino[2,3-h]quinoline-2,4(3H,5H)-dione;
(B118) 5-[4-(6-chloro-2-hydroxybenzoylamino)phenyl]-1H-naphtho[1,2-b][1,4]diazepine-2,4(3H,5H)-dione;
(B119) 5-[4-(2-hydroxy-6-methoxybenzoylamino)phenyl]-1H-naphtho[1,2-b][1,4]diazepine-2,4(3H,5H)-dione;
(B120) 5-[4-[2-methoxy-6-(trifluoromethyl)benzoylamino]phenyl]-1H-naphtho[1,2-b][1,4]diazepine-2,4(3H,5H)-dione;
(B121) 5-[4-[2-hydroxy-6-(trifluoromethyl)benzoylamino]phenyl]-1H-naphtho[1,2-b][1,4]diazepine-2,4(3H,5H)-dione;
(B122) 5-[4-[(2-isopropenylbenzoyl)amino]phenyl]-1,2,3,4,8,9,10,11-octahydronaphtho[1,2-b][1,4]diazepine-2,4(3H,5H)-dione;
(B123) 5-[4-[(2-isopropylbenzoyl)amino]phenyl]-1,2,3,4,8,9,10,11-octahydronaphtho[1,2-b][1,4]diazepine-2,4(3H,5H)-dione;
(B124) 5-[4-[2-chloro-5-(methylthio)benzoylamino]phenyl]-1H-naphtho[1,2-b][1,4]diazepine-2,4(3H,5H)-dione;
(B125) 5-[4-[2-(methylthio)benzoylamino]phenyl]-1H-naphtho[1,2-b][1,4]diazepine-2,4(3H,5H)-dione;
(B126) 5-[4-[3-(methylthio)benzoylamino]phenyl]-1H-naphtho[1,2-b][1,4]diazepine-2,4(3H,5H)-dione;
(B127) 5-[4-[2-ethyl-6-methoxybenzoylamino]phenyl]-1H-naphtho[1,2-b][1,4]diazepine-2,4(3H,5H)-dione;
(B128) 5-[4-(3-methanesulfonylbenzoylamino)phenyl]-1H-naphtho[1,2-b][1,4]diazepine-2,4(3H,5H)-dione;
(B129) 6-ethyl-1-[4-(2-iodobenzoyl)aminophenyl]-1H-1,5-benzodiazepine-2,4(3H,5H)-dione;
(B130) 5-[4-[2-ethyl-6-hydroxybenzoylamino]phenyl]-1H-naphtho[1,2-b][1,4]diazepine-2,4(3H,5H)-dione;
(B131) 5-[4-(3-methanesulfinylbenzoylamino)phenyl]-1H-naphtho[1,2-b][1,4]diazepine-2,4(3H,5H)-dione;
(B132) 5-[4-(2-chloro-5-methanesulfinylbenzoylamino)phenyl]-1H-naphtho[1,2-b][1,4]diazepine-2,4(3H,5H)-dione;
(B133) 5-[4-(2-methanesulfinylbenzoylamino)phenyl]-1H-naphtho[1,2-b][1,4]diazepine-2,4(3H,5H)-dione;
(B134) 5-[4-[[2-(4-morpholinyl)acetyl]amino]phenyl]-1,2,3,4,8,9,10,11-octahydronaphtho[1,2-b][1,4]diazepine-2,4(3H,5H)-dione hydrochloride;
(B135) 5-[4-(2-chloro-6-methoxybenzoylamino)phenyl]-1,3-dihydronaphtho[1,2-e]-1,4-diazepine-2-one;
(B136) 5-[4-[[(3-chloropyridin-2-yl)carbonyl]amino]phenyl]-1H-naphtho[1,2-b][1,4]diazepine-2,4(3H,5H)-dione;
(B137) 5-[4-(2-chloro-6-hydroxybenzoylamino)phenyl]-1,3-dihydronaphtho[1,2-e]-1,4-diazepine-2-one;
(B138) 5-[4-(3-chloro-2-methoxybenzoylamino)phenyl]-1H-naphtho[1,2-b][1,4]diazepine-2,4(3H,5H)-dione;
(B139) 5-[4-[(3-methylpyridin-2-yl)carbonylamino]phenyl]-1H-naphtho[1,2-b][1,4]diazepine-2,4(3H,5H)-dione;
(B140) 5-[4-[[(3-chloropyridin-2-yl)carbonyl]amino]phenyl]-1,2,3,4,8,9,10,11-octahydronaphtho[1,2-b][1,4]diazepine-2,4(3H,5H)-dione;
(B141) 5-[4-(3-chloro-2-hydroxybenzoylamino)phenyl]-1H-naphtho[1,2-b][1,4]diazepine-2,4(3H,5H)-dione;
(B142) 5-[4-[[(3-hydroxypyridin-2-yl)carbonyl]amino]phenyl]-1H-naphtho[1,2-b][1,4]diazepine-2,4(3H,5H)-dione;
(B143) 5-[4-[(3-vinylpyridin-2-yl)carbonylamino]phenyl]-1H-naphtho[1,2-b][1,4]diazepine-2,4(3H,5H)-dione;
(B144) 5-[4-[(3-ethylpyridin-2-yl)carbonylamino]phenyl]-1H-naphtho[1,2-b][1,4]diazepine-2,4(3H,5H)-dione;
(B145) N-[4-(2,4-dioxo-1,2,3,4-tetrahydronaphtho-[1,2-b][1,4]-diazepin-5-yl)phenyl]-2-nitrobenzenesulfonamide;
(B146) N-[4-(2,4-dioxo-1,2,3,4-tetrahydronaphtho[1,2-b][1,4]diazepin-5-yl)phenyl]benzenesulfonamide;
(B147) 3-bromo-N-[4-(2,4-dioxo-1,2,3,4-tetrahydronaphtho[1,2-b][1,4]diazepin-5-yl)phenyl]benzenesulfonamide;
(B148) N-[4-(2,4-dioxo-1,2,3,4-tetrahydronaphtho[1,2-b][1,4]diazepin-5-yl)phenyl]-3-methoxybenzenesulfonamide;
(B149) N-[3-(2-oxo-2,3-dihydro-1H-naphtho[1,2-e][1,4]diazepin-5-yl)phenyl]benzenesulfonamide;
(B150) N-[3-(2,4-dioxo-1,2,3,4-tetrahydronaphtho-[1,2-b][1,4]-diazepin-5-yl)phenyl]-2-nitrobenzenesulfonamide;
(B151) N-[4-(2,4-dioxo-1,2,3,4,8,9,10,11-octahydro-naphtho[1,2-b][1,4]-diazepin-5-yl)phenyl]-2-nitrobenzenesulfonamide;
(B152) N-[4-(2,4-dioxo-1,2,3,4,8,9,10,11-octahydro-naphtho[1,2-b][1,4]-diazepin-5-yl)phenyl]-N-methyl-2-nitrobenzenesulfonamide;
(B153) N-[3-(2,4-dioxo-1,2,3,4-tetrahydronaphtho-[1,2-b][1,4]-diazepin-5-yl)phenyl]-N-methyl-2-nitrobenzenesulfonamide;
(B154) 4-(2,4-dioxo-1,2,3,4,8,9,10,11-octahydronaphtho[1,2-b][1,4]diazepin-5-yl)-N-phenylbenzenesulfonamide;
(B155) N-[3-(2,4-dioxo-1,2,3,4-tetrahydronaphtho[1,2-b][1,4]diazepin-5-yl)phenyl]-2-naphthalenesulfonamide;
(B156) N-[3-(2,4-dioxo-1,2,3,4-tetrahydronaphtho[1,2-b][1,4]diazepin-5-yl)phenyl]-1-naphthalenesulfonamide;
(B157) N-[4-(2,4-dioxo-1,2,3,4-tetrahydronaphtho[1,2-b][1,4]diazepin-5-yl)phenyl]cyclohexanesulfonamide;
(B158) N-[4-(2,4-dioxo-1,2,3,4-tetrahydronaphtho[1,2-b][1,4]diazepin-5-yl)phenyl]-3-pyridinesulfonamide hydrochloride;
(B159) N-[4-(2,4-dioxo-1,2,3,4-tetrahydronaphtho[1,2-b][1,4]diazepin-5-yl)phenyl]-4-isopropylbenzenesulfonamide;
(B160) N-[4-(2,4-dioxo-1,2,3,4-tetrahydronaphtho[1,2-b][1,4]diazepin-5-yl)phenyl]phenylmethanesulfonamide;
(B161) N-[4-(2,4-dioxo-1,2,3,4-tetrahydronaphtho[1,2-b][1,4]diazepin-5-yl)phenyl]-2-thiophenesulfonamide;
(B162) N-[4-(2,4-dioxo-1,2,3,4-tetrahydronaphtho[1,2-b][1,4]diazepin-5-yl)phenyl]-2-naphthalenesulfonamide;
(B163) 4-(2,4-dioxo-1,2,3,4,8,9,10,11-octahydronaphtho[1,2-b][1,4]diazepin-5-yl)phenyl 3-bromobenzenesulfonate;
(B164) N-benzyl-N-[4-(1-benzyl-2,4-dioxo-1,2,3,4-tetrahydronaphtho[1,2-b][1,4]diazepin-5-yl)phenyl]-2-nitrobenzenesulfonamide;
(B165) N-benzyl-N-[4-(2,4-dioxo-1,2,3,4-tetrahydronaphtho[1,2-b][1,4]diazepin-5-yl)phenyl]-2-nitrobenzenesulfonamide;
(B166) 3-bromo-N-[4-(2,4-dioxo-1,2,3,4-tetrahydronaphtho[1,2-b][1,4]diazepin-5-yl)phenyl]-N-methylbenzenesulfonamide;
(B167) N-[4-(2,4-dioxo-1,2,3,4-tetrahydronaphtho-[1,2-b][1,4]-diazepin-5-yl)phenyl]-N-methyl-2-nitrobenzenesulfonamide;
(B168) N-[4-(2,4-dioxo-1,2,3,4-tetrahydronaphtho-[1,2-b][1,4]-diazepin-5-yl)phenyl]-N-(2-hydroxyethyl)-2-nitrobenzenesulfonamide;
(B169) N-[4-(7-chloro-2,4-dioxo-2,3,4,5-tetrahydro-1H-benzo[b][1,4]diazepin-1-yl)phenyl]benzenesulfonamide;
(B170) N-[4-(7-bromo-2,4-dioxo-2,3,4,5-tetrahydro-1H-benzo[b][1,4]diazepin-1-yl)phenyl]benzenesulfonamide;
(B171) N-[4-[(2,4-dioxo-7-(trifluoromethyl)-2,3,4,5-tetrahydro-1H-benzo[b][1,4]diazepin-1-yl)]phenyl]benzenesulfonamide;
(B172) N-[4-(2,4-dioxo-2,3,4,5-tetrahydro-1H-benzo[b][1,4]diazepin-1-yl)phenyl]benzenesulfonamide;
(B173) 1-(2-chlorophenyl)-N-[4-(2,4-dioxo-1,2,3,4-tetrahydronaphtho[1,2-b][1,4]diazepin-5-yl)phenyl]methanesulfonamide;
(B174) 1-(3-bromophenyl)-N-[4-(2,4-dioxo-1,2,3,4-tetrahydronaphtho[1,2-b][1,4]diazepin-5-yl)phenyl]methanesulfonamide;
(B175) N-[4-(2,4-dioxo-1,2,3,4-tetrahydronaphtho-[1,2-b][1,4]-diazepin-5-yl)phenyl]-2-trifluoromethylbenzenesulfonamide;
(B176) N-[4-(7-bromo-6-methyl-2,4-dioxo-2,3,4,5-tetrahydro-1H-benzo[b][1,4]diazepin-1-yl)phenyl]benzenesulfonamide;
(B177) 1-(2-chlorophenyl)-N-[4-(2,4-dioxo-1,2,3,4,8,9,10,11-octahydronaphtho[1,2-b][1,4]diazepin-5-yl)phenyl]methanesulfonamide;
(B178) 3-bromo-N-[4-(2,4-dioxo-1,2,3,4,8,9,10,11-octahydronaphtho[1,2-b][1,4]diazepin-5-yl)phenyl]benzenesulfonamide;
(B179) N-[4-(2,4-dioxo-1,2,3,4,8,9,10,11-octahydronaphtho[1,2-b][1,4]diazepin-5-yl)phenyl]-3-methoxybenzenesulfonamide;
(B180) 1-(2-bromophenyl)-N-[4-(2,4-dioxo-1,2,3,4-tetrahydronaphtho[1,2-b][1,4]diazepin-5-yl)phenyl]methanesulfonamide;
(B181) N-[4-(2,4-dioxo-1,2,3,4-tetrahydronaphtho[1,2-b][1,4]diazepin-5-yl)phenyl]-1-(2-methylphenyl)methanesulfonamide;
(B182) N-[4-(2,4-dioxo-1,2,3,4-tetrahydronaphtho[1,2-b][1,4]diazepin-5-yl)phenyl]-1-(2-nitrophenyl)methanesulfonamide;
(B183) N-[4-(2,4-dioxo-1,2,3,4-tetrahydronaphtho[1,2-b][1,4]diazepin-5-yl)phenyl]-2-phenylethanesulfonamide;
(B184) 1-(2,3-dichlorophenyl)-N-[4-(2,4-dioxo-1,2,3,4,8,9,10,11-octahydronaphtho[1,2-b][1,4]diazepin-5-yl)phenyl]methanesulfonamide;
(B185) 1-(2-chlorophenyl)-N-[4-(2,4-dioxo-7-methoxy-1H-benzo[1,2-b][1,4]diazepin-1-yl)phenyl]methanesulfonamide;
(B186) 1-(2-chlorophenyl)-N-[4-(2,4-dioxo-7-hydroxy-1H-benzo[1,2-b][1,4]diazepin-1-yl)phenyl]methanesulfonamide;
(B187) 1-(4-chlorophenyl)-N-[4-(2,4-dioxo-1,2,3,4-tetrahydronaphtho[1,2-b][1,4]diazepin-5-yl)phenyl]methanesulfonamide;
(B188) 1-(2-chlorophenyl)-N-[4-(2,4-dioxo-1,2,3,4-tetrahydronaphtho[1,2-b][1,4]diazepin-5-yl)benzyl]methanesulfonamide;
(B189) 1-(2-chlorophenyl)-N-[4-(2,4-dioxo-1,2,3,4-tetrahydronaphtho[1,2-b][1,4]diazepin-5-yl)-2-methoxyphenyl]methanesulfonamide;
(B190) 1-(2-chlorophenyl)-N-[4-(2,4-dioxo-1,2,3,4-tetrahydronaphtho[1,2-b][1,4]diazepin-5-yl)-2-hydroxyphenyl]methanesulfonamide;
(B191) 1-(2,6-dichlorophenyl)-N-[4-(2,4-dioxo-1,2,3,4,8,9,10,11-octahydronaphtho[1,2-b][1,4]diazepin-5-yl)phenyl]methanesulfonamide;
(B192) 1-(2-chlorophenyl)-N-[4-(2,4-dioxo-6-methyl-1H-benzo[1,2-b][1,4]diazepin-1-yl)phenyl]methanesulfonamide;
(B193) 1-(2-chlorophenyl)-N-[3-(2,4-dioxy-1,2,3,4-tetrahydronaphtho[1,2-b][1,4]diazepin-5-yl)propyl]methanesulfonamide;
(B194) 1-(2-chlorophenyl)-N-[2-(2,4-dioxo-1,2,3,4-tetrahydronaphtho[1,2-b][1,4]diazepin-5-yl)ethyl]methanesulfonamide;
(B195) N-[4-(2,4-dioxo-1,2,3,4,8,9,10,11-octahydronaphtho[1,2-b][1,4]diazepin-5-yl)phenyl]-1-(2-iodophenyl)methanesulfonamide;
(B196) 1-(2-chlorophenyl)-N-[4-(2,4-dioxo-1,2,3,4,8,9,10,11-octahydronaphtho[1,2-b][1,4]diazepin-5-yl)phenyl]-N-methylmethanesulfonamide;
(B197) 1-(2-chlorophenyl)-N-[4-(2-oxo-2,3-dihydro-1H-naphtho[1,2-e][1,4]diazepin-5-yl)phenyl]methanesulfonamide;
(B198) 1-[2-(trifluoromethyl)phenyl]-N-[4-(2,4-dioxo-1,2,3,4-tetrahydronaphtho[1,2-b][1,4]diazepin-5-yl)phenyl]phenyl-N-methylmethanesulfonamide;
(B199) 1-(2-ethylphenyl)-N-[4-(2,4-dioxo-1,2,3,4-tetrahydronaphtho[1,2-b][1,4]diazepin-5-yl)phenyl]phenyl-N-methylmethanesulfonamide;
(B200) 1-(2,3-dimethylphenyl)-N-[4-(2,4-dioxo-1,2,3,4-tetrahydronaphtho[1,2-b][1,4]diazepin-5-yl)phenyl]phenyl-N-methylmethanesulfonamide;
(B201) 2-(2-chlorophenyl)-N-[4-(2,4-dioxo-1,2,3,4-tetrahydronaphtho[1,2-b][1,4]diazepin-5-yl)phenyl]phenyl-N-methylethanesulfonamide;
(B202) 1-(2-nitrophenyl)-N-[4-(2,4-dioxo-1,2,3,4-tetrahydronaphtho[1,2-b][1,4]diazepin-5-yl)phenyl]phenyl-N-methylmethanesulfonamide;
(B203) 1-(2-aminophenyl)-N-[4-(2,4-dioxo-1,2,3,4-tetrahydronaphtho[1,2-b][1,4]diazepin-5-yl)phenyl]phenyl-N-methylmethanesulfonamide;
(B204) 1-(2-dimethylaminophenyl)-N-[4-(2,4-dioxo-1,2,3,4-tetrahydronaphtho[1,2-b][1,4]diazepin-5-yl)phenyl]phenyl-N-methylmethanesulfonamide;
(B205) 5-[4-[(pyridin-4-yl)carbonylamino]phenyl]-1H-naphtho[1,2-b][1,4]diazepine-2,4(3H,5H)-dione hydrochloride;
(B206) 5-[4-[2-[(pyridin-3-yl)oxy]acetylamino]phenyl]-1H-naphtho[1,2-b][1,4]diazepine-2,4(3H,5H)-dione hydrochloride;
(B207) 5-[4-[(pyridin-3-yl)carbonylamino]phenyl]-1H-naphtho[1,2-b][1,4]diazepine-2,4(3H,5H)-dione hydrochloride;
(B208) 5-[4-[(2-methylpyridin-3-yl)carbonylamino]phenyl]-1H-naphtho[1,2-b][1,4]diazepine-2,4(3H,5H)-dione hydrochloride;
(B209) 5-[4-[(2-chloropyridin-3-yl)carbonylamino]phenyl]-1H-naphtho[1,2-b][1,4]diazepine-2,4(3H,5H)-dione;
(B210) 5-[4-[2-[(pyridin-2-yl)oxy]acetylamino]phenyl]-1H-naphtho[1,2-b][1,4]diazepine-2,4(3H,5H)-dione;
(B211) 5-[4-[[4-(trifluoromethyl)pyridin-3-yl]carbonylamino]phenyl]-1H-naphtho[1,2-b][1,4]diazepine-2,4(3H,5H)-dione;
(B212) 5-[4-[(2-chloropyridin-3-yl)carbonylamino]phenyl]-1H-[1,4]diazepino[2,3-f]isoquinoline-2,4(3H,5H)-dione;
(B213) 5-[4-[(2-chloropyridin-3-yl)carbonylamino]phenyl]-8 , 9, 10, 11-tetrahydro-1H-[1,4]diazepino[2,3-f]isoquinoline-2,4(3H,5H)-dione; and
(B214) 5-[4-[(2-isopropylbenzoyl)amino]phenyl]-1H-naphtho[1,2-b][1,4]diazepine-2,4(3H,5H)-dione.

8. The pharmaceutical composition according to any one of claims 1 to 4, wherein the compound is a compound selected from the group consisting of the following (C1) to (C297):
(C1) N-[4-(3-chloro-5-cyanophenoxy)-3-sulfamoylphenyl]-2-(2-chlorophenyl)acetamide;
(C2) 2-(2-chlorophenyl)-N-4-[3-(dimethylamino)phenoxy]-3-sulfamoylphenylacetamide;
(C3) 2-(2-chlorophenyl)-N-4-[(2-chloropyridin-4-yl)oxy]-3-sulfamoylphenylacetamide;
(C4) 2-(2-chlorophenyl)-N-[4-(3-isopropylphenoxy)-3-sulfamoylphenyl]acetamide;
(C5) 2-(2-chlorophenyl)-N-3-sulfamoyl-4-[3-(trifluoromethyl)phenoxy]phenylacetamide;
(C6) 2-(2-chlorophenyl)-N-3-sulfamoyl-4-[3-(trifluoromethoxy)phenoxy]phenylacetamide;
(C7) N-[4-(3-acetylphenoxy)-3-sulfamoylphenyl]-2-(2-chlorophenyl) acetamide;
(C8) N-[4-(1,3-benzodioxol-5-yloxy)-3-sulfamoylphenyl]-2-(2-chlorophenyl)acetamide;
(C9) N-[4-(3-acetamidophenoxy)-3-sulfamoylphenyl]-2-(2-chlorophenyl) acetamide;
(C10) 2-(2-chlorophenyl)-N-[4-(2-fluorophenoxy)-3-sulfamoylphenyl]acetamide;
(C11) 2-(2-chlorophenyl)-N-[4-(3-fluorophenoxy)-3-sulfamoylphenyl]acetamide;
(C12) 2-(2-chlorophenyl)-N-[4-(4-fluorophenoxy)-3-sulfamoylphenyl]acetamide;
(C13) 2-(2-chlorophenyl)-N-[4-(pyridin-2-yloxy)-3-sulfamoylphenyl]acetamide;
(C14) 2-(2-chlorophenyl)-N-(4-phenoxy-3-sulfamoylphenyl)acetamide;
(C15) 2-(2-chlorophenyl)-N-[4-(3-cyanophenoxy)-3-sulfamoylphenyl]acetamide;
(C16) 2-(2-chlorophenyl)-N-4-[3-(methylsulfonyl)phenoxy]-3-sulfamoylphenylacetamide;
(C17) 3-(4-[(2-chlorophenyl)acetyl]amino-2-sulfamoylphenoxy)benzamide;
(C18) 2-(2-chlorophenyl)-N-[4-(3-methylphenoxy)-3-sulfamoylphenyl]acetamide;
(C19) 2-(2-chlorophenyl)-N-[4-(pyrimidin-5-yloxy)-3-sulfamoylphenyl]acetamide;
(C20) 2-(2-chlorophenyl)-N-3-sulfamoyl-4-[3-(4H-1,2,4-triazol-4-yl)phenoxy]phenylacetamide;
(C21) 2-(2-chlorophenyl)-N-3-sulfamoyl-4-[3-(1H-tetrazol-5-yl)phenoxy]phenylacetamide;
(C22) 2-(2-chlorophenyl)-N-[4-(3-methoxyphenoxy)-3-sulfamoylphenyl]acetamide;
(C23) 2-(2-chlorophenyl)-N-[4-(4-methoxyphenoxy)-3-sulfamoylphenyl]acetamide;
(C24) 2-(2-chlorophenyl)-N-4-[3-(difluoromethoxy)phenoxy]-3-sulfamoylphenylacetamide;
(C25) 2-(2-chlorophenyl)-N-[4-(3,4-dicyanophenoxy)-3-sulfamoylphenyl]acetamide;
(C26) 2-(2-chlorophenyl)-N-4-[3-(morpholin-4-yl)phenoxy]-3-sulfamoylphenylacetamide;
(C27) 2-(2-chlorophenyl)-N-[4-(3-4-[(2-chlorophenyl)acetyl]piperazin-1-ylphenoxy)-3-sulfamoylphenyl]acetamide;
(C28) 2-(2-chlorophenyl)-N-[4-(pyridin-3-yloxy)-3-sulfamoylphenyl]acetamide;
(C29) 2-(2-chlorophenyl)-N-4-[(5-chloropyridin-3-yl)oxy]-3-sulfamoylphenylacetamide;
(C30) 2-(2-chlorophenyl)-N-[4-(4-cyanophenoxy)-3-sulfamoylphenyl]acetamide;
(C31) 2-(2-chlorophenyl)-N-4-[3-(difluoromethyl)phenoxy]-3-sulfamoylphenylacetamide;
(C32) 2-(2-chlorophenyl)-N-[4-(2-methoxyphenoxy)-3-sulfamoylphenyl]acetamide;
(C33) 2-(2-chlorophenyl)-N-[4-(3,5-dicyanophenoxy)-3-sulfamoylphenyl]acetamide;
(C34) 2-(2-chlorophenyl)-N-[4-(5-cyano-2-methoxyphenoxy)-3-sulfamoylphenyl]acetamide;
(C35) 2-(2-chlorophenyl)-N-4-[(2,5-dichloropyridin-3-yl)oxy]-3-sulfamoylphenylacetamide;
(C36) 2-(2-chlorophenyl)-N-4-[(5,6-dichloropyridin-3-yl)oxy]-3-sulfamoylphenylacetamide;
(C37) 3-(4-[(2-chlorophenyl)acetyl]amino-2-sulfamoylphenoxy)-N-cyclopropylbenzamide;
(C38) 2-(2-chlorophenyl)-N-4-[(3-chloropyridin-2-yl)oxy]-3-sulfamoylphenylacetamide;
(C39) 2-(2-chlorophenyl)-N-4-[(4-chloropyridin-2-yl)oxy]-3-sulfamoylphenylacetamide;
(C40) 2-(2-chlorophenyl)-N-4-[(6-chloropyridin-2-yl)oxy]-3-sulfamoylphenylacetamide;
(C41) 2-(2-chlorophenyl)-N-4-[3-(1-methyl-4,5-dihydro-1H-imidazol-2-yl)phenoxy]-3-sulfamoylphenylacetamide;
(C42) 2-(2-chlorophenyl)-N-4-[4-(1H-imidazol-1-yl)phenoxy]-3-sulfamoylphenylacetamide;
(C43) 2-(2-chlorophenyl)-N-4-[4-(2-oxopyrrolidin-1-yl)phenoxy]-3-sulfamoylphenylacetamide;
(C44) 2-(2-chlorophenyl)-N-4-[4-(morpholin-4-yl)phenoxy]-3-sulfamoylphenylacetamide;
(C45) 2-(2-chlorophenyl)-N-[4-(5-cyano-2-methylphenoxy)-3-sulfamoylphenyl]acetamide;
(C46) 2-(2-chlorophenyl)-N-[4-(3-cyano-2-methylphenoxy)-3-sulfamoylphenyl]acetamide;
(C47) 2-(2-chlorophenyl)-N-[4-(3-cyano-4-fluorophenoxy)-3-sulfamoylphenyl]acetamide;
(C48) N-4-[(5-chloro-2-cyanopyridin-3-yl)oxy]-3-sulfamoylphenyl-2-(2-chlorophenyl)acetamide;
(C49) 2-(2-chlorophenyl)-N-4-[3-(piperidin-1-yl)phenoxy]-3-sulfamoylphenylacetamide;
(C50) 2-(2-chlorophenyl)-N-4-[3-(2-oxopyrrolidin-1-yl)phenoxy]-3-sulfamoylphenylacetamide;
(C51) 2-(2-chlorophenyl)-N-4-[3-(2-oxo-1,3-oxazolidin-3-yl)phenoxy]-3-sulfamoylphenylacetamide;
(C52) 2-(2-chlorophenyl)-N-4-[3-(morpholin-4-ylcarbonyl)phenoxy]-3-sulfamoylphenylacetamide;
(C53) 2-(2-chlorophenyl)-N-4-[(4-methyltetrahydro-2H-pyran-4-yl)methoxy]-3-sulfamoylphenylacetamide;
(C54) 2-(2-chlorophenyl)-N-4-[(4-fluorotetrahydro-2H-pyran-4-yl)methoxy]-3-sulfamoylphenylacetamide;
(C55) 2-(2-chlorophenyl)-N-4-[(4-cyanotetrahydro-2H-pyran-4-yl)methoxy]-3-sulfamoylphenylacetamide;
(C56) 2-(2-chlorophenyl)-N-(3-sulfamoyl-4-[2-(trifluoromethyl)pyrimidin-5-yl]oxyphenyl)acetamide;
(C57) 2-(2-chlorophenyl)-N-4-[(2-isopropylpyrimidin-5-yl)oxy]-3-sulfamoylphenylacetamide;
(C58) 2-(2-chlorophenyl)-N-4-[(2-cyclopropyl-4-methylpyrimidin-5-yl)oxy]-3-sulfamoylphenylacetamide;
(C59) N-[4-(3-bromophenoxy)-3-sulfamoylphenyl]-2-(2-chlorophenyl) acetamide;
(C60) N-[4-(4-bromophenoxy)-3-sulfamoylphenyl]-2-(2-chlorophenyl) acetamide;
(C61) 2-(2-chlorophenyl)-N-4-[3-(2-methyl-1,3-thiazol-4-yl)phenoxy]-3-sulfamoylphenylacetamide;
(C62) 2-(2-chlorophenyl)-N-4-[4-(5-oxopyrrolidin-2-yl)phenoxy]-3-sulfamoylphenylacetamide;
(C63) 2-(2-chlorophenyl)-N-4-[4-(2-oxo-1,3-oxazolidin-3-yl)phenoxy]-3-sulfamoylphenylacetamide;
(C64) 2-(2-chlorophenyl)-N-3-sulfamoyl-4-[4-(1,3-thiazol-2-yl)phenoxy]phenylacetamide;
(C65) N-[4-(2-chlorophenoxy)-3-sulfamoylphenyl]-2-(2-chlorophenyl) acetamide;
(C66) N-[4-(4-chlorophenoxy)-3-sulfamoylphenyl]-2-(2-chlorophenyl) acetamide;
(C67) 2-(2-chlorophenyl)-N-4-[3-(piperidin-1-ylcarbonyl)phenoxy]-3-sulfamoylphenylacetamide;
(C68) 2-(2-chlorophenyl)-N-3-sulfamoyl-4-[4-(tetrahydrofuran-3-yl)phenoxy]phenylacetamide;
(C69) 2-(2-chlorophenyl)-N-[4-(3-cyano-5-fluorophenoxy)-3-sulfamoylphenyl]acetamide;
(C70) N-[4-(2-methoxyphenoxy)-3-sulfamoylphenyl]-2-phenylacetamide;
(C71) N-[4-(2-methoxyphenoxy)-3-sulfamoylphenyl]-2-[4-(trifluoromethyl)phenyl]acetamide;
(C72) N-3-sulfamoyl-4-[2-(trifluoromethoxy)phenoxy]phenyl-2-[4-(trifluoromethyl)phenyl]acetamide;
(C73) N-[4-(2-chlorophenoxy)-3-sulfamoylphenyl]-2-[4-(trifluoromethyl)phenyl]acetamide;
(C74) 2-phenyl-N-3-sulfamoyl-4-[2-(trifluoromethoxy)phenoxy]phenylacetamide;
(C75) 2-(2-chlorophenyl)-N-4-[(2-oxo-1,2-dihydropyridin-3-yl)oxy]-3-sulfamoylphenylacetamide;
(C76) N-[4-(2-chlorophenoxy)-3-sulfamoylphenyl]-2-phenylacetamide;
(C77) N-[4-(4-chlorophenoxy)-3-sulfamoylphenyl]-2-phenylacetamide;
(C78) N-4-[(5-chloropyridin-3-yl)oxy]-3-sulfamoylphenyl-2-phenylacetamide;
(C79) 2-(2-chlorophenyl)-N-4-[(2-chloropyrimidin-5-yl)oxy]-3-sulfamoylphenylacetamide;
(C80) 2-(2-chlorophenyl)-N-4-[(5-fluoropyridin-3-yl)oxy]-3-sulfamoylphenylacetamide;
(C81) 2-(2-chlorophenyl)-N-4-[(6-chloropyridin-3-yl)oxy]-3-sulfamoylphenylacetamide;
(C82) N-[2-chloro-4-(3-chlorophenoxy)-5-sulfamoylphenyl]-2-(2-chlorophenyl)acetamide;
(C83) N-[2-chloro-4-(3-chlorophenoxy)-5-sulfamoylphenyl]-2-(2-chloro-3-fluorophenyl)acetamide;
(C84) N-[4-(3-chlorophenoxy)-3-sulfamoylphenyl]-2-(3-fluorophenyl) acetamide;
(C85) N-[4-(3-chlorophenoxy)-3-sulfamoylphenyl]-2-(4-fluorophenyl) acetamide;
(C86) N-[4-(3-chlorophenoxy)-3-sulfamoylphenyl]-2-[2-(trifluoromethyl)phenyl]acetamide;
(C87) N-[4-(3-chlorophenoxy)-3-sulfamoylphenyl]-2-(2-isopropylphenyl) acetamide;
(C88) N-[4-(3-chlorophenoxy)-3-sulfamoylphenyl]-2-(2-ethoxyphenyl) acetamide;
(C89) N-[4-(3-chlorophenoxy)-3-sulfamoylphenyl]-2-[2-(difluoromethyl)phenyl]acetamide;
(C90) N-[4-(3-chlorophenoxy)-3-sulfamoylphenyl]-2-2-[(trifluoromethyl)sulfanyl]phenylacetamide;
(C91) 2-(2-bromophenyl)-N-[4-(3-chlorophenoxy)-3-sulfamoylphenyl]acetamide;
(C92) N-[4-(3-chlorophenoxy)-3-sulfamoylphenyl]-2-(4-methylpyridin-3-yl)acetamide;
(C93) N-[4-(3-chlorophenoxy)-3-sulfamoylphenyl]-2-(2-chloropyridin-3-yl)acetamide;
(C94) N-[4-(3-chlorophenoxy)-3-sulfamoylphenyl]-2-(2-chlorophenyl)-2,2-difluoroacetamide;
(C95) 2-(2-chloro-4-methylphenyl)-N-[4-(3-chlorophenoxy)-3-sulfamoylphenyl]acetamide;
(C96) 2-(2-chloro-6-methylphenyl)-N-[4-(3-chlorophenoxy)-3-sulfamoylphenyl]acetamide;
(C97) 2-(2-chloro-5-methylphenyl)-N-[4-(3-chlorophenoxy)-3-sulfamoylphenyl]acetamide;
(C98) 2-(2-chloro-3-fluorophenyl)-N-[4-(3-chlorophenoxy)-3-sulfamoylphenyl]acetamide;
(C99) 2-(2-chloro-5-fluorophenyl)-N-[4-(3-chlorophenoxy)-3-sulfamoylphenyl]acetamide;
(C100) 2-(2-chloro-6-fluorophenyl)-N-[4-(3-chlorophenoxy)-3-sulfamoylphenyl]acetamide;
(C101) 2-(2-chloro-6-methoxyphenyl)-N-[4-(3-chlorophenoxy)-3-sulfamoylphenyl]acetamide;
(C102) 2-(2-chloro-5-methoxyphenyl)-N-[4-(3-chlorophenoxy)-3-sulfamoylphenyl]acetamide;
(C103) N-[4-(3-chlorophenoxy)-3-sulfamoylphenyl]-2-(2,3-dichlorophenyl) acetamide;
(C104) N-[4-(3-chlorophenoxy)-3-sulfamoylphenyl]-2-(2,6-dichlorophenyl) acetamide;
(C105) N-[4-(3-chlorophenoxy)-3-sulfamoylphenyl]-2-[2-(trifluoromethoxy)phenyl]acetamide;
(C106) N-[4-(3-chlorophenoxy)-3-sulfamoylphenyl]-2,2-difluoro-2-phenylacetamide;
(C107) N-[4-(3-chlorophenoxy)-3-sulfamoylphenyl]-2-[2-chloro-3-(trifluoromethyl)phenyl]acetamide;
(C108) N-[4-(3-chlorophenoxy)-3-sulfamoylphenyl]-2-[2-chloro-6-(trifluoromethyl)phenyl]acetamide;
(C109) N-[4-(3-chlorophenoxy)-3-sulfamoylphenyl]-2-[2-chloro-5-(trifluoromethyl)phenyl]acetamide;
(C110) N-[4-(3-chlorophenoxy)-3-sulfamoylphenyl]-2-(2,4-dichlorophenyl) acetamide;
(C111) N-[4-(3-chlorophenoxy)-3-sulfamoylphenyl]-2-(4,6-dichloropyridin-3-yl)acetamide;
(C112) N-[4-(3-chlorophenoxy)-3-sulfamoylphenyl]-2-(3-chloropyridin-2-yl)acetamide;
(C113) N-[4-(3-chlorophenoxy)-3-sulfamoylphenyl]-2-[2-(difluoromethoxy)phenyl]acetamide;
(C114) N-[4-(3-chlorophenoxy)-3-sulfamoylphenyl]-2-(2,5-dichlorophenyl)acetamide;
(C115) 2-[6-chloro-2,3-difluoro-4-(trifluoromethyl)phenyl]-N-[4-(3-chlorophenoxy)-3-sulfamoylphenyl]acetamide;
(C116) N-[4-(3-chlorophenoxy)-3-sulfamoylphenyl]-2-[4-(trifluoromethyl)phenyl]acetamide;
(C117) 2-(5-bromo-2-chlorophenyl)-N-[4-(3-chlorophenoxy)-3-sulfamoylphenyl]acetamide;
(C118) 2-(4-bromo-2-chloro-5-methylphenyl)-N-[4-(3-chlorophenoxy)-3-sulfamoylphenyl]acetamide;
(C119) N-[4-(3-chlorophenoxy)-3-sulfamoylphenyl]-2-(3-chloropyridin-4-yl)acetamide;
(C120) 2-(2-chloro-6-fluoro-3-methylphenyl)-N-[4-(3-chlorophenoxy)-3-sulfamoylphenyl]acetamide;
(C121) 2-(6-chloro-2-fluoro-3-methylphenyl)-N-[4-(3-chlorophenoxy)-3-sulfamoylphenyl]acetamide;
(C122) 2-(2-chloro-3,6-difluorophenyl)-N-[4-(3-chlorophenoxy)-3-sulfamoylphenyl]acetamide;
(C123) 2-(2-chloro-4,5-difluorophenyl)-N-[4-(3-chlorophenoxy)-3-sulfamoylphenyl]acetamide;
(C124) N-[4-(3-chlorophenoxy)-3-sulfamoylphenyl]-2-(2,3-dichloro-6-fluorophenyl)acetamide;
(C125) N-[4-(3-chlorophenoxy)-3-sulfamoylphenyl]-2-(2,3,6-trichlorophenyl)acetamide;
(C126) N-[4-(3-chlorophenoxy)-3-sulfamoylphenyl]-2-(2,6-dichloro-4-methylphenyl)acetamide;
(C127) N-[4-(3-chlorophenoxy)-3-sulfamoylphenyl]-2-[2,3-dichloro-6-(trifluoromethyl)phenyl]acetamide;
(C128) N-[4-(3-chlorophenoxy)-3-sulfamoylphenyl]-2-(2,6-dichloro-3-methylphenyl)acetamide;
(C129) N-[4-(3-chlorophenoxy)-3-sulfamoylphenyl]-2-(2,6-dichloro-3-cyclopropylphenyl)acetamide;
(C130) N-[4-(3-chlorophenoxy)-3-sulfamoylphenyl]-2-[2,6-dichloro-3-(trifluoromethyl)phenyl]acetamide;
(C131) 2-(3-bromo-2,6-dichlorophenyl)-N-[4-(3-chlorophenoxy)-3-sulfamoylphenyl]acetamide;
(C132) 2-(3-bromo-2-chloro-6-methylphenyl)-N-[4-(3-chlorophenoxy)-3-sulfamoylphenyl]acetamide;
(C133) 2-(3-bromo-6-chloro-2-methylphenyl)-N-[4-(3-chlorophenoxy)-3-sulfamoylphenyl]acetamide;
(C134) N-[4-(3-chlorophenoxy)-3-sulfamoylphenyl]-2-[2-chloro-5-(1,1,2,2-tetrafluoroethoxy)phenyl]acetamide;
(C135) N-[4-(3-chlorophenoxy)-3-sulfamoylphenyl]-2-[2-chloro-4-(trifluoromethyl)phenyl]acetamide;
(C136) 2-(2-chlorophenyl)-N-(4-[3-(methylsulfonyl)benzyl]oxy-3-sulfamoylphenyl)acetamide;
(C137) 2-(2-chlorophenyl)-N-4-[(2-fluorobenzyl)oxy]-3-sulfamoylphenylacetamide;
(C138) 2-(2-chlorophenyl)-N-4-[(4-cyanobenzyl)oxy]-3-sulfamoylphenylacetamide;
(C139) N-4-[(3-chlorobenzyl)oxy]-3-sulfamoylphenyl-2-(2-chlorophenyl) acetamide;
(C140) 2-(2-chlorophenyl)-N-4-[(3-methoxybenzyl)oxy]-3-sulfamoylphenylacetamide;
(C141) N-[4-(benzyloxy)-3-sulfamoylphenyl]-2-(2-chlorophenyl) acetamide;
(C142) 2-(2-chlorophenyl)-N-4-[(3-cyanobenzyl)oxy]-3-sulfamoylphenylacetamide;
(C143) 2-(2-chlorophenyl)-N-4-[(4-fluorobenzyl)oxy]-3-sulfamoylphenylacetamide;
(C144) N-4-[(2-chlorobenzyl)oxy]-3-sulfamoylphenyl-2-(2-chlorophenyl) acetamide;
(C145) 2-(2-chlorophenyl)-N-4-[(2-cyanobenzyl)oxy]-3-sulfamoylphenylacetamide;
(C146) N-[4-(benzyloxy)-3-sulfamoylphenyl]-2-phenylacetamide;
(C147) 2-(2-chlorophenyl)-N-(4-[4-(methylsulfonyl)benzyl]oxy-3-sulfamoylphenyl)acetamide;
(C148) 2-(2-chlorophenyl)-N-[4-(1-phenylethoxy)-3-sulfamoylphenyl]acetamide;
(C149) 2-(2-chlorophenyl)-N-[4-(1-phenylethoxy)-3-sulfamoylphenyl]acetamide;
(C150) 2-(2-chlorophenyl)-N-[4-(pyridin-3-ylmethoxy)-3-sulfamoylphenyl]acetamide;
(C151) 2-(2-chlorophenyl)-N-[4-(pyridin-2-ylmethoxy)-3-sulfamoylphenyl]acetamide;
(C152) 2-(2-chlorophenyl)-N-[4-(pyridin-4-ylmethoxy)-3-sulfamoylphenyl]acetamide;
(C153) N-[4-(pyridin-2-ylmethoxy)-3-sulfamoylphenyl]-2-[4-(trifluoromethyl)phenyl]acetamide;
(C154) 2-(2-chlorophenyl)-N-[4-(pyrimidin-4-ylmethoxy)-3-sulfamoylphenyl]acetamide;
(C155) 2-(2-chlorophenyl)-N-[4-(pyrimidin-2-ylmethoxy)-3-sulfamoylphenyl]acetamide;
(C156) 2-(2-chlorophenyl)-N-[4-(2-phenylethoxy)-3-sulfamoylphenyl]acetamide;
(C157) 2-(2-chlorophenyl)-N-4-[2-(3-chlorophenyl)ethoxy]-3-sulfamoylphenylacetamide;
(C158) 2-(2-chlorophenyl)-N-[4-(cyclobutylmethoxy)-3-sulfamoylphenyl]acetamide;
(C159) 2-(2-chlorophenyl)-N-[4-(oxetan-2-ylmethoxy)-3-sulfamoylphenyl]acetamide;
(C160) 2-(2-chlorophenyl)-N-[4-(oxetan-3-ylmethoxy)-3-sulfamoylphenyl]acetamide;
(C161) 2-(2-chlorophenyl)-N-[4-(cyclopentylmethoxy)-3-sulfamoylphenyl]acetamide;
(C162) 2-(2-chlorophenyl)-N-[3-sulfamoyl-4-(tetrahydrofuran-2-ylmethoxy)phenyl]acetamide;
(C163) 2-(2-chlorophenyl)-N-[3-sulfamoyl-4-(tetrahydrofuran-3-ylmethoxy)phenyl]acetamide;
(C164) 2-(2-chloro-5-fluorophenyl)-N-[3-sulfamoyl-4-(tetrahydro-2H-pyran-4-ylmethoxy)phenyl]acetamide;
(C165) 2-(2-chlorophenyl)-N-[3-sulfamoyl-4-(tetrahydro-2H-pyran-4-ylmethoxy)phenyl]acetamide;
(C166) 2-(2-chlorophenyl)-N-[3-sulfamoyl-4-(tetrahydro-2H-pyran-3-ylmethoxy)phenyl]acetamide;
(C167) 2-(2-chloro-6-fluorophenyl)-N-[3-sulfamoyl-4-(tetrahydro-2H-pyran-4-ylmethoxy)phenyl]acetamide;
(C168) 2-(2-chloro-3-fluorophenyl)-N-[3-sulfamoyl-4-(tetrahydro-2H-pyran-4-ylmethoxy)phenyl]acetamide;
(C169) 2-(2-chlorophenyl)-N-5-sulfamoyl-6-[3-(trifluoromethyl)phenoxy]pyridin-3-ylacetamide;
(C170) 2-phenyl-N-5-sulfamoyl-6-[3-(trifluoromethyl)phenoxy]pyridin-3-ylacetamide;
(C171) N-[4-(3-chlorophenoxy)-3-fluoro-5-sulfamoylphenyl]-2-phenylacetamide;
(C172) N-[4-(3-chlorophenoxy)-3-fluoro-5-sulfamoylphenyl]-2-(2-methylphenyl)acetamide;
(C173) N-[4-(3-chlorophenoxy)-3-fluoro-5-sulfamoylphenyl]-2-(3-methylphenyl)acetamide;
(C174) 2-(2-chlorophenyl)-N-4-[3-(3-oxomorpholin-4-yl)phenoxy]-3-sulfamoylphenylacetamide;
(C175) 2-(2-chlorophenyl)-N-4-[4-(3-oxomorpholin-4-yl)phenoxy]-3-sulfamoylphenylacetamide;
(C176) 2-(2-chlorophenyl)-N-4-[4-(2-oxopiperidin-1-yl)phenoxy]-3-sulfamoylphenylacetamide;
(C177) 2-(2-chlorophenyl)-N-4-[3-(2-oxopiperidin-1-yl)phenoxy]-3-sulfamoylphenylacetamide;
(C178) 2-(2-chlorophenyl)-N-4-[3-(prop-1-en-2-yl)phenoxy]-3-sulfamoylphenylacetamide;
(C179) 2-(2-chlorophenyl)-N-4-[2-(prop-1-en-2-yl)phenoxy]-3-sulfamoylphenylacetamide;
(C180) N-[4-(3-chlorophenoxy)-3-sulfamoylphenyl]-2-(4-methylphenyl) acetamide;
(C181) N-[4-(3-chlorophenoxy)-3-sulfamoylphenyl]-2-(4-chlorophenyl) acetamide;
(C182) N-[4-(3-chlorophenoxy)-3-sulfamoylphenyl]-2-(pyridin-3-yl)acetamide;
(C183) N-[4-(3-chlorophenoxy)-3-sulfamoylphenyl]-2-(2-methylphenyl) acetamide;
(C184) N-[4-(3-chlorophenoxy)-3-sulfamoylphenyl]-2-(3-methylphenyl) acetamide;
(C185) N-[4-(3-chlorophenoxy)-3-sulfamoylphenyl]-2-phenylpropanamide;
(C186) N-[4-(3-chlorophenoxy)-3-sulfamoylphenyl]-2-(pyridin-2-yl)acetamide;
(C187) N-[4-(3-chlorophenoxy)-3-sulfamoylphenyl]-2-(3-chlorophenyl) acetamide;
(C188) N-[4-(3-chlorophenoxy)-3-sulfamoylphenyl]-2-(2-chlorophenyl) acetamide;
(C189) N-[4-(3-chlorophenoxy)-3-sulfamoylphenyl]-2-(pyridin-4-yl)acetamide;
(C190) N-[4-(3-chlorophenoxy)-3-sulfamoylphenyl]-2-(6-methylpyridin-2-yl)acetamide;
(C191) N-[4-(3-chlorophenoxy)-3-sulfamoylphenyl]-2-(4-methoxyphenyl) acetamide;
(C192) N-[4-(3-chlorophenoxy)-3-sulfamoylphenyl]-2-(3-methoxyphenyl) acetamide;
(C193) N-[4-(3-chlorophenoxy)-3-sulfamoylphenyl]-2-(2-methoxyphenyl) acetamide;
(C194) N-[4-(3-chlorophenoxy)-3-sulfamoylphenyl]-2-(5-methylpyridin-2-yl)acetamide;
(C195) (2S)-N-[4-(3-chlorophenoxy)-3-sulfamoylphenyl]-2-phenylpropanamide;
(C196) (2R)-N-[4-(3-chlorophenoxy)-3-sulfamoylphenyl]-2-phenylpropanamide;
(C197) N-[4-(3-chlorophenoxy)-3-sulfamoylphenyl]-2-(2-chlorophenyl)propanamide;
(C198) 2-(2-[4-(3-chlorophenoxy)-3-sulfamoylphenyl]amino-2-oxoethyl)-N-(2-methoxyethyl)-N-methylbenzamide;
(C199) 2-(2-[4-(3-chlorophenoxy)-3-sulfamoylphenyl]amino-2-oxoethyl)-N,N-dimethylbenzamide;
(C200) N-[4-(cyclohexyloxy)-3-sulfamoylphenyl]-2-phenylacetamide;
(C201) 2-(2-chlorophenyl)-N-[4-(cyclohexyloxy)-3-sulfamoylphenyl]acetamide;
(C202) 3-(2-[4-(3-chlorophenoxy)-3-sulfamoylphenyl]amino-2-oxoethyl)-N-(2-methoxyethyl)benzamide;
(C203) 3-(2-[4-(3-chlorophenoxy)-3-sulfamoylphenyl]amino-2-oxoethyl)-N,N-dimethylbenzamide;
(C204) 3-(2-[4-(3-chlorophenoxy)-3-sulfamoylphenyl]amino-2-oxoethyl)-N-methylbenzamide;
(C205) N-[4-(cyclobutyloxy)-3-sulfamoylphenyl]-2-phenylacetamide;
(C206) 2-(2-chlorophenyl)-N-[4-(cyclobutyloxy)-3-sulfamoylphenyl]acetamide;
(C207) 2-phenyl-N-[3-sulfamoyl-4-(tetrahydro-2H-pyran-4-yloxy)phenyl]acetamide;
(C208) 2-(2-chlorophenyl)-N-[3-sulfamoyl-4-(tetrahydro-2H-pyran-4-yloxy)phenyl]acetamide;
(C209) 3-(2-[4-(3-chlorophenoxy)-3-sulfamoylphenyl]amino-2-oxoethyl)-N-(2-methoxyethyl)-N-methylbenzamide;
(C210) N-[4-(3-chlorophenoxy)-3-sulfamoylphenyl]-2-(5-chloropyridin-2-yl)acetamide;
(C211) N-[4-(3-chlorophenoxy)-3-sulfamoylphenyl]-2-[3-(2-methoxyethoxy)phenyl]acetamide;
(C212) N-[4-(3-chlorophenoxy)-3-sulfamoylphenyl]-2-[2-(2-methoxyethoxy)phenyl]acetamide;
(C213) N-[4-(3-chlorophenoxy)-3-sulfamoylphenyl]-2-[3-(2-hydroxyethoxy)phenyl]acetamide;
(C214) N-[4-(3-chlorophenoxy)-3-sulfamoylphenyl]-2-[2-(2-hydroxyethoxy)phenyl]acetamide;
(C215) N-[4-(3-chlorophenoxy)-3-sulfamoylphenyl]-2-(2-fluorophenyl) acetamide;
(C216) N-[4-(oxetan-3-yloxy)-3-sulfamoylphenyl]-2-phenylacetamide;
(C217) 2-(2-chlorophenyl)-N-[4-(oxetan-3-yloxy)-3-sulfamoylphenyl]acetamide;
(C218) N-[4-(cyclopentyloxy)-3-sulfamoylphenyl]-2-phenylacetamide;
(C219) 2-(2-chlorophenyl)-N-[4-(cyclopentyloxy)-3-sulfamoylphenyl]acetamide;
(C220) N-4-[(1-methylpiperidin-3-yl)oxy]-3-sulfamoylphenyl-2-phenylacetamide;
(C221) 2-(2-chlorophenyl)-N-4-[(1-methylpiperidin-3-yl)oxy]-3-sulfamoylphenylacetamide;
(C222) N-4-[(1-methylpyrrolidin-3-yl)oxy]-3-sulfamoylphenyl-2-phenylacetamide;
(C223) 2-(2-chlorophenyl)-N-4-[(1-methylpyrrolidin-3-yl)oxy]-3-sulfamoylphenylacetamide;
(C224) N-[4-(4-chlorophenoxy)-3-sulfamoylphenyl]-2-(4-fluorophenyl) acetamide;
(C225) N-[4-(3-chlorophenoxy)-3-sulfamoylphenyl]-2-(4-cyanophenyl)acetamide;
(C226) N-[4-(3-chlorophenoxy)-3-sulfamoylphenyl]-2-(2-cyanophenyl)acetamide;
(C227) N-[4-(3-chlorophenoxy)-3-sulfamoylphenyl]-2-(3-cyanophenyl)acetamide;
(C228) N-[4-(4-chlorophenoxy)-3-sulfamoylphenyl]-2-[4-(trifluoromethyl)phenyl]acetamide;
(C229) N-[4-(4-chlorophenoxy)-3-sulfamoylphenyl]-2-(4-chlorophenyl) acetamide;
(C230) N-[4-(4-chlorophenoxy)-3-sulfamoylphenyl]-2-(4-methoxyphenyl) acetamide;
(C231) N-[4-(4-chlorophenoxy)-3-sulfamoylphenyl]-2-(2-fluorophenyl) acetamide;
(C232) 2-(2-chloro-4-fluorophenyl)-N-[4-(4-chlorophenoxy)-3-sulfamoylphenyl]acetamide;
(C233) 2-(2-chlorophenyl)-N-4-[(1,1-dioxidetetrahydrothiophen-3-yl)oxy]-3-sulfamoylphenylacetamide;
(C234) 2-(2-chlorophenyl)-N-4-[(1-methyl-1H-pyrazol-4-yl)oxy]-3-sulfamoylphenylacetamide;
(C235) N-[4-(4-chlorophenoxy)-3-sulfamoylphenyl]-2-[4-(difluoromethyl)phenyl]acetamide;
(C236) 2-(2-chloro-4-methoxyphenyl)-N-[4-(4-chlorophenoxy)-3-sulfamoylphenyl]acetamide;
(C237) 2-(2-chlorophenyl)-N-4-[(1-methyl-1H-pyrazol-3-yl)oxy]-3-sulfamoylphenylacetamide;
(C238) 2-(2-chlorophenyl)-N-4-[(1-methyl-1H-pyrazol-5-yl)oxy]-3-sulfamoylphenylacetamide;
(C239) 2-(2-chlorophenyl)-N-4-[(1-methylpiperidin-4-yl)oxy]-3-sulfamoylphenylacetamide;
(C240) 2-(2-chlorophenyl)-N-(4-[5-methyl-2-(pyridin-3-yl)-1,3-thiazol-4-yl]oxy-3-sulfamoylphenyl)acetamide;
(C241) N-[4-(3-chlorophenoxy)-2-methyl-5-sulfamoylphenyl]-2-(2-chlorophenyl)acetamide;
(C242) 2-(2-chlorophenyl)-N-{4-[(1-oxidetetrahydrothiophen-3-yl)oxy]-3-sulfamoylphenyl}acetamide;
(C243) N-[4-(4-chlorophenoxy)-3-sulfamoylphenyl]-2-[2,6-dichloro-4-(trifluoromethyl)phenyl]acetamide;
(C244) N-[4-(4-chlorophenoxy)-3-sulfamoylphenyl]-2-(2,5-dichloro-4-cyanophenyl)acetamide;
(C245) N-[4-(3-chlorophenoxy)-3-sulfamoylphenyl]-2-phenylacetamide;
(C246) N-[4-(cyclopropylmethoxy)-3-sulfamoylphenyl]-2-phenylacetamide;
(C247) N-[4-(3,5-dimethylphenoxy)-3-sulfamoylphenyl]-2-phenylacetamide;
(C248) N-[4-(2,4-difluorophenoxy)-3-sulfamoylphenyl]-2-phenylacetamide;
(C249) N-[4-(4-fluorophenoxy)-3-sulfamoylphenyl]-2-phenylacetamide;
(C250) N-[4-(3-fluorophenoxy)-3-sulfamoylphenyl]-2-phenylacetamide;
(C251) N-[4-(3-methoxyphenoxy)-3-sulfamoylphenyl]-2-phenylacetamide;
(C252) N-[4-(2-fluoro-5-methylphenoxy)-3-sulfamoylphenyl]-2-phenylacetamide;
(C253) 2-phenyl-N-3-sulfamoyl-4-[4-(trifluoromethoxy)phenoxy]phenylacetamide;
(C254) 2-phenyl-N-3-sulfamoyl-4-[3-(trifluoromethyl)phenoxy]phenylacetamide;
(C255) N-[4-(3,5-dimethoxyphenoxy)-3-sulfamoylphenyl]-2-phenylacetamide;
(C256) N-[4-(3-cyanophenoxy)-3-sulfamoylphenyl]-2-phenylacetamide;
(C257) N-[4-(3-chlorophenoxy)-3-sulfamoylphenyl]-2-(4-hydroxyphenyl) acetamide;
(C258) 2-(2-chloro-6-methoxy-4-methylphenyl)-N-[4-(3-chlorophenoxy)-3-sulfamoylphenyl]acetamide;
(C259) 2-(2-chloro-6-fluorophenyl)-N-[4-(3-chlorophenoxy)-3-sulfamoylphenyl]propanamide;
(C260) 2-(2-chloro-4,6-difluorophenyl)-N-[4-(3-chlorophenoxy)-3-sulfamoylphenyl]acetamide;
(C261) N-[4-(3-chlorophenoxy)-3-sulfamoylphenyl]-2-(2,6-dichlorophenyl)propanamide;
(C262) 2-(2-chlorophenyl)-N-4-[(2H5)phenyloxy]-3-sulfamoylphenylacetamide;
(C263) 2-(2-chlorophenyl)-N-(4-{[4-chloro(2H4)phenyl]oxy}-3-sulfamoylphenyl)acetamide;
(C264) 2-(2-chlorophenyl)-N-(4-{[2-chloro(2H4)phenyl]oxy}-3-sulfamoylphenyl)acetamide;
(C265) 2-(2-chlorophenyl)-N-4-[4-(2-hydroxypropan-2-yl)phenoxy]-3-sulfamoylphenylacetamide;
(C266) 2-(2-chlorophenyl)-N-4-[(2,2-dimethyltetrahydro-2H-pyran-4-yl)methoxy]-3-sulfamoylphenylacetamide;
(C267) 2-(2-chlorophenyl)-N-{4-[(1R,5S,6r)-3-oxabicyclo[3.1.0]hex-6-ylmethoxy]-3-sulfamoylphenyl}acetamide;
(C268) 2-(2-chlorophenyl)-N-4-[(4-chlorotetrahydro-2H-pyran-4-yl)methoxy]-3-sulfamoylphenylacetamide;
(C269) 2-(2-chlorophenyl)-N-[4-(1,4-dioxane-2-ylmethoxy)-3-sulfamoylphenyl]acetamide;
(C270) 2-(2-chlorophenyl)-N-3-sulfamoyl-4-[(2,2,6,6-tetramethyltetrahydro-2H-pyran-4-yl)oxy]phenylacetamide;
(C271) N-[4-(3-chlorophenoxy)-3-methyl-5-sulfamoylphenyl]-2-(2-chlorophenyl)acetamide;
(C272) N-[4-(3-chlorophenoxy)-3-methyl-5-sulfamoylphenyl]-2-phenylacetamide;
(C273) methyl 2-(4-[(2-chlorophenyl)acetyl]amino-2-sulfamoylphenoxy)benzoate;
(C274) methyl 4-(4-[(2-chlorophenyl)acetyl]amino-2-sulfamoylphenoxy)benzoate;
(C275) 2-(2-chlorophenyl)-N-4-[3-(2-hydroxypropan-2-yl)phenoxy]-3-sulfamoylphenylacetamide;
(C276) 2-(2-chlorophenyl)-N-4-[2-(2-hydroxypropan-2-yl)phenoxy]-3-sulfamoylphenylacetamide;
(C277) N-[4-(4-chlorophenoxy)-3-sulfamoylphenyl]-2-(2,3-dihydro-1,4-benzodioxin-6-yl)acetamide;
(C278) 2-(7-chloro-2,3-dihydro-1,4-benzodioxin-6-yl)-N-[4-(3-chlorophenoxy)-3-sulfamoylphenyl]acetamide;
(C279) 2-(5-chloro-2,3-dihydro-1-benzofuran-4-yl)-N-[4-(3-chlorophenoxy)-3-sulfamoylphenyl]acetamide;
(C280) 2-(2-fluorophenyl)-N-[3-sulfamoyl-4-(tetrahydro-2H-pyran-4-ylmethoxy)phenyl]acetamide;
(C281) N-[3-sulfamoyl-4-(tetrahydro-2H-pyran-4-ylmethoxy)phenyl]-2-[2-(trifluoromethyl)phenyl]acetamide;
(C282) 2-[2-(difluoromethyl)phenyl]-N-[3-sulfamoyl-4-(tetrahydro-2H-pyran-4-ylmethoxy)phenyl]acetamide;
(C283) 2-(2-chloro-4-fluorophenyl)-N-[3-sulfamoyl-4-(tetrahydro-2H-pyran-4-ylmethoxy)phenyl]acetamide;
(C284) 2-(2-chlorophenyl)-N-(3-sulfamoyl-4-{[6-(trifluoromethyl)pyridin-3-yl]oxy}phenyl)acetamide;
(C285) 2-(2-chlorophenyl)-N-(4-{[5-chloro-4-(trifluoromethyl)pyridin-2-yl]oxy}-3-sulfamoyl-phenyl)acetamide;
(C286) N-[4-(3-chlorophenoxy)-3-sulfamoylphenyl]-2-phenyl(2H2)acetamide;
(C287) N-{4-[(6-chloro-5-fluoropyridin-3-yl)oxy]-3-sulfamoylphenyl}-2-(2-chlorophenyl)acetamide;
(C288) 2-(2-chlorophenyl)-N-{4-[(4,4-difluoro-1-hydroxycyclohexyl)methoxy]-3-sulfamoyl-phenyl}acetamide;
(C289) 2-(2-chlorophenyl)-N-{4-[(1-hydroxycyclohexyl)methoxy]-3-sulfamoylphenyl}acetamide;
(C290) N-[4-(3-chlorophenoxy)-3-fluoro-5-sulfamoylphenyl]-2-(2-fluorophenyl)acetamide;
(C291) N-[4-(3-chlorophenoxy)-3-fluoro-5-sulfamoylphenyl]-2-[2-(difluoromethyl)phenyl]acetamide;
(C292) N-[4-(3-chlorophenoxy)-3-fluoro-5-sulfamoylphenyl]-2-(2-chlorophenyl)acetamide;
(C293) 2-(2-chloro-5-fluorophenyl)-N-[4-(3-chlorophenoxy)-3-fluoro-5-sulfamoylphenyl]acetamide;
(C294) N-[6-(3-chlorophenoxy)-5-sulfamoylpyridin-3-yl]-2-(2-fluorophenyl)acetamide;
(C295) N-[6-(3-chlorophenoxy)-5-sulfamoylpyridin-3-yl]-2-[2-(trifluoromethyl)phenyl]acetamide;
(C296) N-[6-(3-chlorophenoxy)-5-sulfamoylpyridin-3-yl]-2-[2-(difluoromethyl)phenyl]acetamide; and
(C297) 2-(2-chloro-5-fluorophenyl)-N-[6-(3-chlorophenoxy)-5-sulfamoylpyridin-3-yl]acetamide.

9. The pharmaceutical composition according to any one of claims 1 to 4, wherein the compound is a compound selected from the group consisting of the following (D1) to (D558):
(D1) 2-(2-chlorophenyl)-N-[4-(2-oxopyridin-1(2H)-yl)-3-sulfamoylphenyl]acetamide;
(D2) N-[4-(4-chloro-2-oxopyridin-1(2H)-yl)-3-sulfamoylphenyl]-2-(2-chlorophenyl)acetamide;
(D3) 2-(2-chlorophenyl)-N-[4-(3,5-dichloro-2-oxopyridin-1(2H)-yl)-3-sulfamoylphenyl]acetamide;
(D4) N-[4-(3-chloro-2-oxopyridin-1(2H)-yl)-3-sulfamoylphenyl]-2-(2-chlorophenyl)acetamide;
(D5) 2-(2-chlorophenyl)-N-[4-(3-methyl-1H-1,2,4-triazol-1-yl)-3-sulfamoylphenyl]acetamide;
(D6) 2-(2-chlorophenyl)-N-[4-(5-methyl-1H-1,2,4-triazol-1-yl)-3-sulfamoylphenyl]acetamide;
(D7) 2-(2-chlorophenyl)-N-{3-sulfamoyl-4-[3-(trifluoromethyl)-1H-1,2,4-triazol-1-yl]phenyl}acetamide;
(D8) 2-(2-chlorophenyl)-N-{4-[5-methyl-3-(trifluoromethyl)-1H-1,2,4-triazol-1-yl]-3-sulfamoylphenyl}acetamide;
(D9) 2-(2-chlorophenyl)-N-[4-(1H-imidazol-1-yl)-3-sulfamoylphenyl]acetamide;
(D10) 2-(2-chlorophenyl)-N-{3-sulfamoyl-4-[4-(trifluoromethyl)-1H-imidazol-1-yl]phenyl}acetamide;
(D11) 2-(2-chlorophenyl)-N-{3-sulfamoyl-4-[3-(trifluoromethyl)-1H-pyrazol-1-yl]phenyl}acetamide;
(D12) 2-(2-chlorophenyl)-N-{4-[3-(difluoromethyl)-1H-pyrazol-1-yl]-3-sulfamoylphenyl}acetamide;
(D13) 2-(2-chlorophenyl)-N-{4-[5-cyclopropyl-3-(difluoromethyl)-1H-pyrazol-1-yl]-3-sulfamoylphenyl}acetamide;
(D14) 2-(2-chlorophenyl)-N-{4-[4-methyl-3-(trifluoromethyl)-1H-pyrazol-1-yl]-3-sulfamoylphenyl}acetamide;
(D15) 2-(2-chlorophenyl)-N-{4-[5-methyl-3-(trifluoromethyl)-1H-pyrazol-1-yl]-3-sulfamoylphenyl}acetamide;
(D16) 2-(2-chlorophenyl)-N-[3-sulfamoyl-4-(1H-1,2,4-triazol-1-yl)phenyl]acetamide;
(D17) 2-(2-chlorophenyl)-N-{4-[3-(difluoromethyl)-1H-1,2,4-triazol-1-yl]-3-sulfamoylphenyl}acetamide;
(D18) N-{4-[3-(difluoromethyl)-1H-1,2,4-triazol-1-yl]-3-sulfamoylphenyl}-2-(2-fluorophenyl)acetamide;
(D19) 2-(2-chlorophenyl)-N-{3-sulfamoyl-4-[4-(trifluoromethyl)-1H-pyrazol-1-yl]phenyl}acetamide;
(D20) 2-(2-fluorophenyl)-N-{3-sulfamoyl-4-[4-(trifluoromethyl)-1H-pyrazol-1-yl]phenyl}acetamide;
(D21) 2-[2-(difluoromethyl)phenyl]-N-{3-sulfamoyl-4-[4-(trifluoromethyl)-1H-pyrazol-1-yl]phenyl}acetamide;
(D22) N-{3-sulfamoyl-4-[4-(trifluoromethyl)-1H-pyrazol-1-yl]phenyl}-2-[2-(trifluoromethyl)phenyl]acetamide;
(D23) N-[4-(3-tert-butyl-1H-1,2,4-triazol-1-yl)-3-sulfamoylphenyl]-2-(2-chlorophenyl)acetamide;
(D24) 2-(2-chlorophenyl)-N-[4-(3-chloro-1H-1,2,4-triazol-1-yl)-3-sulfamoylphenyl]acetamide;
(D25) 2-(2-chlorophenyl)-N-[4-(4-chloro-1H-pyrazol-1-yl)-3-sulfamoylphenyl]acetamide;
(D26) 2-(2-chlorophenyl)-N-[4-(4-fluoro-1H-pyrazol-1-yl)-3-sulfamoylphenyl]acetamide;
(D27) 2-(2-chlorophenyl)-N-[4-(4-isopropoxy-1H-pyrazol-1-yl)-3-sulfamoylphenyl]acetamide;
(D28) N-[4-(4-bromo-1H-pyrazol-1-yl)-3-sulfamoylphenyl]-2-(2-chlorophenyl)acetamide;
(D29) 2-(2-chlorophenyl)-N-[4-(3-isobutyl-1H-1,2,4-triazol-1-yl)-3-sulfamoylphenyl]acetamide;
(D30) 2-(2-chlorophenyl)-N-{4-[4-(methylsulfanyl)-1H-pyrazol-1-yl]-3-sulfamoylphenyl}acetamide;
(D31) 2-(2-chlorophenyl)-N-[4-(4-methoxy-1H-pyrazol-1-yl)-3-sulfamoylphenyl]acetamide;
(D32) N-[4-(1H-benzoimidazol-1-yl)-3-sulfamoylphenyl]-2-(2-chlorophenyl)acetamide;
(D33) N-[4-(4-chloro-1H-imidazol-1-yl)-3-sulfamoylphenyl]-2-(2-chlorophenyl)acetamide;
(D34) 2-(2-chlorophenyl)-N-{4-[3-(dimethylamino)-1H-1,2,4-triazol-1-yl]-3-sulfamoylphenyl}acetamide;
(D35) 2-(2-chlorophenyl)-N-[4-(3-ethyl-1H-1,2,4-triazol-1-yl)-3-sulfamoylphenyl]acetamide;
(D36) (2-chlorophenyl)-N-[4-(3-cyclopropyl-1H-1,2,4-triazol-1-yl)-3-sulfamoylphenyl]acetamide;
(D37) 2-(2-chlorophenyl)-N-[4-(5-cyclopropyl-1H-1,2,4-triazol-1-yl)-3-sulfamoylphenyl]acetamide;
(D38) 2-(2-chlorophenyl)-N-{4-[3-(methoxymethyl)-1H-1,2,4-triazol-1-yl]-3-sulfamoylphenyl}acetamide;
(D39) 2-(2-chlorophenyl)-N-[4-(4-cyano-1H-pyrazol-1-yl)-3-sulfamoylphenyl]acetamide;
(D40) N-[4-(4-tert-butyl-1H-imidazol-1-yl)-3-sulfamoylphenyl]-2-(2-chlorophenyl)acetamide;
(D41) 2-(2-chlorophenyl)-N-[4-(3-cyano-1H-1,2,4-triazol-1-yl)-3-sulfamoylphenyl]acetamide;
(D42) N-[4-(4-bromo-1H-imidazol-1-yl)-3-sulfamoylphenyl]-2-(2-chlorophenyl)acetamide;
(D43) 2-(2-chlorophenyl)-N-[4-(3H-imidazo[4,5-b]pyridin-3-yl)-3-sulfamoylphenyl]acetamide;
(D44) 2-(2-chlorophenyl)-N-[4-(1H-imidazo[4,5-b]pyridin-1-yl)-3-sulfamoylphenyl]acetamide;
(D45) 2-(2-chlorophenyl)-N-[4-(1H-imidazo[4,5-c]pyridin-1-yl)-3-sulfamoylphenyl]acetamide;
(D46) 2-(2-chlorophenyl)-N-[4-(3H-imidazo[4,5-c]pyridin-3-yl)-3-sulfamoylphenyl]acetamide;
(D47) 2-(2-chlorophenyl)-N-[4-(2,4-dimethyl-1H-imidazol-1-yl)-3-sulfamoylphenyl]acetamide;
(D48) 2-(2-fluorophenyl)-N-[4-(4-fluoro-1H-pyrazol-1-yl)-3-sulfamoylphenyl]acetamide;
(D49) N-[4-(4-chloro-1H-pyrazol-1-yl)-3-sulfamoylphenyl]-2-[2-(difluoromethyl)phenyl]acetamide;
(D50) N-[4-(4-chloro-1H-pyrazol-1-yl)-3-sulfamoylphenyl]-2-(2-fluorophenyl)acetamide;
(D51) 2-[2-(difluoromethyl)phenyl]-N-[4-(4-fluoro-1H-pyrazol-1-yl)-3-sulfamoylphenyl]acetamide;
(D52) N-[4-(4-cyano-1H-pyrazol-1-yl)-3-sulfamoylphenyl]-2-(2-fluorophenyl)acetamide;
(D53) 2-(2-methoxyphenyl)-N-{3-sulfamoyl-4-[3-(trifluoromethyl)-1H-1,2,4-triazol-1-yl]phenyl}acetamide;
(D54) 2-(2-fluorophenyl)-N-{3-sulfamoyl-4-[3-(trifluoromethyl)-1H-1,2,4-triazol-1-yl]phenyl}acetamide;
(D55) 2-(3-fluorophenyl)-N-{3-sulfamoyl-4-[3-(trifluoromethyl)-1H-1,2,4-triazol-1-yl]phenyl}acetamide;
(D56) 2-(2-chloro-4-fluorophenyl)-N-{3-sulfamoyl-4-[3-(trifluoromethyl)-1H-1,2,4-triazol-1-yl]phenyl}acetamide;
(D57) 2-[2-(difluoromethoxy)phenyl]-N-{3-sulfamoyl-4-[3-(trifluoromethyl)-1H-1,2,4-triazol-1-yl]phenyl}acetamide;
(D58) 2-(2-chloro-5-fluorophenyl)-N-{3-sulfamoyl-4-[3-(trifluoromethyl)-1H-1,2,4-triazol-1-yl]phenyl}acetamide;
(D59) 2-(3-chloropyridin-4-yl)-N-{3-sulfamoyl-4-[3-(trifluoromethyl)-1H-1,2,4-triazol-1-yl]phenyl}acetamide;
(D60) 2-[4-(difluoromethyl)phenyl]-N-{3-sulfamoyl-4-[3-(trifluoromethyl)-1H-1,2,4-triazol-1-yl]phenyl}acetamide;
(D61) 2-(2-chlorophenyl)-2,2-difluoro-N-{3-sulfamoyl-4-[3-(trifluoromethyl)-1H-1,2,4-triazol-1-yl]phenyl}acetamide;
(D62) N-[4-(4-bromo-1H-pyrazol-1-yl)-3-sulfamoylphenyl]-2-(2-chlorophenyl)-2,2-difluoroacetamide;
(D63) N-[4-(4-bromo-1H-pyrazol-1-yl)-3-sulfamoylphenyl]-2-(2-fluorophenyl)acetamide;
(D64) N-[4-(4-bromo-1H-pyrazol-1-yl)-3-sulfamoylphenyl]-2-[2-(trifluoromethyl)phenyl]acetamide;
(D65) N-[4-(4-bromo-1H-pyrazol-1-yl)-3-sulfamoylphenyl]-2-(3-fluorophenyl)acetamide;
(D66) N-[4-(4-bromo-1H-pyrazol-1-yl)-3-sulfamoylphenyl]-2-(2-methylphenyl)acetamide;
(D67) N-[4-(4-bromo-1H-pyrazol-1-yl)-3-sulfamoylphenyl]-2-(2-chloropyridin-3-yl)acetamide;
(D68) N-[4-(4-bromo-1H-pyrazol-1-yl)-3-sulfamoylphenyl]-2-(2-chloro-4-fluorophenyl)acetamide;
(D69) N-[4-(4-bromo-1H-pyrazol-1-yl)-3-sulfamoylphenyl]-2-[2-(difluoromethoxy)phenyl]acetamide;
(D70) N-[4-(4-bromo-1H-pyrazol-1-yl)-3-sulfamoylphenyl]-2-[2-(trifluoromethoxy)phenyl]acetamide;
(D71) N-[4-(4-bromo-1H-pyrazol-1-yl)-3-sulfamoylphenyl]-2-(2-chloro-4,5-difluorophenyl)acetamide;
(D72) N-[4-(4-bromo-1H-pyrazol-1-yl)-3-sulfamoylphenyl]-2-(3-chloropyridin-4-yl)acetamide;
(D73) N-[4-(4-bromo-1H-pyrazol-1-yl)-3-sulfamoylphenyl]-2-[4-(difluoromethyl)phenyl]acetamide;
(D74) 2-(2-bromophenyl)-N-[4-(4-chloro-1H-pyrazol-1-yl)-3-sulfamoylphenyl]acetamide;
(D75) N-[4-(4-chloro-1H-pyrazol-1-yl)-3-sulfamoylphenyl]-2-(2-methoxyphenyl)acetamide;
(D76) 2-(4-chlorophenyl)-N-[4-(4-chloro-1H-pyrazol-1-yl)-3-sulfamoylphenyl]acetamide;
(D77) 2-(2-chloro-6-fluorophenyl)-N-[4-(4-chloro-1H-pyrazol-1-yl)-3-sulfamoylphenyl]acetamide;
(D78) N-[4-(4-chloro-1H-pyrazol-1-yl)-3-sulfamoylphenyl]-2-(2-nitrophenyl)acetamide;
(D79) N-[4-(4-chloro-1H-pyrazol-1-yl)-3-sulfamoylphenyl]-2-(2,4-dichlorophenyl)acetamide;
(D80) N-[4-(4-chloro-1H-pyrazol-1-yl)-3-sulfamoylphenyl]-2-(2,6-dichlorophenyl)acetamide;
(D81) N-[4-(4-chloro-1H-pyrazol-1-yl)-3-sulfamoylphenyl]-2-(3,4-difluorophenyl)acetamide;
(D82) 2-(3-chlorophenyl)-N-[4-(4-chloro-1H-pyrazol-1-yl)-3-sulfamoylphenyl]acetamide;
(D83) N-[4-(4-chloro-1H-pyrazol-1-yl)-3-sulfamoylphenyl]-2-(3,5-difluorophenyl)acetamide;
(D84) N-[4-(4-chloro-1H-pyrazol-1-yl)-3-sulfamoylphenyl]-2-(4-fluorophenyl)acetamide;
(D85) N-[4-(4-chloro-1H-pyrazol-1-yl)-3-sulfamoylphenyl]-2-(2-hydroxyphenyl)acetamide;
(D86) N-[4-(4-chloro-1H-pyrazol-1-yl)-3-sulfamoylphenyl]-2-(4-hydroxyphenyl)acetamide;
(D87) N-[4-(4-chloro-1H-pyrazol-1-yl)-3-sulfamoylphenyl]-2-(2,3-difluorophenyl)acetamide;
(D88) 2-(2-chloro-4-fluorophenyl)-N-[4-(4-chloro-1H-pyrazol-1-yl)-3-sulfamoylphenyl]acetamide;
(D89) N-[4-(4-chloro-1H-pyrazol-1-yl)-3-sulfamoylphenyl]-2-(2-chloropyridin-3-yl)acetamide;
(D90) N-[4-(4-chloro-1H-pyrazol-1-yl)-3-sulfamoylphenyl]-2-[2-chloro-3-(trifluoromethyl)phenyl]acetamide;
(D91) N-[4-(4-chloro-1H-pyrazol-1-yl)-3-sulfamoylphenyl]-2-[2-(difluoromethoxy)phenyl]acetamide;
(D92) N-[4-(4-chloro-1H-pyrazol-1-yl)-3-sulfamoylphenyl]-2-[2-(trifluoromethoxy)phenyl]acetamide;
(D93) 2-(2-chloro-4,5-difluorophenyl)-N-[4-(4-chloro-1H-pyrazol-1-yl)-3-sulfamoylphenyl]acetamide;
(D94) 2-(2-chloro-4-methoxyphenyl)-N-[4-(4-chloro-1H-pyrazol-1-yl)-3-sulfamoylphenyl]acetamide;
(D95) 2-(2-chloro-6-fluoro-3-methylphenyl)-N-[4-(4-chloro-1H-pyrazol-1-yl)-3-sulfamoylphenyl]acetamide;
(D96) 2-(2-chloro-3,6-difluorophenyl)-N-[4-(4-chloro-1H-pyrazol-1-yl)-3-sulfamoylphenyl]acetamide;
(D97) 2-(2-chloro-5-methylphenyl)-N-[4-(4-chloro-1H-pyrazol-1-yl)-3-sulfamoylphenyl]acetamide;
(D98) 2-(2-chloro-5-fluorophenyl)-N-[4-(4-chloro-1H-pyrazol-1-yl)-3-sulfamoylphenyl]acetamide;
(D99) N-[4-(4-chloro-1H-pyrazol-1-yl)-3-sulfamoylphenyl]-2-(2,5-dichlorophenyl)acetamide;
(D100) N-[4-(4-chloro-1H-pyrazol-1-yl)-3-sulfamoylphenyl]-2-(2-isopropylphenyl)acetamide;
(D101) 2-(2-chloro-5-methoxyphenyl)-N-[4-(4-chloro-1H-pyrazol-1-yl)-3-sulfamoylphenyl]acetamide;
(D102) 2-(2-chloro-4,6-difluorophenyl)-N-[4-(4-chloro-1H-pyrazol-1-yl)-3-sulfamoylphenyl]acetamide;
(D103) N-[4-(4-chloro-1H-pyrazol-1-yl)-3-sulfamoylphenyl]-2-[2-chloro-6-(trifluoromethyl)phenyl]acetamide;
(D104) 2-(5-bromo-4-fluoro-2-methylphenyl)-N-[4-(4-chloro-1H-pyrazol-1-yl)-3-sulfamoylphenyl]acetamide;
(D105) 2-(2-chloro-6-methoxyphenyl)-N-[4-(4-chloro-1H-pyrazol-1-yl)-3-sulfamoylphenyl]acetamide;
(D106) N-[4-(4-chloro-1H-pyrazol-1-yl)-3-sulfamoylphenyl]-2-(2,6-difluorophenyl)propanamide;
(D107) N-[4-(4-chloro-1H-pyrazol-1-yl)-3-sulfamoylphenyl]-2-[4-(difluoromethyl)phenyl]acetamide;
(D108) 2-(4-chlorophenyl)-N-{3-sulfamoyl-4-[4-(trifluoromethyl)-1H-pyrazol-1-yl]phenyl}acetamide;
(D109) 2-(2-chlorophenyl)-N-[4-(4-chloro-1H-pyrazol-1-yl)-3-sulfamoylphenyl]-2,2-difluoroacetamide;
(DIIO) 2-(2-nitrophenyl)-N-{3-sulfamoyl-4-[4-(trifluoromethyl)-1H-pyrazol-1-yl]phenyl}acetamide;
(Dill) 2-(2,4-dichlorophenyl)-N-{3-sulfamoyl-4-[4-(trifluoromethyl)-1H-pyrazol-1-yl]phenyl}acetamide;
(D112) 2-(2-methoxyphenyl)-N-{3-sulfamoyl-4-[4-(trifluoromethyl)-1H-pyrazol-1-yl]phenyl}acetamide;
(D113) 2-(2-chloro-6-fluorophenyl)-N-{3-sulfamoyl-4-[4-(trifluoromethyl)-1H-pyrazol-1-yl]phenyl}acetamide;
(D114) 2-(3-fluorophenyl)-N-{3-sulfamoyl-4-[4-(trifluoromethyl)-1H-pyrazol-1-yl]phenyl}acetamide;
(D115) 2-(3,5-difluorophenyl)-N-{3-sulfamoyl-4-[4-(trifluoromethyl)-1H-pyrazol-1-yl]phenyl}acetamide;
(D116) 2-(2,6-dichlorophenyl)-N-{3-sulfamoyl-4-[4-(trifluoromethyl)-1H-pyrazol-1-yl]phenyl}acetamide;
(D117) 2-(2-bromophenyl)-N-{3-sulfamoyl-4-[4-(trifluoromethyl)-1H-pyrazol-1-yl]phenyl}acetamide;
(D118) 2-(3,4-difluorophenyl)-N-{3-sulfamoyl-4-[4-(trifluoromethyl)-1H-pyrazol-1-yl]phenyl}acetamide;
(D119) 2-(4-fluorophenyl)-N-{3-sulfamoyl-4-[4-(trifluoromethyl)-1H-pyrazol-1-yl]phenyl}acetamide;
(D120) 2-(3-chlorophenyl)-N-{3-sulfamoyl-4-[4-(trifluoromethyl)-1H-pyrazol-1-yl]phenyl}acetamide;
(D121) 2-(4-methoxyphenyl)-N-{3-sulfamoyl-4-[4-(trifluoromethyl)-1H-pyrazol-1-yl]phenyl}acetamide;
(D122) 2-(2,3-difluorophenyl)-N-{3-sulfamoyl-4-[4-(trifluoromethyl)-1H-pyrazol-1-yl]phenyl}acetamide;
(D123) 2-(2-methylphenyl)-N-{3-sulfamoyl-4-[4-(trifluoromethyl)-1H-pyrazol-1-yl]phenyl}acetamide;
(D124) 2-(3-methylphenyl)-N-{3-sulfamoyl-4-[4-(trifluoromethyl)-1H-pyrazol-1-yl]phenyl}acetamide;
(D125) 2-[2-(difluoromethoxy)phenyl]-N-{3-sulfamoyl-4-[4-(trifluoromethyl)-1H-pyrazol-1-yl]phenyl}acetamide;
(D126) 2-(2-chloropyridin-3-yl)-N-{3-sulfamoyl-4-[4-(trifluoromethyl)-1H-pyrazol-1-yl]phenyl}acetamide;
(D127) 2-(2-chloro-4-fluorophenyl)-N-{3-sulfamoyl-4-[4-(trifluoromethyl)-1H-pyrazol-1-yl]phenyl}acetamide;
(D128) 2-(2-chloro-5-methylphenyl)-N-{3-sulfamoyl-4-[4-(trifluoromethyl)-1H-pyrazol-1-yl]phenyl}acetamide;
(D129) N-{3-sulfamoyl-4-[4-(trifluoromethyl)-1H-pyrazol-1-yl]phenyl}-2-[2-(trifluoromethoxy)phenyl]acetamide;
(D130) 2-(2-chloro-4,5-difluorophenyl)-N-{3-sulfamoyl-4-[4-(trifluoromethyl)-1H-pyrazol-1-yl]phenyl}acetamide;
(D131) 2-[2-chloro-3-(trifluoromethyl)phenyl]-N-{3-sulfamoyl-4-[4-(trifluoromethyl)-1H-pyrazol-1-yl]phenyl}acetamide;
(D132) 2-(2-chloro-6-fluoro-3-methylphenyl)-N-{3-sulfamoyl-4-[4-(trifluoromethyl)-1H-pyrazol-1-yl]phenyl}acetamide;
(D133) 2-(2-chloro-3,6-difluorophenyl)-N-{3-sulfamoyl-4-[4-(trifluoromethyl)-1H-pyrazol-1-yl]phenyl}acetamide;
(D134) 2-[2-chloro-6-(trifluoromethyl)phenyl]-N-{3-sulfamoyl-4-[4-(trifluoromethyl)-1H-pyrazol-1-yl]phenyl}acetamide;
(D135) 2-(2-chloro-4-methoxyphenyl)-N-{3-sulfamoyl-4-[4-(trifluoromethyl)-1H-pyrazol-1-yl]phenyl}acetamide;
(D136) 2-(2,5-dichlorophenyl)-N-{3-sulfamoyl-4-[4-(trifluoromethyl)-1H-pyrazol-1-yl]phenyl}acetamide;
(D137) 2-(2-isopropylphenyl)-N-{3-sulfamoyl-4-[4-(trifluoromethyl)-1H-pyrazol-1-yl]phenyl}acetamide;
(D138) 2-(2-chloro-5-methoxyphenyl)-N-{3-sulfamoyl-4-[4-(trifluoromethyl)-1H-pyrazol-1-yl]phenyl}acetamide;
(D139) 2-(2-chloro-5-fluorophenyl)-N-{3-sulfamoyl-4-[4-(trifluoromethyl)-1H-pyrazol-1-yl]phenyl}acetamide;
(D140) N-[4-(4-fluoro-1H-pyrazol-1-yl)-3-sulfamoylphenyl]-2-[2-(trifluoromethyl)phenyl]acetamide;
(D141) 2-(5-bromo-4-fluoro-2-methylphenyl)-N-{3-sulfamoyl-4-[4-(trifluoromethyl)-1H-pyrazol-1-yl]phenyl}acetamide;
(D142) 2-(2-chloro-6-methoxyphenyl)-N-{3-sulfamoyl-4-[4-(trifluoromethyl)-1H-pyrazol-1-yl]phenyl}acetamide;
(D143) 2-(2-chloro-4,6-difluorophenyl)-N-{3-sulfamoyl-4-[4-(trifluoromethyl)-1H-pyrazol-1-yl]phenyl}acetamide;
(D144) 2-(3-fluorophenyl)-N-[4-(4-fluoro-1H-pyrazol-1-yl)-3-sulfamoylphenyl]acetamide;
(D145) 2-(3,4-difluorophenyl)-N-[4-(4-fluoro-1H-pyrazol-1-yl)-3-sulfamoylphenyl]acetamide;
(D146) 2-(3,5-difluorophenyl)-N-[4-(4-fluoro-1H-pyrazol-1-yl)-3-sulfamoylphenyl]acetamide;
(D147) N-[4-(4-fluoro-1H-pyrazol-1-yl)-3-sulfamoylphenyl]-2-(2-hydroxyphenyl)acetamide;
(D148) 2-(3-chlorophenyl)-N-[4-(4-fluoro-1H-pyrazol-1-yl)-3-sulfamoylphenyl]acetamide;
(D149) 2-(4-fluorophenyl)-N-[4-(4-fluoro-1H-pyrazol-1-yl)-3-sulfamoylphenyl]acetamide;
(D150) N-[4-(4-fluoro-1H-pyrazol-1-yl)-3-sulfamoylphenyl]-2-(4-hydroxyphenyl)acetamide;
(D151) N-[4-(4-fluoro-1H-pyrazol-1-yl)-3-sulfamoylphenyl]-2-(3-methylphenyl)acetamide;
(D152) N-[4-(4-fluoro-1H-pyrazol-1-yl)-3-sulfamoylphenyl]-2-(4-methylphenyl)acetamide;
(D153) N-[4-(4-fluoro-1H-pyrazol-1-yl)-3-sulfamoylphenyl]-2-(4-methoxyphenyl)acetamide;
(D154) N-[4-(4-fluoro-1H-pyrazol-1-yl)-3-sulfamoylphenyl]-2-(2-methylphenyl)acetamide;
(D155) 2-(2,3-difluorophenyl)-N-[4-(4-fluoro-1H-pyrazol-1-yl)-3-sulfamoylphenyl]acetamide;
(D156) 2-(2-ethoxyphenyl)-N-[4-(4-fluoro-1H-pyrazol-1-yl)-3-sulfamoylphenyl]acetamide;
(D157) 2-[2-(difluoromethoxy)phenyl]-N-[4-(4-fluoro-1H-pyrazol-1-yl)-3-sulfamoylphenyl]acetamide;
(D158) 2-(2-chloropyridin-3-yl)-N-[4-(4-fluoro-1H-pyrazol-1-yl)-3-sulfamoylphenyl]acetamide;
(D159) 2-(2-chloro-6-fluoro-3-methylphenyl)-N-[4-(4-fluoro-1H-pyrazol-1-yl)-3-sulfamoylphenyl]acetamide;
(D160) N-[4-(4-fluoro-1H-pyrazol-1-yl)-3-sulfamoylphenyl]-2-[2-(trifluoromethoxy)phenyl]acetamide;
(D161) 2-(2-chloro-4,5-difluorophenyl)-N-[4-(4-fluoro-1H-pyrazol-1-yl)-3-sulfamoylphenyl]acetamide;
(D162) 2-[2-chloro-3-(trifluoromethyl)phenyl]-N-[4-(4-fluoro-1H-pyrazol-1-yl)-3-sulfamoylphenyl]acetamide;
(D163) 2-(2,5-dichlorophenyl)-N-[4-(4-fluoro-1H-pyrazol-1-yl)-3-sulfamoylphenyl]acetamide;
(D164) 2-(2-chloro-3,6-difluorophenyl)-N-[4-(4-fluoro-1H-pyrazol-1-yl)-3-sulfamoylphenyl]acetamide;
(D165) 2-(2-chloro-5-methylphenyl)-N-[4-(4-fluoro-1H-pyrazol-1-yl)-3-sulfamoylphenyl]acetamide;
(D166) 2-(5-bromo-4-fluoro-2-methylphenyl)-N-[4-(4-fluoro-1H-pyrazol-1-yl)-3-sulfamoylphenyl]acetamide;
(D167) N-[4-(4-fluoro-1H-pyrazol-1-yl)-3-sulfamoylphenyl]-2-(2-isopropylphenyl)acetamide;
(D168) 2-(2-chloro-5-fluorophenyl)-N-[4-(4-fluoro-1H-pyrazol-1-yl)-3-sulfamoylphenyl]acetamide;
(D169) 2-[2-chloro-6-(trifluoromethyl)phenyl]-N-[4-(4-fluoro-1H-pyrazol-1-yl)-3-sulfamoylphenyl]acetamide;
(D170) N-[4-(4-chloro-1H-pyrazol-1-yl)-3-sulfamoylphenyl]-2-(4-methoxyphenyl)acetamide;
(D171) 2-(2,6-difluorophenyl)-N-[4-(4-fluoro-1H-pyrazol-1-yl)-3-sulfamoylphenyl]propanamide;
(D172) N-[4-(4-chloro-1H-pyrazol-1-yl)-3-sulfamoylphenyl]-2-(4-methylphenyl)acetamide;
(D173) N-[4-(4-chloro-1H-pyrazol-1-yl)-3-sulfamoylphenyl]-2-(2-methylphenyl)acetamide;
(D174) N-[4-(4-chloro-1H-pyrazol-1-yl)-3-sulfamoylphenyl]-2-(3-methylphenyl)acetamide;
(D175) N-[4-(4-chloro-1H-pyrazol-1-yl)-3-sulfamoylphenyl]-2-(2-ethoxyphenyl)acetamide;
(D176) 2-(2-ethoxyphenyl)-N-{3-sulfamoyl-4-[4-(trifluoromethyl)-1H-pyrazol-1-yl]phenyl}acetamide;
(D177) 2-(2-bromophenyl)-N-[4-(4-fluoro-1H-pyrazol-1-yl)-3-sulfamoylphenyl]acetamide;
(D178) 2-(2,4-dichlorophenyl)-N-[4-(4-fluoro-1H-pyrazol-1-yl)-3-sulfamoylphenyl]acetamide;
(D179) 2-(2,6-dichlorophenyl)-N-[4-(4-fluoro-1H-pyrazol-1-yl)-3-sulfamoylphenyl]acetamide;
(D180) N-[4-(4-cyano-1H-pyrazol-1-yl)-3-sulfamoylphenyl]-2-(2-nitrophenyl)acetamide;
(D181) 2-(4-chlorophenyl)-N-[4-(4-cyano-1H-pyrazol-1-yl)-3-sulfamoylphenyl]acetamide;
(D182) N-[4-(4-cyano-1H-pyrazol-1-yl)-3-sulfamoylphenyl]-2-(2-methoxyphenyl)acetamide;
(D183) 2-(2-chloro-6-fluorophenyl)-N-[4-(4-cyano-1H-pyrazol-1-yl)-3-sulfamoylphenyl]acetamide;
(D184) N-[4-(4-cyano-1H-pyrazol-1-yl)-3-sulfamoylphenyl]-2-[2-(trifluoromethyl)phenyl]acetamide;
(D185) N-[4-(4-cyano-1H-pyrazol-1-yl)-3-sulfamoylphenyl]-2-(3-fluorophenyl)acetamide;
(D186) N-[4-(4-cyano-1H-pyrazol-1-yl)-3-sulfamoylphenyl]-2-(2,4-dichlorophenyl)acetamide;
(D187) N-[4-(4-cyano-1H-pyrazol-1-yl)-3-sulfamoylphenyl]-2-(2,6-dichlorophenyl)acetamide;
(D188) 2-(2-bromophenyl)-N-[4-(4-cyano-1H-pyrazol-1-yl)-3-sulfamoylphenyl]acetamide;
(D189) N-[4-(4-cyano-1H-pyrazol-1-yl)-3-sulfamoylphenyl]-2-(3,4-difluorophenyl)acetamide;
(D190) N-[4-(4-cyano-1H-pyrazol-1-yl)-3-sulfamoylphenyl]-2-(3,5-difluorophenyl)acetamide;
(D191) 2-(3-chlorophenyl)-N-[4-(4-cyano-1H-pyrazol-1-yl)-3-sulfamoylphenyl]acetamide;
(D192) N-[4-(4-cyano-1H-pyrazol-1-yl)-3-sulfamoylphenyl]-2-(4-fluorophenyl)acetamide;
(D193) N-[4-(4-cyano-1H-pyrazol-1-yl)-3-sulfamoylphenyl]-2-(2-hydroxyphenyl)acetamide;
(D194) N-[4-(4-cyano-1H-pyrazol-1-yl)-3-sulfamoylphenyl]-2-(4-hydroxyphenyl)acetamide;
(D195) N-[4-(4-cyano-1H-pyrazol-1-yl)-3-sulfamoylphenyl]-2-(4-methylphenyl)acetamide;
(D196) N-[4-(4-cyano-1H-pyrazol-1-yl)-3-sulfamoylphenyl]-2-(4-methoxyphenyl)acetamide;
(D197) N-[4-(4-cyano-1H-pyrazol-1-yl)-3-sulfamoylphenyl]-2-(2-methylphenyl)acetamide;
(D198) N-[4-(4-cyano-1H-pyrazol-1-yl)-3-sulfamoylphenyl]-2-(3-methylphenyl)acetamide;
(D199) N-[4-(4-cyano-1H-pyrazol-1-yl)-3-sulfamoylphenyl]-2-(2-ethoxyphenyl)acetamide;
(D200) N-[4-(4-cyano-1H-pyrazol-1-yl)-3-sulfamoylphenyl]-2-(2,3-difluorophenyl)acetamide;
(D201) 2-(2-chloropyridin-3-yl)-N-[4-(4-cyano-1H-pyrazol-1-yl)-3-sulfamoylphenyl]acetamide;
(D202) 2-(2-chloro-4-fluorophenyl)-N-[4-(4-cyano-1H-pyrazol-1-yl)-3-sulfamoylphenyl]acetamide;
(D203) N-[4-(4-cyano-1H-pyrazol-1-yl)-3-sulfamoylphenyl]-2-[2-(difluoromethoxy)phenyl]acetamide;
(D204) N-[4-(4-cyano-1H-pyrazol-1-yl)-3-sulfamoylphenyl]-2-[2-(trifluoromethoxy)phenyl]acetamide;
(D205) 2-(2-chloro-4,5-difluorophenyl)-N-[4-(4-cyano-1H-pyrazol-1-yl)-3-sulfamoylphenyl]acetamide;
(D206) 2-[2-chloro-3-(trifluoromethyl)phenyl]-N-[4-(4-cyano-1H-pyrazol-1-yl)-3-sulfamoylphenyl]acetamide;
(D207) 2-(2-chloro-6-fluoro-3-methylphenyl)-N-[4-(4-cyano-1H-pyrazol-1-yl)-3-sulfamoylphenyl]acetamide;
(D208) 2-(2-chloro-3,6-difluorophenyl)-N-[4-(4-cyano-1H-pyrazol-1-yl)-3-sulfamoylphenyl]acetamide;
(D209) 2-(2-chloro-5-methylphenyl)-N-[4-(4-cyano-1H-pyrazol-1-yl)-3-sulfamoylphenyl]acetamide;
(D210) 2-(2-chloro-4-methoxyphenyl)-N-[4-(4-cyano-1H-pyrazol-1-yl)-3-sulfamoylphenyl]acetamide;
(D211) N-[4-(4-cyano-1H-pyrazol-1-yl)-3-sulfamoylphenyl]-2-(2,5-dichlorophenyl)acetamide;
(D212) 2-(5-chloro-2-methoxyphenyl)-N-[4-(4-cyano-1H-pyrazol-1-yl)-3-sulfamoylphenyl]acetamide;
(D213) N-[4-(4-cyano-1H-pyrazol-1-yl)-3-sulfamoylphenyl]-2-[2-(propan-2-yl)phenyl]acetamide;
(D214) 2-(2-chloro-5-fluorophenyl)-N-[4-(4-cyano-1H-pyrazol-1-yl)-3-sulfamoylphenyl]acetamide;
(D215) 2-[2-chloro-6-(trifluoromethyl)phenyl]-N-[4-(4-cyano-1H-pyrazol-1-yl)-3-sulfamoylphenyl]acetamide;
(D216) 2-(2-chloro-6-methoxyphenyl)-N-[4-(4-cyano-1H-pyrazol-1-yl)-3-sulfamoylphenyl]acetamide;
(D217) 2-(2-chloro-4,6-difluorophenyl)-N-[4-(4-cyano-1H-pyrazol-1-yl)-3-sulfamoylphenyl]acetamide;
(D218) 2-(3-chloropyridin-4-yl)-N-[4-(4-cyano-1H-pyrazol-1-yl)-3-sulfamoylphenyl]acetamide;
(D219) N-[4-(4-cyano-1H-pyrazol-1-yl)-3-sulfamoylphenyl]-2-[4-(difluoromethyl)phenyl]acetamide;
(D220) 2-(2-chlorophenyl)-N-[4-(4-cyano-1H-pyrazol-1-yl)-3-sulfamoylphenyl]-2,2-difluoroacetamide;
(D221) 2-(2-fluorophenyl)-N-{3-sulfamoyl-4-[5-(trifluoromethyl)pyridin-3-yl]phenyl}acetamide;
(D222) N-{3-sulfamoyl-4-[5-(trifluoromethyl)pyridin-3-yl]phenyl}-2-[2-(trifluoromethoxy)phenyl]acetamide;
(D223) N-{4-[1-(difluoromethyl)-1H-pyrazol-4-yl]-3-sulfamoylphenyl}-2-(2-methylphenyl)acetamide;
(D224) N-{4-[1-(difluoromethyl)-1H-pyrazol-4-yl]-3-sulfamoylphenyl}-2-[2-(trifluoromethyl)phenyl]acetamide;
(D225) 2-[2-(difluoromethyl)phenyl]-N-{4-[1-(difluoromethyl)-1H-pyrazol-4-yl]-3-sulfamoylphenyl}acetamide;
(D226) N-{4-[1-(difluoromethyl)-1H-pyrazol-4-yl]-3-sulfamoylphenyl}-2-(2-methoxyphenyl)acetamide;
(D227) N-{4-[1-(difluoromethyl)-1H-pyrazol-4-yl]-3-sulfamoylphenyl}-2-(4-fluorophenyl)acetamide;
(D228) 2-(2-chloro-5-fluorophenyl)-N-{4-[1-(difluoromethyl)-1H-pyrazol-4-yl]-3-sulfamoylphenyl}acetamide;
(D229) 2-(2,3-dichlorophenyl)-N-{4-[1-(difluoromethyl)-1H-pyrazol-4-yl]-3-sulfamoylphenyl}acetamide;
(D230) 2-(3-chloropyridin-4-yl)-N-{4-[1-(difluoromethyl)-1H-pyrazol-4-yl]-3-sulfamoylphenyl}acetamide;
(D231) 2-(2-chlorophenyl)-N-[4-(3-cyclobutyl-1H-1,2,4-triazol-1-yl)-3-sulfamoylphenyl]acetamide;
(D232) N-[4-(4-acetyl-1H-pyrazol-1-yl)-3-sulfamoylphenyl]-2-(2-chlorophenyl)acetamide;
(D233) 2-(2-chlorophenyl)-N-[4-(3-isopropyl-1H-1,2,4-triazol-1-yl)-3-sulfamoylphenyl]acetamide;
(D234) N-[4-(4-chloro-1H-pyrazol-1-yl)-3-sulfamoylphenyl]-2-[2-(trifluoromethyl)phenyl]acetamide;
(D235) ethyl 1-(4-{[(2-chlorophenyl)acetyl]amino}-2-sulfamoylphenyl)-1H-pyrazole-4-carboxylate;
(D236) ethyl 1-(4-{[(2-fluorophenyl)acetyl]amino}-2-sulfamoylphenyl)-1H-pyrazole-4-carboxylate;
(D237) 2-(2-fluorophenyl)-N-{4-[4-(2-hydroxypropan-2-yl)-1H-pyrazol-1-yl]-3-sulfamoylphenyl}acetamide;
(D238) 2-(2-chlorophenyl)-N-{4-[4-(2-hydroxypropan-2-yl)-1H-pyrazol-1-yl]-3-sulfamoylphenyl}acetamide;
(D239) 2-(2-chloropyridin-3-yl)-N-{3-sulfamoyl-4-[3-(trifluoromethyl)-1H-1,2,4-triazol-1-yl]phenyl}acetamide;
(D240) 2-(2-chlorophenyl)-N-methyl-N-{3-sulfamoyl-4-[4-(trifluoromethyl)-1H-pyrazol-1-yl]phenyl}acetamide;
(D241) 2-(2-chlorophenyl)-N-{3-sulfamoyl-4-[4-(trifluoromethyl)-2H-1,2,3-triazol-2-yl]phenyl}acetamide;
(D242) 2-(2-chlorophenyl)-N-{3-sulfamoyl-4-[4-(trifluoromethyl)-1H-1,2,3-triazol-1-yl]phenyl}acetamide;
(D243) 2-(2-chloro-5-cyanophenyl)-N-{3-sulfamoyl-4-[4-(trifluoromethyl)-1H-pyrazol-1-yl]phenyl}acetamide;
(D244) 2-(2-chlorophenyl)-N-{3-cyano-5-sulfamoyl-4-[4-(trifluoromethyl)-1H-pyrazol-1-yl]phenyl}acetamide;
(D245) 2-(5-bromo-2-chlorophenyl)-N-{3-sulfamoyl-4-[4-(trifluoromethyl)-1H-pyrazol-1-yl]phenyl}acetamide;
(D246) N-[4-(3-chloro-1H-1,2,4-triazol-1-yl)-3-sulfamoylphenyl]-2-(2-fluorophenyl)acetamide;
(D247) 2-(2-chlorophenyl)-N-[3-cyano-4-(4-fluoro-1H-pyrazol-1-yl)-5-sulfamoylphenyl]acetamide;
(D248) 2-(2-chlorophenyl)-N-[4-(4-methyl-1H-pyrazol-1-yl)-3-sulfamoylphenyl]acetamide;
(D249) 2-(2-chlorophenyl)-N-[4-(3-methoxy-1H-1,2,4-triazol-1-yl)-3-sulfamoylphenyl]acetamide;
(D250) 2-(2-chlorophenyl)-N-[4-(4-cyclopropyl-1H-imidazol-1-yl)-3-sulfamoylphenyl]acetamide;
(D251) 2-(2-chlorophenyl)-N-[4-(4-methyl-1H-imidazol-1-yl)-3-sulfamoylphenyl]acetamide;
(D252) 2-(2-chlorophenyl)-N-[4-(3-cyclopropyl-1H-pyrazol-1-yl)-3-sulfamoylphenyl]acetamide;
(D253) 2-(2-chlorophenyl)-N-[4-(2H-pyrazolo[3,4-b]pyridin-2-yl)-3-sulfamoylphenyl]acetamide;
(D254) 2-(2-chlorophenyl)-N-[4-(2H-pyrazolo[3,4-c]pyridin-2-yl)-3-sulfamoylphenyl]acetamide;
(D255) 2-(2-chlorophenyl)-N-[4-(2H-pyrazolo[4,3-b]pyridin-2-yl)-3-sulfamoylphenyl]acetamide;
(D256) 2-(2-chlorophenyl)-N-{4-[4-(2-methoxyethyl)-1H-pyrazol-1-yl]-3-sulfamoylphenyl}acetamide;
(D257) 2-(2-chlorophenyl)-N-[4-(3-fluoro-1H-pyrazol-1-yl)-3-sulfamoylphenyl]acetamide;
(D258) 2-(2-chlorophenyl)-N-(4-{4-[(2,2-difluoroethyl)amino]-1H-pyrazol-1-yl}-3-sulfamoylphenyl) acetamide;
(D259) 2-(2-chlorophenyl)-N-{4-[4-(2,2-difluoroethyl)-1H-pyrazol-1-yl]-3-sulfamoylphenyl}acetamide;
(D260) 2-(2-chlorophenyl)-N-[4-(4-{[(2,2-difluoroethyl)amino]methyl}-1H-pyrazol-1-yl)-3-sulfamoylphenyl]acetamide;
(D261) 2-(2-chlorophenyl)-N-{3-sulfamoyl-4-[5-(trifluoromethyl)-1,2,4-oxadiazol-3-yl]phenyl}acetamide;
(D262) 2-(2-fluorophenyl)-N-[4-(4-methyl-1H-pyrazol-1-yl)-3-sulfamoylphenyl]acetamide;
(D263) N-[4-(4-cyclopropyl-1H-imidazol-1-yl)-3-sulfamoylphenyl]-2-(2-fluorophenyl)acetamide;
(D264) N-[4-(3-cyclopropyl-1H-pyrazol-1-yl)-3-sulfamoylphenyl]-2-(2-fluorophenyl)acetamide;
(D265) 2-(2-fluorophenyl)-N-{4-[4-(2-methoxyethyl)-1H-pyrazol-1-yl]-3-sulfamoylphenyl}acetamide;
(D266) 2-(2-chlorophenyl)-N-(2-sulfamoylbiphenyl-4-yl)acetamide;
(D267) 2-(2-chlorophenyl)-N-{4-[1-(cyclopropylmethyl)-1H-pyrazol-4-yl]-3-sulfamoylphenyl}acetamide;
(D268) 2-(2-chlorophenyl)-N-{4-[1-(2-methoxyethyl)-1H-pyrazol-4-yl]-3-sulfamoylphenyl}acetamide;
(D269) 2-(2-chlorophenyl)-N-{4-[1-(piperidin-4-yl)-1H-pyrazol-4-yl]-3-sulfamoylphenyl}acetamide;
(D270) 2-(2-fluorophenyl)-N-{4-[1-(piperidin-4-yl)-1H-pyrazol-4-yl]-3-sulfamoylphenyl}acetamide;
(D271) N-{4-[1-(azetidin-3-yl)-1H-pyrazol-4-yl]-3-sulfamoylphenyl}-2-(2-chlorophenyl)acetamide;
(D272) 2-(3-chlorophenyl)-N-(2-sulfamoylbiphenyl-4-yl)acetamide;
(D273) 2-(2-chlorophenyl)-N-[4-(3-methyl-1H-pyrazol-1-yl)-3-sulfamoylphenyl]acetamide;
(D274) 2-(2-chlorophenyl)-N-[4-(6-chloropyridin-3-yl)-3-sulfamoylphenyl]acetamide;
(D275) 2-(2-chlorophenyl)-N-[4-(3,5-dimethyl-1,2-oxazol-4-yl)-3-sulfamoylphenyl]acetamide;
(D276) 2-(2-chlorophenyl)-N-(4-{1-[(3-methyloxetan-3-yl)methyl]-1H-pyrazol-4-yl}-3-sulfamoylphenyl)acetamide;
(D277) 2-(2-chloro-4-fluorophenyl)-N-{4-[1-(2-methoxyethyl)-1H-pyrazol-4-yl]-3-sulfamoylphenyl}acetamide;
(D278) 2-(2-chloro-4-fluorophenyl)-N-[4-(1-cyclopropyl-1H-pyrazol-4-yl)-3-sulfamoylphenyl]acetamide;
(D279) 2-(2-chlorophenyl)-N-(3-sulfamoyl-4-{1-[2-(trifluoromethoxy)ethyl]-1H-pyrazol-4-yl}phenyl)acetamide;
(D280) 2-(2-chlorophenyl)-N-[4-(1-cyclobutyl-1H-pyrazol-4-yl)-3-sulfamoylphenyl]acetamide;
(D281) 2-(2-chlorophenyl)-N-(4-{1-[2-(pyrrolidin-1-yl)ethyl]-1H-pyrazol-4-yl}-3-sulfamoylphenyl)acetamide;
(D282) 2-(2-chlorophenyl)-N-[4-(5-cyanopyridin-3-yl)-3-sulfamoylphenyl]acetamide;
(D283) 2-(2-chlorophenyl)-N-(4-{1-[oxetan-2-ylmethyl]-1H-pyrazol-4-yl}-3-sulfamoylphenyl)acetamide;
(D284) 2-(2-chlorophenyl)-N-{3-sulfamoyl-4-[2-(2,2,2-trifluoroethoxy)pyrimidin-5-yl]phenyl}acetamide;
(D285) 2-(2-chlorophenyl)-N-[4-(2-methoxypyrimidin-5-yl)-3-sulfamoylphenyl]acetamide;
(D286) 2-(2-chlorophenyl)-N-(4-{1-[2-(propan-2-yloxy)ethyl]-1H-pyrazol-4-yl}-3-sulfamoylphenyl)acetamide;
(D287) 2-(2-chlorophenyl)-N-{4-[1-(3-hydroxy-3-methylbutyl)-1H-pyrazol-4-yl]-3-sulfamoylphenyl}acetamide;
(D288) 2-(2-chlorophenyl)-N-{4-[5-(2-hydroxypropan-2-yl)-1-methyl-1H-pyrazol-3-yl]-3-sulfamoylphenyl}acetamide;
(D289) 2-(2-chlorophenyl)-N-{4-[5-(difluoromethoxy)pyridin-3-yl]-3-sulfamoylphenyl}acetamide;
(D290) N-[4-(2-chloro-5-methoxypyridin-3-yl)-3-sulfamoylphenyl]-2-(2-chlorophenyl)acetamide;
(D291) 2-(2-chlorophenyl)-N-{4-[1-(2-hydroxyethyl)-1H-pyrazol-4-yl]-3-sulfamoylphenyl}acetamide;
(D292) N-[4-(5-tert-butyl-1H-pyrazol-3-yl)-3-sulfamoylphenyl]-2-(2-chlorophenyl)acetamide;
(D293) N-[4-(1-benzyl-1H-pyrazol-4-yl)-3-sulfamoylphenyl]-2-(2-chlorophenyl)acetamide;
(D294) 2-(2-chlorophenyl)-N-[4-(6-methylpyridazin-4-yl)-3-sulfamoylphenyl]acetamide;
(D295) 2-(2-chlorophenyl)-N-{3-sulfamoyl-4-[6-(trifluoromethyl)pyridin-2-yl]phenyl}acetamide;
(D296) N-{4-[1-(difluoromethyl)-1H-pyrazol-4-yl]-3-sulfamoylphenyl}-2-(2-fluorophenyl)acetamide;
(D297) 2-(2-chlorophenyl)-N-{6-[1-(difluoromethyl)-1H-pyrazol-4-yl]-5-sulfamoylpyridin-3-yl}acetamide;
(D298) N-[4-(6-chloro-5-methylpyridin-3-yl)-3-sulfamoylphenyl]-2-(2-chlorophenyl)acetamide;
(D299) 2-(2-chlorophenyl)-N-{4-[2-(cyclopropylamino)pyrimidin-5-yl]-3-sulfamoylphenyl}acetamide;
(D300) 2-(2-chlorophenyl)-N-{3-sulfamoyl-4-[1-(tetrahydrofuran-3-yl)-1H-pyrazol-4-yl]phenyl}acetamide;
(D301) 2-(2-chlorophenyl)-N-{4-[2-(methylamino)pyrimidin-5-yl]-3-sulfamoylphenyl}acetamide;
(D302) 2-(2-chlorophenyl)-N-[6-(1-methyl-1H-pyrazol-4-yl)-5-sulfamoylpyridin-3-yl]acetamide;
(D303) 2-(2-chlorophenyl)-N-{4-[1-(2,2-difluoroethyl)-1H-pyrazol-4-yl]-3-sulfamoylphenyl}acetamide;
(D304) N-{3-sulfamoyl-4-[5-(trifluoromethyl)pyridin-3-yl]phenyl}-2-[2-(trifluoromethyl)phenyl]acetamide;
(D305) 2-(2-chlorophenyl)-N-[3-sulfamoyl-5'-(trifluoromethyl)-2,3'-bipyridin-5-yl]acetamide;
(D306) 2-(2-chlorophenyl)-N-{3-sulfamoyl-4-[2-(trifluoromethyl)pyrimidin-5-yl]phenyl}acetamide;
(D307) 2-(2-chlorophenyl)-N-{3-sulfamoyl-4-[5-(trifluoromethoxy)pyridin-3-yl]phenyl}acetamide;
(D308) 2-(2-chlorophenyl)-N-[4-(2-cyclopropylpyrimidin-5-yl)-3-sulfamoylphenyl]acetamide;
(D309) 2-(2-chlorophenyl)-N-[4-(2-ethoxypyrimidin-5-yl)-3-sulfamoylphenyl]acetamide;
(D310) 2-(2-chlorophenyl)-N-{4-[2-(propan-2-ylamino)pyrimidin-5-yl]-3-sulfamoylphenyl}acetamide;
(D311) 2-(2-chlorophenyl)-N-{4-[2-(propan-2-yloxy)pyrimidin-5-yl]-3-sulfamoylphenyl}acetamide;
(D312) 2-(2-chlorophenyl)-N-{4-[2-(ethylamino)pyrimidin-5-yl]-3-sulfamoylphenyl}acetamide;
(D313) 2-(2-chlorophenyl)-N-[4-(2-methylpyrimidin-5-yl)-3-sulfamoylphenyl]acetamide;
(D314) 2-(2-chlorophenyl)-N-{4-[2-(propylamino)pyrimidin-5-yl]-3-sulfamoylphenyl}acetamide;
(D315) 2-(2-chlorophenyl)-N-(3-sulfamoyl-4-{2-[(2,2,2-trifluoroethyl)amino]pyrimidin-5-yl}phenyl)acetamide;
(D316) 2-(2-chlorophenyl)-N-{4-[2-(cyclobutyloxy)pyrimidin-5-yl]-3-sulfamoylphenyl}acetamide;
(D317) N-[4-(2-chloro-4-methylpyrimidin-5-yl)-3-sulfamoylphenyl]-2-(2-chlorophenyl)acetamide;
(D318) 2-(2-chlorophenyl)-N-{3-sulfamoyl-4-[4-(trifluoromethyl)pyridin-2-yl]phenyl}acetamide;
(D319) 2-(2-chlorophenyl)-N-[4-(5-chloropyridin-2-yl)-3-sulfamoylphenyl]acetamide;
(D320) 2-(2-chlorophenyl)-N-[4-(1,2-dimethyl-1H-imidazol-4-yl)-3-sulfamoylphenyl]acetamide;
(D321) N-{6-[1-(difluoromethyl)-1H-pyrazol-4-yl]-5-sulfamoylpyridin-3-yl}-2-(2-fluorophenyl)acetamide;
(D322) 2-(2-chlorophenyl)-N-{4-[5-(pyrrolidin-1-yl)pyridin-3-yl]-3-sulfamoylphenyl}acetamide;
(D323) 2-(2-chlorophenyl)-N-[4-(1-cyclopropyl-1H-pyrazol-4-yl)-3-sulfamoylphenyl]-2-hydroxyethanamide;
(D324) 2-(2-chlorophenyl)-N-{4-[1-(difluoromethyl)-1H-pyrazol-4-yl]-3-sulfamoylphenyl}-2-hydroxyethanamide;
(D325) 2-(2-chlorophenyl)-N-[4-(5-chloropyridin-3-yl)-3-sulfamoylphenyl]acetamide;
(D326) 2-(2-chlorophenyl)-N-{4-[1-(difluoromethyl)-1H-pyrazol-4-yl]-3-sulfamoylphenyl}acetamide;
(D327) 2-(2-chlorophenyl)-N-[4-(5-fluoropyridin-3-yl)-3-sulfamoylphenyl] acetamide;
(D328) 2-(2-chlorophenyl)-N-{4-[1-(2-methylpropyl)-1H-pyrazol-4-yl]-3-sulfamoylphenyl}acetamide;
(D329) 2-(2-chlorophenyl)-N-[4-(1-cyclopentyl-1H-pyrazol-4-yl)-3-sulfamoylphenyl]acetamide;
(D330) 2-(2-chlorophenyl)-N-[2'-fluoro-3'-(propan-2-yloxy)-2-sulfamoylbiphenyl-4-yl]acetamide;
(D331) 2-(2-chlorophenyl)-N-{3-sulfamoyl-4-[5-(trifluoromethyl)pyridin-3-yl]phenyl}acetamide;
(D332) N-[4-(6-chloro-5-methoxypyridin-3-yl)-3-sulfamoylphenyl]-2-(2-chlorophenyl)acetamide;
(D333) 2-(2-chloro-6-fluorophenyl)-N-[4-(1-cyclopropyl-1H-pyrazol-4-yl)-3-sulfamoylphenyl]acetamide;
(D334) 2-(2-chloro-3-fluorophenyl)-N-[4-(1-methyl-1H-pyrazol-4-yl)-3-sulfamoylphenyl]acetamide;
(D335) N-[4-(1-tert-butyl-1H-pyrazol-4-yl)-3-sulfamoylphenyl]-2-(2-chlorophenyl)acetamide;
(D336) 2-(2-chlorophenyl)-N-{4-[2-(propan-2-yloxy)pyridin-3-yl]-3-sulfamoylphenyl}acetamide;
(D337) 2-(2-chloro-3-fluorophenyl)-N-[4-(1-cyclopropyl-1H-pyrazol-4-yl)-3-sulfamoylphenyl]acetamide;
(D338) 2-(2-fluorophenyl)-N-[4-(pyridin-3-yl)-3-sulfamoylphenyl]acetamide;
(D339) 2-(2-chloro-6-fluorophenyl)-N-[4-(1-methyl-1H-pyrazol-4-yl)-3-sulfamoylphenyl]acetamide;
(D340) 2-(2-chlorophenyl)-N-[3'-fluoro-5'-(2-hydroxypropan-2-yl)-2-sulfamoylbiphenyl-4-yl]acetamide;
(D341) 2-(2-chlorophenyl)-N-[4-(5-methoxypyridin-3-yl)-3-sulfamoylphenyl]acetamide;
(D342) 2-(2-chlorophenyl)-N-[3'-(2-hydroxypropan-2-yl)-2-sulfamoylbiphenyl-4-yl]acetamide;
(D343) 2-(2-fluorophenyl)-N-{4-[1-(propan-2-yl)-1H-pyrazol-4-yl]-3-sulfamoylphenyl}acetamide;
(D344) N-[4-(1-cyclopropyl-1H-pyrazol-4-yl)-3-sulfamoylphenyl]-2-(2-fluorophenyl)acetamide;
(D345) 2-(2-chlorophenyl)-N-{3-sulfamoyl-4-[1-(tetrahydro-2H-pyran-2-yl)-1H-pyrazol-4-yl]phenyl}acetamide;
(D346) N-[4-(1-tert-butyl-1H-pyrazol-4-yl)-3-sulfamoylphenyl]-2-(2-fluorophenyl)acetamide;
(D347) N-[3'-fluoro-5'-(2-hydroxypropan-2-yl)-2-sulfamoylbiphenyl-4-yl]-2-(2-fluorophenyl)acetamide;
(D348) N-[4-(1-cyclopentyl-1H-pyrazol-4-yl)-3-sulfamoylphenyl]-2-(2-fluorophenyl)acetamide;
(D349) 2-(2-chlorophenyl)-N-[4-(1-cyclopropyl-1H-pyrazol-4-yl)-3-sulfamoylphenyl]acetamide;
(D350) 2-(2-chloro-6-fluorophenyl)-N-{4-[1-(2-methoxyethyl)-1H-pyrazol-4-yl]-3-sulfamoylphenyl}acetamide;
(D351) 2-(2-chlorophenyl)-N-[4-(1,3-dimethyl-1H-pyrazol-5-yl)-3-sulfamoylphenyl]acetamide;
(D352) 2-(2-chlorophenyl)-N-(4'-chloro-2-sulfamoylbiphenyl-4-yl)acetamide;
(D353) 2-(2-chlorophenyl)-N-{4-[1-methyl-3-(trifluoromethyl)-1H-pyrazol-5-yl]-3-sulfamoylphenyl}acetamide;
(D354) 2-(2-chlorophenyl)-N-[4-(pyridin-3-yl)-3-sulfamoylphenyl]acetamide;
(D355) 2-(2-chlorophenyl)-N-{4-[1-methyl-3-(trifluoromethyl)-1H-pyrazol-4-yl]-3-sulfamoylphenyl}acetamide;
(D356) 2-(2-chlorophenyl)-N-(3'-chloro-2-sulfamoylbiphenyl-4-yl)acetamide;
(D357) 2-(2-chlorophenyl)-N-(4-{1-[(2,2-dichlorocyclopropyl)methyl]-1H-pyrazol-4-yl}-3-sulfamoylphenyl) acetamide;
(D358) 2-(2-chlorophenyl)-N-[4-(1,3-dimethyl-1H-pyrazol-4-yl)-3-sulfamoylphenyl]acetamide;
(D359) 2-(2-chlorophenyl)-N-{4-[1-(propan-2-yl)-1H-pyrazol-4-yl]-3-sulfamoylphenyl}acetamide;
(D360) 2-(2-chlorophenyl)-N-[4-(1-methyl-1H-pyrazol-4-yl)-3-sulfamoylphenyl]acetamide;
(D361) 2-(2-chlorophenyl)-N-{4-[1-(2-hydroxy-3,3-dimethylbutyl)-1H-pyrazol-4-yl]-3-sulfamoylphenyl}acetamide;
(D362) 2-(2-fluorophenyl)-N-[4-(5-fluoropyridin-3-yl)-3-sulfamoylphenyl]acetamide;
(D363) 2-(2-chlorophenyl)-N-[4-(5-methyl-1-phenyl-1H-pyrazol-4-yl)-3-sulfamoylphenyl]acetamide;
(D364) 4'-{[(2-chlorophenyl)acetyl]amino}-N-[2-(dimethylamino)ethyl]-2'-sulfamoylbiphenyl-3-carboxamide;
(D365) 2-(2-chlorophenyl)-N-[4-(pyrazolo[1,5-a]pyrimidin-3-yl)-3-sulfamoylphenyl]acetamide;
(D366) 2-(2-chlorophenyl)-N-{4-[5-(pyrrolidin-1-ylcarbonyl)pyridin-3-yl]-3-sulfamoylphenyl}acetamide;
(D367) 2-(2-chlorophenyl)-N-[4-(1H-pyrazol-4-yl)-3-sulfamoylphenyl]acetamide;
(D368) 2-(2-fluorophenyl)-N-{4-[1-methyl-3-(trifluoromethyl)-1H-pyrazol-5-yl]-3-sulfamoylphenyl}acetamide;
(D369) 2-(2-fluorophenyl)-N-{4-[1-(2-methoxyethyl)-1H-pyrazol-4-yl]-3-sulfamoylphenyl}acetamide;
(D370) 2-(2-chlorophenyl)-N-{4-[1-(2-hydroxy-2-methylpropyl)-1H-pyrazol-4-yl]-3-sulfamoylphenyl}acetamide;
(D371) 2-(5-chloro-2-fluorophenyl)-N-[4-(1-cyclopropyl-1H-pyrazol-4-yl)-3-sulfamoylphenyl]acetamide;
(D372) 2-(2-chlorophenyl)-N-[4-(6-methylpyridin-3-yl)-3-sulfamoylphenyl]acetamide;
(D373) 2-(2-chlorophenyl)-N-{4-[1-(oxetan-3-yl)-1H-pyrazol-4-yl]-3-sulfamoylphenyl}acetamide;
(D374) 2-(2-fluorophenyl)-N-[4-(1-methyl-1H-pyrazol-4-yl)-3-sulfamoylphenyl]acetamide;
(D375) N-[4-(1,3-dimethyl-1H-pyrazol-4-yl)-3-sulfamoylphenyl]-2-(2-fluorophenyl)acetamide;
(D376) N-[4-(5-cyclopropyl-1,3,4-oxadiazol-2-yl)-3-sulfamoylphenyl]-2-(2-fluorophenyl)acetamide;
(D377) 2-(2-chlorophenyl)-N-[4-(5-cyclopropyl-1,3,4-oxadiazol-2-yl)-3-sulfamoylphenyl]acetamide;
(D378) N-{4-[4-(difluoromethyl)-1H-pyrazol-1-yl]-3-sulfamoylphenyl}-2-(2-fluorophenyl)acetamide;
(D379) 2-(2-chlorophenyl)-N-{4-[4-(difluoromethyl)-1H-pyrazol-1-yl]-3-sulfamoylphenyl}acetamide;
(D380) 2-(2-chlorophenyl)-N-{5-sulfamoyl-6-[4-(trifluoromethyl)-1H-pyrazol-1-yl]pyridin-3-yl}acetamide;
(D381) 2-(2-fluorophenyl)-N-{5-sulfamoyl-6-[4-(trifluoromethyl)-1H-pyrazol-1-yl]pyridin-3-yl}acetamide;
(D382) N-[6-(4-cyano-1H-pyrazol-1-yl)-5-sulfamoylpyridin-3-yl]-2-(2-fluorophenyl)acetamide;
(D383) 2-(2-chlorophenyl)-N-[6-(4-cyano-1H-pyrazol-1-yl)-5-sulfamoylpyridin-3-yl]acetamide;
(D384) 2-(2-fluorophenyl)-N-[6-(4-fluoro-1H-pyrazol-1-yl)-5-sulfamoylpyridin-3-yl]acetamide;
(D385) 2-(2-chlorophenyl)-N-[6-(4-fluoro-1H-pyrazol-1-yl)-5-sulfamoylpyridin-3-yl]acetamide;
(D386) N-[6-(4-bromo-1H-pyrazol-1-yl)-5-sulfamoylpyridin-3-yl]-2-(2-fluorophenyl)acetamide;
(D387) 2-(2-chlorophenyl)-N-[6-(4-chloro-1H-pyrazol-1-yl)-5-sulfamoylpyridin-3-yl]acetamide;
(D388) N-[6-(4-chloro-1H-pyrazol-1-yl)-5-sulfamoylpyridin-3-yl]-2-(2-fluorophenyl)acetamide;
(D389) N-[6-(4-bromo-1H-pyrazol-1-yl)-5-sulfamoylpyridin-3-yl]-2-(2-chlorophenyl)acetamide;
(D390) 2-(2-fluorophenyl)-N-{3-sulfamoyl-4-[5-(trifluoromethyl)-1,3,4-oxadiazol-2-yl]phenyl}acetamide;
(D391) 2-(2-chlorophenyl)-N-{3-sulfamoyl-4-[5-(trifluoromethyl)-1,3,4-oxadiazol-2-yl]phenyl}acetamide;
(D392) 2-(2-chlorophenyl)-N-{4-[3-(pyridin-3-yl)-1H-1,2,4-triazol-1-yl]-3-sulfamoylphenyl}acetamide;
(D393) 2-(2-fluorophenyl)-N-[4-(1H-pyrazol-1-yl)-3-sulfamoylphenyl]acetamide;
(D394) N-[4-(4-tert-butyl-1H-pyrazol-1-yl)-3-sulfamoylphenyl]-2-(2-chlorophenyl)acetamide;
(D395) 2-(2-chlorophenyl)-N-[4-(1H-indazol-1-yl)-3-sulfamoylphenyl]acetamide;
(D396) 2-(2-chlorophenyl)-N-[4-(2H-indazol-2-yl)-3-sulfamoylphenyl]acetamide;
(D397) 2-(2-fluorophenyl)-N-[4-(2H-indazol-2-yl)-3-sulfamoylphenyl]acetamide;
(D398) 2-(2-fluorophenyl)-N-[4-(1H-indazol-1-yl)-3-sulfamoylphenyl]acetamide;
(D399) 1-(4-{[(2-chlorophenyl)acetyl]amino}-2-sulfamoylphenyl)-N,N-dimethyl-1H-pyrazole-4-carboxamide;
(D400) N-[1-(4-{[(2-chlorophenyl)acetyl]amino}-2-sulfamoylphenyl)-1H-pyrazol-4-yl]cyclopropanecarboxamide;
(D401) 2-(2-chlorophenyl)-N-{4-[4-(pyrazin-2-yl)-1H-pyrazol-1-yl]-3-sulfamoylphenyl}acetamide;
(D402) 2-(2-chlorophenyl)-N-{4-[4-(pyridin-2-yl)-1H-pyrazol-1-yl]-3-sulfamoylphenyl}acetamide;
(D403) 2-(2-chlorophenyl)-N-{4-[4-(pyridin-3-yl)-1H-pyrazol-1-yl]-3-sulfamoylphenyl}acetamide;
(D404) 2-(2-chlorophenyl)-N-{4-[4-(pyrimidin-4-yl)-1H-pyrazol-1-yl]-3-sulfamoylphenyl}acetamide;
(D405) 2-(2-fluorophenyl)-N-{4-[4-(pyrimidin-4-yl)-1H-pyrazol-1-yl]-3-sulfamoylphenyl}acetamide;
(D406) 2-(4-methoxyphenyl)-N-{3-sulfamoyl-4-[4-(trifluoromethyl)-2H-1,2,3-triazol-2-yl]phenyl}acetamide;
(D407) 2-(4-methoxyphenyl)-N-{3-sulfamoyl-4-[4-(trifluoromethyl)-1H-1,2,3-triazol-1-yl]phenyl}acetamide;
(D408) 2-(4-methylphenyl)-N-{3-sulfamoyl-4-[4-(trifluoromethyl)-2H-1,2,3-triazol-(D)2-yl]phenyl}acetamide;
(D409) 2-(4-methylphenyl)-N-{3-sulfamoyl-4-[4-(trifluoromethyl)-1H-1,2,3-triazol-1-yl]phenyl}acetamide;
(D410) 2-[4-(difluoromethyl)phenyl]-N-{3-sulfamoyl-4-[4-(trifluoromethyl)-2H-1,2,3-triazol-2-yl]phenyl}acetamide;
(D411) 2-[4-(difluoromethyl)phenyl]-N-{3-sulfamoyl-4-[4-(trifluoromethyl)-1H-1,2,3-triazol-1-yl]phenyl}acetamide;
(D412) 2-(2-fluorophenyl)-N-{3-sulfamoyl-4-[4-(trifluoromethyl)-2H-1,2,3-triazol-2-yl]phenyl}acetamide;
(D413) 2-(2-fluorophenyl)-N-{3-sulfamoyl-4-[4-(trifluoromethyl)-1H-1,2,3-triazol-1-yl]phenyl}acetamide;
(D414) 2-[2-chloro-4-(trifluoromethyl)phenyl]-N-{3-sulfamoyl-4-[4-(trifluoromethyl)-2H-1,2,3-triazol-2-yl]phenyl}acetamide;
(D415) 2-[2-chloro-4-(trifluoromethyl)phenyl]-N-{3-sulfamoyl-4-[4-(trifluoromethyl)-1H-1,2,3-triazol-1-yl]phenyl}acetamide;
(D416) 2-(2,4-dichlorophenyl)-N-{3-sulfamoyl-4-[4-(trifluoromethyl)-2H-1,2,3-triazol-2-yl]phenyl}acetamide;
(D417) 2-(2,4-dichlorophenyl)-N-{3-sulfamoyl-4-[4-(trifluoromethyl)-1H-1,2,3-triazol-1-yl]phenyl}acetamide;
(D418) 2-(2-chlorophenyl)-N-[3-sulfamoyl-4-(1,3-thiazol-5-yl)phenyl]acetamide;
(D419) 2-(2-chlorophenyl)-N-[3-sulfamoyl-4-(1,2-thiazol-3-yl)phenyl]acetamide;
(D420) 2-(2-chlorophenyl)-N-[4-(2-methyl-1,3-thiazol-4-yl)-3-sulfamoylphenyl]acetamide;
(D421) 2-(2-chlorophenyl)-N-[4-(2-methoxy-1,3-thiazol-4-yl)-3-sulfamoylphenyl]acetamide;
(D422) 2-(2-chlorophenyl)-N-[4-(2-methoxy-1,3-thiazol-5-yl)-3-sulfamoylphenyl]acetamide;
(D423) 2-(2-chlorophenyl)-N-[4-(2-methyl-1,3-thiazol-5-yl)-3-sulfamoylphenyl]acetamide;
(D424) 2-(2-chlorophenyl)-N-[4-(3-methyl-1,2-thiazol-5-yl)-3-sulfamoylphenyl]acetamide;
(D425) 2-(2-chlorophenyl)-N-[4-(4-methyl-1,3-thiazol-2-yl)-3-sulfamoylphenyl]acetamide;
(D426) 2-(2-chlorophenyl)-N-[3-sulfamoyl-4-(1,2-thiazol-4-yl)phenyl]acetamide;
(D427) 2-(2-chlorophenyl)-N-[3-sulfamoyl-4-(1,3-thiazol-2-yl)phenyl]acetamide;
(D428) 2-(2-chlorophenyl)-N-[4-(4-cyano-1,3-thiazol-2-yl)-3-sulfamoylphenyl]acetamide;
(D429) 2-(2-chlorophenyl)-N-{4-[2-(difluoromethyl)-1,3-thiazol-5-yl]-3-sulfamoylphenyl}acetamide;
(D430) 2-(2-chlorophenyl)-N-[4-(2-cyclopropyl-1,3-thiazol-5-yl)-3-sulfamoylphenyl]acetamide;
(D431) 2-(2-chlorophenyl)-N-[4-(2-cyclopropyl-1,3-thiazol-4-yl)-3-sulfamoylphenyl]acetamide;
(D432) 2-(2-chlorophenyl)-N-[4-(4-methyl-1,3-oxazol-2-yl)-3-sulfamoylphenyl]acetamide;
(D433) 2-(2-chlorophenyl)-N-{3-sulfamoyl-4-[2-(trifluoromethyl)-1,3-thiazol-4-yl]phenyl}acetamide;
(D434) 2-(2-chlorophenyl)-N-{3-sulfamoyl-4-[4-(trifluoromethyl)-1,3-thiazol-2-yl]phenyl}acetamide;
(D435) 2-(2-chlorophenyl)-N-{4-[2-(2-hydroxypropan-2-yl)-1,3-thiazol-5-yl]-3-sulfamoylphenyl}acetamide;
(D436) 2-(2-chlorophenyl)-N-{4-[2-(2-hydroxypropan-2-yl)-1,3-thiazol-4-yl]-3-sulfamoylphenyl}acetamide;
(D437) 2-(2-chlorophenyl)-N-[3-sulfamoyl-4-(1,3-thiazol-4-yl)phenyl]acetamide;
(D438) 2-(2-chlorophenyl)-N-[4-(5-cyclopropyl-1,2-oxazol-3-yl)-3-sulfamoylphenyl]acetamide;
(D439) 2-(2-chlorophenyl)-N-[4-(2-cyclopropyl-1,3-oxazol-5-yl)-3-sulfamoylphenyl]acetamide;
(D440) 2-(2-chlorophenyl)-N-(4-{4-[(3,3-difluoroazetidin-1-yl)carbonyl]-1H-pyrazol-1-yl}-3-sulfamoylphenyl)acetamide;
(D441) N-{4-[4-(azetidin-1-ylcarbonyl)-1H-pyrazol-1-yl]-3-sulfamoylphenyl}-2-(2-chlorophenyl)acetamide;
(D442) 2-(2-chlorophenyl)-N-{4-[4-(pyrrolidin-1-ylcarbonyl)-1H-pyrazol-1-yl]-3-sulfamoylphenyl}acetamide;
(D443) 2-(2-chlorophenyl)-N-(4-{4-[(3,3-difluoropyrrolidin-1-yl)carbonyl]-1H-pyrazol-1-yl}-3-sulfamoylphenyl) acetamide;
(D444) 2-(4-chlorophenyl)-N-{3-sulfamoyl-4-[5-(trifluoromethyl)pyridin-3-yl]phenyl}acetamide;
(D445) 2-(2-chloro-6-fluorophenyl)-N-{3-sulfamoyl-4-[5-(trifluoromethyl)pyridin-3-yl]phenyl}acetamide;
(D446) N-{3-sulfamoyl-4-[5-(trifluoromethyl)pyridin-3-yl]phenyl}-2-[4-(trifluoromethyl)phenyl]acetamide;
(D447) 2-(3-fluorophenyl)-N-{3-sulfamoyl-4-[5-(trifluoromethyl)pyridin-3-yl]phenyl}acetamide;
(D448) 2-(2,4-dichlorophenyl)-N-{3-sulfamoyl-4-[5-(trifluoromethyl)pyridin-3-yl]phenyl}acetamide;
(D449) 2-(2-bromophenyl)-N-{3-sulfamoyl-4-[5-(trifluoromethyl)pyridin-3-yl]phenyl}acetamide;
(D450) 2-(2,4-difluorophenyl)-N-{3-sulfamoyl-4-[5-(trifluoromethyl)pyridin-3-yl]phenyl}acetamide;
(D451) 2-(3,4-difluorophenyl)-N-{3-sulfamoyl-4-[5-(trifluoromethyl)pyridin-3-yl]phenyl}acetamide;
(D452) 2-(3,5-difluorophenyl)-N-{3-sulfamoyl-4-[5-(trifluoromethyl)pyridin-3-yl]phenyl}acetamide;
(D453) 2-(3-chlorophenyl)-N-{3-sulfamoyl-4-[5-(trifluoromethyl)pyridin-3-yl]phenyl}acetamide;
(D454) 2-(4-methylphenyl)-N-{3-sulfamoyl-4-[5-(trifluoromethyl)pyridin-3-yl]phenyl}acetamide;
(D455) 2-(4-methoxyphenyl)-N-{3-sulfamoyl-4-[5-(trifluoromethyl)pyridin-3-yl]phenyl}acetamide;
(D456) 2-(2-fluoro-4-methylphenyl)-N-{3-sulfamoyl-4-[5-(trifluoromethyl)pyridin-3-yl]phenyl}acetamide;
(D457) 2-(2-fluoro-4-methoxyphenyl)-N-{3-sulfamoyl-4-[5-(trifluoromethyl)pyridin-3-yl]phenyl}acetamide;
(D458) 2-[4-(difluoromethyl)phenyl]-N-{3-sulfamoyl-4-[5-(trifluoromethyl)pyridin-3-yl]phenyl}acetamide;
(D459) 2-(4-chlorophenyl)-N-[4-(6-chloropyridin-3-yl)-3-sulfamoylphenyl]acetamide;
(D460) N-[4-(6-chloropyridin-3-yl)-3-sulfamoylphenyl]-2-(2-fluorophenyl)acetamide;
(D461) 2-(2-chloro-6-fluorophenyl)-N-[4-(6-chloropyridin-3-yl)-3-sulfamoylphenyl]acetamide;
(D462) N-[4-(6-chloropyridin-3-yl)-3-sulfamoylphenyl]-2-[4-(trifluoromethyl)phenyl]acetamide;
(D463) N-[4-(6-chloropyridin-3-yl)-3-sulfamoylphenyl]-2-[2-(trifluoromethyl)phenyl]acetamide;
(D464) N-[4-(6-chloropyridin-3-yl)-3-sulfamoylphenyl]-2-(3-fluorophenyl)acetamide;
(D465) N-[4-(6-chloropyridin-3-yl)-3-sulfamoylphenyl]-2-(2,4-dichlorophenyl)acetamide;
(D466) N-[4-(6-chloropyridin-3-yl)-3-sulfamoylphenyl]-2-(2,4-difluorophenyl)acetamide;
(D467) 2-(2-bromophenyl)-N-[4-(6-chloropyridin-3-yl)-3-sulfamoylphenyl]acetamide;
(D468) N-[4-(6-chloropyridin-3-yl)-3-sulfamoylphenyl]-2-(3,4-difluorophenyl)acetamide;
(D469) N-[4-(6-chloropyridin-3-yl)-3-sulfamoylphenyl]-2-(4-methylphenyl)acetamide;
(D470) N-[4-(6-chloropyridin-3-yl)-3-sulfamoylphenyl]-2-(4-methoxyphenyl)acetamide;
(C471) N-[4-(6-chloropyridin-3-yl)-3-sulfamoylphenyl]-2-(2-fluoro-4-methylphenyl)acetamide;
(D472) N-[4-(6-chloropyridin-3-yl)-3-sulfamoylphenyl]-2-(2-fluoro-4-methoxyphenyl)acetamide;
(D473) N-[4-(6-chloropyridin-3-yl)-3-sulfamoylphenyl]-2-[4-(difluoromethyl)phenyl]acetamide;
(D474) N-[4-(6-chloropyridin-3-yl)-3-sulfamoylphenyl]-2-[2-chloro-4-(trifluoromethyl)phenyl]acetamide;
(D475) 2-(2,4-dichlorophenyl)-N-[4-(5-fluoropyridin-3-yl)-3-sulfamoylphenyl]acetamide;
(D476) 2-(2-fluoro-4-methylphenyl)-N-[4-(5-fluoropyridin-3-yl)-3-sulfamoylphenyl]acetamide;
(D477) 2-[4-(difluoromethyl)phenyl]-N-[4-(5-fluoropyridin-3-yl)-3-sulfamoylphenyl]acetamide;
(D478) 2-(2-chlorophenyl)-N-[4-(4-cyclopropyl-1H-pyrazol-1-yl)-3-sulfamoylphenyl]acetamide;
(D479) N-[4-(4,6-difluoro-2H-benzotriazol-2-yl)-3-sulfamoylphenyl]-2-(2-fluorophenyl)acetamide;
(D480) N-[4-(4,6-difluoro-1H-benzotriazol-1-yl)-3-sulfamoylphenyl]-2-(2-fluorophenyl)acetamide;
(D481) 2-(2-chlorophenyl)-N-[4-(4,6-difluoro-1H-benzotriazol-1-yl)-3-sulfamoylphenyl]acetamide;
(D482) 2-(2-chlorophenyl)-N-[4-(4,6-difluoro-2H-benzotriazol-2-yl)-3-sulfamoylphenyl]acetamide;
(D483) 2-(2-chlorophenyl)-N-{3-sulfamoyl-4-[2-(trifluoromethyl)-1,3-thiazol-5-yl]phenyl}acetamide;
(D484) 2-(2-chlorophenyl)-N-[4-(4-cyano-1H-pyrazol-1-yl)-5-sulfamoyl-2-(trifluoromethyl)phenyl]acetamide;
(D485) 2-[2-chloro-5-(trifluoromethyl)phenyl]-N-[4-(4-cyano-1H-pyrazol-1-yl)-3-sulfamoylphenyl]acetamide;
(D486) 2-[2-chloro-5-(trifluoromethyl)phenyl]-N-[4-(4-cyano-1H-pyrazol-1-yl)-5-sulfamoyl-2-(trifluoromethyl)phenyl]acetamide;
(D487) 2-(2-chlorophenyl)-N-[4-(4-cyano-1H-pyrazol-1-yl)-3-sulfamoyl-2-(trifluoromethyl)phenyl]acetamide;
(D488) 2-[2-chloro-3-(trifluoromethyl)phenyl]-N-[4-(4-cyano-1H-pyrazol-1-yl)-5-sulfamoyl-2-(trifluoromethyl)phenyl]acetamide;
(D489) 2-(2-chlorophenyl)-N-{4-[1-(difluoromethyl)-1H-pyrazol-4-yl]-3-sulfamoyl-2-(trifluoromethyl)phenyl}acetamide;
(D490) 2-[2-chloro-3-(trifluoromethyl)phenyl]-N-{4-[1-(difluoromethyl)-1H-pyrazol-4-yl]-3-sulfamoylphenyl}acetamide;
(D491) 2-(2-chlorophenyl)-N-{4-[1-(difluoromethyl)-1H-pyrazol-4-yl]-5-sulfamoyl-2-(trifluoromethyl)phenyl}acetamide;
(D492) 2-(2-chlorophenyl)-N-{4-[1-(difluoromethyl)-5-(trifluoromethyl)-1H-pyrazol-4-yl]-3-sulfamoylphenyl}acetamide;
(D493) 2-(2-chlorophenyl)-N-{4-[1-(difluoromethyl)-1H-pyrazol-4-yl]-2-fluoro-3-sulfamoylphenyl}acetamide;
(D494) 2-(2-chlorophenyl)-N-{4-[1-(difluoromethyl)-1H-pyrazol-4-yl]-2-fluoro-5-sulfamoylphenyl}acetamide;
(D495) 2-(2-chlorophenyl)-N-{4-[2-(dimethylamino)-1,3-thiazol-4-yl]-3-sulfamoylphenyl}acetamide;
(D496) 2-(2-chlorophenyl)-N-[3-sulfamoyl-4-(1,2-thiazol-5-yl)phenyl]acetamide;
(D497) 1-(4-{[(2-chlorophenyl)acetyl]amino}-2-sulfamoylphenyl)-N-cyclopropyl-N-methyl-1H-pyrazol-4-carboxamide;
(D498) 2-(2-chlorophenyl)-2-hydroxy-N-{3-sulfamoyl-4-[4-(trifluoromethyl)-1H-pyrazol-1-yl]phenyl}ethanamide;
(D499) 2-(2-chlorophenyl)-N-{3-chloro-5-sulfamoyl-4-[4-(trifluoromethyl)-1H-pyrazol-1-yl]phenyl}acetamide;
(D500) 2-(2-chlorophenyl)-N-[4-(4-cyano-3-hydroxy-1H-pyrazol-1-yl)-3-sulfamoylphenyl]acetamide;
(D501) 2-(2-chlorophenyl)-N-[4-(1H-pyrazolo[4,3-c]pyridin-1-yl)-3-sulfamoylphenyl]acetamide;
(D502) 2-(2-chlorophenyl)-N-[4-(4,5-dimethyl-1,3-thiazol-2-yl)-3-sulfamoylphenyl]acetamide;
(D503) 2-(2-chlorophenyl)-N-[4-(2,4-dimethyl-1,3-thiazol-5-yl)-3-sulfamoylphenyl]acetamide;
(D504) 2-(2-chlorophenyl)-N-[4-(4-methyl-1,3-thiazol-5-yl)-3-sulfamoylphenyl]acetamide;
(D505) N-{4-(4-amino-1H-pyrazol-1-yl)-3-[(2,4-dimethoxybenzyl)sulfamoyl]phenyl}-2-(2-chlorophenyl)acetamide;
(D506) N-[1-(4-{[(2-chlorophenyl)acetyl]amino}-2-sulfamoylphenyl)-1H-pyrazol-4-yl]-2,2-difluoroacetamide;
(D507) N-[1-(4-{[(2-chlorophenyl)acetyl]amino}-2-sulfamoylphenyl)-1H-pyrazol-4-yl]-3,3,3-trifluoropropanamide;
(D508) N-[1-(4-{[(2-chlorophenyl)acetyl]amino}-2-sulfamoylphenyl)-1H-pyrazol-4-yl]-3,3,3-trifluoro-2-methylpropanamide (racemate);
(D509) N-[1-(4-{[(2-chlorophenyl)acetyl]amino}-2-sulfamoylphenyl)-1H-pyrazol-4-yl]-3,3,3-trifluoro-2-methylpropanamide (enantiomer A);
(D510) N-[1-(4-{[(2-chlorophenyl)acetyl]amino}-2-sulfamoylphenyl)-1H-pyrazol-4-yl]-3,3,3-trifluoro-2-methylpropanamide (enantiomer B);
(D511) 2-(2-chlorophenyl)-N-(4-{4-[(cis)-2,5-dimethylpyrrolidin-1-yl]-1H-pyrazol-1-yl}-3-sulfamoylphenyl) acetamide;
(D512) 2-(2-chlorophenyl)-N-(4-{4-[(trans)-2,5-dimethylpyrrolidin-1-yl]-1H-pyrazol-1-yl}-3-sulfamoylphenyl)acetamide (enantiomer A);
(D513) 2-(2-chlorophenyl)-N-(4-{4-[(trans)-2,5-dimethylpyrrolidin-1-yl]-1H-pyrazol-1-yl}-3-sulfamoylphenyl)acetamide (enantiomer B);
(D514) N-(4-{4-[(2,2-difluoroethyl)amino]-1H-pyrazol-1-yl}-3-sulfamoylphenyl)-2-(2-fluorophenyl)acetamide;
(D515) 2-(2-chlorophenyl)-N-(3-sulfamoyl-4-{4-[(2,2,2-trifluoroethyl)amino]-1H-pyrazol-1-yl}phenyl)acetamide;
(D516) 2-(2-chlorophenyl)-N-[4-(4-isopropyl-1H-pyrazol-1-yl)-3-sulfamoylphenyl]acetamide;
(D517) 2-(2-fluorophenyl)-N-[4-(4-isopropyl-1H-pyrazol-1-yl)-3-sulfamoylphenyl]acetamide;
(D518) 2-(2-chlorophenyl)-N-{3-sulfamoyl-4-[4-(2,2,2-trifluoroethyl)-1H-pyrazol-1-yl]phenyl}acetamide;
(D519) 2-(2-fluorophenyl)-N-{3-sulfamoyl-4-[4-(2,2,2-trifluoroethyl)-1H-pyrazol-1-yl]phenyl}acetamide;
(D520) 2-(2-chlorophenyl)-N-[4-(5-cyclopropyl-1,2,4-oxadiazol-3-yl)-3-sulfamoylphenyl]acetamide;
(D521) 2-(2-chlorophenyl)-N-{3-sulfamoyl-4-[3-(trifluoromethyl)-1,2,4-oxadiazol-5-yl]phenyl}acetamide;
(D522) 2-(2-chlorophenyl)-N-{4-[5-(difluoromethyl)-1,3,4-oxadiazol-2-yl]-3-sulfamoylphenyl}acetamide;
(D523) N-{4-[5-(difluoromethyl)-1,3,4-oxadiazol-2-yl]-3-sulfamoylphenyl}-2-(2-fluorophenyl)acetamide;
(D524) 2-(2-chlorophenyl)-N-[4-(5-methyl-1,3,4-oxadiazol-2-yl)-3-sulfamoylphenyl]acetamide;
(D525) 2-(2-fluorophenyl)-N-[4-(5-methyl-1,3,4-oxadiazol-2-yl)-3-sulfamoylphenyl]acetamide;
(D526) N-[4-(5-methyl-1,3,4-oxadiazol-2-yl)-3-sulfamoylphenyl]-2-(4-methylphenyl)acetamide;
(D527) 2-(2-chlorophenyl)-N-[4-(1H-pyrrol-3-yl)-3-sulfamoylphenyl]acetamide;
(D528) 2-(2-chlorophenyl)-N-{4-[5-(difluoroacetyl)-1H-pyrrol-3-yl]-3-sulfamoylphenyl}acetamide;
(D529) 2-(2-chlorophenyl)-N-[4-(1-methyl-1H-pyrrol-3-yl)-3-sulfamoylphenyl]acetamide;
(D530) 2-(2-chlorophenyl)-N-[4-(5-cyano-1-methyl-1H-pyrrol-2-yl)-3-sulfamoylphenyl]acetamide;
(D531) 2-(2-chlorophenyl)-N-{3-sulfamoyl-2-(trifluoromethyl)-4-[4-(trifluoromethyl)-1H-pyrazol-1-yl]phenyl}acetamide;
(D532) 2-(2-chlorophenyl)-N-{5-sulfamoyl-2-(trifluoromethyl)-4-[4-(trifluoromethyl)-1H-pyrazol-1-yl]phenyl}acetamide;
(D533) 2-[2-chloro-3-(trifluoromethyl)phenyl]-N-{5-sulfamoyl-2-(trifluoromethyl)-4-[4-(trifluoromethyl)-1H-pyrazol-1-yl]phenyl}acetamide;
(D534) 2-[2-chloro-5-(trifluoromethyl)phenyl]-N-{5-sulfamoyl-2-(trifluoromethyl)-4-[4-(trifluoromethyl)-1H-pyrazol-1-yl]phenyl}acetamide;
(D535) 2-[2-chloro-6-(trifluoromethyl)phenyl]-N-[4-(4-fluoro-1H-pyrazol-1-yl)-5-sulfamoyl-2-(trifluoromethyl)phenyl]acetamide;
(D536) 2-[2-chloro-3-(trifluoromethyl)phenyl]-N-[4-(4-fluoro-1H-pyrazol-1-yl)-5-sulfamoyl-2-(trifluoromethyl)phenyl]acetamide;
(D537) 2-[2-chloro-5-(trifluoromethyl)phenyl]-N-[4-(4-fluoro-1H-pyrazol-1-yl)-5-sulfamoyl-2-(trifluoromethyl)phenyl]acetamide;
(D538) 2-[2-chloro-4-(trifluoromethyl)phenyl]-N-[4-(4-fluoro-1H-pyrazol-1-yl)-5-sulfamoyl-2-(trifluoromethyl)phenyl]acetamide;
(D539) N-[4-(3-tert-butyl-4-cyano-1H-pyrazol-1-yl)-3-sulfamoylphenyl]-2-(2-chlorophenyl)acetamide;
(D540) N-[4-(3-bromo-4-cyano-1H-pyrazol-1-yl)-3-sulfamoylphenyl]-2-(2-chlorophenyl)acetamide;
(D541) 2-(2-chlorophenyl)-N-{4-[4-chloro-3-(trifluoromethyl)-1H-pyrazol-1-yl]-3-sulfamoylphenyl}acetamide;
(D542) 2-(2-chlorophenyl)-N-[4-(3,4-dimethyl-1H-pyrazol-1-yl)-3-sulfamoylphenyl]acetamide;
(D543) N-[4-(4-chloro-3,5-dimethyl-1H-pyrazol-1-yl)-3-sulfamoylphenyl]-2-(2-chlorophenyl)acetamide;
(D544) 2-(2-chlorophenyl)-N-[4-(1H-pyrazol-1-yl)-3-sulfamoylphenyl]acetamide;
(D545) 2-(2-chlorophenyl)-N-[4-(3-cyano-5-methyl-1H-pyrazol-1-yl)-3-sulfamoylphenyl]acetamide;
(D546) 2-(2-chlorophenyl)-N-[4-(3-hydroxy-5-methyl-1H-pyrazol-1-yl)-3-sulfamoylphenyl]acetamide;
(D547) 2-(2-chlorophenyl)-N-[4-(4-cyano-5-methyl-1H-pyrazol-1-yl)-3-sulfamoylphenyl]acetamide;
(D548) 2-(2-chlorophenyl)-N-[4-(4-cyano-3-methyl-1H-pyrazol-1-yl)-3-sulfamoylphenyl]acetamide;
(D549) 2-(2-chlorophenyl)-N-{4-[4-(morpholin-4-yl)-1,3-thiazol-2-yl]-3-sulfamoylphenyl}acetamide;
(D550) 2-(2-chlorophenyl)-N-{4-[5-(morpholin-4-yl)-1,3-thiazol-2-yl]-3-sulfamoylphenyl}acetamide;
(D551) 2-(2-chlorophenyl)-N-[4-(5-methyl-1,3-thiazol-2-yl)-3-sulfamoylphenyl]acetamide;
(D552) 2-(2-chlorophenyl)-N-[4-(pyridin-4-yl)-3-sulfamoylphenyl]acetamide;
(D553) 1-(4-{[(2-chlorophenyl)acetyl]amino}-2-sulfamoylphenyl)-1H-pyrazol-4-carboxamide;
(D554) 2-(2-chloro-3-hydroxyphenyl)-N-[4-(4-cyano-1H-pyrazol-1-yl)-3-sulfamoylphenyl]acetamide;
(D555) 2-(2-chloro-4-hydroxyphenyl)-N-[4-(4-cyano-1H-pyrazol-1-yl)-3-sulfamoylphenyl]acetamide;
(D556) 2-(2-chloro-5-hydroxyphenyl)-N-[4-(4-cyano-1H-pyrazol-1-yl)-3-sulfamoylphenyl]acetamide;
(D557) 2-(2-chloro-6-hydroxyphenyl)-N-[4-(4-cyano-1H-pyrazol-1-yl)-3-sulfamoylphenyl]acetamide; and
(D558) 2-(2-chlorophenyl)-N-[4-(4-cyano-1H-pyrazol-1-yl)-3-sulfamoylphenyl]-2-hydroxyacetamide.
